(19) Europäisches Patentamt
European Patent Office
Office européen des brevets

(11) EP 2 562 265 A1

(12) EUROPEAN PATENT APPLICATION

(43) Date of publication:
27.02.2013 Bulletin 2013/09

(51) Int Cl.:
*C12Q 1/68* (2006.01)

(21) Application number: 11178334.6

(22) Date of filing: 22.08.2011

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicants:
- Lead Discovery Center GmbH
  44227 Dortmund (DE)
- Max-Planck-Gesellschaft zur Förderung der Wissenschaften e.V.
  80539 München (DE)

(72) Inventors:
- Choidas, Axel, Dr.
  58313 Herdecke (DE)
- Klebl, Bert
  44559 Dortmund (DE)
- Habenberger, Peter
  44139 Dortmund (DE)
- Eickhoff, Jan
  58313 Herdecke (DE)
- Thomas, Roman
  53332 Bornheim (DE)
- Heuckmann, Johannes
  50937 Köln (DE)

(74) Representative: Vossius & Partner
Siebertstrasse 4
81675 München (DE)

# (54) Susceptibility to selective CDK9 inhibitors

(57) The present invention relates to a method of selecting (a) cell(s), (a) tissue(s) or (a) cell culture(s) with susceptibility to a selective CDK9 inhibitor. Also a method for determining the responsiveness of a mammalian tumor cell or cancer cell to treatment with a selective CDK9 inhibitor is described herein. In particular, the present invention provides for an in vitro method for the identification of a responder for or a patient sensitive to a selective CDK9 inhibitor, whereby the patient is suspected to suffer from NUT midline carcinoma (NMC). The present invention also relates to a method of monitoring or predicting the efficacy of a treatment of NUT midline carcinoma (NMC), wherein treatment with a selective CDK9 inhibitor is in particular envisaged. Also the use of a (transgenic) non-human animal or a (transgenic) cell having at least one rearrangement in the NUT gene for screening and/or validation of a medicament for the treatment NUT midline carcinoma (NMC) is described. Furthermore, a kit useful for carrying out the methods described herein as well as an oligo- or polynucleotide capable of detecting rearrangements in the NUT gene are provide.



Printed by Jouve, 75001 PARIS (FR)

## Description

The present invention relates to a method of selecting (a) cell(s), (a) tissue(s) or (a) cell culture(s) with susceptibility to a selective CDK9 inhibitor. Also a method for determining the responsiveness of a mammalian tumor cell or cancer cell to treatment with a selective CDK9 inhibitor is described herein. In particular, the present invention provides for an in vitro method for the identification of a responder for or a patient sensitive to a selective CDK9 inhibitor, whereby the patient is suspected to suffer from NUT midline carcinoma (NMC). The present invention also relates to a method of monitoring or predicting the efficacy of a treatment of NUT midline carcinoma (NMC), wherein treatment with a selective CDK9 inhibitor is in particular envisaged. Also the use of a (transgenic) non-human animal or a (transgenic) cell having at least one rearrangement in the NUT gene for screening and/or validation of a medicament for the treatment NUT midline carcinoma (NMC) is described. Furthermore, a kit useful for carrying out the methods described herein as well as an oligo- or polynucleotide capable of detecting rearrangements in the NUT gene are provided.

NUT midline carcinomas (subsequently referred to as "NMC") is a highly lethal cancer that has previously been described to occur in young adults and children; see French (2004) J Clin Oncology 22(20), 4135-4139. However, recent publications indicate that NMC occurs in children and adults of all ages; see French (2010) J Clin Pathol 63: 492-496. NMC is a disease which is genetically defined by rearrangements in the nuclear protein in testis (NUT) gene on chromosome 15q14 most commonly in a balanced translocation with the BRD4 gene or the BRD3 gene. A corresponding rearrangement has first been disclosed in a cell line termed Ty-82 which had been derived from a 22-year old woman with undifferentiated thymic carcinoma; see Kuzume (1992) Int J Cancer 50, 259-264. Later, it has been found that this translocation involving rearrangement in the NUT gene is characteristic for a particularly aggressive form of a midline carcinoma and the term NUT midline carcinoma has been coined; see French (2001) Am J Pathol 159(6), 1987-1992. NMC as a genetically defined disease does not arise from a specific organ. Most cases occur in the mediastinum and upper aerodigestive tract, but in some cases tumors have arisen in bone, bladder, abdominal retroperitoneum, pancrease and salivary glands; see French (2010), Cancer Genetics and Cytogenetics 203, 16-20 and Ziai (2010) Head and Neck Pathol 4, 163-168.

In about two thirds of NMC cases NUT is fused to BRD4 on chromosome 19; see French (2003) Cancer Res 63, 304-307 and French (2008) Oncogene 27, 2237-2242. French (2008) found that in certain cases NUT may also be fused to BRD3. Further, the authors of this document investigated the functional role of BRD-NUT fusion proteins using an siRNA assay for silencing expression. It was found that the suppression of expression of such fusion genes results in squameous differentiation and cell cycle arrest and it was concluded that BRD-NUT fusion proteins contribute to carcinogenesis. It has been suggested in the art that NUT rearrangement is a very early, possible tumour-initiating event; see French (2010) J Clin Pathol (loc. cit.).

NUT rearrangements are restricted to NMC and, therefore, the diagnosis of NMC is not in question once NUT rearrangement has been detected by immunuohistochemical testing (e.g. FISH) or by molecular testing like detection of the expression of NUT fusion genes, in particular BRD4-NUT fusion genes, BRD3-NUT fusion genes or fusions of NUT with other uncharacterised genes (termed NUT-variant fusion genes). The expression of such fusion genes goes along with corresponding NUT rearrangements. Also NMC diagnosis via detection of NUT expression with a NUT specific monoclonal antibody has been disclosed in the art; see Haack (2009) Am J Surg Pathol 33(7), 984-991. Thus, the challenge is not the diagnosis of NMC but rather the decision to perform the diagnosis on subject suspected of suffering from NMC.

Generally, it is believed that NMC is a rare type of cancer; however, most cases of NMC currently go unrecognized due to its lack of characteristic histological features; see French (2010) J Clin Pathol loc. cit. NMCs are often mistaken for other cancer types such as thymic carcinoma, squamous cell carcinoma of the head and neck, lung carcinoma, Ewing sarcoma, and acute leukemia; see Schwartz (2011) Cancer Res 71(7), 2686-2696. French (2010) J Clin Pathol loc. cit. has proposed to consider any poorly differentiated, monomorphic, midline neoplasm that does not stain for lineage-specific markers for NUT rearrangement testing. Many patients with presently undiagnosed NMC would profit enormously from diagnosis and subsequent effective treatment of NMC.

Unfortunately, an effective therapy of NMC is presently not available resulting in a low survival rate (1 survival out of 22 reported cases) and a mean survival of less than 1 year (9.5 months) despite aggressive chemotherapy and radiation treatment, as summarized in Table 1 of French (2010) J Clin Pathol, loc. cit. and French (2010), Cancer Genetics and Cytogenetics 203, 16-20. Further, numerous NMC tumors might not be treated at all or treatment might commence late due to a late or absent NMC diagnosis. Though reliable diagnosis of NMC is, in principle, available, there is, thus, a need in the art for the early identification of patients eligible for efficient treatment of NMC. Clearly, in vitro methods for the identification of susceptible cells are also needed.

Potential therapies of NMC have been proposed in the art. Schwartz (loc.cit.) has investigated the mechanism underlying an NMC subtype that is characterized by the expression of the BRD4-NUT fusion gene. Schwartz found that expression of BRD4-NUT is associated with globally decreased histone deacetylation and transcriptional repression. Therefore, the authors of this document suggest the use of histone deacetylase inhibitors (HDACi) such as vorinostat and romidepsin in order to revert this effect and to thereby treat NMC. Schwartz also suggests the use of small molecule

bromodomain inhibitors (Brdi) to target BRD4-NUT; yet, the authors emphasize that the most specific targeting of BRD4-NUT would be directed at NUT and that the potential difficulties in identifying deliverable NUT-directed inhibitors may be facilitated by the recent development of stapled peptides. In line with Schwartz (loc. cit.) the international patent application WO 2010/011700 describes the use of compounds, in particular histone deacetylase inhibitors, that promote increased acetylation of histones for the treatment of a cancer characterized by NUT or BRD chromosomal rearrangments. Also Filippakopoulos (2010) propose the BRD4-NUT fusion as therapeutic target in NMC using a BRD4-directed inhibitor termed JQ1 (a thieno-triazolo-1,4-diazepine).

Alsarraj (2011) Cancer Res (author manuscript accepted for publication, doi:10.1158/0008-5472.CAN-10-4417) explores the role of the bromodomain-containing chromatin modifying factor BRD4 in development of cancer. Alsarraj describes that ectopic Brd4 expression represses primary tumor growth and that Brd4 activation is predictive for good outcome in human breast cancer; the authors of this document speculate that the tumor- and the metastasis-suppressive properties of Brd4 may be associated with the presence of a proline-rich region in the C-terminal part of one isoform while the extreme C-terminal domain containing a P-TEFb binding region is found to act as a metastasis enhancer. However, Alsarraj does not suggest a potential treatment of cancer and is not concerned at all with NMC.

Thus, the technical problem underlying the present invention is the provision of means and methods allowing the therapeutic intervention in NMC.

The technical problem is solved by provision of the embodiments characterized in the claims.

Accordingly, the present invention relates to a method of selecting (a) cell(s), (a) tissue(s) or (a) cell culture(s) with susceptibility to a selective CDK9 inhibitor, comprising the steps:

(a) determining the presence of a rearrangement in the NUT gene in said cell, tissue or cell culture;
(b) selecting (a) cell(s), tissue(s) or cell culture(s) with at least one rearrangement in the NUT gene;
(c) contacting said cell(s), tissue(s) or cell culture(s) with a selective CDK9 inhibitor; and
(d) evaluating viability of said cell(s), tissue(s) or cell culture(s) contacted with said selective CDK9 inhibitor, wherein a decreased viability is indicative for susceptibility to said selective CDK9 inhibitor.

As used herein, the term "cell, tissue or cell culture" is not only limited to isolated cells, tissues or cell cultures but also comprises the use of samples, i.e. biological, medical or pathological samples that consist of fluids that comprise such cells, tissues or cell cultures. Such a fluid may be a body fluid or also excrements and may also be a culture sample, like the culture medium from cultured cells or cultured tissues. The body fluids may comprise, but are not limited to blood, serum, plasma, urine, saliva, synovial fluid, spinal fluid, cerebrospinal fluid, tears, stool and the like.

Accordingly, the gist of the present invention lies in the finding that the herein provided methods allow for the determination of the susceptibility of a given cell, tissue or cells in a tissue, (or a cell culture or individual cells in such a cell culture, or as will be explained below, (a) cell(s), tissue or cell culture in a biological/medical/pathological sample) for the anti-cancer or anti-proliferative treatment with a selective CDK9 inhibitor.

As shown in the appended examples, it was surprisingly found that cells that comprise a rearrangement in the NUT gene are in particular susceptible to a selective CDK9 inhibitor. The examples provided herein also show that selective CDK9 inhibitor can successfully be employed in the treatment of NUT midline carcinoma (NMC) which is, by definition, characterized by rearrangements in the NUT gene. Nothing in the art suggested the use of selective CDK9 inhibitors in this context.

Therefore, the present invention does not only provide for a method for selecting cells/tissues/cell cultures which are susceptible to (a) selective CDK9 inhibitor(s), but also for an in vitro method for assessing an individual, i.e. a human or animal patient, for its potential responsiveness to treatment of NMC with (a) selective CDK9 inhibitor(s). Thus, the present invention provides not only for the possibility to select cells, tissues and cell cultures that are susceptible for selective CDK9 inhibitor treatment (i.e. the selection of e.g. research tools whereon (a) novel selective CDK9 inhibitor (s) may be tested or which are useful in screening methods for compounds that are suspected to function as (a) selective CDK9 inhibitor(s)) but also for a method to evaluate whether a given patient, preferably a human patient, in need of treatment of NMC, is a responder for selective CDK9 inhibitor treatment. Preferably, the responsiveness of a given patient to Cpd B2 and Cpd B 1 is tested. These and further selective CDK9 inhibitor that may be tested are described herein below in more detail. Further it is expected that the use of CDK9 inhibitors, especially of the selective CDK9 inhibitors is associated with less side effects.

The selection method of a responding cell/tissue/cell culture or a responding patient comprises a step wherein (a) cell(s), tissue(s) or cell culture(s) with at least one rearrangement in the NUT gene is selected. Said rearrangement is indicative for susceptibility to a selective CDK9 inhibitor. The term "rearrangement in the NUT gene" refers to any rearrangement in the NUT gene that is characteristic for NUT midline carcinoma (NMC) or a rearrangement resulting in the expression of a Brd/Nut fusion protein. Exemplary "rearrangments in the NUT gene" as well as methods for their detection are known in the art (see, for example, French (2010) J Clin Pathol, loc. cit.) and also described herein.

In an alternative embodiment, the present invention relates in particular to a method for determining the responsiveness of a mammalian tumor cell or cancer cell to a selective CDK9 inhibitor, said method comprising determining the presence of at least one rearrangement in the NUT gene in said tumor cell, wherein said rearrangement in the NUT gene is indicative of whether the cell is likely to respond or is responsive to the selective CDK9 inhibitor. Such a determination may take place on an individual, isolated tumor cell. Such an evaluation may also be carried out on biological/medical/pathological samples, like body fluids, isolated body tissue samples and the like, wherein said samples preferably comprise cells or cell debris to be analyzed.

As pointed out above, there is a need for stratification of patients who are to be subjected to an anti-NMC therapy with selective CDK9 inhibitors and distinguishing between selective CDK9 inhibitor "responder" and "non-responder" patients.

Therefore, subject of the present invention is also a method for diagnosing an individual who is to be subjected to or is being subjected to an anti-NMC treatment to asses the responsiveness to selective CDK9 inhibitor prior, during and/or after selective CDK9 inhibitor treatment which comprises the steps of (a) detection of at least one rearrangement in the NUT gene in a biological/medical/pathological sample wherein the presence of said at least one rearrangement in the NUT gene is indicative for the responsiveness to a selective CDK9 inhibitor treatment prior, during and after treatment with such selective CDK9 inhibitor; and (b) sorting the individual into responder or non-responder based on detection of said at least one rearrangment in the NUT gene. The presence of at least one rearrangement in the NUT gene as defined herein correlates preferably significantly ($p<0.05$) with a responsiveness to a selective CDK9 inhibitor/susceptibility to a selective CDK9 inhibitor.

The identification of rearrangments in the NUT gene as markers for susceptibility of (tumor) cell(s) to a selective CDK9 inhibitor provides an effective therapeutic approach for patients suffering from cancer characterized by the presence such rearrangments (i.e. NMC). Treatment of susceptible patients with a selective CDK9 inhibitor may lead to an increase in clinical response rate and/or an increase in survival. For example, the clinical response rate may increase to at least 80 %.

As mentioned above, rearrangements in the NUT gene have never been disclosed in context with susceptibility to selective CDK9 inhibitors. At most, HDAC inhibitors, BRD inhbitors or NUT inhbitors have been proposed in context of the development of potential NMC therapies; see Schwartz, loc. cit.

Patients suffering from cancer with (a) rearrangement(s) in the NUT gene (like patients suffering from NMC) have a particularly low survival rate and a bad prognosis and know therapies are not effective. These patients will, therefore, profit enormously from the herein provided therapy with selective CDK9 and the identification of rearrangments in the NUT gene as markers for susceptibility to selective CDK9 inhibitors. Further, the herein described methods allow the identification of responders for/patients sensitive to an CDK9 inhibitor prior to initiation of clinical trials involving patients.

The terms "susceptibility to a selective CDK9 inhibitor" and "responsiveness to treatment with a selective CDK9 inhibitor" are used interchangeably in context of the present invention. Any explanations given herein in respect to "susceptibility to a selective CDK9 inhibitor" also apply to "responsiveness to treatment with a selective CDK9 inhibitor", mutatis mutandis, and vice versa. Technical means and methods for determining the susceptibility to drugs are known in the art. Such methods comprise, inter alia, cell or tissue culture experiments. It is also envisaged herein that two or more different selective CDK9 inhibitors (i.e. selective CDK9 inhibitors having different chemical formulae, optionally non-structurally related selective CDK9 inhibitors) may be tested simultaneously. However, it is preferred herein that only one selective CDK9 inhibitor is tested at one time. Preferred selective CDK9 inhibitors to be used and tested in the present invention are described herein below.

The selection methods or method for determining the responsiveness to treatment with a selective CDK9 inhibitor provided herein may comprise a contacting step/exposing step which is explained in more detail herein below.

These above-mentioned methods may also comprise an evaluation/determination step, which may, for example, include determining the viability of the cell(s), tissue(s) or cell culture(s) contacted with/exposed to a selective CDK9 inhibitor or (a) mammalian cell(s) treated with a selective CDK9 inhibitor. For example, (a) cell(s), (a) tissue(s) or (a) cell culture(s) described herein above may show decreased viability upon contacting/exposing/treating with a selective CDK9 inhibitor. Preferably, the cell(s), tissue(s) or cell culture(s) may show an at least 10 %, 20 %, 30 %, 40 %, 50 %, 60 %, 70 %, 80 %, and most preferably, at least 90 % reduction in viability compared to control cell(s), tissue(s) or cell culture (s) not contacted/exposed/treated with a selective CDK9 inhibitor. Preferably, the control cell(s), (a) tissue(s) or (a) cell culture(s) will be identical to the cell(s), (a) tissue(s) or (a) cell culture(s) to be tested as described herein with the only exception that the control (s), (a) tissue(s) or (a) cell culture(s) are not contacted with/exposed to the selective CDK9 inhibitor.

Thus, (a) cell(s), (a) tissue(s) or (a) cell culture(s) contacted/exposed/treated with a selective CDK9 inhibitor and showing, for example, a decreased proliferation as described herein above, can be considered as being susceptible to a selective CDK9 inhibitor. Correspondingly, (a) mammalian cell(s) treated with a selective CDK9 inhibitor showing such a decreased proliferation can be considered as responsive to treatment with a selective CDK9 inhibitor.

The step of determining the presence of a rearrangement in the NUT gene is described herein below. As already

mentioned above, it has been surprisingly found in context of this invention that the presence of such a a rearrangement in the NUT gene is indicative of whether (a) cell(s), (a) tissue(s) or (a) cell culture(s) contacted/treated with or exposed to selective CDK9 inhibitor is susceptible to said inhibitor or responsive to treatment with a selective CDK9 inhibitor. A reduction in viability may, for example, be reflected in a decreased proliferation, such as 10 %, 20 %, 30 %, 40 %, 50 %, 60 %, 70 %, 80 %, and most preferably, 90 % reduction in proliferation compared to control cell(s), tissue(s) or cell culture(s) not contacted/exposed/treated with a selective CDK9 inhibitor The decreased proliferation may be quantitated, for example, by measuring the total cell volume, tissue volume or cell culture volume using standard techniques.
The difference in proliferation between contacted/exposed/treated cell(s), tissue(s) or cell culture(s) and corresponding controls as defined herein may, for example, be evaluated/determined by measuring the volume of the cell(s), tissue(s) or cell culture(s) taking advantage of standard techniques. Said evaluation/determination may be performed in various points in time, for example, 15 minutes, 30 minutes, 60 minutes, 2 hours, 5 hours, 18 hours, 24 hours, 2 days, 3 days, 4 days, 5 days, six days and/or seven days after contacting/treating said cell(s), tissue(s) or cell culture(s) with a selective CDK9 inhibitor or exposing said cell(s), tissue(s) or cell culture(s) to a selective CDK9 inhibitor. It is envisaged herein that said evaluation/determination may be performed repeatedly, for example, at 15 minutes, 30 minutes and 60 minutes after said contacting/exposing/treating. It is of note that said cell(s), tissue(s) or cell culture(s) may be contacted /treated not only once with said selective CDK9 inhibitor or exposed to said selective CDK9 inhibitor but several times (e.g. 2 times, 3 times, 5 times, 10 times or 20 times) under various conditions (e.g. same concentration of inhibitor, different concentration of inhibitor, inhibitor comprised in a composition with different stabilizers, diluents, and/or carriers and the like). Accordingly, said optionally repeated evaluation/determination may be performed after the final contacting/treating with or exposing to said selective CDK9 inhibitor or in between said above-mentioned various contacting/exposing/ treating steps. The explanations given herein above in respect of the exemplary determination/evaluation step, comprising determining the proliferation of the cell(s), tissue(s) or cell culture(s) contacted with/exposed to an selective CDK9 inhibitor apply to other determination/evaluation steps described herein (e.g. determining the level of NUT expression, expression of NUT fusion genes like BRD4-NUT fusion genes or BRD3-NUT fusion genes and the like) and further determination/evaluation steps a person skilled in the art will be aware of, such as assays that quantitate the induction of apoptotic cell death, senescence or any other cell biology phenotype that is associated with decreased viability or proliferation of tumor cells.

These selection methods or method for determining the responsiveness to treatment with a selective CDK9 inhibitor may also comprise determinining the level of NUT activity or activity of NUT fusion genes, wherein the activity of NUT activity or activity of NUT fusion genes is indicative whether the cell(s), tissue(s) or cell culture(s) is (are) susceptible to a selective CDK9 inhibitor or is (are) responsive to treatment with a selective CDK9 inhibitor. The term "activity" used herein comprises, for example, determining the activity at the protein level and/or the determination of the expression level (e.g. mRNA or protein). Methods for determining the activity as defined herein are well known in the art and also described herein below.

As used herein, a kinase "inhibitor" refers to any compound capable of downregulating, decreasing, suppressing or otherwise regulating the amount and/or activity of a kinase. Inhibition of these kinases can be achieved by any of a variety of mechanisms known in the art, including, but not limited to binding directly to the kinase polypeptide, denaturing or otherwise inactivating the kinase, or inhibiting the expression of the gene (e.g., transcription to mRNA, translation to a nascent polypeptide, and/or final polypeptide modifications to a mature protein), which encodes the kinase. Generally, kinase inhibitors may be proteins, polypeptides, nucleic acids, small molecules, or other chemical moieties.
As used herein the term "inhibiting" or "inhibition" refers to the ability of a compound to downregulate, decrease, reduce, suppress, inactivate, or inhibit at least partially the activity of an enzyme, or the expression of an enzyme or protein and/or the virus replication.

The term "CDK9 inhibitor" means accordingly in this context a compound capable of inhibiting the expression and/or activity of "CDK9" defined herein above. An CDK9 inhibitor may, for example, interfere with transcription of a CDK9 gene, processing (e.g. splicing, export from the nucleus and the like) of the gene product (e.g. unspliced or partially spliced mRNA) and/or translation of the gene product (e.g. mature mRNA). The CDK9 inhibitor may also interfere with further modification (like phosphorylation) of the polypeptide/protein encoded by the CDK9 gene and thus completely or partially inhibit the activity of the CDK9 protein as described herein above. Furthermore, the CDK9 inhibitor may interfere with interactions of the CDK9 protein with other proteins.

In accordance with the above, the compounds according to the general formula (I) disclosed herein below as well as pharmaceutically acceptable salts thereof are used as an inhibitor for a protein kinase, preferably as an inhibitor for a cellular protein kinase.
In a preferred embodiment of this aspect said cellular protein kinase consists of Cyclin-dependent protein kinases (CDKs). The cyclin-dependent protein kinase can be selected from the group comprising: CDK1, CDK2, CDK3, CDK4, CDK5, CDK6, CDK7, CDK8, CDK9, CDK10, CDK11, CrkRS (Crk7, CDC2-related protein kinase 7), CDKL1 (cyclin-dependent kinase-like 1); KKIALRE, CDKL2 (cyclin-dependent kinase-like 2), KKIAMRE, CDKL3 (cyclin-dependent kinase-like 3), NKIAMRE, CDKL4, similar to cyclin-dependent kinase-like 1, CDC2L1 (cell division cycle 2-like 1), PITSLRE B, CDC2L1

(cell division cycle 2-like 1), PITSLRE A, CDC2L5 (cell division cycle 2-like 5), PCTK1 (PCTAIRE protein kinase 1), PCTK2 (PCTAIRE protein kinase 2), PCTK3 (PCTAIRE protein kinase 3) or PFTK1 (PFTAIRE protein kinase 1).
In a particularly preferred embodiment said cyclin-dependent protein kinase is CDK9. Thus, the compounds according to the general formula (I) as well as pharmaceutically acceptable salts thereof are, in a very preferred embodiment, used as an inhibitor for CDK9, in particular as a selective CDK9 inhibitor.

Furthermore, in another particularly preferred embodiment the compounds according to the invention show a high potency (demonstrated by a low $IC_{50}$ value) for inhibiting CDK9 activity. In context of the present invention, the $IC_{50}$ value with respect to CDK9 can be determined by the methods described in the method section of PCT patent application PCT/EP2011/001445 which is incorporated herein by reference in its entirety. Preferably, it is determined according to the method described in section 3.6 of said PCT patent application PCT/EP2011/001445.

Surprisingly it turned out that the compounds according to the general formula (I) as well as pharmaceutically acceptable salts thereof selectively inhibit CDK9 in comparison to other protein kinases and in comparison to other cyclin-dependent protein kinases. Thus, the compounds according to the general formula (I) as well as pharmaceutically acceptable salts thereof are used as selective inhibitors for CDK9.

Particularly preferred compounds of the present invention according to formula (I) show a stronger CDK9 than CDK2 inhibition. In context of the present invention, the $IC_{50}$ value with respect to CDK2 can be determined by the methods described in the method section of PCT patent application PCT/EP2011/001445. Preferably, it is determined according to the method described in section 3.5 of PCT/EP2011/001445.

Selectivity expresses the biologic fact that at a given compound concentration enzymes (or proteins) are affected to different degrees. In the case of enzymes selective inhibition can be defined as preferred inhibition by a compound at a given concentration. Or in other words, an enzyme is selectively inhibited over another enzyme when there is a concentration which results in inhibition of the first enzyme whereas the second enzyme is not affected. To compare compound effects on different enzymes it is crucial to employ similar assay formats, such as the LANCE assay as described in more detail below.

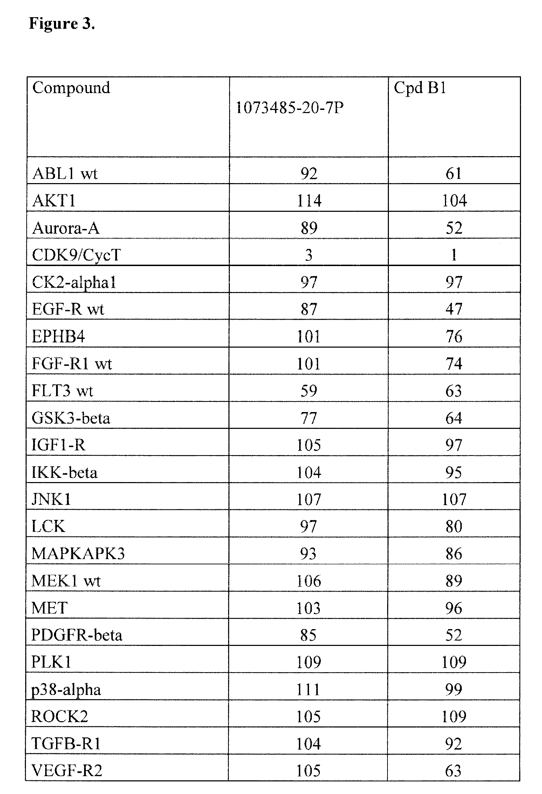

The inhibitors to be used herein are preferably specific for CDK9, i.e. the compounds specifically inhibit CDK9. This is inter alia shown in Figure 3 where the inhibiting effect of exemplary compounds on CDK9 is demonstrated.
A radiometric protein kinase assay (33PanQinase®) Activity Assay) was used for measuring the kinase activity of protein kinases employing exemplary CDK9 inhibitors to be used in the present invention (see Figure **3**). The low kinase activities of CDK9 show that exemplary compounds potently inhibit CDK9. Activities of other kinases are not inhibited.

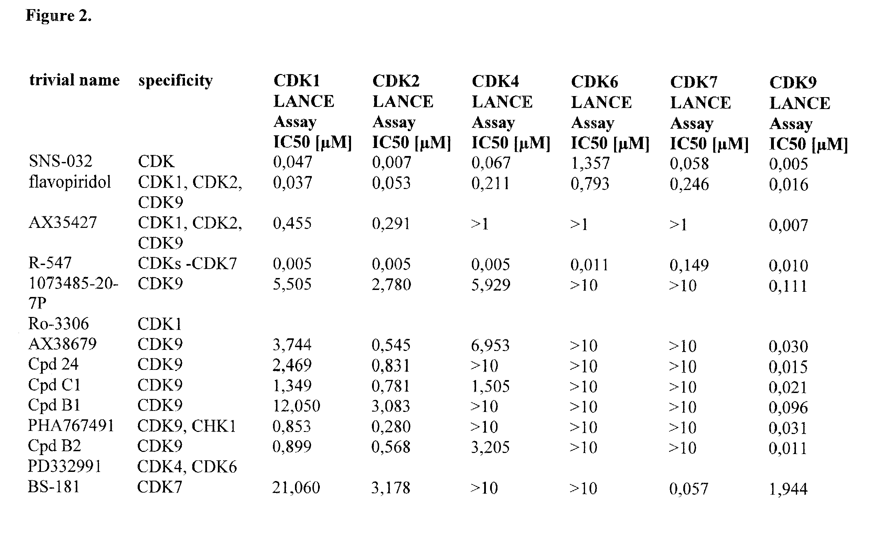

In the experimental part selectivity of the herein provided inhibitors for CDK9 is shown using, inter alia, the well known Lance Assay; see Figure **2.** The Lance assay has been described for example in Moshinsky et al.; 2003 (A widely applicable, high-throughput TR-FRET assay for the measurement of kinase autophosphorylation: VEGFR-2 as a prototype. Moshinsky DJ, Ruslim L, Blake RA, Tang F. J Biomol Screen. 2003 Aug;8(4):447-52). The Lance Ultra KinaSelect Assay may be used to determine half maximal inhibitory concentration ($IC_{50}$) of inhibitor compounds and CDK/Cyclin complexes. The principle behind this enzymatic assay is based upon the phosphorylation of the Ulight-Peptide Substrat. It is detected by using a specific EU-labeled anti-phospho peptide antibody. The binding of the Eu labeled anti-phospho peptide antibody to the phosphorylated U*Light* labeled peptide gives rise to a FRET-signal. Binding of an inhibitor to the kinase prevents phosphorylation of the U*light*-MBP Substrat, resulting in a loss of FRET. Based on these results, the IC50 value can be determined.

The Lance assay and the $^{33}$PanQinase® assay may be performed as follows:

Typically such experiments are started by generation of compounds which are serially diluted in multi titer plates in dimethylsulfoxide (DMSO). In the next step, working solutions for the enzymes, the substrates (protein and ATP separately) are generated in enzyme buffer. The preparation of the assay plate (definition of positive and negative control, reference inhibitors, test compounds and the pipetting of all solutions and compounds except the ATP working solution) is done within the next step. Finally the reaction is started by the addition of the ATP working solution. All pipetting steps can be done manually or by the help of robotics. Within the incubation of 1 h at room temperature the enzyme catalyzes the generation of phosphorylated substrate. This reaction is more ore less inhibited by the added compounds. Finally, to stop the reaction and to detect phosphorylated substrate detection buffer (see material and methods) is added followed by another incubation of 1 h. The data is evaluated by measuring the FRET-Signal. Data is processed by subtraction of the backgroung signal (negative control) from all investigated activities. These activities are set into relation to the positive control. Altogether this is shown by the following equation:

resulting activity (%) = 100 X [(signal of compound – signal of negative control) / (signal of positive control – signal of negative control)]

Further analysis steps include the determination of IC50 values by using the activities of a dose response experiment and an algorithm (equation #205 in Excel fit) for calculation.

A similar experimental procedure is performed when the resulting activity within $^{33}$PanQinase® assay is done. In advance buffers are prepared but in this case the pipetting sequence is first ATP solution diluted with assay buffer, DMSO or compound solution. The reaction (1h at 30°C) is started by addition of a substrate-kinase mix. During the incubation the kinase phosphorylates the substrate (different for each kinase). Due to the fact that the ATP solution contains $^{33}$P-labelled ATP the substrate proteins are labeled with $^{33}$P. The reaction is stopped by addition of excess $H_3PO_4$. If the reaction is performed in plates binding substrate proteins, said plates are washed to reduce unspecific signals (mainly not used ATP). The incorporation of $^{33}$P into substarte proteins is a direct measure of activity of the respective kinase. Therefore, the incorporated radioactivity is detected by scintillation counting. Data is evaluated, processed and analyzed as described for the LANCE assays.

From Figure 2 it can be deduced that a known CDK7-inhibitor (BS-181) has an IC50 value of 1.944 in the CDK9 Lance Assay. As shown in the experimental part, the IC50 value determined for exemplary selective CDK9 inhibitors, for example according to the Lance Assay, is low, preferably below 0.2 μM, more preferably, below 0.15 μM, 0.14 μM, 0.13 μM, 0.12 μM or even lower. More preferably, the IC50 value is below 0.1 μM, 0.095 μM, 0.090 μM, 0.085 μM, 0.080 μM, 0.075 μM, 0.070 μM, 0.065 μM, 0.060 μM, 0.055 μM, 0.050 μM, 0.045 μM, 0.040 μM, 0.035 μM, 0.030 μM , or even below 0.025 μM, wherein the lower values are preferred over the higher values. Even more preferably, the IC50 value is below 0.024 μM, 0.023 μM, 0.022 μM, 0.021 μM, 0.020 μM, 0.019 μM, 0.018 μM, 0.017 μM, 0.016 μM, 0.015 μM, 0.014 μM, 0.013 μM, 0.012 μM, or 0.011 μM. The IC50 value may even be lower, for example, below 0.010 μM, 0.009 μM, 0.008 μM, 0.007 μM, 0.006 μM, or 0.005 μM. Generally, the lower values are preferred herein over the higher values.

It is preferred herein that the ratio of IC50 values of selective CDK9-inhibitors determined according to the CDK9 Lance assay and IC50 values of selective CDK9-inhibitors determined according to the CDK1 Lance assay, CDK2 Lance assay, CDK4 Lance assay, and/or the CDK6 Lance assay is about 1:10 or lower. A ratio of 1:10 or lower also indicates selectivity of the inhibitor for CDK9. More preferred is a ratio of 1:10, 1:20, 1:30, 1:40, 1:50, 1:60, 1:70, 1:80, 1:90 or 1:100 or even lower.

The following selective CDK9 inhibitors are preferably used in accordance with the present invention; these and further selective CDK9 inhibitors for use in the present invention are described in PCT/EP2011/001445, EP10075131.2 (filing date 22.03.2010) EP11075037.9 (filing date 02.03.2011) and EP11075038.7 (filing date 02.03.2011) which are incorporated herein by reference in their entirety.

The disubstituted triazine compounds to be used according to the present invention are defined by the general formula (I)

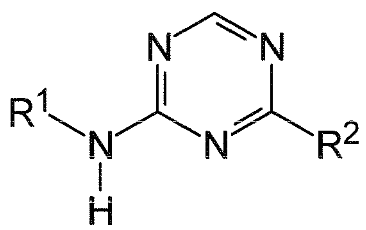

Formula (I)

wherein

**$R^1$** is

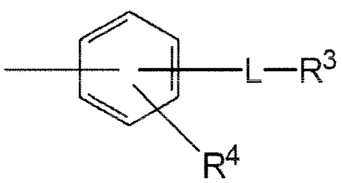 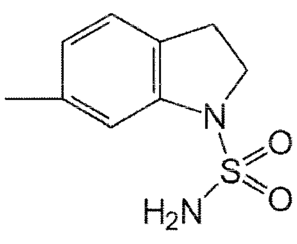 or 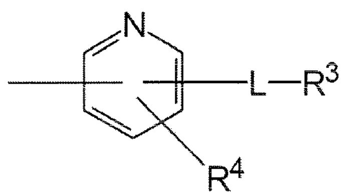

**L** is a bond or $-CR^5R^6-$; $-CR^5R^6-CR^7R^8-$; $-CR^5R^6-CR^7R^8-CR^9R^{10}-$,

**L** is a bond or $-CR^5R^6-$, $-CR^5R^6-CR^7-R^8-$, $CR^5R^6-CR^7R^8-CR^9-R^{10}-$, $-CR^5R^6-CR^7R^8-CR^9R^{10}-CR^{11}R^{12}-$;

**$R^5$ - $R^{12}$** represent independently of each other -H, $-CH_3$, $-C_2H_5$, $-C_3H_7$, -F, -Cl, -Br, -I;

**$R^3$** is selected from -H, $NO_2$, $NH_2$, -CN, -F, -Cl, -Br, -I, $-CH_3$, $-C_2H_5$, -Ph, $-C_3H_7$, $-CH(CH_3)_2$, $-C_4H_9$, $-CH_2-CH(CH_3)_2$, $-CH(CH_3)-C_2H_5$, $-C(CH_3)_3$, $-O-CH_3$, $-O-C_2H_5$, $-O-C_3H_7$, $-O-CH(CH_3)_2$, $-O-C_4H_9$, $-O-CH_2-CH(CH_3)_2$, $-O-CH(CH_3)-C_2H_5$, $-O-C(CH_3)_3$, $-CR^{13}R^{14}R^{21}$, $-CR^{13}R^{14}-CR^{15}R^{16}R^{21}$, $-O-CR^{13}R^{14}R^{21}$, $-CR^{13}R^{14}-CR^{15}R^{16}-CR^{17}R^{18}R^{21}$, $-CR^{13}-R^{14}-CR^{15}R^{16}-CR^{17}R^{18}-CR^{19}R^{20}R^{21}$, $-O-CR^{13}R^{14}-CR^{15}R^{16}R^{21}$, $-O-CR^{13}R^{14}-CR^{15}R^{16}-CR^{17}R^{18}R^{21}$, $-SO_2R^{22}$, $-CO-NR^{23}R^{24}$, $-NR^{25}COR^{22}$, $-O-CR^{13}R^{14}-CR^{15}R^{16}-CR^{17}R^{18}-CR^{19}R^{20}R^{21}$, $-NR^{25}SO_2NR^{23}R^{24}$, $-NR^{25}SO_2R^{22}$, $-NR^{25}-CONR^{23}R^{24}$, $-SO_2NR^{23}R^{24}$, $-SO(NR^{26})R^{27}$, -NH-CO-NH-Ph;

**$R^{13}$ - $R^{21}$, $R^{29}$ - $R^{32}$** and **$R^{33}$ - $R^{48}$** represent independently of each other -H, -F, -Cl, -Br, -I;

**$R^{26}$** is -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CR^{13}R^{14}R^{21}$, $-COR^{28}$, $-CR^{13}R^{14}-CR^{15}R^{16}R^{21}$, $-CR^{13}R^{14}-CR^{15}R^{16}-CR^{17}R^{18}-CR^{19}R^{20}-CR^{29}R^{30}R^{21}$, $-CR^{13}R^{14}-CR^{15}R^{16}-CR^{17}R^{18}R^{21}$, $-CR^{13}R^{14}-CR^{15}R^{16}-CR^{17}R^{18}-CR^{19}R^{20}R^{21}$, $-CR^{13}R^{14}-CR^{15}R^{16}-CR^{17}R^{18}-R^{19}R^{20}-CR^{29}R^{30}-CR^{31}R^{32}R^{21}$, $-COOR^{28}$,

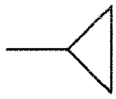 , 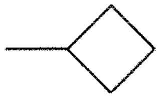 , 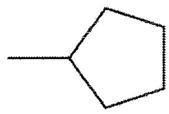 , 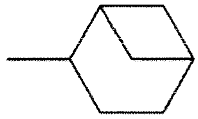 ,

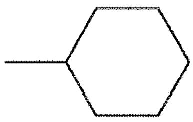 , 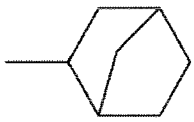 , 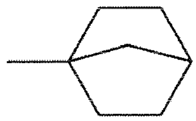 , 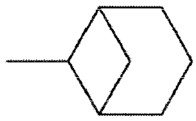 ,

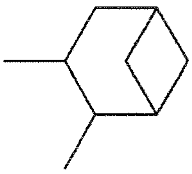 , 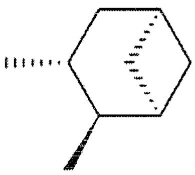 , 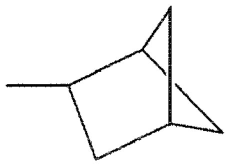 , 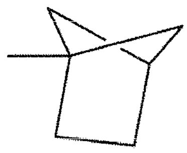 ,

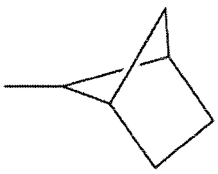 , 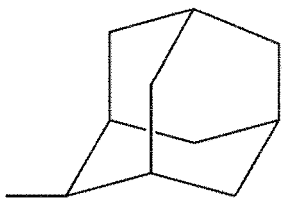 , 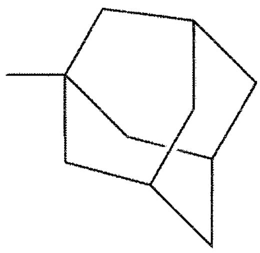 ,

these $C_3$-$C_6$-cycloalkyl groups may further be substituted by one, two, three, four, five or more substituents selected from the group consisting of $R^{33}$ - $R^{48}$;

**$R^{22}$**, **$R^{27}$**, and **$R^{28}$** are independently selected from $-CR^{49}R^{50}R^{51}$, $-CR^{49}R^{50}-CR^{52}R^{53}R^{51}$, $-CR^{49}R^{50}-CR^{52}R^{53}-CR^{54}R^{55}-CR^{56}R^{57}-CR^{58}R^{59}R^{51}$, $-CR^{49}R^{50}-CR^{52}R^{53}-CR^{54}R^{55}R^{51}$, $-CR^{49}R^{50}-CR^{52}R^{53}-CR^{54}R^{55}-CR^{56}R^{57}R^{51}$, $O-CR^{49}R^{50}-CR^{52}R^{53}-CR^{54}R^{55}-CR^{56}R^{57}-CR^{58}R^{59}-CR^{60}R^{61}R^{51}$, $-CH_2Ph$; $-CH_2Ph$ the phenyl group of which may further be substituted by one, two, three, four or five substituents selected from the group consisting of $R^5$ - $R^{12}$;

$C_3$-$C_6$-cycloalkyl groups listed for $R^{26}$, which may further be substituted by one, two, three, four, five or more substituents selected the group consisting of $R^{33}$ - $R^{48}$;

**$R^{49}$ - $R^{61}$** represent independently of each other -H, $-CH_3$, $-C_2H_5$, $-C_3H_7$, $-C_4H_9$, -F, -Cl, -Br, -I, -OH, $-NO_2$, $-NH_2$;

**$R^{23}$** and **$R^{24}$** are independently selected from -H, $-CR^{49}R^{50}R^{51}$, $-CR^{49}R^{50}$-$CR^{52}R^{53}R^{51}$, $-CR^{49}R^{50}$-$CR^{52}R^{53}$-$CR^{54}R^{55}$-$CR^{56}R^{57}$-$CR^{58}R^{59}R^{51}$, $-CR^{49}R^{50}$-$CR^{52}R^{53}$-$CR^{54}R^{55}R^{51}$, $-CR^{49}R^{50}$-$CR^{52}R^{53}$-$CR^{54}R^{55}$-$CR^{56}R^{57}R^{51}$, $-CR^{49}R^{50}$-$CR^{52}R^{53}$-$CR^{54}R^{55}$-$CR^{56}R^{57}$-$CR^{58}R^{59}$-$CR^{60}R^{61}R^{51}$, $-CR^{49}R^{50}$-$CR^{52}R^{53}$-O-$R^{51'}$, $-CR^{49}R^{50}$-$CR^{52}R^{53}$-$CR^{54}R^{55}$-O-$R^{51'}$, $-CR^{49}R^{50}$-$CR^{52}R^{53}$-$NR^{51'}R^{51''}$, $-CR^{49}R^{50}$-$CR^{52}R^{53}$-$CR^{54}R^{55}NR^{51'}R^{51''}$, $-CR^{49}R^{50}$-$CR^{52}R^{53}$-$CR^{54}R^{55}$-$CR^{56}R^{57}$-$NR^{51'}R^{51''}$, $-CR^{49}R^{50}$-$CR^{52}R^{53}$-$CR^{54}R^{55}$-$CR^{56}R^{57}$-$CR^{58}R^{59}$-$NR^{51'}R^{51''}$,
phenyl, substituted phenyl, benzyl, substituted benzyl, or both residues **$R^{23}$** and **$R^{24}$** together form with the nitrogen atom to which they are attached a azetidine, pyrrolidine, piperidine, piperazine, azepane, or morpholine ring;

**$R^{51'}$** and **$R^{51''}$** represent independently of each other -H, $-CH_3$, $-C_2H_5$, $-C_3H_7$, $-C_4H_9$, $-CH_2Ph$, $-COOC(CH_3)_3$, $-COOCH_3$, $-COOCH_2CH_3$, $-COOCH_2CH_2CH_3$, $-COOCH(CH_3)_2$, $-COOCH_2Ph$, $-COCH_3$;

and **$R^{25}$** is selected from -H, $-CH_3$, $-C_2H_5$, $-C_3H_7$, $-CH(CH_3)_2$, $-C_4H_9$, $-CH_2$-$CH(CH_3)_2$, $-CH(CH_3)$-$C_2H_5$ or $-C(CH_3)_3$;

**$R^4$** is selected from -H, $NO_2$, $NH_2$, -CN, -F, -Cl, -Br, -I, $-CONH_2$, $-SO_2CH_3$, $-SO_2C_2H_5$, $-SO_2C_3H_7$, -NH-$SO_2$-$CH_3$, -NH-$SO_2$-$C_2H_5$, NH-$SO_2$-$C_3H_7$, NHCO-$CH_3$, -NHCO-$C_2H_5$, -NHCO-$C_3H_7$, $-SO_2NR^{23}R^{24}$, $-CH_2$-$SO_2NR^{23}R^{24}$, $-C_2H_4$-$SO_2NR^{23}R^{24}$, $-C_3H_6$-$SO_2NR^{23}R^{24}$, $-SO_2NH_2$, $-CH_2$-$SO_2NH_2$, $-C_2H_4$-$SO_2NH_2$, $-C_3H_6$-$SO_2NH_2$,

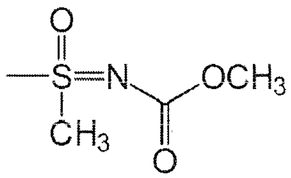 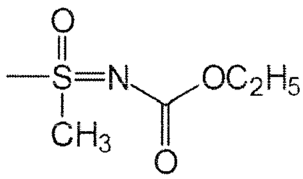 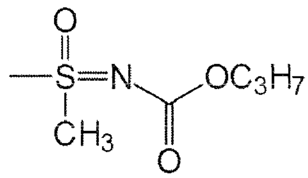

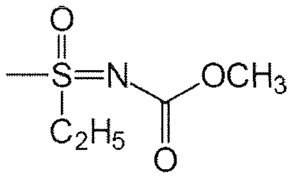 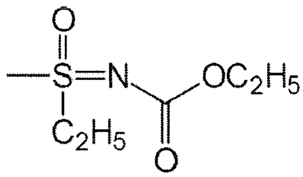 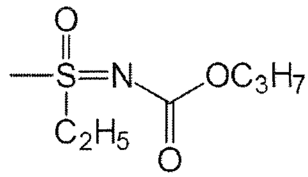

$-CR^{62}R^{63}R^{64}$, $-CR^{62}R^{63}$-$CR^{66}R^{66}$-$CR^{67}R^{68}$-$CR^{69}R^{70}R^{64}$, -O-$CR^{62}R^{63}$-$CR^{65}R^{66}R^{64}$, -O-$CR^{62}R^{63}$-$R^{65}R^{66}$-$CR^{67}R^{68}$-$R^{64}$, $-OR^{62}R^{63}$-$CR^{65}R^{66}$-$R^{67}R^{68}R^{64}$, -O-$CR^{62}R^{63}$-$CR^{65}R^{66}$-$CR^{67}R^{68}$-$CR^{69}R^{70}R^{64}$, $-CR^{62}R^{63}$-$CR^{65}R^{66}R^{64}$, -O-$CR^{62}R^{63}$-$CR^{65}R^{66}$-$CR^{67}R^{68}$-$CR^{69}R^{70}$-$CR^{71}$-$R^{72}R^{64}$, -O-$CR^{62}R^{63}R^{64}$, -O-$CR^{62}R^{63}$-$CR^{65}R^{66}$-$CR^{67}R^{68}$-$CR^{69}R^{70}$-$CR^{71}R^{72}$-$CR^{73}R^{74}R^{64}$, $-CR^{62}R^{63}$-$CR^{65}R^{66}$-$CR^{67}R^{68}$-$CR^{69}R^{70}$-$R^{71}R^{72}R^{64}$-, $-CR^{62}R^{63}$-$CR^{65}R^{66}$-$CR^{67}R^{68}$-$CR^{69}R^{70}$-$CR^{71}R^{72}$-$CR^{73}R^{74}R^{64}$, $-OCH_2Ph$,

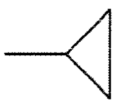, 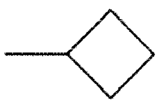, 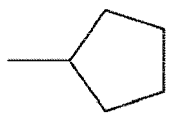, 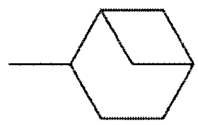,

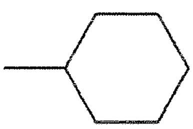, 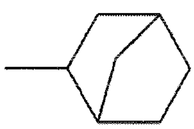, 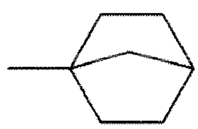, 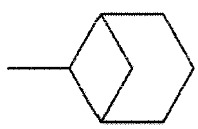,

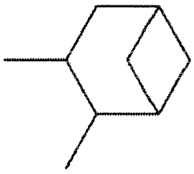 , 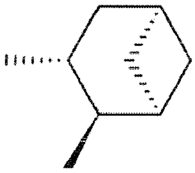 , 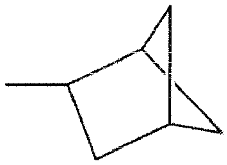 , 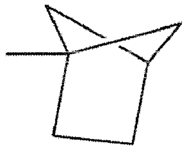 ,

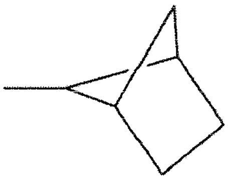 , 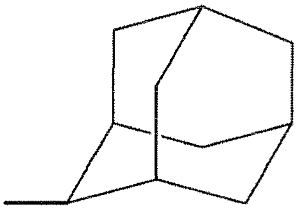 , 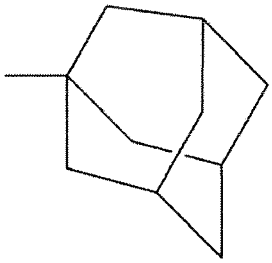 ,

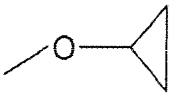 , 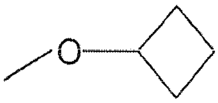 , 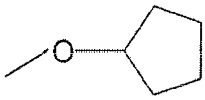 , 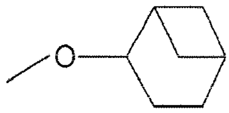 ,

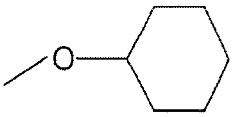 , 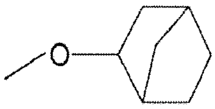 , 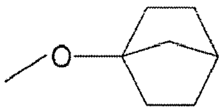 , 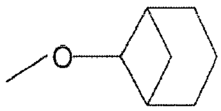 ,

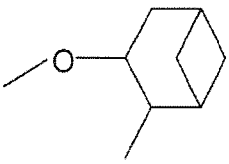 , 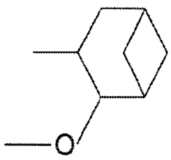 , 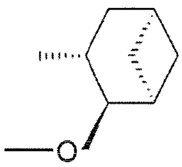 , 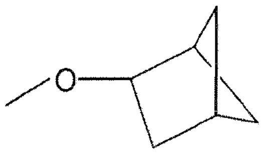 ,

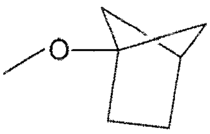 , 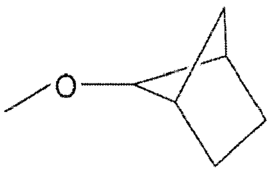 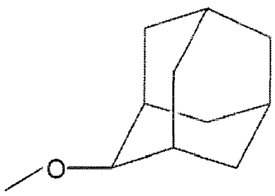 , 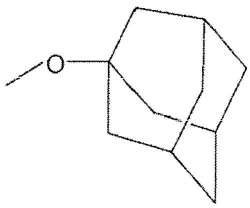 ,

these $C_3$-$C_6$-cycloalkoxy groups and $C_3$-$C_6$-cycloalkyl groups may further be substituted by one, two, three, four, five or more substituents selected from the group consisting of $R^{33}$- $R^{48}$;

**$R^{62}$- $R^{74}$** represent independently of each other -H, -cyclo-$C_3H_5$, -cyclo-$C_4H_7$, -cyclo-$C_5H_9$, -$CR^{75}R^{76}R^{77}$, -$CR^{75}R^{76}$-$CR^{78}R^{79}R^{77}$, -$CR^{75}R^{76}$-$CR^{78}R^{79}$-$CR^{80}R^{81}R^{77}$, -$CR^{75}R^{76}$-$CR^{78}R^{79}$-$CR^{80}R^{79}$-$CR^{82}R^{81}R^{77}$, -F, -Cl, -Br, -I, -Ph;

**$R^{75}$ - $R^{82}$** represent independently of each other -H, -F, -Cl, -Br, -I, -$NH_2$;

**$R^4$** together with $R^{22}$, $R^{23}$, $R^{24}$, or $R^{25}$ may form a group -$CH_2CH_2$- or -$CH_2CH_2CH_2$- if $R^4$ is attached ortho to -L-$R^3$;

**$R^2$** is

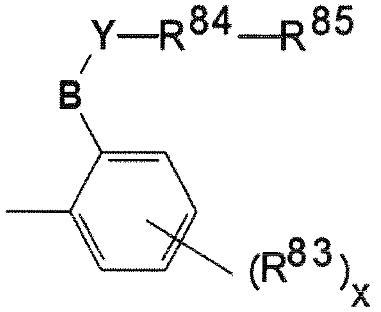

or

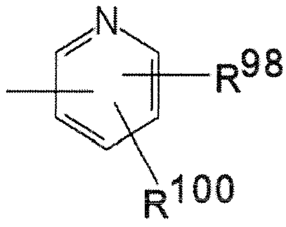

;

**$R^{83}$** is selected from -H, -OH, $-NO_2$, -CN, -F, -Cl, -Br, -I, $-NR^{23'}R^{24'}$, $-CF_3$, $-CR^{62}R^{63}R^{64}$, $-CR^{62}R^{63}-NR^{23'}R^{24'}$, $-CR^{62}R^{63}-R^{65}R^{66}R^{64}$, $-CR^{62}R^{63}-CR^{65}R^{66}-NR^{23}R^{24'}$, $-CR^{62}R^{63}-CR^{65}R^{66}-CR^{67}R^{68}R^{64}$, $-R^{62}R^{63}-CR^{65}R^{66}-CR^{67}-R^{68}-NR^{23'}R^{23'}R^{24'}$, $-O-CR^{62}R^{63}R^{64}$, $-O-CR^{62}R^{63}-CR^{65}R^{66}-R^{64}$, $-O-CR^{62}R^{63}-CR^{65}R^{66}-CR^{67}R^{68}R^{64}$, -CHO, $-CH_2-OH$, $-CR^{23'}O$, $-CH_2OR^{23'}$;

**$R^{23'}$** and **$R^{24'}$** represent independently of each other -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$; -(cyclo-$C_3H_5$);

**x** is a value between 0 and 3;

**B** is a bond, $-CR^{86}R^{87}-$, $-CR^{86}R^{87}-CR^{88}R^{89}-$, $-CR^{86}R^{87}-CR^{88}R^{89}-CR^{90}R^{91}-$, $-CR^{86}R^{87}-CR^{88}R^{89}-CR^{90}R^{91}-CR^{92}R^{93}-$ $-CR^{86}R^{87}-CR^{88}R^{89}-CR^{90}R^{91}-CR^{92}R^{93}-CR^{94}R^{95}-$, - $CR^{86}R^{87}-CR^{88}R^{89}-CR^{90}R^{91}-CR^{92}R^{93}-CR^{94}R^{95}-CR^{96}R^{97}-$;

**$R^{86}$ - $R^{97}$** represent independently of each other -H, $-CH_3$, $-C_2H$-5, $-C_3H_7$, $-C_4H_9$, -F, -Cl, -Br, -I;

**Y** is a bond, -O-, -S-, -SO-, $-SO_2-$, $-SO_2NH-$, $NHSO_2-$, -CO-, -COO-, -OOC-, -CONH-, -NHCO-, -NH-, $-N(CH_3)-$, -NH-CO-NH-, -O-CO-NH-, -NH-CO-O-;

**$R^{84}$** is selected from a bond, $-CR^{86}R^{87}-$, $-CR^{86}R^{87}-CR^{88}R^{89}-CR^{90}R^{91}-$, $-CR^{86}R^{87}-CR^{88}R^{89}-CR^{90}R^{91}-CR^{92}R^{93}-$, $-CR^{86}R^{87}-CR^{88}R^{89}-CR^{90}R^{91}-CR^{92}R^{93}-CR^{94}R^{95}-$ , $-CR^{86}R^{87}-CR^{88}R^{89}-$ , $-CR^{86}R^{87}-CR^{88}R^{89}-CR^{90}R^{91}-CR^{92}R^{93}-CR^{94}R^{95}-CR^{96}R^{97}-$;

**$R^{85}$** is selected from

(i) -H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -Ph, -OPh, $-OCH_2$-Ph, $-OCPh_3$, -SH, $-SCH_3$, $-SC_2H_5$, $-SC_3H_7$, -S-cyclo-$C_3H_5$, $-SCH(CH_3)_2$, $-SC(CH_3)_3$, $-SC_4H_9$, $-NO_2$, -F, -Cl, -Br, -I, $-P(O)(OH)_2$, $-P(O)(OCH_3)_2$, $-P(O)(OC_2H_5)_2$, $-P(O)(OCH(_CH_3)_2)_2$, $-Si(CH_3)_2(C(CH_3)_3)$, $-Si(C_2H_5)_3$, $-Si(CH_3)_3$, -CN, -CHO, $-COCH_3$, $-COC_2H_5$, $-COC_3H_7$, -CO-cyclo-$C_3H_5$, $-COCH(CH_3)_2$, $-COC(CH_3)_3$, $-COC_4H_9$, -COOH, $-COOCH_3$, $-COOC_2H_5$, $-COOC_3H_7$, $-COOC_4H_9$, -COO-cyclo-$C_3H_5$, $-COOCH(CH_3)_2$, $-COOC(CH_3)_3$, -OOC-$CH_3$, -OOC-$C_2H_5$, -OOC-$C_3H_7$, -OOC-$C_4H_9$, -OOC-cyclo-$C_3H_5$,- OOC-$CH(CH_3)_2$, -OOC-$C(CH_3)_3$, -CON-$R^{23'}R^{24'}$, $NHCOCH_3$, $-NHCOC_2H_5$, $-NHCOC_3H_7$, -NHCO-cyclo-$C_3H_5$, -NHCO-$CH(CH_3)_2$, $-NHCOC_4H_9$, -NHCO-$C(CH_3)_3$, -NHCO-$OCH_3$, -NHCO-$OC_2H_5$, -NHCO-$OC_3H_7$, -NHCO-O-cyclo-$C_3H_5$, -NHCO-$OC_4H_9$, -NHCO-OCH $(CH_3)_2$, -NHCO-$OC(CH_3)_3$, -NHCO-$OCH_2$Ph, $-NR^{23}R^{24}$, $-CF_3$, $-SOCH_3$, $-SOC_2H_5$, $-SOC_3H_7$, -SO-cyclo-$C_3H_5$, $-SOCH(CH_3)_2$, $-SOC(CH_3)_3$, $-SO_2CH_3$, $-SO_2C_2H_5$, $-SO_2C_3H_7$, $-SO_2$-cyclo-$C_3H_5$, $-SO_2CH(CH_3)_2$, $-SO_2C_4H_9$, $-SO_2C(CH_3)_3$, $-SO_3H$, $-SO_2NR^{23'}R^{24'}$, $-OCF_3$, $-OC_2F_5$, -O-$COOCH_3$, -O-$COOC_2H_5$, -O-$COOC_3H_7$, -O-COO-cyclo-$C_3H_5$, -O-$COOC_4H_9$, -O-$COOCH(CH_3)_2$, -O-$COOCH_2$Ph, -O-$COOC(CH_3)_3$, -NH-CO-$NH_2$, -NH-CO-$NHCH_3$, -NH-CO-$NHC_2H_5$, -NH-CO-$NHC_3H_7$, -NH-CO-$NHC_4H_9$, -NH-CO-NH-cyclo-$C_3H_5$, -NH-CO-NH[CH $(CH_3)_2$], -NH-CO-NH[$C(CH_3)_3$], -NH-CO-$N(CH_3)_2$, -NH-CO-$N(C_2H_5)_2$, -NH-CO-$N(C_3H_7)_2$, -NH-CO-$N(C_4H_9)_2$, -NH-CO-N(cyclo-$C_3H_5$)$_2$, -NH-CO-N[$CH(CH_3)_2$]$_2$, -NH-CO-N[$C(CH_3)_3$]$_2$, -NH-C(=NH)-$NH_2$, -NH-C(=NH)-$NHCH_3$, -NH-C(=NH)-$NHC_2H_5$, -NH-C(=NH)-$NHC_3H_7$, -NH-C(=NH)-$NHC_4H_9$, -NH-C(=NH)-NH-cyclo-$C_3H_5$, -O-$CH_2$-cyclo-$C_3H_5$, -NH-C(=NH)-NH[$CH(CH_3)_2$], -NH-C(=NH)-NH[$C(CH_3)_3$], -NH-C(=NH)-$N(CH_3)_2$, -NH-C(=NH)-$N(C_2H_5)_2$, -NH-C(=NH)-$N(C_3H_7)_2$, -NH-C(=NH)-N(cyclo-$C_3H_5$)$_2$, -NH-C(=NH)-$N(C_4H_9)_2$, -NH-C(=NH)--N[CH $(CH_3)_2$]$_2$, -NH-C(=NH)-N[$C(CH_3)_3$]$_2$, -O-CO-$NH_2$, -O-CO-$NHCH_3$, -O-CO-$NHC_2H_5$, -O-CO-$NHC_3H_7$, -O-CO-$NHC_4H_9$, -O-CO-NH-cyclo-$C_3H_5$, -O-CO-NH[$CH(CH_3)_2$], -O-CO-NH[$C(CH_3)_3$], -O-CO-$N(CH_3)_2$, -O-CO-N

$(C_2H_5)_2$, $-O-CO-N(C_3H_7)_2$, $-O-CO-N(C_4H_9)_2$, $-O-CO-N(cyclo-C_3H_5)_2$, $-O-CO-N[CH(CH_3)_2]_2$, $-O-CO-N[C(CH_3)_3]_2$,

(ii) an aromatic or heteroaromatic mono- or bicyclic ring selected from 2-thienyl, 3-thienyl, 2-furanyl, 3-furanyl, 2-oxazolyl, 3-oxazolyl, 4-oxazolyl, 2-thiazolyl, 3-thiazolyl, 4-thiazolyl, 1-pyrazolyl, 3-pyrazolyl, 4-pyrazolyl, 5-pyrazolyl, 1-imidazolyl, 2-imidazolyl, 4-imidazolyl, 5-imidazolyl, phenyl, 1-naphthyl, 2-naphthyl, 2-pyridyl, 3-pyridyl, 4-pyridyl, 2-pyrimidinyl, 4-pyrimidinyl, 5-pyrimidinyl, 2-pyrazinyl, 3-pyridazinyl, 4-pyridazinyl, 1,3,5-triazin-2-yl,

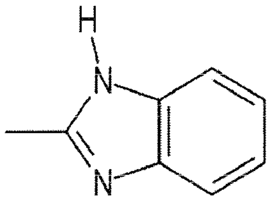 , 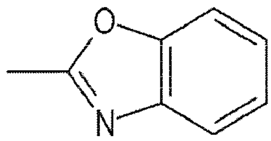 , 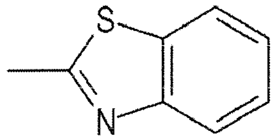 ,

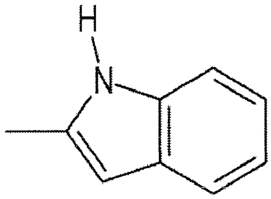 , 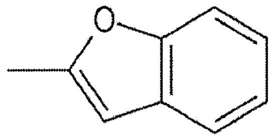 , 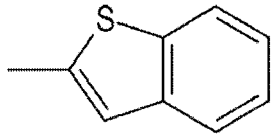 ,

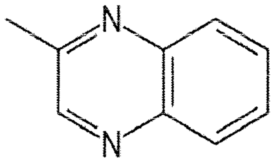 , 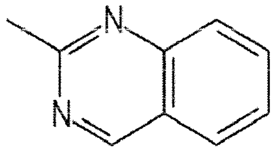 , 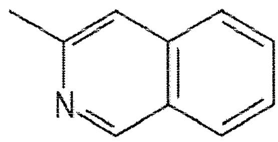 ,

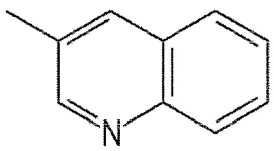 , 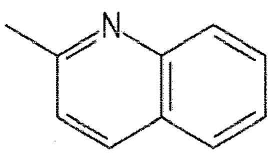 , 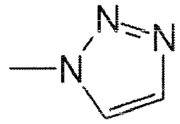 ,

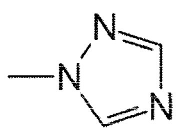 , 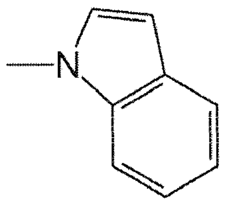 , 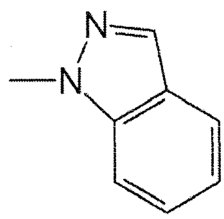 , 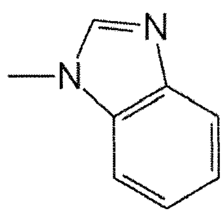 ,

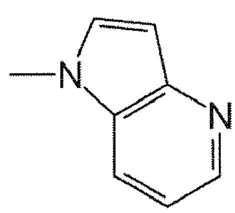 , 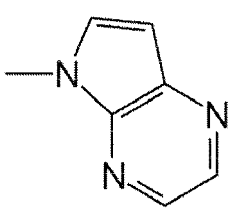 , 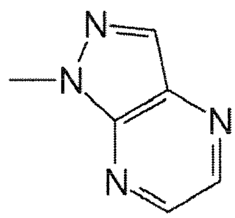 , 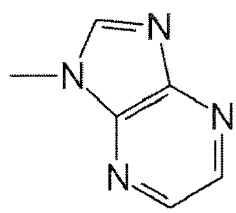 ,

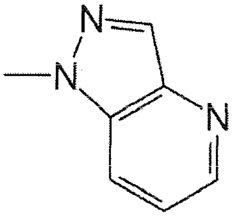 , 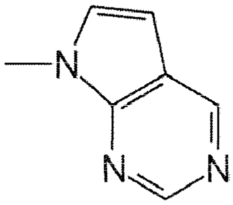 , 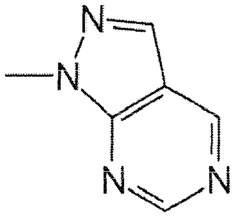 , 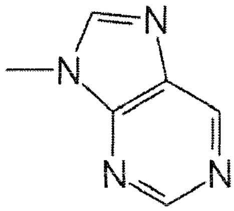 ,

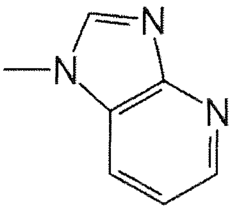 , 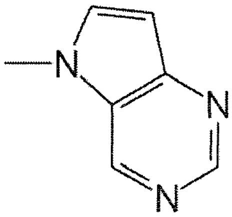 , 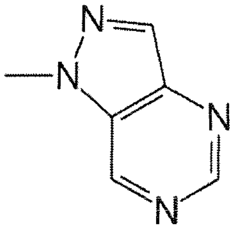 , 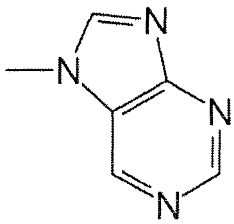 ,

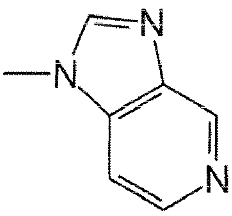 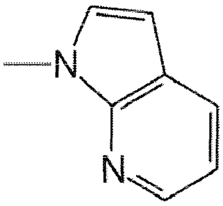 , 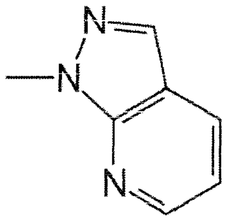 , 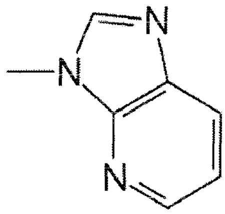 ,

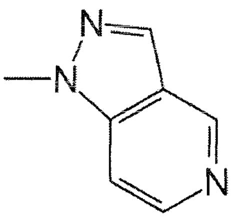 , 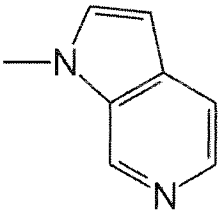 , 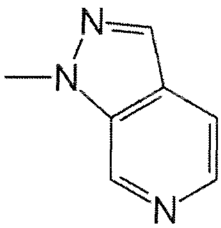 , 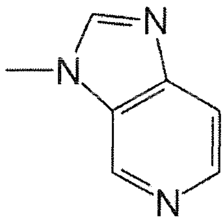 ,

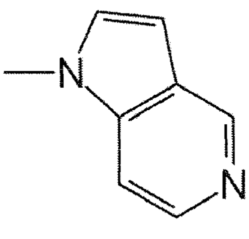 , 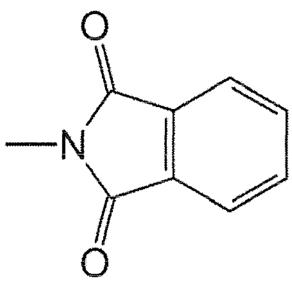 ,

which optionally may be substituted by one or two substituents selected from -F, -Cl, -Br, -I, $-OCH_3$, $-CH_3$, $-NO_2$, -CN, $-CF_3$;

(iii) a saturated ring selected from

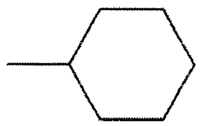,
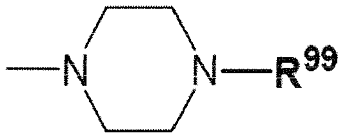,
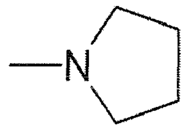,

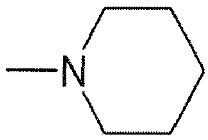,
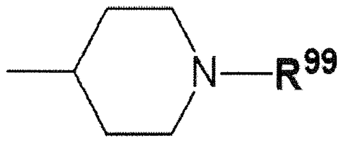,
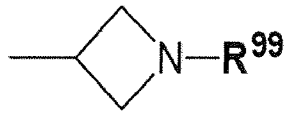,

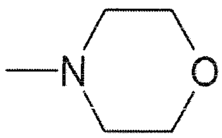,
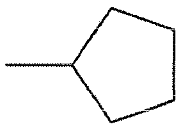,
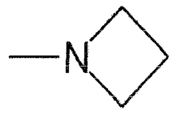;

**$R^{99}$** represents -H, $-CH_3$, $-CH_2Ph$, $-COOC(CH_3)_3$, $-COOCH_3$, $-COOCH_2CH_3$, $-COOCH_2CH_2CH_3$, $-COOCH(CH_3)_2$, $-COOCH_2Ph$, $-COCH_3$;

the group $-B-Y-R^{84}-R^{85}$ together with one substituent $R^{83}$ may form a group $-OCH_2O-$, if $R^{13}$ is attached in position ortho to $-B-Y-R^{84}-R^{85}$;

with the proviso that $R^{83}$ is not -H, if the group $-B-Y-R^{84}-R^{85}$ is hydrogen.

**$R^{98}$** is selected from $-NO_2$, -CN, -F, -Cl, -Br, -I, $-NH_2$, -OH, $-CR^{62}R^{63}-CR^{65}R^{66}-CR^{67}R^{68}-CR^{69}R^{70}R^{64}$, $-O-CR^{62}R^{63}R^{64}$, $-O-CR^{62}R^{63}-CR^{65}R^{66}R^{64}$, $-O-CR^{62}R^{63}-CR^{65}R^{66}-CR^{67}R^{68}R^{64}$, $-O-CR^{62}R^{63}-CR^{65}R^{66}-CR^{67}R^{68}-CR^{69}R^{70}R^{64}$, $-O-CR^{62}R^{63}-CR^{65}R^{66}-CR^{67}R^{68}-CR^{69}R^{70}-CR^{71}R^{72}R^{64}$, $-CR^{62}R^{63}-CR^{65}R^{66}-CR^{67}R^{68}R^{64}$, $-O-CR^{62}R^{63}-CR^{65}R^{66}-CR^{67}R^{68}-CR^{69}R^{70}-CR^{71}R^{72}-CR^{73}R^{74}R^{64}$, $-CR^{62}R^{63}-CR^{65}R^{66}R^{64}$, $-CR^{62}R^{63}-O-CR^{65}R^{66}-CR^{67}R^{68}-CR^{69}R^{70}R^{64}$, $-CR^{62}R^{63}-O-CR^{65}R^{66}-CR^{67}R^{68}R^{64}$, $-CR^{62}R^{63}-O-CR^{65}R^{66}-CR^{67}R^{68}-CR^{69}R^{70}-CR^{71}R^{72}R^{64}$, $-CR^{62}R^{63}-O-CR^{65}R^{66}R^{64}$, $-CR^{62}R^{63}-O-CR^{65}R^{66}-CR^{67}R^{68}-CR^{69}R^{70}-CR^{71}R^{72}-CR^{73}R^{74}R^{64}$, $-CR^{62}R^{63}R^{64}$, $-CR^{62}R^{63}-CR^{65}R^{66}-CR^{67}R^{68}-CR^{69}R^{70}-CR^{71}R^{72}R^{64}$, $-OCH_2Ph$, $-OCH_2-CH_2-Ph$, $-CH_2-O-CH_2-Ph$, $-CR^{62}R^{63}-CR^{65}R^{66}-CR^{67}R^{68}-CR^{69}R^{70}-CR^{71}R^{72}-CR^{73}R^{74}R^{64}$;
with the proviso that $R^{98}$ is attached to a position ortho to the bond between the pyridine and the triazine ring if $R^{98}$ is not an amino group in para position to the bond between the pyridine and the triazine ring;

**$R^{100}$** is selected from -H, $-NO_2$, -CN, -F, -Cl, -Br, -I, $NH_2$, -OH, $-CF_3$, $-CH_3$, $-CH_2CH_3$, $-CH_2CH_2CH_3$, $-CH(CH_3)_2$, $-OCH_3$, $-OCH_2CH_3$, $-OCH_2CH_2CH_3$, $-OCH(CH_3)_2$, $-OCF_3$, $-OCH_2Ph$;
and with the proviso that if $R^1$ is a phenyl moiety and $R^2$ is also a phenyl moiety a chloro substituent is only allowed on the $R^1$ phenyl moiety or on the $R^2$ phenyl moiety but not on both simultaneously;
and with the proviso that the compound 4-[4-(2-benzoylaminophenyl)-[1,3,5]triazin-2-ylamino]benzamide is excluded;

and enantiomers, stereoisomeric forms, mixtures of enantiomers, diastereomers, mixtures of diastereomers, prodrugs, hydrates, solvates, acid salt forms, tautomers, and racemates of the above mentioned compounds and pharmaceutically acceptable salts or salts of solvates thereof.
The expression prodrug is defined as a substance, which is applied in an inactive or significantly less active form. Once applied and incorporated, the prodrug is metabolized in the body *in vivo* into the active compound.
The expression tautomer is defined as an organic compound that is interconvertible by a chemical reaction called tautomerization. Tautomerization can be catalyzed preferably by bases or acids or other suitable compounds.
Preferred are compounds having the general formula (I):

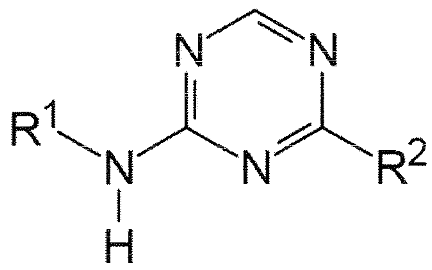

Formula (I)

wherein

**$R^1$** represents

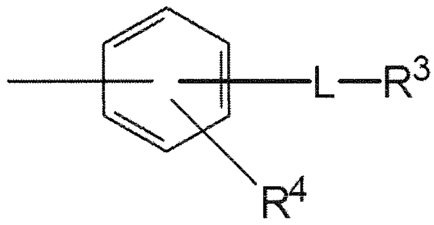

in which

**L** is a bond, $-CH_2-$, $-CH_2CH_2-$, or $-CH_2-$, particularly preferred $-CH_2-$;

**$R^3$** is $-SO_2NH_2$, $-SO_2NH(CH_3)$, $-SO_2N(CH_3)_2$, $-SO_2NH(CH_2CH_2OCH_3)$, $-NHSO_2CH_3$, $-NHSO_2CH_2CH_3$, $-NHSO_2CH_2CH_2CH_3$, $-NHSO_2CF_3$, $-SO_2CH_3$, $-NHSO_2NH_2$, $-SO(NH)CH_3$, particularly preferred $-SO_2NH_2$;

**$R^4$** is -H, $-CH_3$, -F, -Cl, or $-CF_3$, particularly preferred -H;

**$R^2$** represents

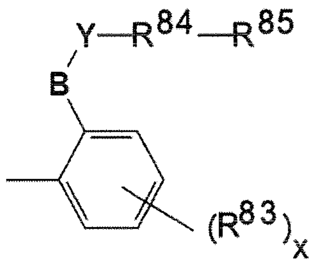

or

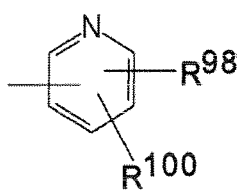

;

in which the group **$-B-Y-R^{84}-R^{85}$** is $-OCH_3$, $-OCH_2CH_3$, $-OCH_2CH_2CH_3$, $-OCH_2CH_2CH_2CH_3$, $-OCH(CH_3)_2$, -OPh, $-OCH_2Ph$, $-OCH_2$(4-pyridyl), particularly preferred $-OCH_3$;

**$R^{83}$** is -H, -F, or -Cl;

x is 0, 1, or 2;

**$R^{98}$** is $-OCH_3$ and **$R^{100}$** is -H, provided that $R^{98}$ is attached to a position ortho to the bond between the pyridine and the triazine ring.

In more preferred compounds of Formula (I)
the substituent **$-L-R^3$** is $-SO_2NH_2$, $-CH_2SO_2NH_2$, $-CH_2CH_2SO_2NH_2$, $-CF_2SO_2NH_2$, $-NHSO_2NH_2$, $-CH_2NHSO_2NH_2$,

$-SO_2CH_3$, $-SO(NH)CH_3$, $-CH_2SO(NH)CH_3$,
and **$R^4$** is -H;
**$R^2$** is 2-methoxyphenyl, 4-fluoro-2-methoxyphenyl, or 6-fluoro-2-methoxyphenyl.

Preferred are compounds of general formula (I), wherein $R^1$ is

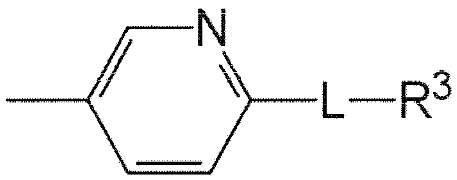

and wherein L is a bond or is $-CH_2-$ or $-CH_2CH_2-$ and $R^3$ has the meanings as defined heren and more preferably $R^3$ represents $-SO_2R^{22}$ or $-SO_2NR^{23}R^{24}$, wherein $R^{22}$, $R^{23}$ and $R^{24}$ have the meanings as defined herein and preferably $R^{22}$, $R^{23}$ and $R^{24}$ represent independently of each other -H $-CF_3$, $-CH_3$, $-CH_2CH_3$, $-CH_2CH_2CH_3$, $-CH_2CH_2CH_2CH_3$, $-CH(CH_3)_2$, $-CH_2-NH_2$, $-CH_2-CH_2-NH_2$, $-CH_2-CH_2-CH_2-NH_2$, $-CH_2-CH_2-CH_2-CH_2-NH_2$, $-CH_2NH-CO-O-C(CH_3)_3$, $-CH_2-CH_2-NH-CO-O-C(CH_3)_3$, $-CH_2-CH_2-CH_2-NH-CO-O-C(CH_3)_3$, $-CH_2-CH_2-CH_2-CH_2-NH-CO-O-C(CH_3)_3$.

Also preferred are compounds of general formula (I), wherein **L** is a bond, $-CH_2-$, $-CH_2CH_2-$, $-CH_2CH_2CH_2-$, or $-CF_2-$, more preferred $-CH_2-$ or $-CH_2CH_2-$.

Preferred are compounds of general formula (I), wherein **$R^2$** is

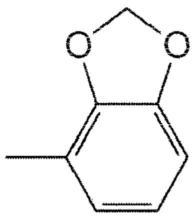
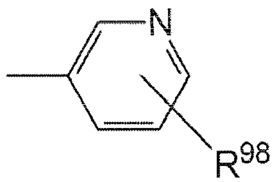
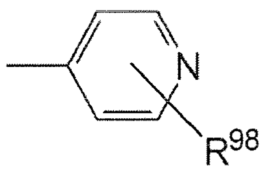

If residue **$R^2$** is a phenyl ring, it is preferred that the substituent **$B-Y-R^{84}-R^{85}$** in ortho position of the linkage to the triazine core is not hydrogen and if that substituent is hydrogen, **$R^{83}$** is not hydrogen and moreover that at least one substituent **$R^{83}$** is in ortho position of the linkage to the triazine core. Thus one substituent of **$B-Y-R^{84}-R^{85}$** and **$R^{83}$** has to be different from hydrogen so that **$R^2$** cannot be an unsubstituted phenyl ring. Moreover it is preferred that $R^{85}$ is not -H, if B, Y and $R^{84}$ are bonds and $R^{83}$ is different from hydrogen. If two substituents are present, it is preferred that the second substituent is in meta position or para position of the linkage to the triazine core. If a third substituent is present the substitution pattern 2,3,5 or 2,3,4 are preferred. Fluorine is a preferred second and/or third substituent and is preferably in meta or para position of the linkage to the triazine core. Thus, the following residues **$R^2$** are preferred:

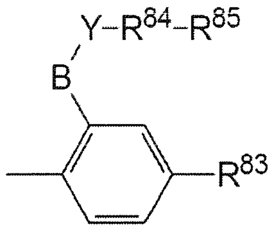 , 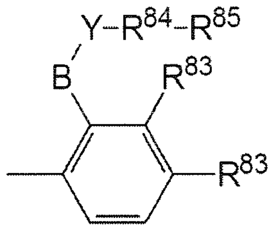 , 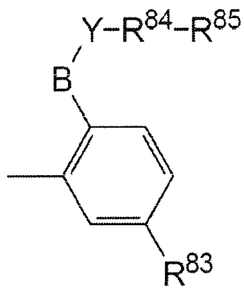 , 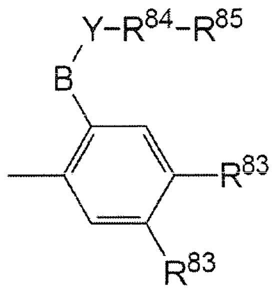 .

If residue **$R^2$** is a pyridyl ring it is preferred that one substituent of **$R^{98}$** is in ortho position of the linkage to the triazine core. Preferred are the following **$R^2$** residues:

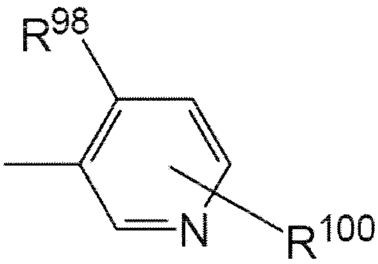
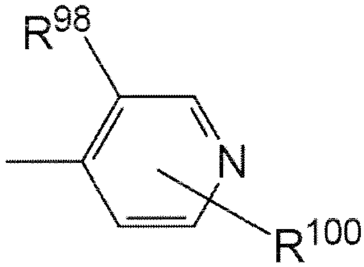
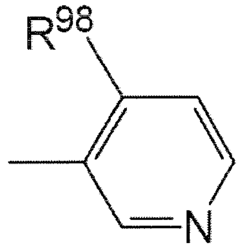

Also preferred are compounds of general formula (I), wherein $\mathbf{R^{85}}$ is

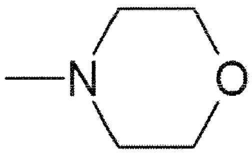
,
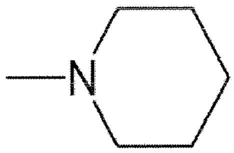
,
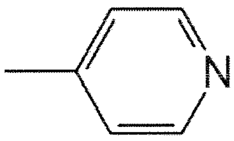
.

$\mathbf{R^3}$ is preferably selected from -H, $-NO_2$, $NH_2$, -CN, -F, -Cl, -Br, -I, $-CH_3$, $-C_2H_5$, - Ph, $-C_3H_7$, $-CH(CH_3)_2$, $-C_4H_9$, $-CH_2-CH(CH_3)_2$, $-CH(CH_3)-C_2H_5$, $-C(CH_3)_3$, $-O-CH_3$, $-O-C_2H_5$, $-O-_3H_7$, $-O-CH(CH_3)_2$, $-O-C_4H_9$, $-O-CH_2-CH(CH_3)_2$, $-O-CH(CH_3)-C_2H_5$, $-O-C(CH_3)_3$, $-SO_2R^{22}$ and $-SO_2NR^{23}R^{24}$.

$\mathbf{R^{26}}$ is preferably selected from -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, -cyclo-$C_3H_5$, -cyclo-$C_4H_7$ and -cyclo-$C_5H_9$.

Moreover compounds of general formula (I), wherein $\mathbf{R^{22}}$, $\mathbf{R^{23}}$, $\mathbf{R^{24}}$, $\mathbf{R^{27}}$ and $\mathbf{R^{28}}$ are independently of each other selected from -H, $-CH_3$, $-C_2H_5$, $-C_3H_7$, $-C_4H_9$ or $-CH_2Ph$.

Preferably $\mathbf{R^{62}}$ - $\mathbf{R^{74}}$ represent independently of each other -H, -Ph, -cyclo-$C_3H_5$, -cyclo-$C_4H_7$, $-CH_3$, $-C_2H_5$, $-C_3H_7$, $-C_4H_9$, -cyclo-$C_5H_9$, -F, -Cl, -Br or -I.

Furthermore preferred are compounds of general formula (I), wherein $\mathbf{R^4}$ is selected from -H, - $NO_2$, $-NH_2$, -CN, -F, -Cl, -Br, -I, -cyclo-$C_3H_5$, -cyclo-$C_4H_7$, -cyclo-$C_5H_9$, $-CH_3$, $-C_2H_5$, $-C_3H_7$, $-CH(CH_3)_2$, $-C_4H_9$, $-CONH_2$, $-SO_2CH_3$, $-SO_2C_2H_5$, $-SO_2C_3H_7$, $-NH-SO_2-CH_3$, $-NH-SO_2-C_2H_5$, $-NH-SO_2-C_3H_7$, $-NHCO-CH_3$, $-NHCO-C_2H_5$, $-NHCO-C_3H_7$, $-SO_2NR^{23}R^{24}$, $-CH_2-SO_2NR^{23}R^{24}$, $-C_2H_4-SO_2NR^{23}R^{24}$, $-C_3H_6-SO_2NR^{23}R^{24}$, $-SO_2NH_2$, $-CH_2-SO_2NH_2$, $-C_2H_4-SO_2NH_2$, $-C_3H_6-SO_2NH_2$,

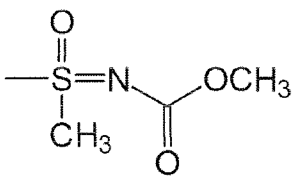
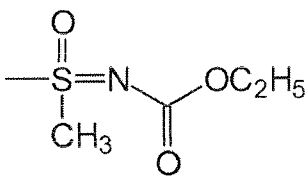
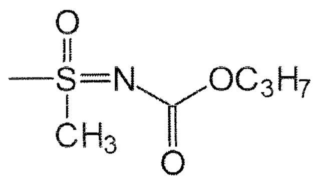

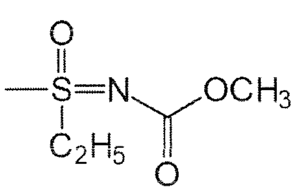
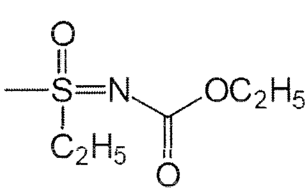
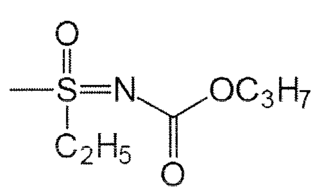

$-CH(CH_3)-C_2H_5$ $-C(CH_3)_3$, $-C_5H_{11}$, $-CH_2-CH_2-CH_2-CH_2R^{64}$, $-O-CH_2-CH_2R^{64}$, $-CH_2R^{64}$, $-O-CH_2-CH_2-CH_2R^{64}$, $-CH_2-CH_2-CH_2R^{64}$, $-O-CH_2-CH_2-CH_2-CH_2R^{64}$, $-CH_2-$ $CH_2R^{64}$, $-O-CH_2-CH_2-CH_2-CH_2-CH_2R^{64}$, $-CH_2-CH_2-CH_2CH_2-CH_2R^{64}$, $-O-CH_2-CH_2-CH_2-CH_2-CH_2-CH_2R^{64}$, $-CH_2-CH_2-CH_2-CH_2-CH_2-CH_2R^{64}$, $-OCH_2Ph$, $-O-CH_2R^{64}$, wherein $\mathbf{R^{64}}$ represents -Ph, -F, -Cl, -Br or -I. Preferred are compounds wherein, $\mathbf{R^4}$ is selected from $-NO_2$,

$-NH_2$, $-CONH_2$, $-SO_2CH_3$, $-SO_2C_2H_5$, $-SO_2C_3H_7$, $-NH-SO_2-CH_3$, $-NH-SO_2-C_2H_5$, $-NH-SO_2-C_3H_7$, $-NHCO-CH_3$, $-NHCO-C_2H_5$, $-NHCO-C_3H_7$, $-SO_2NR^{23}R^{24}$, $-CH_2-SO_2NR^{23}R^{24}$, $-C_2H_4-SO_2NR^{23}R^{24}$, $-C_3H_6-SO_2NR^{23}R^{24}$, $-SO_2NH_2$, $-CH_2-SO_2NH_2$, $-C_2H_4-SO_2NH_2$, $-C_3H_6-SO_2NH_2$,

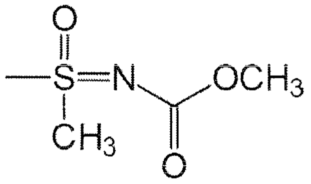
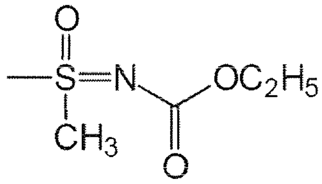
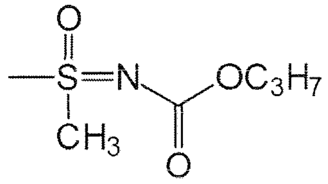

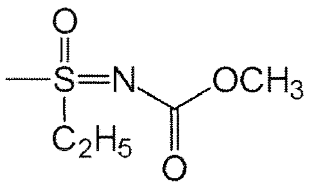
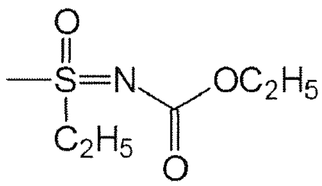
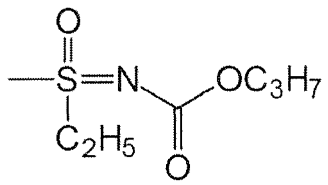

Moreover it is especially preferred that not both substituents $-L-R^3$ and $-R^4$ are hydrogen. Thus it is preferred that the phenyl substituent $R^1$ and the pyridyl substituent $R^1$ have at least one substituent and preferably one substituent in meta position and most preferably the preferred substituents mentioned above for $-L-R^3$ and $-R^4$ in meta position and especially preferred for $-R^4$ in meta position. Consequently the following $\mathbf{R^1}$ residues are preferred and especially preferred are the following substituents $\mathbf{R^1}$ with the preferred substituents for $-L-R^3$ and $-R^4$:

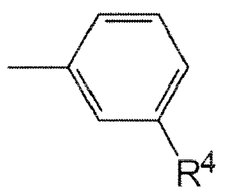
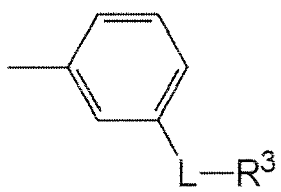

Also preferred are compounds of general formula (I), wherein $\mathbf{R^{83}}$ is -H, -OH, $-NO_2$, -CN, - F, -Cl, -Br, -I, $-NH_2$, $-NH(CH_3)$, $-N(CH_3)_2$, $-NH(C_2H_5)$, $-N(C_2H_5)_2$, $-CF_3$, $-CH_3$, $-C_2H_5$, $-C_3H_7$, $-CH(CH_3)_2$, $-C_4H_9$, $-C(CH_3)_3$, $-CH_2-NH_2$, $-CH_2-NH(CH_3)$, $-CH_2-N(CH_3)_2$, $-CH_2-NH(C_2H_5)$, $-CH_2-N(C_2H_5)_2$, $-CH_2-CH_2-NH_2$, - $CH_2-CH_2-NH(CH_3)$, $-CH_2-CH_2-N(CH_3)_2$, $-CH_2-CH_2-NH(C_2H_5)$, $-CH_2-CH_2-N(C_2H_5)_2$, - $CH_2-CH_2-CH_2-NH_2$, $-CH_2-CH_2-CH_2-NH(CH_3)$, $-CH_2-CH_2-CH_2-N(CH_3)_2$, $-CH_2-CH_2-CH_2-NH(C_2H_5)$, $-CH_2-CH_2-CH_2-N(C_2H_5)_2$, $-O-CH_3$, $-O-CH_2-CH_3$, $-O-CH_2-CH_2-CH_3$, -CHO, $-CH_2OH$, $-CO-CH_3$, $-CO-CH_2-CH_3$, $-CO-CH_2-CH_2-CH_3$, $-CO-CH_2-CH_2-CH_2-CH_3$, $-CH_2O-CH_3$, $-CH_2O-CH_2-CH_3$, $-CH_2O-CH_2-CH_2-CH_3$, $-CH_2F$, $-CH_2Cl$, $-CH_2Br$, $-CH_2-CH_2F$, $-CH_2-CH_2Cl$, $-CH_2-CH_2Br$, $-CH_2-CH_2-CH_2F$, $-CH_2-CH_2-CH_2Cl$, $-CH_2-CH_2-CH_2Br$.

Moreover compounds of general formula (I) are preferred, wherein **B** represents a bond, $-CH_2-$, - $CH_2CH_2-$, $-CH_2CH_2CH_2-$, $-CH_2CH_2CH_2CH_2-$, $-CH_2CH_2CH_2CH_2CH_2-$ and/or wherein **Y** represents a bond, -O-, or -NH-.

In addition compounds of general formula (I) are preferred, wherein $\mathbf{R^{84}}$ represents a bond, $-CH_2-$, $-CH_2CH_2-$, $-CH_2CH_2CH_2-$, $-CH_2CH_2CH_2CH_2-$, $-CH_2CH_2CH_2CH_2CH_2-$.

Preferred are also compounds of general formula (I), wherein $\mathbf{R^{85}}$ is -H, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -Ph, -OPh, $-OCH_2-Ph$, $-OCPh_3$, $-NO_2$, -F, -Cl, -Br, -I, -CN, -CHO, $-COCH_3$, $-COC_2H_5$, $-COC_3H_7$, -CO-cyclo-$C_3H_5$, $-COCH(CH_3)_2$, $-COC(CH_3)_3$, $-COC_4H_9$, -COOH, $-COOCH_3$, $-COOC_2H_5$, $-COOC_3H_7$, $-COOC_4H_9$, -COO-cyclo-$C_3H_5$, $-COOCH(CH_3)_2$, $-COOC(CH_3)_3$, $-OOC-CH_3$, $-OOC-C_2H_5$, $-OOC-C_3H_7$, $-OOC-C_4H_9$, -OOC-cyclo-$C_3H_5$, $-OOC-CH(CH_3)_2$, $-OOC-C(CH_3)_3$, $-CONR^{23'}R^{24'}$, $-NHCOCH_3$, $-NHCOC_2H_5$, $-NHCOC_3H_7$, -NHCO-cyclo-$C_3H_5$, $-NHCO-CH(CH_3)_2$, $-NHCOC_4H_9$, $-NHCO-C(CH_3)_3$, $-NHCO-OCH_3$, $-NHCO-OC_2H_5$, $-NHCO-OC_3H_7$, -NHCO-O-cyclo-$C_3H_5$, $-NHCO-OC_4H_9$, $-NHCO-OCH(CH_3)_2$, $-NHCO-OC(CH_3)_3$, $-NHCO-OCH_2Ph$, $-NR^{23}R^{24}$, $-CF_3$, $-SOCH_3$, $-SOC_2H_5$, $-SOC_3H_7$, -SO-cyclo-$C_3H_5$, $-SOCH(CH_3)_2$, - $SOC(CH_3)_3$, $-SO_2CH_3$, $-SO_2C_2H_5$, $-SO_2C_3H_7$, $-SO_2$-cyclo-$C_3H_5$, $-SO_2CH(CH_3)_2$, $-SO_2C_4H_9$, $-SO_2C(CH_3)_3$, $-SO_3H$, $-SO_2NR^{23'}R^{24'}$, $-OCF_3$, $-OC_2F_5$, $-NH-CO-NH_2$, $-NH-CO-NHCH_3$, $-NH-CO-NHC_2H_5$, $-NH-CO-NHC_3H_7$, $-NH-CO-NHC_4H_9$, -NH-CO-NH-cyclo-$C_3H_5$, $-NH-CO-NH[CH(CH_3)_2]$, $-NH-CO-NH[C(CH_3)_3]$, $-NH-CO-N(CH_3)_2$, $-NH-CO-N(C_2H_5)_2$, $-NH-CO-N(C_3H_7)_2$, $-O-CO-NH_2$, $-O-CO-NHCH_3$, $-O-CO-NHC_2H_5$, $-O-CO-NHC_3H_7$, $-O-CO-NHC_4H_9$, -O-CO-NH-cyclo-$C_3H_5$, $-O-CO-NH[CH(CH_3)_2]$, -O-CO-NH

$[C(CH_3)_3]$, $-O-CO-N(CH_3)_2$, $-O-CO-N(C_2H_5)_2$, $-O-CO-N(C_3H_7)_2$, $-O-CO-N(C_4H_9)_2$, $-O-CO-N(cyclo-C_3H_5)_2$, $-O-CO-N[CH(CH_3)_2]_2$, $-O-CO-N[C(CH_3)_3]_2$, 2-thienyl, 3-thienyl, 2-furanyl, 3-furanyl, 2-oxazolyl, 3-oxazolyl, 4-oxazolyl, 2-thiazolyl, 3-thiazolyl, 4-thiazolyl, 1-pyrazolyl, 3-pyrazolyl, 4-pyrazolyl, 5-pyrazolyl, 1-imidazolyl, 2-imidazolyl, 4-imidazolyl, 5-imidazolyl, phenyl, 1-naphthyl, 2-naphthyl, 2-pyridyl, 3-pyridyl, 4-pyridyl, 2-pyrimidinyl, 4-pyrimidinyl, 5-pyrimidinyl, 2-pyrazinyl, 3-pyridazinyl, 4-pyridazinyl, 1,3,5-triazin-2-yl,

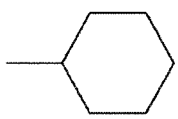 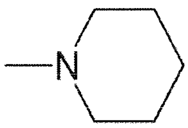 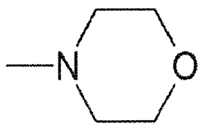 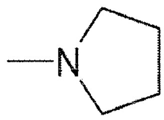 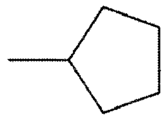

with the proviso that $R^{83}$ is not -H, if the group $-B-Y-R^{84}-R^{85}$ is hydrogen.

Also preferred are compounds of general formula (I), wherein $\mathbf{R^{98}}$ is $-NO_2$, -CN, -F, -Cl, -Br, -I, $NH_2$, -OH, $-CF_3$, $-CH_3$, $-C_2H_5$, $-C_3H_7$, $-CH(CH_3)_2$, $-C_4H_9$, $-C(CH_3)_3$, $-CH_2-NH_2$, $-CH_2-NH(CH_3)$, $-CH_2-N(CH_3)_2$, $-CH_2-NH(C_2H_5)$, $-CH_2-N(C_2H_5)_2$, $-CH_2-CH_2-NH_2$, $-CH_2-CH_2-NH(CH_3)$, $-CH_2-CH_2-N(CH_3)_2$, $-CH_2-CH_2-NH(C_2H_5)$, $-CH_2-CH_2-N(C_2H_5)_2$, $-CH_2-CH_2-CH_2-NH_2$, $-CH_2-CH_2-CH_2-NH(CH_3)$, $-CH_2-CH_2-CH_2-N(CH_3)_2$, $-CH_2-CH_2-CH_2-NH(C_2H_5)$, $-CH_2-CH_2-CH_2-N(C_2H_5)_2$, $-O-CH_3$, $-O-CH_2-CH_3$, $-O-CH_2-CH_2-CH_3$, $-CH_2O-CH_3$, $-CH_2O-CH_2-CH_3$, $-CH_2O-CH_2-CH_2-CH_3$, $-CH_2F$, $-CH_2Cl$, $-CH_2Br$, $-CH_2-CH_2F$, $-CH_2-CH_2Cl$, $-CH_2-CH_2Br$, $-CH_2-CH_2-CH_2F$, $-CH_2-CH_2-CH_2Cl$, $-CH_2-CH_2-CH_2Br$, $-OCH_2Ph$, $-OCH_2-CH_2-Ph$, $-CH_2-O-CH_2-Ph$.

Moreover especially preferred are compounds of the general formula (I), wherein

$\mathbf{R^1}$ is

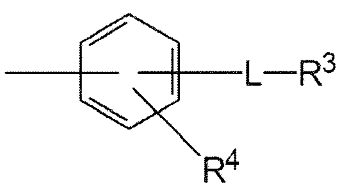 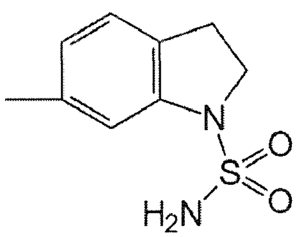 or 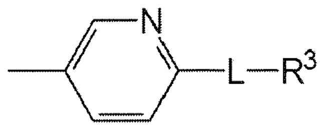

**L** is a bond, $-CH_2-$, or $-CH_2CH_2-$;

$\mathbf{R^3}$ is -H, $-SO_2NR^{23}R^{24}$, $-CONR^{23}R^{24}$, $-NO_2$, $-NH_2$, $-NHSO_2R^{22}$, $-NHCOR^{22}$, $-SO_2R^{22}$, -NH-CO-NH-Ph, or -Ph,

$\mathbf{R^4}$ is -H, $-CH_2-SO_2NR^{23}R^{24}$, $-SO_2NR^{23}R^{24}$, $-CONH_2$, $-C_2H_4-SO_2NR^{23}R^{24}$, $-NH-SO_2-CH_3$, $-NH-SO_2-C_2H_5$, $-NH-SO_2-C_3H_7$, $-NHCO-CH_3$, $-NHCO-C_2H_5$, $-NO_2$, $-NH_2$, $-SO_2CH_3$, or

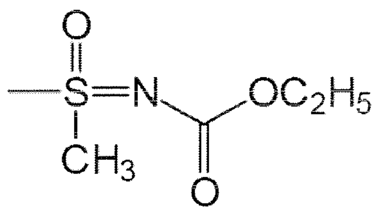

$\mathbf{R^{23}}$ and $\mathbf{R^{24}}$ are independently selected from -H, $-CH_3$, $-C_2H_5$, $-C_3H_7$, $-(cyclo-C_3H_5)$, $-CH_2-CH_2-CH_2-CH_2-NH_2$, or $-CH_2-CH_2-CH_2-CH_2-NH-COOC(CH_3)_3$,

$\mathbf{R^2}$ represents

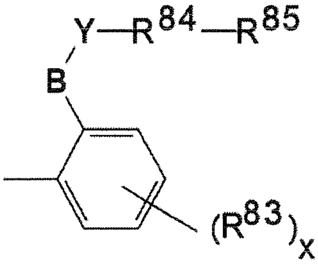 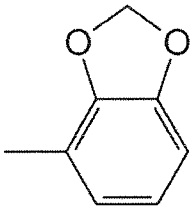 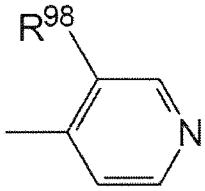

or

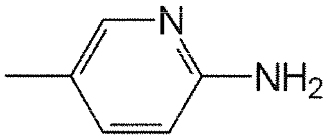

**B** is a bond or $-CH_2-$;

**Y** is a bond, -O-, or NH-;

**R^83** is selected from -H, -CN, -F, -Cl, $-O-CR^{62}R^{63}R^{64}$, $-CF_3$, $-CH_2OR^{23'}$, $-CR^{23'}O$, $-CR^{62}R^{63}-NR^{23'}R^{24'}$, $-CR^{62}R^{63}R^{64}$;

**R^23'** and **R^24'** represent independently of each other -H, $-CH_3$, $-(cyclo-C_3H_5)$;

**R^62-R^64** represent independently of each other -H, $-CH_3$, -Ph, -F, $-(cyclo-C_3H_5)$;

**R^84** is selected from a bond, $-CH_2-$, or $-CH_2-CH_2-CH_2-CH_2-$;

**R^85** is selected from -H, $-CF_3$, $-OCH_3$, $-OCH(CH_3)_2$, -CN, $-NHCOCH_3$, $-OCH_2-(cyclo-C_3H_5)$, $-NR^{23}R^{24}$, -Ph, -OPh, $-NHCO-OC(CH_3)_3$,

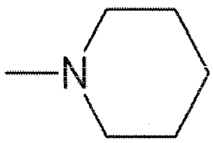 , 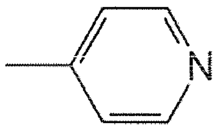 , 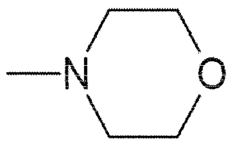 ;

**R^98** represents $-OCH_3$;

and salts, solvates or salts of solvates of the afore-mentioned compounds and especially the hydrochloride salt or the trifluoroacetate salt of these compounds.

Moreover especially preferred are compounds of the general formula (I), wherein

**R^1** is

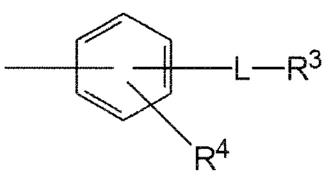 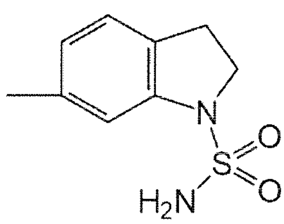 or 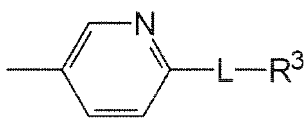

**L** is a bond, $-CH_2-$, or $-CH_2CH_2-$;

**$R^3$** is -H, $-SO_2NH_2$, $-CONH_2$, $-NO_2$, $-NH_2$, $-NH-SO_2-CH_3$, $-NH-SO_2-C_3H_7$, $-NHCO-CH_3$, $-SO_2CH_3$, -Ph, $-SO_2-NH-CH_2-CH_2-CH_2-CH_2-NH-COOC(CH_3)_3$, NH-CO-NH-Ph, or $-SO_2-NH-CH_2-CH_2-CH_2-CH_2-NH_2$,

**$R^4$** is -H, $-CH_2-SO_2NH_2$, $-SO_2NH_2$, $-C_2H_4-SO_2NH_2$, $-CONH_2$, $-NH-SO_2-CH_3$, $-NH-SO_2-C_3H_7$, $-NHCO-CH_3$, $-NO_2$, $-NH_2$, $-SO_2CH_3$, or

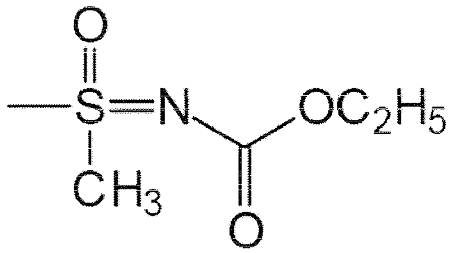

**$R^2$** represents

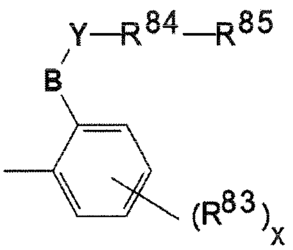
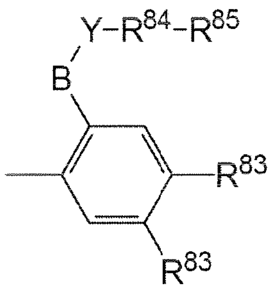
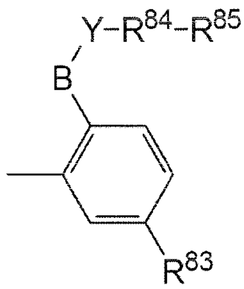
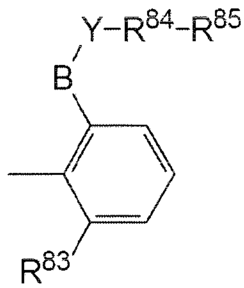

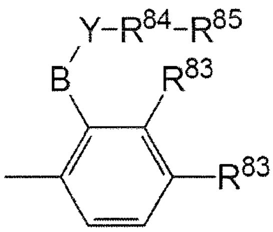
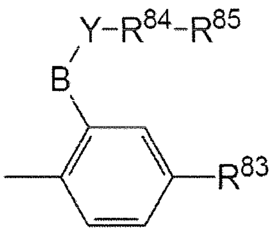
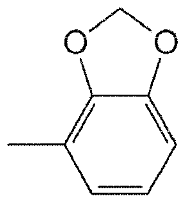
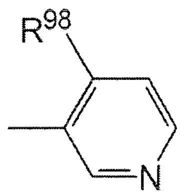

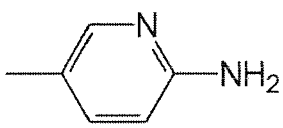
or
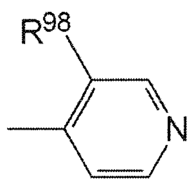

**B** is a bond or $-CH_2-$;

**Y** is a bond, -O-, or -NH-;

**$R^{83}$** is selected from -H, -F, -Cl, $-O-CH_3$, $-O-C_2H_5$, $-OCH_2-(cyclo-C_3H_5)$, -CN, $-CF_3$, $-CH_2OH$, -CHO, $-CH_2-NH(cyclo-C_3H_5)$, $-CH_2-NH(CH_3)$, $-CF_3$;

**$R^{84}$** is selected from a bond, $-CH_2-$, or $-CH_2-CH_2-CH_2-CH_2-$;

**$R^{85}$** is selected from -H, $-CF_3$, $-OCH_3$, $-OCH(CH_3)_2$, -CN, $NHCOCH_3$, $-OCH_2-(cyclo-C_3H_5)$, $NH_2$, $NH-(cyclo-C_3H_5)$,

Ph, -OPh, $-NHCO-OC(CH_3)_3$,

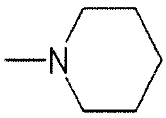, 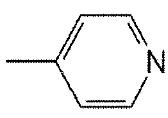, 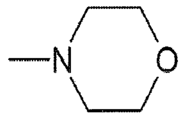;

**$R^{98}$** represents $-OCH_3$;
and salts, solvates or salts of solvates of the afore-mentioned compounds and especially the hydrochloride salt or the trifluoroacetate salt of these compounds.

In a particularly preferred embodiment the present invention concerns compounds of formula (I), wherein

**$R^1$** represents

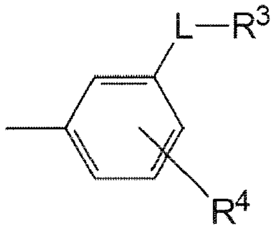

in which
the substituent $-L-R^3$ is $-SO_2NH_2$ or $-CH_2SO_2NH_2$,

$R^4$ is -H;

$R^2$ represents 2-methoxyphenyl, 4-fluoro-2-methoxyphenyl or 2-benzyloxyphenyl,

or their salts, solvates or salts of solvates and especially the hydrochloride salt or the trifluoroacetate salt.

In another particularly preferred embodiment the present invention concerns compounds of formula (I) selected from 3-[(4-(2-Methoxyphenyl)-1,3,5-triazin-2-yl)amino]benzenemethanesulfonamide (B1), 3-[(4-(2-Methoxyphenyl)-1,3,5-triazin-2-yl)amino]benzenesulfonamide (C1), 3-[(4-(4-Fluoro-2-methoxyphenyl)-1,3,5-triazin-2-yl)amino]benzenemethanesulfonamide (B2), 1-[(4-(2-Benzyloxyphenyl)-1,3,5-triazin-2-yl)amino]benzenemethanesulfonamide (B13), or their salts, solvates or salts of solvates and especially the hydrochloride salt or the trifluoroacetate salt.

In another particularly preferred embodiment the present invention concerns 3-[(4-(4-Fluoro-2-methoxyphenyl)-1,3,5-triazin-2-yl)amino]benzenemethanesulfonamide, or its salts, solvates or salts of solvates and especially the hydrochloride salt or the trifluoroacetate salt.

In another particularly preferred embodiment the present invention concerns 1-(3-{[4-(4-fluoro-2-methoxyphenyl)-1,3,5-triazin-2-yl]amino}phenyl)methanesulfonamide hydro-chloride.

Excluded are these compounds from the present invention, wherein $-R^4$ and $-L-R^3$ are methoxy or ethoxy groups.

Excluded from the present invention are also compounds, wherein $R^1$ is

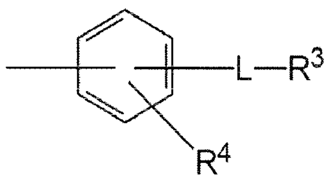

and wherein **$R^2$** is

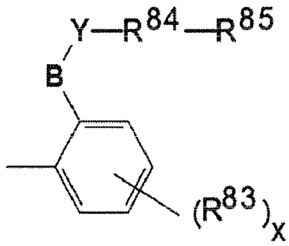

and wherein one of $-R^4$ and $-L-R^3$ is a chloro substituent and wherein one of $B-Y-R^{84}-R^{85}$ and $-R^{83}$ is also a chloro substituent. More general, compounds of general formula (I) with two or more chloro substituents are not preferred and might be excluded.

If the group $B-Y-R^{84}-R^{85}$ represents the substituent -NH-CO-Ph, the phenyl moiety $R^1$ has at least one substituent which is not in para position to the bond between the phenyl moiety $R^1$ and the triazine ring or the substituent $-L-R^3$, wherein L is a bond is different from the substituent $-CO-NH_2$. In addition the following compound is excluded from the scope of the present invention by disclaimer:

4-[4-(2-benzoylaminophenyl)-[1,3,5]triazin-2-ylamino]benzamide

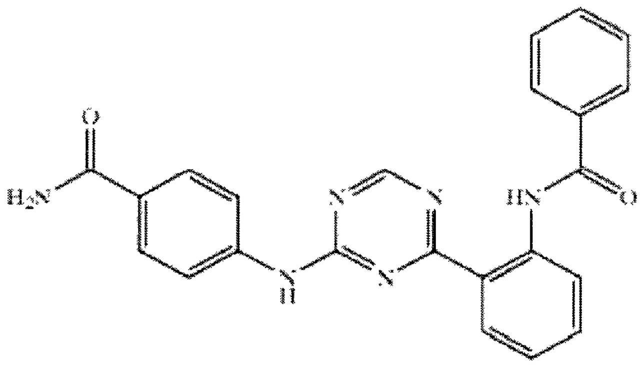

In a further aspect of the present invention, the novel compounds according to the general formula (I) represent chiral compounds. The novel compounds according to the general formula (I) represent a racemate, or a S or a R enantiomer or a mixture of isomers.

In yet another preferred embodiment of the present invention, the compound according to the general formula (I) is selected from the group of compounds depicted in the following Table 1.

**Table 1**

| Compound No. | Structure | Nomenclature |
|---|---|---|
| **B1** | 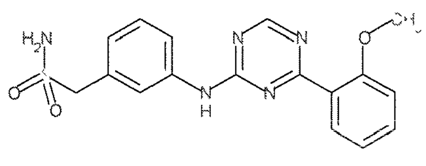 | 3-[(4-(2-Methoxyphenyl)-1,3,5-triazin-2-yl) amino]benzenemethanesulfonamide |
| **B2** | 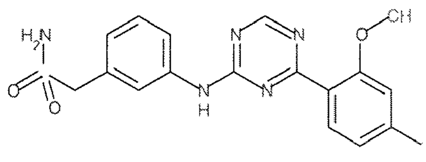 | 3-[(4-(4-Fluoro-2-methoxyphenyl)-1,3,5-triazin-2-yl)amino] benzenemethanesulfonamide |

(continued)

| Compound No. | Structure | Nomenclature |
|---|---|---|
| B3 | 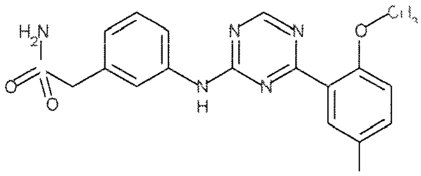 | 3-[(4-(5-Fluoro-2-methoxyphenyl)-1,3,5-triazin-2-yl)amino] benzenemethanesulfonamide |
| B4 | 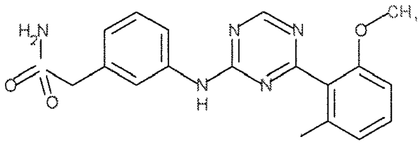 | 3-[(4-(6-Fluoro-2-methoxyphenyl)-1,3,5-triazin-2-yl)amino] benzenemethanesulfonamide |
| B5 | 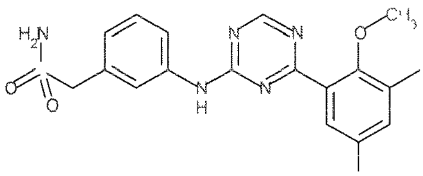 | 3-[(4-(3,5-Difluoro-2-methoxyphenyl)-1,3,5-triazin-2-yl)amino] benzenemethanesulfonamide |
| B6 | 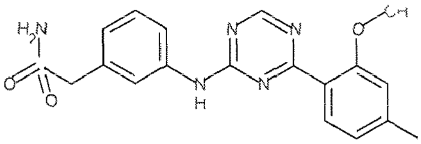 | 3-[(4-(4-Chloro-2-methoxyphenyl)-1,3,5-triazin-2-yl)amino] benzenemethanesulfonamide |
| B7 | 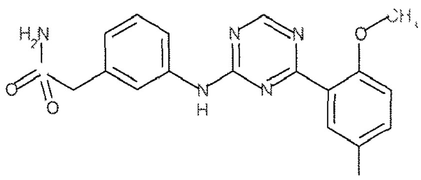 | 3-[(4-(5-Chloro-2-methoxyphenyl)-1,3,5-triazin-2-yl)amino] benzenemethanesulfonamide |
| B8 | 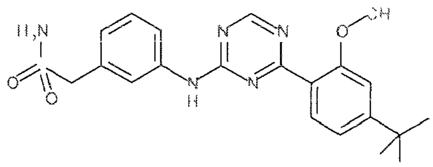 | 3-[(4-(2-Methoxy-4-trifluoromethyl-phenyl)-1,3,5-triazin-2-yl)amino] benzenemethanesulfonamide |
| B9 | 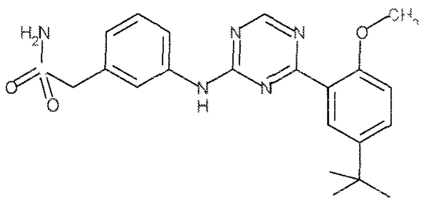 | 3-[(4-(2-Methoxy-5-trifluoromethyl-phenyl)-1,3,5-triazin-2-yl)amino] benzenemethanesulfonamide |

(continued)

| Compound No. | Structure | Nomenclature |
|---|---|---|
| **B10** | 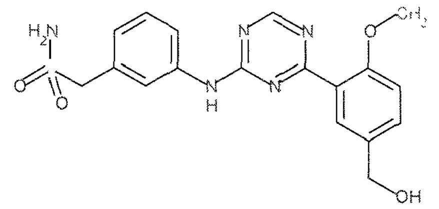 | 3-[(4-(5-Hydroxymethyl-2-methoxyphenyl)-1,3,5-triazin-2-yl)amino] benzenemethanesulfonamide |
| **B11** | 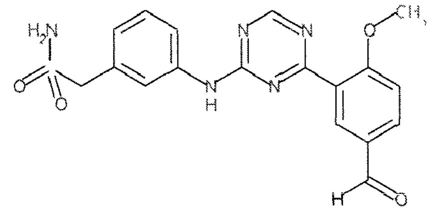 | 3-[(4-(5-Formyl-2-methoxyphenyl)-1,3,5-triazin-2-yl)amino] benzenemethanesulfonamide |
| **B12** | 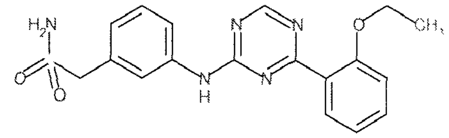 | 3-[(4-(2-Ethoxyphenyl)-1,3,5-triazin-2-yl)amino] benzenemethanesulfonamide |
| **B13** | 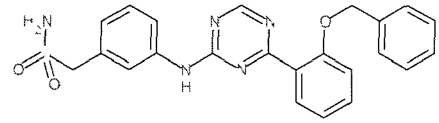 | 3-[(4-(2-Benzyloxyphenyl)-1,3,5-triazin-2-yl) amino] benzenemethanesulfonamide |
| **B14** | 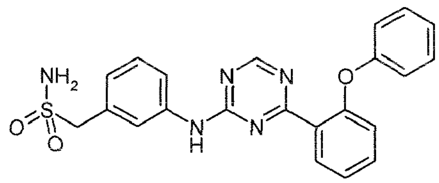 | 1-(3-{[4-(2-phenoxyphenyl)-1,3,5-triazin-2-yl] amino}phenyl)methanesulfonamide |
| **B15** | 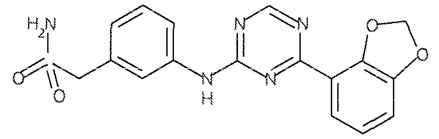 | 3-[(4-(1,3-Benzodioxol-4-yl)-1,3,5-triazin-2-yl) amino]benzenemethanesulfonamide |
| **B16** | 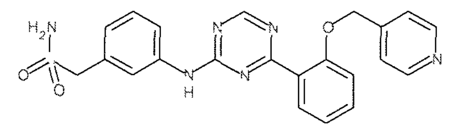 | 3-[(4-(2-((4-Pyridinyl)methoxy)phenyl)-1,3,5-triazin-2-yl)amino] benzenemethanesulfonamide |

(continued)

| Compound No. | Structure | Nomenclature |
|---|---|---|
| **B17** | 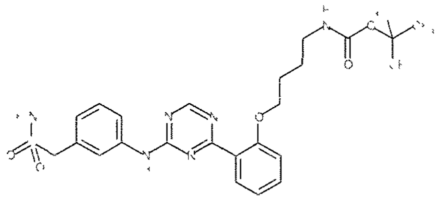 | 3-[(4-(2-(4-(tert-Butoxycarbonylamino)butoxy) phenyl)-1,3,5-triazin-2-yl)amino] benzenemethanesulfonamide |
| **B18** | 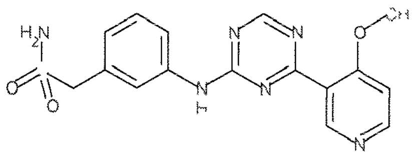 | 3-[(4-(4-Methoxypyridin-3-yl)-1,3,5-triazin-2-yl) amino]benzenemethanesulfonamide |
| **B19** | 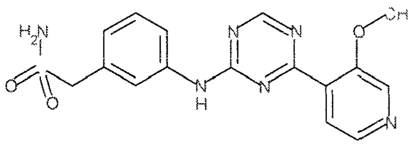 | 3-[(4-(3-Methoxypyridin-4-yl)-1,3,5-triazin-2-yl) amino]benzenemethanesulfonamide |
| **B20** | 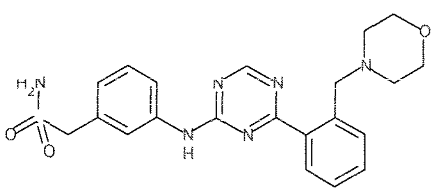 | 3-[(4-(2-((Morpholin-4-yl)methyl)phenyl)-1,3,5-triazin-2-yl)amino] benzenemethanesulfonamide |
| **B21** | 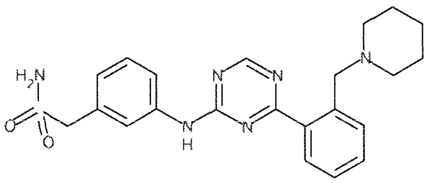 | 3-[(4-(2-((Piperidin-1-yl)methyl)phenyl)-1,3,5-triazin-2-yl)amino] benzenemethanesulfonamide |
| **B22** | 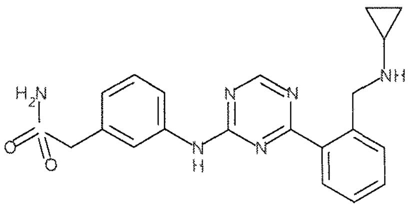 | 3-[(4-(2-(Cyclopropylamino-methyl)phenyl)-1,3,5-triazin-2-yl)amino] benzenemethanesulfonamide |
| **B23** | 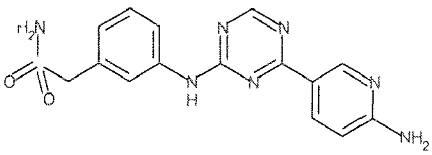 | 3-[(4-(6-Aminopyridin-3-yl)-1,3,5-triazin-2-yl) amino]benzenemethanesulfonamide |
| **B24** | 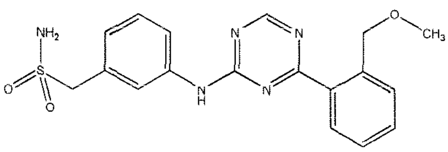 | 3-[(4-(2-(Methoxymethyl)phenyl)-1,3,5-triazin-2-yl)amino]benzenemethanesulfonamide |

(continued)

| Compound No. | Structure | Nomenclature |
|---|---|---|
| C1 | 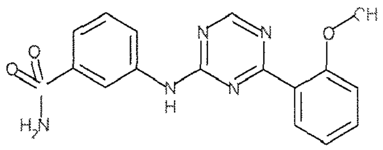 | 3-[(4-(2-Methoxyphenyl)-1,3,5-triazin-2-yl) amino]benzenesulfonamide |
| D1 | 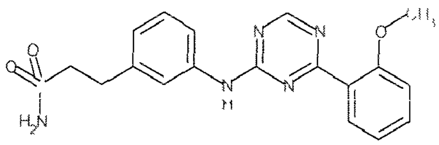 | 2-[3-((4-(2-Methoxyphenyl)-1,3,5-triazin-2-yl) amino)phenyl]ethanesulfonamide |
| D2 | 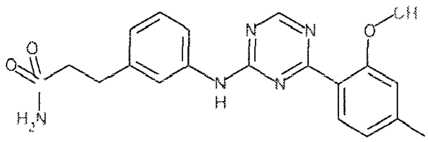 | 2-[3-((4-(4-Fluoro-2-methoxyphenyl)-1,3,5-triazin-2-yl)amino)phenyl]ethanesulfonamide |
| E1 | 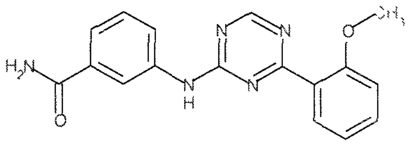 | 3-[(4-(2-Methoxyphenyl)-1,3,5-triazin-2-yl) amino]benzamide |
| F1 | 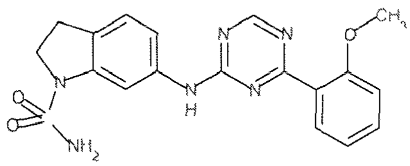 | 6-[(4-(2-Methoxyphenyl)-1,3,5-triazin-2-yl) amino]-2,3-dihydro-1H-indole-1-sulfonamide |
| G1 | 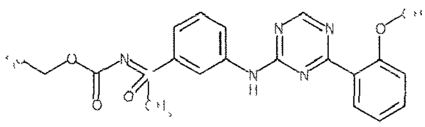 | rac-S-[3-((4-(2-Methoxyphenyl)-1,3,5-triazin-2-yl)amino)phenyl]-N-ethoxycarbonyl-S-methyl-sulfoximide |
| H1 | 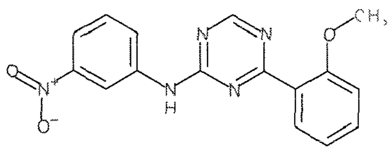 | 4-(2-Methoxyphenyl)-N-(3-nitrophenyl)-1,3,5-triazine-2-amine |
| I1 | 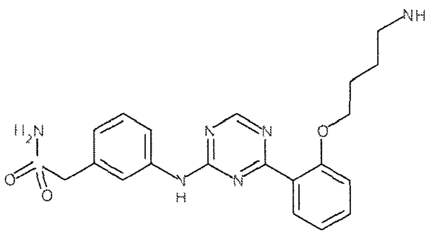 | 3-[(4-(2-(4-Aminobutoxy)phenyl)-1,3,5-triazin-2-yl)amino]benzenemethanesulfonamide |
| J1 | 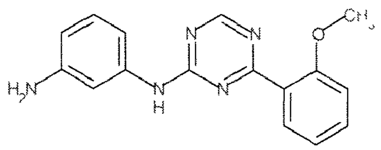 | N-(3-Aminophenyl)-4-(2-methoxyphenyl)-1,3,5-triazine-2-amine |

(continued)

| Compound No. | Structure | Nomenclature |
|---|---|---|
| K1 | 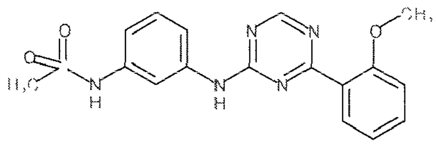 | N-[3-((4-(2-Methoxyphenyl)-1,3,5-triazin-2-yl) amino)phenyl]-methanesulfonamide |
| L1 | 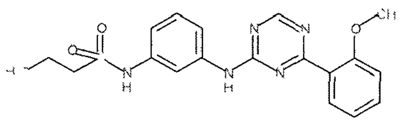 | N-[3-((4-(2-Methoxyphenyl)-1,3,5-triazin-2-yl) amino)phenyl]-propanesulfonamide |
| M1 | 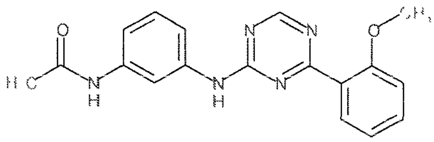 | N-[3-((4-(2-Methoxyphenyl)-1,3,5-triazin-2-yl) amino)phenyl]acetamide |
| N1 | 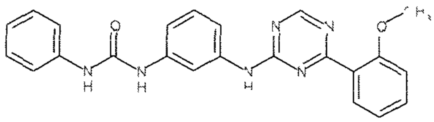 | N-[3-((4-(2-Methoxypheny)-1,3,5-triazin-2-yl) amino)phenyl]-N'-phenyl-urea |
| O1 | 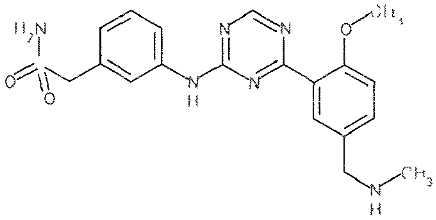 | 3-[(4-(2-Methoxy-5-(methylamino-methyl) phenyl)-1,3,5-triazin-2-yl)amino] benzenemethanesulfonamide |
| P1 | 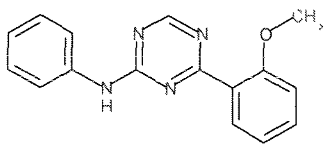 | 4-(2-Methoxyphenyl)-N-phenyl-1,3,5-triazine-2-amine |
| Q1 | 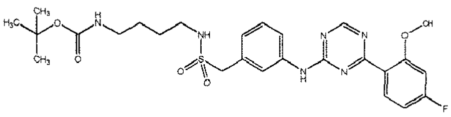 | tert-Butyl [4-((3-((4-(4-Fluoro-2-methoxyphenyl)-1,3,5-triazin-2-yl)amino) phenyl)phethylsulfonamido) butyl]carbamate |
| R1 | 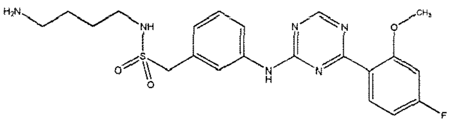 | N-(4-Aminobutyl)-1-[3-((4-(4-fluoro-2-methoxyphenyl)-1,3,5-triazin-2-yl)amino) phenyl]methanesulfonamide |
| S1 | 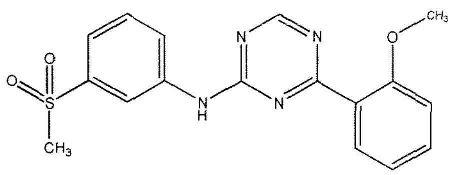 | 4-(2-Methoxyphenyl)-N-(3-(methylsulfonyl) phenyl)-1,3,5-triazin-2-amine |

(continued)

| Compound No. | Structure | Nomenclature |
|---|---|---|
| **T1** | 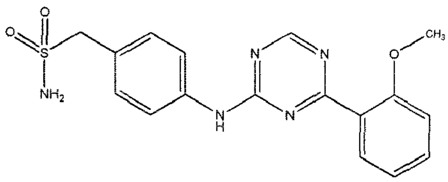 | 4-[(4-(2-Methoxyphenyl)-1,3,5-triazin-2-yl) amino]benzenemethane-sulfonamide |
| **U1** | 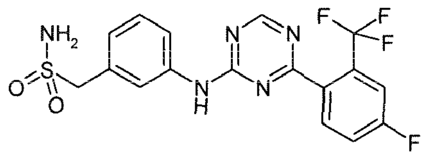 | 1-[3-({4-[4-fluoro-2-(trifluoromethyl)phenyl]-1,3,5-triazin-2-yl}amino)phenyl] methanesulfonamide |
| **U2** | 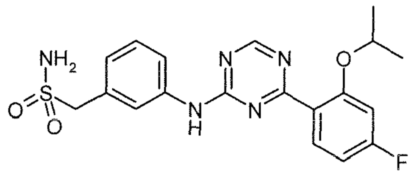 | 1-[3-({4-[4-fluoro-2-(propan-2-yloxy)phenyl]-1,3,5-triazin-2-yl}amino)phenyl] methanesulfonamide |
| **U3** | 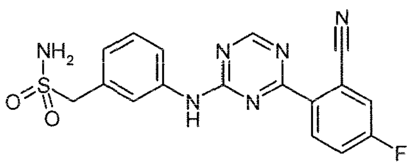 | 1-(3-{[4-(2-cyano-4-fluorophenyl)-1,3,5-triazin-2-yl]amino}phenyl)methanesulfonamide |
| **U4** | 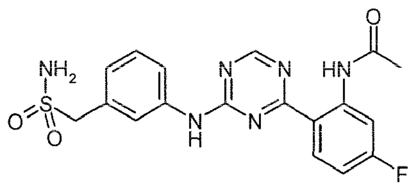 | N-[5-fluoro-2-(4-{[3-(sulfamoylmethyl)phenyl] amino}-1,3,5-triazin-2-yl)phenyl]acetamide |
| **U5** | 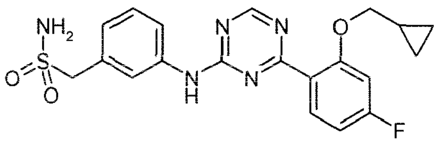 | 1-[3-({4-[2-(cyclopropylmethoxy)-4-fluorophenyl]-1,3,5-triazin-2-yl}amino)phenyl] methanesulfonamide |
| **U6** | 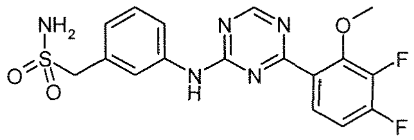 | 1-(3-{[4-(3,4-difluoro-2-methoxyphenyl)-1,3,5-triazin-2-yl]amino}phenyl)methanesulfonamide |
| **U7** | 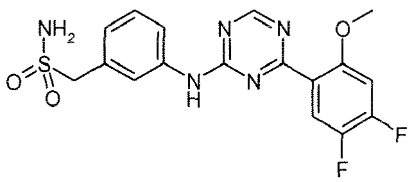 | 1-(3-{[4-(4,5-difluoro-2-methoxyphenyl)-1,3,5-triazin-2-yl]amino}phenyl)methanesulfonamide |

(continued)

| Compound No. | Structure | Nomenclature |
|---|---|---|
| **U8** | 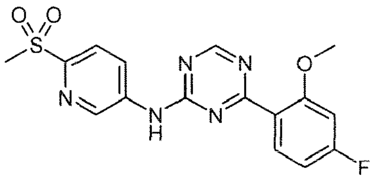 | 4-(4-fluoro-2-methoxyphenyl)-N-[6-(methylsulfonyl)pyridin-3-yl]-1,3,5-triazin-2-amine |
| **B1'** | 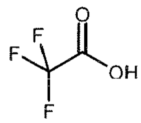 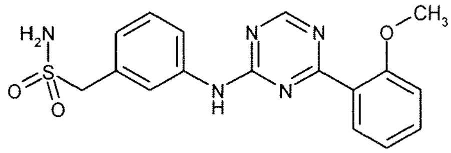 | 3-[(4-(2-Methoxyphenyl)-1,3,5-triazin-2-yl) amino]benzenemethanesulfonamide trifluoroacetic acid salt |
| **B2'** | HCl 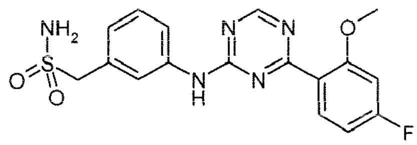 | 1-(3-{[4-(4-fluoro-2-methoxyphenyl)-1,3,5-triazin-2-yl]amino}phenyl)methanesulfonamide hydrochloride |
| **B13'** | 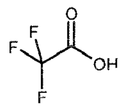 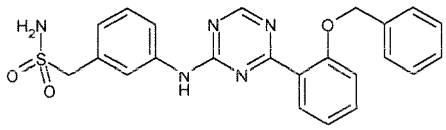 | 3-[(4-(2-Benzyloxyphenyl)-1,3,5-triazin-2-yl) amino] benzenemethanesulfon-amide trifluoroacetic acid salt |
| **C1'** | 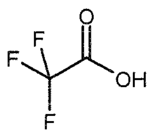 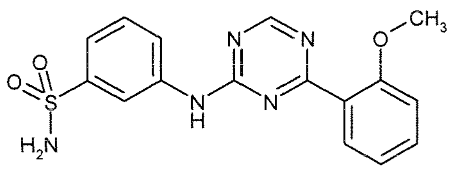 | 3-[(4-(2-Methoxyphenyl)-1,3,5-triazin-2-yl) amino]benzenesulfonamide trifluoroacetic acid salt |
| **B2''** | $CF_3COOH$ 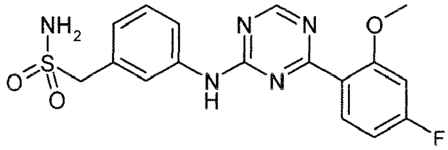 | 1-(3-{[4-(4-fluoro-2-methoxyphenyl)-1,3,5-triazin-2-yl]amino}phenyl)methanesulfonamide trifluoroacetic acid salt |

It is particularly preferred to use the following selective CDK9 inhibitors shown in Table 2 in accordance with the present invention:

**Table 2.**

| Compound No. | Structure | Nomenclature |
|---|---|---|
| **B1** | 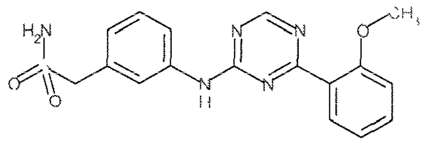 | 3-[(4-(2-Methoxyphenyl)-1,3,5-triazin-2-yl) amino]benzenemethanesulfonamide |
| **B2** | 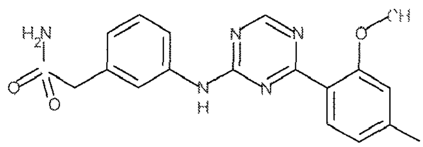 | 3-[(4-(4-Fluoro-2-methoxyphenyl)-1,3,5-triazin-2-yl)amino] benzenemethanesulfonamide |
| **B3** | 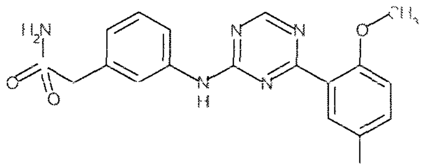 | 3-[(4-(5-Fluoro-2-methoxyphenyl)-1,3,5-triazin-2-yl)amino] benzenemethanesulfonamide |
| **B4** | 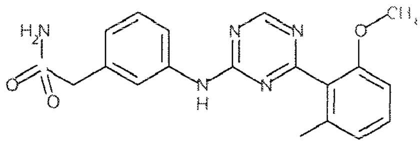 | 3-[(4-(6-Fluoro-2-methoxyphenyl)-1,3,5-triazin-2-yl)amino] benzenemethanesulfonamide |
| **B6** | 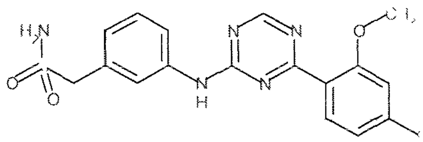 | 3-[(4-(4-Chloro-2-methoxyphenyl)-1,3,5-triazin-2-yl)amino] benzenemethanesulfonamide |
| **B7** | 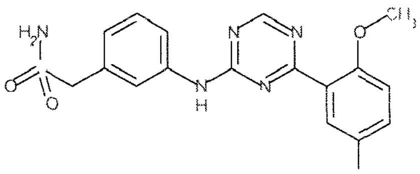 | 3-[(4-(5-Chloro-2-methoxyphenyl)-1,3,5-triazin-2-yl)amino] benzenemethanesulfonamide |
| **B12** | 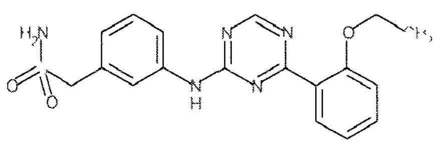 | 3-[(4-(2-Ethoxyphenyl)-1,3,5-triazin-2-yl)amino] benzenemethanesulfonamide |
| **B13** | 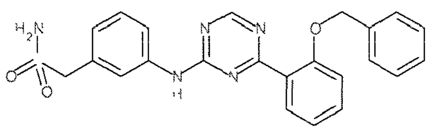 | 3-[(4-(2-Benzyloxyphenyl)-1,3,5-triazin-2-yl) amino] benzenemethanesulfonamide |

(continued)

| Compound No. | Structure | Nomenclature |
|---|---|---|
| **B14** | 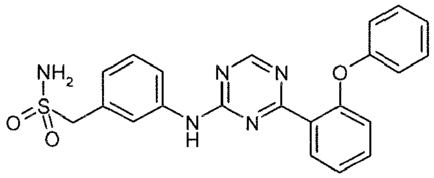 | 1-(3-{[4-(2-phenoxyphenyl)-1,3,5-triazin-2-yl] amino}phenyl)methanesulfonamide |
| **B16** | 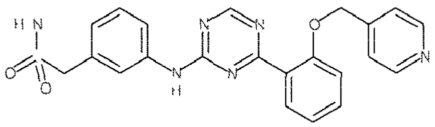 | 3-[(4-(2-((4-Pyridinyl)methoxy)phenyl)-1,3,5-triazin-2-yl)amino] benzenemethanesulfonamide |
| **B17** | 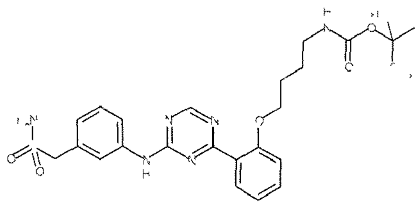 | 3-[(4-(2-(4-(tert-Butoxycarbonylamino)butoxy) phenyl)-1,3,5-triazin-2-yl)amino] benzenemethanesulfonamide |
| **B18** | 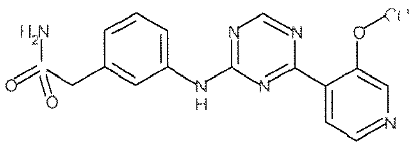 | 3-[(4-(3-Methoxypyridin-4-yl)-1,3,5-triazin-2-yl) amino]benzenemethanesulfonamide |
| **B23** | 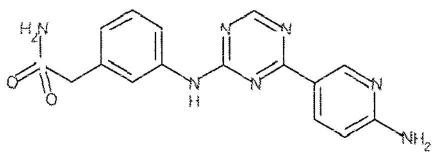 | 3-[(4-(6-Aminopyridin-3-yl)-1,3,5-triazin-2-yl) amino]benzenemethanesulfonamide |
| **B24** | 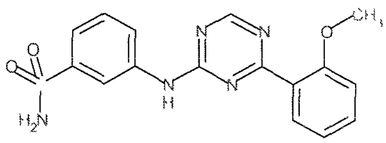 | 3-[(4-(2-Methoxyphenyl)-1,3,5-triazin-2-yl) amino]benzenesulfonamide |
| **C1** | 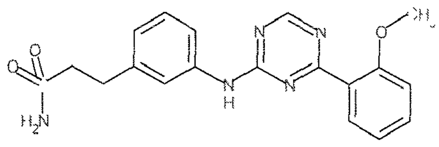 | 2-[3-((4-(2-Methoxyphenyl)-1,3,5-triazin-2-yl) amino)phenyl]ethanesulfonamide |
| **D1** | 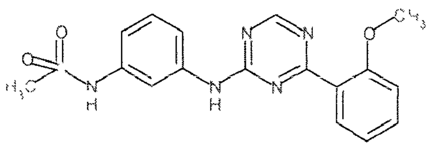 | N-[3-((4-(2-Methoxyphenyl)-1,3,5-triazin-2-yl) amino)phenyl]-methanesulfonamide |

(continued)

| Compound No. | Structure | Nomenclature |
|---|---|---|
| L1 | 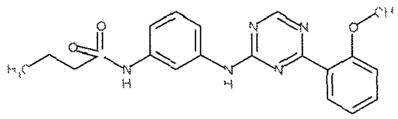 | N-[3-((4-(2-Methoxyphenyl)-1,3,5-triazin-2-yl) amino)phenyl]-propanesulfonamide |
| Q1 | 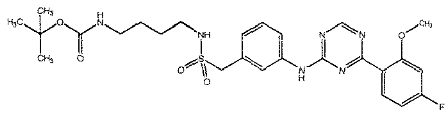 | tert-Butyl [4-((3-((4-(4-Fluoro-2-methoxyphenyl)-1,3,5-triazin-2-yl)amino) phenyl)methylsulfonamido) butyl]carbamate |
| R1 | 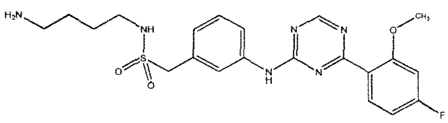 | N-(4-Aminobutyl)-1-[3-((4-(4-fluoro-2-methoxyphenyl)-1,3,5-triazin-2-yl)amino) phenyl]methanesulfonamide |
| S1 | 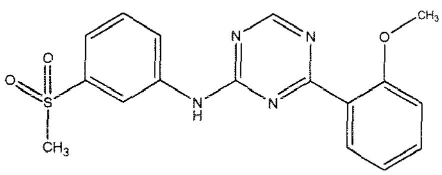 | 4-(2-Methoxyphenyl)-N-(3-(methylsulfonyl) phenyl)-1,3,5-triazin-2-amine |
| U2 | 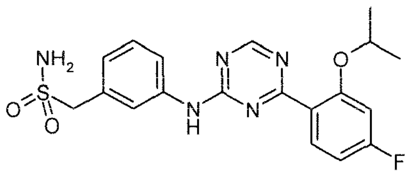 | 1-[3-({4-[4-Fluoro-2-(propan-2-yloxy)phenyl]-1,3,5-triazin-2-yl}amino)phenyl] methanesulfonamide |
| U5 | 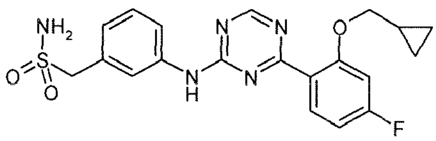 | 1-[3-({4-[2-(Cyclopropylmethoxy)-4-fluorophenyl]-1,3,5-triazin-2-yl}amino)phenyl] methanesulfonamide |
| U7 | 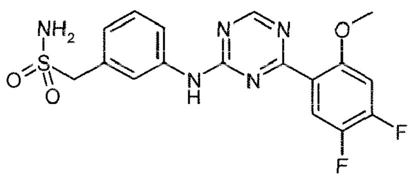 | 1-(3-{[4-(4.5-Difluoro-2-methoxyphenyl)-1,3,5-triazin-2-yl]amino}phenyl)methanesulfonamide |
| 24 | 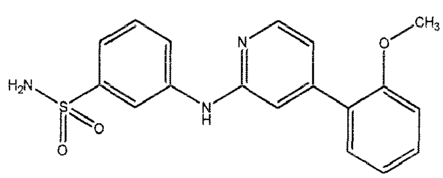 | 3-[(4-(2-Methoxyphenyl)pyridin-2-yl)amino] benzenesulfonamide |
| 25 | 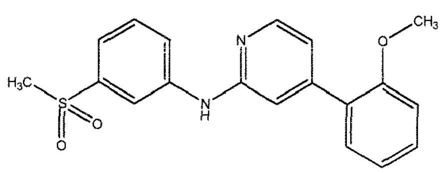 | 4-(2-Methoxyphenyl)-N-(3-(methylsulfonyl) phenyl)pyridin-2-amine |

(continued)

| Compound No. | Structure | Nomenclature |
|---|---|---|
| **26** | 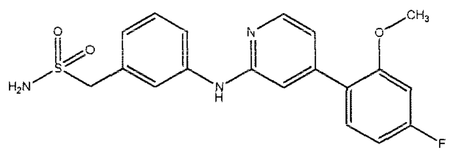 | [3-((4-(4-Fluoro-2-methoxyphenyl)pyridin-2-yl) amino)phenyl]methanesulfonamide |
| **27** | 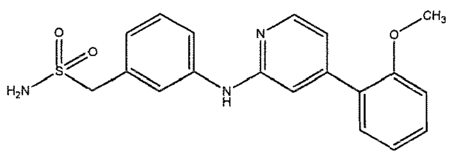 | [3-((4-(2-Methoxyphenyl)pyridin-2-yl)amino) phenyl]methanesultonamide |
| **28** | 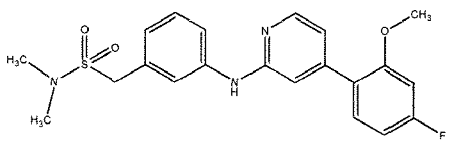 | 1-[3-((4-(4-Fluoro-2-methoxyphenyl)pyridin-2-yl)amino)phenyl]-N,N-dimethylmethanesulfonamide |
| **29** | 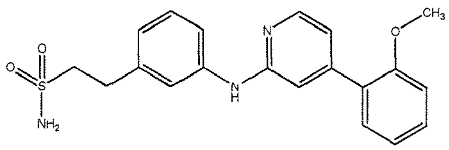 | 2-[3-((4-(2-Methoxyphenyl)pyridin-2-yl)amino) phenyl]ethanesulfonamide |
| **30** | 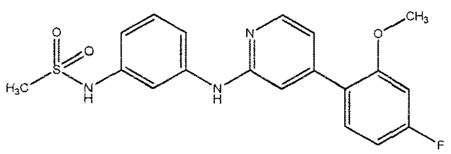 | N-[3-((4-(4-Fluoro-2-methoxyphenyl)pyridin-2-yl)amino)phenyl]methanesulfonamide |
| **31** | 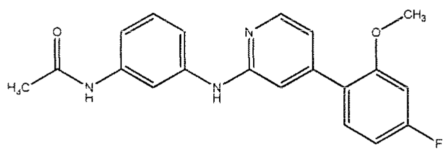 | N-[3-((4-(4-Fluoro-2methoxyphenyl)pyridin-2-yl)amino)phenyl]acetamide |
| **32** | 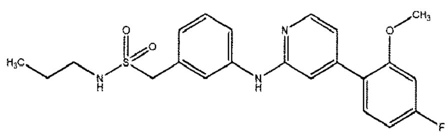 | 1-[3-((4-(4-Fluoro-2-methoxyphenyl)pyridin-2-yl)amino)phenyl]-N-propylmethanesulfonamide |
| **33** | 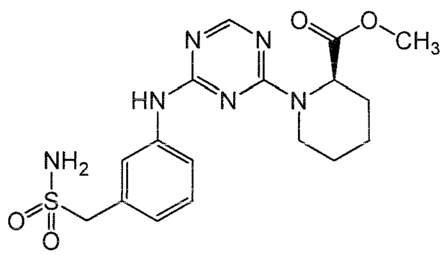 | (R)-Methyl 1-[4-((3-(Sulfamoylmethyl)phenyl) amino)-1,3,5-triazin-2-yl]piperidine-2-carboxylate |
| **34** | 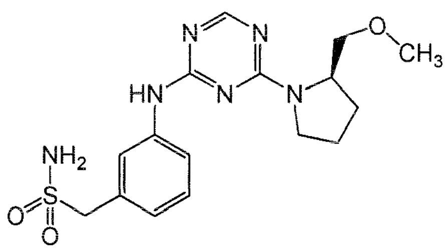 | (R)-3-[(4-(2-(Methoxymethyl)pyrrolidin-1-yl)-1,3,5-triazin-2-yl)amino] benzenemethanesulfonamide |

(continued)

| Compound No. | Structure | Nomenclature |
|---|---|---|
| **35** | 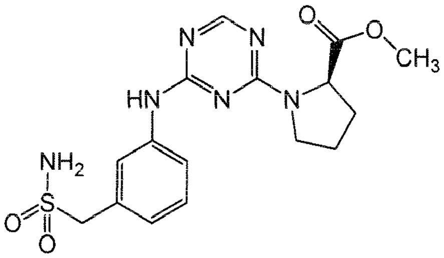 | (R)-Methyl 1-[4-((3-(Sulfamoylmethyl)phenyl) amino)-1,3,5-triazin-2-yl]pyrrolidine-2-carboxylate |
| **36** | 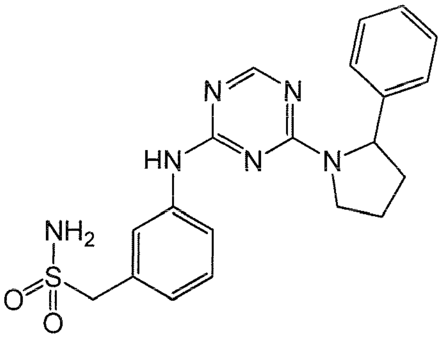 | rac-3-[(4-(2-Phenylpyrrolidin-1-yl)-1,3,5-triazin-2-yl)amino]benzenemethanesulfonamide |
| **37** | 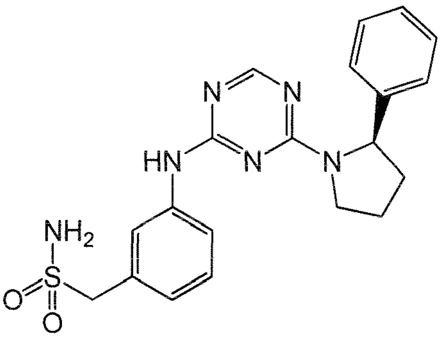 | (R)-3-[(4-(2-Phenylpyrrolidin-1-yl)-1,3,5-triazin-2-yl)amino]benzenemethanesulfonamide |
| **38** | 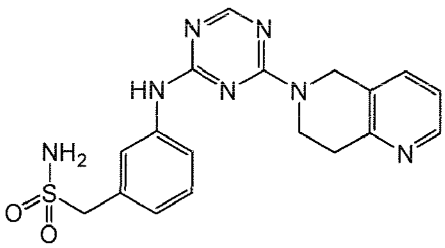 | 3-[(4-(7,8-Dihydro-1,6-naphthyridin-6(5H)-yl)-1,3,5-triazin-2-yl)amino] benzenemethanesulfonamide |
| **39** | 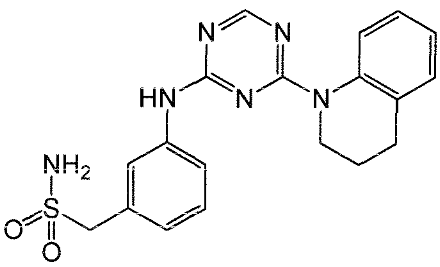 | 3-[(4-(3,4-Dihydroquinolin-1(2H)-yl)-1,3,5-triazin-2-yl)amino] benzenemethanesulfonamide |

(continued)

| Compound No. | Structure | Nomenclature |
|---|---|---|
| **40** | 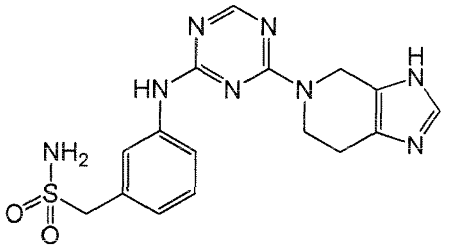 | 3-[(4-(6,7-Dihydro-3H-imidazo[4,5-c]pyridin-5 (4H)-yl)-1,3,5-triazin-2-yl)amino] benzenemethanesulfonamide |
| **41** | 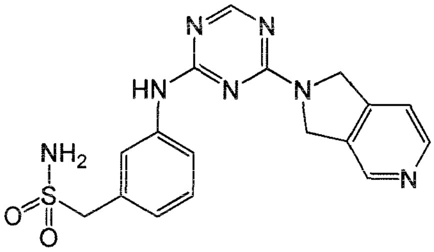 | 3-[(4-(1H-Pyrrolo[3,4-c]pyridin-2(3H)-yl)-1,3,5-triazin-2-yl)amino] benzenemethanesulfonamide |
| **42** | 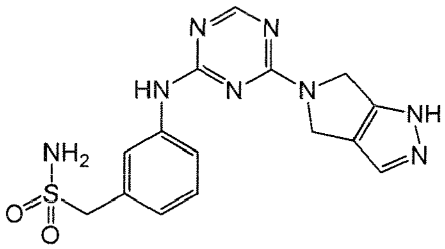 | 3-[(4-(Pyrrolo[3,4-c]pyrazol-5(1H,4H,6H)-yl)-1,3,5-triazin-2yl)amino] benzenemethanesulfonamide |
| **43** | 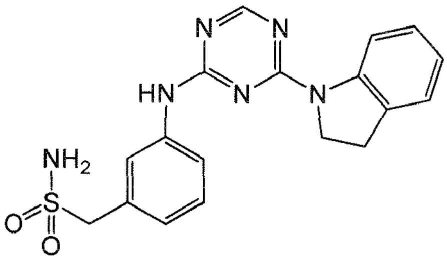 | 3-[(4-(Indolin-1-yl)-1,3,5-triazin-2-yl)amino] benzenemethanesulfonamide |
| **44** | 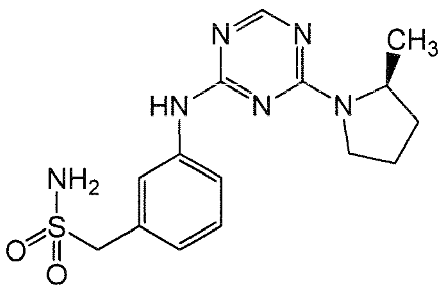 | (S)-3-[(4-(2-Methylpyrrolidin-1-yl)-1,3,5-triazin-2-yl)amino]benzenemethanesulfonamide |

The above and further compounds which can be used in accordance with the present invention are also disclosed in POT/EP2011/001445, EP10075131.2 (filing date 22.03.2010) EP11075037.9 (filing date 02.03.2011) and EP11075038.7 (filing date 02.03.2011) which are incorporated herein by reference in their entirety.

The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic

acid, ethylenesulfonic acid, p-toluenesulfonic acid, naphthylsulfonic acid, sulfanilic acid, camphorsulfonic acid, china acid, mandelic acid, o-methylmandelic acid, hydrogenbenzenesulfonic acid, picric acid, adipic acid, d-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form with a sufficient amount of the desired acid to produce a salt in the conventional manner. Preferred is the mesylate salt, hydrochloride salt and the trifluoroacetate salt and especially preferred is the trifluoroacetate salt and the hydrochloride salt.
In the case the inventive compounds bear acidic groups, salts could also be formed with inorganic or organic bases. Examples for suitable inorganic or organic bases are, for example, NaOH, KOH, $NH_4OH$, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

**Syntheses of compounds**

The synthesis of the inventive disubstituted triazines according to the present invention is preferably carried out according to the general synthetic sequences, shown in Schemes **1** to **3.**

Scheme 1

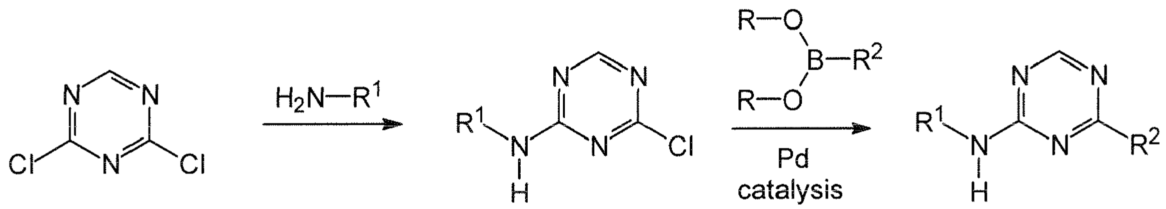


In a first step 2,4-Dichloro-1,3,5-triazine is reacted with anilines $R^1NH_2$ to give 2-arylamino-4-chloro-1,3,5-triazines. The reaction is carried out with one equivalent of the aniline in an inert solvent like DMF, THF, DME, dioxane or an alcohol like isopropanol, or mixtures of such solvents. Preferably the reaction is carried out at a temperature below room temperature in such a way that the reaction mixture is kept homogenous. Preferred conditions use an additional base like triethylamine or N,N-diisopropylethylamine.
In a second step the intermediate 2-arylamino-4-chloro-1,3,5-triazine is reacted with a boronic acid derivative $R^2$-$B(OR)_2$ to give compounds of Formula (I). The boronic acid derivative may be a boronic acid (R = -H) or an ester of the boronic acid, e.g. its isopropyl ester (R = $-CH(CH_3)_2$), preferably an ester derived from pinacol in which the boronic acid intermediate forms a 2-aryl-4,4,5,5-tetramethyl-1,3,2-dioxaborolane (R-R = $-C(CH_3)_2$-$C(CH_3)_2$-). Both R represent independently of each other preferably hydrogen or an alkyl chain with 1-10 carbon atoms or a cycloalkyl chain with 3 to 12 carbon atoms or both residues R represent together a residue derived from pinacol. The coupling reaction is catalyzed by Pd catalysts, e.g. by Pd(0) catalysts like tetrakis(triphenylphosphine)palladium(0) $[Pd(PPh_3)_4]$, tris(dibenzylideneacetone) di-palladium(0) $[Pd_2(dba)_3]$, or by Pd(II) catalysts like dichlorobis(triphenylphosphine)-palladium(II) $[Pd(PPh_3)_2Cl_2]$, palladium(II) acetate and triphenylphosphine or more preferred by [1,1'-bis(diphenylphosphino)ferrocene]palladium dichloride. The reaction is preferably carried out in a mixture of a solvent like dioxane, DMF, DME, THF, or isopropanol with water and in the presence of a base like aqueous sodium bicarbonate or $K_3PO_4$.

Scheme 2

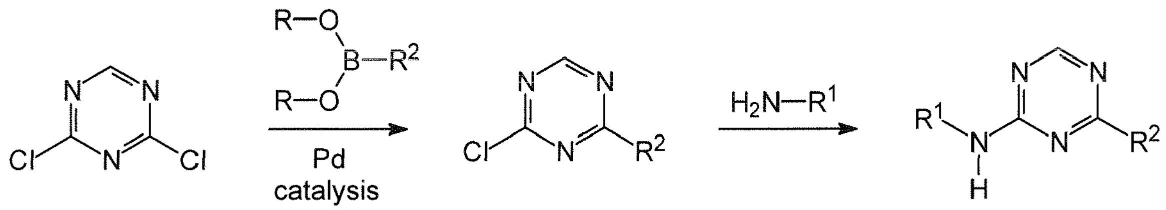


The synthesis of 1,3,5-triazines of Formula (I) starting from 2,4-dichloro-1,3,5-triazine may be carried out in the inverse order of the reaction steps compared to Scheme 1, in such a manner that in a first step the reaction of a triazine

with a boronic acid derivative is followed in a second step by the reaction of the intermediate triazine with an aniline. Preferred conditions for the coupling reaction of the first step are heating the reacting agents in toluene with dichlorobis(triphenylphosphine)palladium(II) [$Pd(PPh_3)_2Cl_2$] as a catalyst in the presence of sodium or potassium carbonate as a base.

Scheme 3

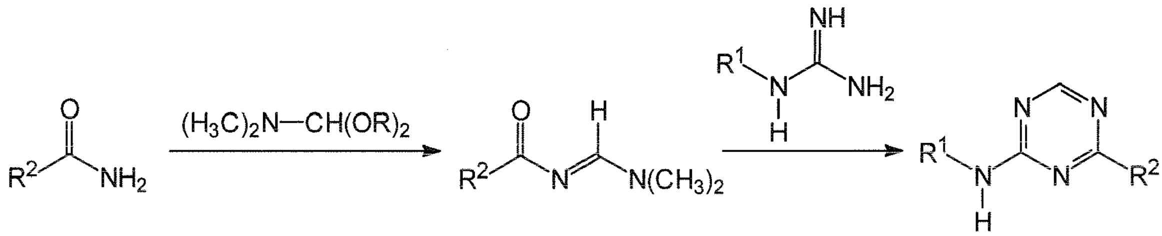


Compounds of Formula (I) may be prepared by the methodology described in J. Org. Chem. 60 (1995), 8428-8430. Primary amides $R^2$-$CONH_2$ are heated with acetals and preferably dialkylacetals of N,N-dimethylformamide, preferably with its dimethyl or diethyl acetal, in particular with the dimethyl acetal (R = $-CH_3$). The intermediate N-acylformamidine is not isolated and subsequently converted to 1,3,5-triazines of Formula (I) by heating with a guanidine $R^1$-NH-C(NH)$NH_2$. Preferably the reaction is carried out by heating the reacting agents in dioxane in the presence of a base like potassium tert-butoxide.

Several compounds of Formula (I) may be prepared by converting substituents which are attached to the aromatic rings $R^1$ and/or $R^2$ to other substituents using standard reactions which are known to the person skilled in the art. For example, a nitro group can be reduced to an amino group, such an amino group can be converted to a sulfonamide by reaction with a sulfonyl chloride, to a carboxamide by reaction with a carbonyl chloride or another activated derivative of a carboxylic acid, to an urea by reaction with an isocyanate. Carbamate substituents may be cleaved to amino groups, in particular tert-butyl carbamates by reaction with acids like trifluoroacetic acid or hydrochloric acid. Formyl groups may be converted to aminomethyl groups by reaction with primary amines under conditions of a reductive amination; see, for example, synthesis of the compounds as shown in Table 2.

Further selective CDK9 inhibitors to be used in accordance with the present invention are well known in the art and are, for example, described in Krystof (2009) Medicinal Research Reviews, DOI 10.1002/med.20172, as well as in international patent applications published as WO 2009/047359, WO 2010/003133, WO 2008/79933 and WO 2411/012661. All these documents are incorporated herein by reference in their entirety.

Potential selective CDK9 inhibitors as defined herein above may be screened/identified by routine assays, such as a radiometric protein kinase assay (33PanQinase® Activity Assay; and/or the well known Lance Assay.

The following exemplary inhibitors can be used in accordance with the present invention, for example, in cotherapy as described herein:

SNS-032: Piperidine-4-carboxylic acid [5-(5-tert-butyl-oxazol-2-ylmethylsulfanyl)-thiazol-2-yl]-amide; Misra RN et al. J Med Chem. 2004, 47(7):1719-28;

flavopiridol: 2-(2-Chloro-phenyl)-5,7-dihydroxy-8-(3-hydroxy-1-methyl-piperidin-4-yl)-chromen-4-one, Lloyd R Kelland. Expert Opinion on Investigational Drugs. 2000, 9(12):2903-2911;

AX35427: N-(5-((6-(3-aminophenyl)pyrimidin-4-yl)amino)-2-methylphenyl)propane-1-sulfonamide, 848637-29-6P in WO 2005026129;

R-547: [4-amino-2-(1-methanesulfonylpiperidin-4-ylamino)pyrimidin-5-yl]-(2,3-difluoro-6-methoxyphenyl)methanone, DePinto W et al., Mol Cancer Ther 2006, 5:2644-2658;

1073485-20-7P: 3-[[6-(2-methoxyphenyl)-4-pyrimidinyl]amino]-Benzenemethanesulfonamide compound 1073485-20-7P in WO 2008132138;

AX38679: 3-((6-(2-methoxyphenyl)pyrimidin-4-yl)amino)benzenesulfonamide, 848637-62-7P in WO 2005026129;

PHA767491: 1,5,6,7-tetrahydro-2-(4-pyridinyl)-4H-pyrrolo[3,2-c]pyridin-4-one, Montagnoli, A. Nat Chem Biol 2008,

4(6) 357-365;

BS181: N5-(6-aminohexyl)-3-(1-methylethyl)-N7-(phenylmethyl), Ali S et al. Cancer Res. 2009, 69(15):6208-15).

DRB:

5,6-dichloro-1-b-ribofuranosyl-benzimidazole; Mancebo HS, Lee G, Flygare J, Tomassini J, Luu P, Zhu Y, Peng J, Blau C, Hazuda D, Price D, Flores O. P-TEFb kinase is required for HIV Tat transcriptional activation in vivo and in vitro. Genes Dev 1997;11:2633-2644;

Roscovitine: 6-Benzylamino-2[(R)-(1'-ethyl-2'-hydroxyethylamino)]-9-isopropylpurine, Meijer, Laurent; Bisagni, Emile; Legraverend, Michel. Purine derivatives with antiproliferative properties, their preparation, and biological uses thereof. PCT Int. Appl. (1997), 52 pp. CODEN: PIXXD2 WO 9720842 A1;

AG-012986:

4-[[4-Amino-5-(2,6-difluorobenzoyl)thiazol-2-yl]amino]-N-((R)-2-dimethylamino-1-methylethyl)benzamide Zhang C, Lundgren K, Yan Z, Arango ME, Price S, Huber A, Higgins J, Troche G, Skaptason J, Koudriakova T, Nonomiya J, Yang M, O'Connor P, Bender S, Los G, Lewis C, Jessen B. Pharmacologic properties of AG-012986, a pan-cyclin-dependent kinase inhibitor with antitumor efficacy. Mol Cancer Ther 2008;7:818-828;

P276-00:

4H-1-Benzopyran-4-one, 2-(2-chlorophenyl)-5,7-dihydroxy-8-[(2R,3S)-2-(hydroxymethyl)-1-methyl-3-pyrrolidinyl]-, hydrochloride (1:1); Joshi, Kalpana S.; Rathos, Maggie J.; Joshi, Rajendra D.; Sivakumar, Meenakshi; Mascarenhas, Malcolm; Kamble, Shrikant; Lal, Bansi; Sharma, Somesh. In vitro antitumor properties of a novel cyclin-dependent kinase inhibitor, P276-00. Molecular Cancer Therapeutics (2007), 6(3), 918-925. CODEN: MCTOCF ISSN:1535-7163. CAN 146:513967 AN 2007:289479;

ZK 304709:

4-[3-Chloro-5-(4-methylpiperazin-1-yl)benzoylamino]-1H-pyrazole-3-carboxylic acid cyclohexylamide Siemeister, G.; Luecking, U.; Wagner, C.; Detjen, K.; Mc Coy, C.; Bosslet, Klaus. Molecular and pharmacodynamic characteristics of the novel multi-target tumor growth inhibitor ZK304709. Biomedicine & Pharmacotherapy (2006), 60(6), 269-272. CODEN: BIPHEX ISSN:0753-3322. CAN 146:176348 AN 2006:831518;

EXEL-8647 and/or EXEL-3700:

Heuer TS. Discovery of Selective CDK9 Small Molecule Inhibitors: CDK9 Inhibition in Tumor Cells is Associated with Inhibition of Proliferation and Induction of Apoptosis. AACR-NCIEORTC International Conference on Molecular Targets and Cancer Therapeutics. Geneva, Switzerland; 21-24 October 2008;

AT7519:

4-(2,6-Dichlorobenzoylamino)-1H-pyrazole-3-carboxylic acid N-(piperidin-4-yl)amide Squires MS, Feltell RE, Lock V, Smith D, Lewis EJ, Higgins J, Yule M, Thompson NT, Cooke L, Croce Della K, Qi W, Lyons JF, Mahadevan D. AT7519, a potent CDK inhibitor, is active in leukemia models and primary CLL patient samples. 49th Annual Meeting and Exposition of American Society for Hematology. Atlanta, GA, 8-11 December 2007;

Compound 7d:

N-[2-(dimethylamino)ethyl]-2-fluoro-4-[[5-fluoro-4-[2-methyl-1-(1-methylethyl)-1H-imidazol-5-yl]-2-pyrimidinyl] amino] ; Jones CD, Andrews DM, Barker AJ, Blades K, Daunt P, East S, Geh C, Graham MA, Johnson KM, Loddick SA, McFarland HM, McGregor A, Moss L, Rudge DA, Simpson PB, Swain ML, Tam KY, Tucker JA, Walker M. The discovery of AZD5597, a potent imidazole pyrimidine amide CDK inhibitor suitable for intravenous dosing. Bioorg Med Chem Lett 2008;18:6369-6373;

AZD5597:

[4-[[5-fluoro-4-[2-methyl-1-(1-methylethyl)-1H-imidazol-5-yl]-2-pyrimidinyl]amino]phenyl] [(3 S)-3-(methylamino)-1-pyrrolidinyl]- Jones CD, Andrews DM, Barker AJ, Blades K, Daunt P, East S, Geh C, Graham MA, Johnson KM, Loddick SA, McFarland HM, McGregor A, Moss L, Rudge DA, Simpson PB, Swain ML, Tam KY, Tucker JA, Walker M. The discovery of AZD5597, a potent imidazole pyrimidine amide CDK inhibitor suitable for intravenous dosing. Bioorg Med Chem Lett 2008;18:6369-6373;

RGB-286638:

N-[1,4-dihydro-3-[4-[[4-(2-methoxyethyl)-1-piperazinyl]methyl]phenyl]-4-oxoindeno[1,2-c]pyrazol-5-yl]-N'-4-morpholinyl-, hydrochloride (1:2) Pharmacological targeting of CDK9 in cardiac hypertrophy Krystof V, Chamrád I, Jorda R, Kohoutek J. Med Res Rev. 2010 Jul;30(4):646-66. Review;

LCQ195/AT9311:

4-(2,6-dichlorobenzamido)-N-(1-(methylsulfonyl)piperidin-4-yl)-1H-pyrazole-3-carboxamide McMillin, Douglas W.; Delmore, Jake; Negri, Joseph; Buon, Leutz; Jacobs, Hannah M.; Laubach, Jacob; Jakubikova, Jana; Ooi, Melissa; Hayden, Patrick; Schlossman, Robert; Munshi, Nikhil c.; Lengauer, Christoph; Richardson, Paul G.; Anderson, Kenneth C.; Mitsiades, Constantine S. Molecular and cellular effects of multi-targeted cyclin-dependent kinase inhibition in myeloma: biological and clinical implications. British Journal of Haematology (2011), 152 (4), 420-432. CODEN: BJHEAL ISSN:0007-1048. AN 2011:307444 CAPLUS.

Particularly preferred compounds for use in the present invention are Cpd 24, Cpd C1, Cpd B1 and Cpd B2 as described and defined herein above.

Also siRNAs/RNAis, antisense molecules and ribozymes directed against nucleic acid molecules encoding CDK9 or its activators Cyclin T or Cyclin K are envisaged as (an) CDK9 inhibitor(s) for the use and the method of the present invention. The above-mentioned antagonist/inhibitor of CDK9 may also be a co-suppressive nucleic acid.
An siRNA approach is, for example, disclosed in Elbashir ((2001), Nature 411, 494-498)). It is also envisaged in accordance with this invention that for example short hairpin RNAs (shRNAs) are employed in accordance with this invention as pharmaceutical composition. The shRNA approach for gene silencing is well known in the art and may comprise the use of st (small temporal) RNAs; see, inter alia, Paddison (2002) Genes Dev. 16, 948-958.
As mentioned above, approaches for gene silencing are known in the art and comprise "RNA"- approaches like RNAi (iRNA) or siRNA. Successful use of such approaches has been shown in Paddison (2002) loc. cit., Elbashir (2002) Methods 26, 199-213; Novina (2002) Mat. Med. June 3, 2002; Donze (2002) Nucl. Acids Res. 30, e46; Paul (2002) Nat. Biotech 20, 505-508; Lee (2002) Nat. Biotech. 20, 500-505; Miyagashi (2002) Nat. Biotech. 20, 497-500; Yu (2002) PNAS 99, 6047-6052 or Brummelkamp (2002), Science 296, 550-553. These approaches may be vectorbased, e.g. the pSUPER vector, or RNA polIII vectors may be employed as illustrated, inter alia, in Yu (2002) loc. cit.; Miyagishi (2002) loc. cit. or Brummelkamp (2002) loc. cit.

However, use of CDK9 inhibitors in accordance with the present invention is not limited to known CDK9 inhibitors. Accordingly, also yet unknown CDK9 inhibitors may be used in accordance with the present invention. Such inhibitors may be identified by the methods described and provided herein and methods known in the art, like high-throughput screening using biochemical assays for inhibition of CDK9. Assays for screening of potential CDK9 inhibitors and, in particular, for identifying selective CDK9 inhibitors as defined herein are shown in the experimental part and described herein above. For example, a radiometric protein kinase assay (33PanQinase® Activity Assay; see Figure **3.** In addition/in the alternative, the well known Lance Assay can also be used; see Figure **2.** Based on his general knowledge a person skilled in the art is easily in the position to identify inhibitors or verify the inhibiting activity of compounds suspected of being CDK9 inhibitors. These tests may be employed on cell(s) or cell culture(s) described in the appended example, but also further cell(s)/tissue(s)/cell culture(s) may be used, such as cell(s)/ tissues/cell culture(s) derived from biopsies.

These selection methods or method for determining the responsiveness to treatment with a selective CDK9 inhibitor may also be used to screen or validate potential selective CDK9 inhibitors. For example, the activity/level of expression of CDK9 may be determined, wherein a lower activity/level of expression of CDK9 compared to a control is indicative for the capacity of a candidate molecule/substance to selectively inhibit CDK9. The term "activity of CDK9" used herein refers to the activity of a CDK9 protein (protein encoded by a CDK9 gene). The term "expression of CDK9" is used herein interchangeably with "expression of CDK9 gene" and refers to the expression of the CDK9 gene. It is to be understood that the activity/expression level of CDK9 determined in (a) cell(s), (a) tissue(s) or (a) cell culture(s) contacted with/exposed to an CDK9 inhibitor is compared with the activity/expression level of CDK9 in (a) control cell (s), (a) tissue(s) or (a) cell culture(s), i.e. cell(s), (a) tissue(s) or (a) cell culture(s) not contacted with/exposed to an CDK9

inhibitor. A skilled person will be aware of means and methods for performing such tests and selecting appropriate controls. Preferably, the control cell(s), (a) tissue(s) or (a) cell culture(s) will be identical to the cell(s), (a) tissue(s) or (a) cell culture(s) to be tested as described herein with the only exception that the control (s), (a) tissue(s) or (a) cell culture (s) are not contacted with/exposed to the CDK9 inhibitor.

Preferably, decreased CDK9 activity/expression levels of CDK9 proteins/polypeptides and/or CDK9 polynucleotides/ nucleic acid molecules are indicative of the capacity of a candidate molecule/substance to selectively inhibit CDK9. It is preferred herein that the CDK9 activity/expression level is decreased by at least 10 %, 20 %, 30 %, 40 %, 50 %, 60 %, 70 %, 80 % and most preferably by at least 90 % in cell(s), (a) tissue(s) or (a) cell culture(s) contacted with/exposed to an CDK9 inhibitor compared with the activity/expression level of CDK9 in (a) control cell(s), (a) control tissue(s) or (a) control cell culture(s). It is of note that the CDK9 activity must not necessarily correlate with the expression level. Thus, it may be, that CDK9 acitivity is decreased in the presence of an CDK9 inhibitor even though CDK9 expression is the same or even increased. However, a person skilled of the art will be aware of this and preferably evaluate CDK9 activity (i.e. activity/function of the CDK9 protein) when determining the capacitiy of a candidate substance to inhibit CDK9.

As mentioned, a person skilled in the art will be aware of corresponding means and methods for detecting and evaluating the CDK9 activity/expression level. Exemplary methods to be used include but are not limited to molecular assessments such as Western Blots, Northern Blots, Real-Time PCR and the like.

If the gene product is an RNA, in particular an mRNA (e.g. unspliced, partially spliced or spliced mRNA), determination can be performed by taking advantage of northern blotting techniques, hybridization on microarrays or DNA chips equipped with one or more probes or probe sets specific for mRNA transcripts or PCR techniques referred to above, like, for example, quantitative PCR techniques, such as Real time PCR. These and other suitable methods for binding (specific) mRNA are well known in the art and are, for example, described in Sambrook and Russell (2001, loc. cit.). A skilled person is capable of determining the amount of the component, in particular said gene products, by taking advantage of a correlation, preferably a linear correlation, between the intensity of a detection signal and the amount of the gene product to be determined.

In case the component is a polypeptide/protein, quantification can be performed by taking advantage of the techniques referred to above, in particular Western blotting techniques. Generally, the skilled person is aware of methods for the quantitation of (a) polypeptide(s)/protein(s). Amounts of purified polypeptide in solution can be determined by physical methods, e.g. photometry. Methods of quantifying a particular polypeptide in a mixture rely on specific binding, e.g of antibodies. Specific detection and quantitation methods exploiting the specificity of antibodies comprise for example immunohistochemistry (*in situ*). Western blotting combines separation of a mixture of proteins by electrophoresis and specific detection with antibodies. Electrophoresis may be multi-dimensional such as 2D electrophoresis. Usually, polypeptides are separated in 2D electrophoresis by their apparent molecular weight along one dimension and by their isoelectric point along the other direction. Alternatively, protein quantitation methods may involve but are not limited to mass spectrometry or enzyme-linked immunosorbant assay methods.

The below explanations concerning the NUT gene and NUT protein, apply, mutatis mutandis, to other nucleic acid sequences and amino acid sequences to be employed in context of the present invention, such as partner genes of NUT in NUT fusion genes like BRD4-NUT fusion genes, BRD3-NUT fusion genes or NUT-variant fusion genes characteristic for NMC. Accordingly, the explanations apply, mutatis mutandis, to members of the BET family (BRD2, BRDT and, in particular, human BRD3 gene and BRD3 protein (SEQ ID NOs: 5 and 6, respectively) and human BRD4 gene and BRD4 protein (SEQ ID NOs: 3 and 4, respectively). The explanations apply also to human CDK9 gene and CDK9 protein (SEQ ID NOs: 7 and 8, respectively), in particular CDK9 proteins to be used in the screening and/or validation of potential selective CDK9 inhibitors as described herein.

The term "NUT gene" ("nuclear protein in testis") as used in context of this invention refers to a gene encoding an unstructured polypeptide of unknown function that is highly expressed in normal spermatids; see Schwartz, loc. cit. It has been reported that the NUT protein binds to the histone acetyltransferase (HAT) p300; see Schwartz, loc. cit.

The nucleic acid sequence of the human NUT gene and the corresponding amino acid sequence is shown in SEQ ID NO: 1 and SEQ ID NO: 2, respectively. Generally, the term NUT used herein refers to any amino acid sequence having (partial) NUT activity as described herein and nucleic acid sequence(s) encoding such (an) amino acid sequence (s).

The nucleic acid sequences of NUT of other mammalian or non-mammalian species (in particular mouse, rat, chimpanzee) than the herein provided sequences for human NUT can be identified by the skilled person using methods known in the art, e.g. by nucleic acid sequencing or using hybridization assays or by using alignments, either manually or by using computer programs such as those mentioned herein below in connection with the definition of the term "hybridization" and degrees of homology. In one embodiment, the nucleic acid sequence encoding for orthologs of human NUT gene is at least 40% homologous to the nucleic acid sequences as shown in SEQ ID NO: 1. More preferably, the nucleic acid sequence encoding for orthologs of the human NUT gene is at least 45%, 50%, 55%, 60%, 65%, 70%, 75%, 80%, 85%, 90%, 91%, 92%, 93%, 94%, 95%, 96%, 97% or 98% homologous to the nucleic acid sequence as shown in SEQ ID NO. 1, wherein the higher values are preferred. Most preferably, the nucleic acid sequence encoding

for orthologs of the human NUT gene is at least 99% homologous to the nucleic acid sequence as shown in SEQ ID NO. 1.

Hybridization assays for the characterization of orthologs of known nucleic acid sequences/promoters are well known in the art; see e.g. Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989). The term "hybridization" or "hybridizes" as used herein may relate to hybridizations under stringent or non-stringent conditions. If not further specified, the conditions are preferably non-stringent. Said hybridization conditions may be established according to conventional protocols described, e.g., in Sambrook (2001) loc. cit.; Ausubel (1989) loc. cit., or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as, for example, the highly stringent hybridization conditions of 0.1 x SSC, 0.1% SDS at 65°C or 2 x SSC, 60°C, 0.1 % SDS. Low stringent hybridization conditions for the detection of homologous or not exactly complementary sequences may, for example, be set at 6 x SSC, 1% SDS at 65°C. As is well known, the length of the probe and the composition of the nucleic acid to be determined constitute further parameters of the hybridization conditions.

In accordance with the present invention, the terms "homology" or "percent homology" or "identical" or "percent identity" or "percentage identity" or "sequence identity" in the context of two or more nucleic acid sequences refers to two or more sequences or subsequences that are the same, or that have a specified percentage of nucleotides that are the same (preferably at least 40% identity, more preferably at least 45%, 50%, 55%, 60%, 65%, 70%, 75%, 80%, 85%, 90%, 91%, 92%, 93%, 94%, 95%, 96%, 97% or 98% identity, most preferably at least 99% identity), when compared and aligned for maximum correspondence over a window of comparison, or over a designated region as measured using a sequence comparison algorithm as known in the art, or by manual alignment and visual inspection. Sequences having, for example, 75% to 90% or greater sequence identity may be considered to be substantially identical. Such a definition also applies to the complement of a test sequence. Preferably the described identity exists over a region that is at least about 15 to 25 nucleotides in length, more preferably, over a region that is at least about 50 to 100 nucleotides in length, more preferably at least about 200 to 400 nucleotides, at least about 300 to 500 nucleotides, at least about 400 to 600 nucleotides in length, at least about 500 to 1000 nucleotides, at least about 800 to 1500 nucleotides, at least about 1000 to 2000 nucleotides, at least about 1500 to 2500 nucleotides or at least about 2000 to 3000 nucleotides. Even more preferably, the described identity exists over a region that is at least about 3000 to 4200 nucleotides in length, more preferably at least about 3200 to 4000 nucleotides, more preferably at least about 3300 to 3900 nucleotides. Most preferably, the described identity exists over a region that is at least about 3350 to 3850 nucleotides in length. In a most preferred embodiment, the described identity exists over the entire length of the nucleotide sequence shown in SEQ ID NO. 1, preferably the region thereof encoding the NUT protein. The coding region ranges from nucleotide 156 to nucleotide 3554 of the nucleotide sequence shown in SEQ ID NO: 1. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul, (1997) Nucl. Acids Res. 25:3389-3402; Altschul (1993) J. Mol. Evol. 36:290-300; Altschul (1990) J. Mol. Biol. 215:403-410). The BLASTN program for nucleic acid sequences uses as defaults a word length (W) of 11, an expectation (E) of 10, M=5, N=4, and a comparison of both strands. The BLOSUM62 scoring matrix (Henikoff (1989) PNAS 89:10915) uses alignments (B) of 50, expectation (E) of 10, M=5, N=4, and a comparison of both strands.

In order to determine whether a nucleotide residue in a nucleic acid sequence corresponds to a certain position in the nucleotide sequence of e.g. SEQ ID NO 1, the skilled person can use means and methods well-known in the art, e.g., alignments, either manually or by using computer programs such as those mentioned herein. For example, BLAST 2.0, which stands for Basic Local Alignment Search Tool BLAST (Altschul (1997), loc. cit.; Altschul (1993), loc. cit.; Altschul (1990), loc. cit.), can be used to search for local sequence alignments. BLAST, as discussed above, produces alignments of nucleotide sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cutoff score set by the user. The BLAST approach is to look for HSPs between a query sequence and a database sequence, to evaluate the statistical significance of any matches found, and to report only those matches, which satisfy the user-selected threshold of significance. The parameter E establishes the statistically significant threshold for reporting database sequence matches. E is interpreted as the upper bound of the expected frequency of chance occurrence of

an HSP (or set of HSPs) within the context of the entire database search. Any database sequence whose match satisfies E is reported in the program output.

Analogous computer techniques using BLAST (Altschul (1997), loc. cit.; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\text{\% sequence identity x \% maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules. Another example for a program capable of generating sequence alignments is the CLUSTALW computer program (Thompson (1994) Nucl. Acids Res. 2:4673-4680) or FASTDB (Brutlag (1990) Comp. App. Biosci. 6:237-245), as known in the art.

Also envisaged herein is not only the use of nucleic acid sequences encoding the NUT gene but also amino acid sequences of NUT protein. In line with the above explanations concerning orthologs/homologs of human NUT gene as shown in SEQ ID NO: 2, also orthologous/homologous amino acid sequences of the human NUT protein may be employed in accordance with the present invention. Accordingly, the terms "homology" or "percent homology" or "identical" or "percent identity" or "percentage identity" or "sequence identity" refer in the context of two or more amino acid sequences to two or more sequences or subsequences that are the same, or that have a specified percentage of amino acids that are the same (preferably at least 40% identity, more preferably at least 45%, 50%, 55%, 60%, 65%, 70%, 75%, 80%, 85%, 90%, 91%, 92% 93%, 94%, 95%, 96%, 97% or 98% identity, most preferably at least 99% identity) compared to the amino acid sequence of human NUT protein as shown in SEQ ID NO. 2.

As mentioned above, the term "rearrangement in the NUT gene" used herein refers to any rearrangement in the NUT gene that is characteristic for NUT midline carcinoma (NMC). Exemplary "rearrangments in the NUT gene" as well as methods for their detection are known in the art (see, for example, French (2010) J Clin Pathol, loc. cit.). For example, the rearrangement can be or can be caused by a translocation of the NUT gene (or a part or fragment thereof). One particularly preferred translocation is the t15;19 translocation known in the art. This translocation has resulted in the formation of a fusion gene of NUT, the so called BRD4-NUT fusion gene. Accordingly, rearrangement in the NUT gene which are or are caused/associated by the formation of a BRD4/NUT fusion gene are particularly preferred in context of the present invention. Also envisaged in this context is the formation of a fusion gene comprising a sequence encoding the complete BRD4 gene, and/or one or more parts or fragments thereof and comprising a sequence encoding the complete NUT gene and/or one or more parts or fragments thereof. The exemplary BRD4-NUT fusion protein is composed of the N-terminal of BRD4 (amino acids 1-720 out of 1372) and almost the entire protein sequence of NUT (amino acids 6-1127). The N-terminal of BRD4 includes bromodomains 1 and 2 and other, less well characterized functional domains.

Though the majority of known NMC cases are associated with the formation of a BRD4-NUT fusion gene, further rearrangments in the NUT gene have been described in the art, and the term "NUT variant fusion gene" has been coined in the art to cover the remaining NMC subtypes.

One exemplary "NUT variant fusion gene" is the so called "BRD3-NUT fusion gene". Accordingly, rearrangements in the NUT gene which are or are caused/associated by the formation of a BRD3/NUT fusion gene are also envisaged in context of the present invention. Again, the formation of a fusion gene comprising a sequence encoding the complete BRD3 gene, and/or one or more parts or fragments thereof and comprising comprising a sequence encoding the complete NUT gene and/or one or more parts or fragments thereof is envisaged herein.

The rearrangements in the NUT gene and optionally mutations/rearrangments/aberrant expression of further genes can be detected by methods known in the art. Such methods are, for example described in French CA, 2010; (NUT midline carcinoma. French CA. Cancer Genet Cytogenet. 2010 Nov;203(1):16-20.) A person skilled in the art is in the position to adapt the methods for detecting rearrangments in genes described in the above-mentioned documents to the rearrangements in the NUT gene described herein and further rearrangments in this gene known in the art. A person skilled in the art will readily understand that also rearrangements in said gene not described herein but known in the art or mutations yet to be identified may also be used in the context of the present invention.

Exemplary, non-limiting methods to be used in the detection of rearrangements in the NUT gene are described in WO 2010/011700, Haack (2009), loc. cit., French (2010) Cancer Genet Cytogenet (loc. cit.) and French (2010) J Clin Pathol (loc. cit.).

Particularly preferred is diagnosis via in situ hybridisation (FISH, CISH and the like), since these methods can detect any rearrangement in the NUT gene. However, also detection of a gene product of the above described NUT fusion genes is envisaged using routine techniques like Northern Blot, Real time PCR and the like. The latter methods are particularly envisaged in the detection of BRD3-NUT transcripts and/or BRD4-NUT transcripts. Also immunohistochemical methods (or other routine methods like Western Blots etc.) may be employed to detect expression products on a protein level. For example, antibodies French (2010) Cancer Genet Cytogenet (loc. cit.) or Haack (2009), loc. cit. describe the use of a diagnostic NUT specific monoclonal antibody, taking advantage of the fact the the native protein is not expressed outside of the testis.
Further methods which are useful for detecting mutations or rearrangments are methods for sequencing of nucleic acids (e.g. Sanger di-deoxy sequencing), "next generation" methods, single molecule sequencing, methods enabling detection of variant alleles/mutations, such as Real-time PCR, PCR-RFLP assay (see Cancer Research 59 (1999), 5169-5175), mass-spectrometric genotyping (e.g. MALDI-TOF), HPLC, enzymatic methods and SSPC (single strand confirmation polymorphism analysis; see Pathol Int (1996) 46, 801-804).
In particular, such methods may include enzymatic amplification of DNA or cDNA fragments using oligonucleotides specifically hybridizing to exonic or intronic parts of the rearranged NUT gene by PCT. Such amplifications may be carried out in two reactions when employing genomic DNA or even in only a single reaction when employing cDNA. The resulting PCR products may be subjected to either conventional Sanger-based dideoxy nucleotide sequencing methods or employing novel parallel sequencing methods ("next generation sequencing") such as those marketed by Roche (454 technology), Illumina (Solexa technology) or ABI (Solid technology). Rearrangements or mutations may be identified from sequence reads by comparison with publicly available gene sequence data bases. Alternatively, mutations may be identified by allele-specific incorporation of probes that can either be detected using enzymatic detection reactions, fluorescence, mass spectrometry or others; see Vogeser (2007) Dtsch Arztebl 104 (31-32), A2194-200.
Paraffin-embedded clinical material may be used in the detection of rearrangements in the NUT gene. Detection may comprise a histolopathology review of the sample to be tested to ensure tumour tissue is present. A commercially available Kit to be used in the detection method is the AllPrep DNA/RNA FFPE Kit form Quiagen (Germany). Further kits to be used for detecting rearrangements in the NUT gene are commercially available.
A positive result in the detection method described above indicates the presence of (a) rearrangement(s) in the NUT gene.

In one embodiment of the present invention, not only the presence of at least one rearrangement in the NUT gene is determined, but also, as a further option, expression of the NOTCH3 gene. It has been shown in the appended examples that cell lines having a NOTCH3 overexpression in addition to a rearrangement in the NUT gene are particularly susceptible to a selective CDK9 inhibitor. A nucleic acid sequence of the human NOTCH3 gene and a corresponding amino acid sequence are depicted in SEQ ID NOs: 11 and 12, respectively.
As mentioned above, (a) tumor cell(s)/tumor(s) with (a) rearrangement(s) in the NUT gene and overexpression of the NOTCH3 gene is (are) sensitive to treatment with selective CDK9 inhibitors. Therefore, it is envisaged that (a) tumor cell(s)/tumor(s) with (a) with (a) rearrangement(s) in the NUT gene and, optionally, overexpression of the NOTCH3 gene might be particularly sensitive to treatment with selective CDK9 inhibitors. Therefore, (a) cell(s), (a) tissue(s) or (a) cell culture selected in accordance with the present method with at least one rearrangement in the NUT gene and overexpression of the NOTCH3 gene might be particularly susceptible to a selective CDK9 inhibitor. Accordingly, treatment of patients with a selective CDK9 (the patients suffering from NMC) may be particularly successful in respect of, for example, prognosis or survival rate.
The determination of the presence of other markers for susceptibility to CDK9 inhibitors in addition to (a) rearrangement in the NUT gene and (as a further option), additionally, overexpression in the NOTCH3 gene is also envisaged herein. Of course, also the presence of markers for susceptibility to other compounds/drugs (e.g. NUT inhibitors; NOTCH3 inhibitors and the like) may be determined in accordance with the present methods. This may be of value in selecting (a) cell(s), (a) tissue(s) or (a) cell culture or in the identification of patients/responders which are not only susceptible/ sensitive to (a) selective CDK9 inhibitor(s) but also to other compounds/drugs (e.g. (an) NUT inhibitor and/or a NOTCH3 inhibitor(s)). These cell(s)/tissue(s)/cell culture(s)/patient(s)/ responder(s) may, for example, be subject to co-therapy/co-treatment with a selective CDK9 inhibitor and a further compound/drug (e.g. (a) NUT inhibitor(s)).
Alternatively, (a) cell(s), (a) tissue(s) or (a) cell culture may be selected or patients/responders be identified which are characterized by the presence only of (a) rearrangement in the NUT gene but not overexpression of the NOTCH3 gene and/or optionally further genes. For example, patients suffering from cancer characterized by the presence of at least one rearrangement in the NUT gene (e.g. NMC) and (a) mutation(s) or overexpreesion of a further gene (e.g. NOTCH3) may only be treated with a selective CDK9 inhibitor but not in co-therapy with NOTCH3 inhibitor and a selective CDK9 inhibitor if the patients are known to be resistant to such NOTCH3 inhibitor. Of course, co-therapy/combination therapy to be used in context of the present invention may also comprise radiation therapy, conventional chemotherapy and the like.

As described herein below and shown in the appended example, these methods for determining the susceptibility to (a) selective CDK9 inhibitor(s)/ responsiveness to treatment with (a) selective CDK9 inhibitor(s) may comprise in a

first step contacting cell(s), tissue(s) or cell culture(s) with a selective CDK9 inhibitor or exposing cell(s), tissue(s) or cell culture(s) to selective CDK9 inhibitor. A person skilled in the art knows how the contacting with/exposing to a selective CDK9 inhibitor is to be performed. For example, the cell(s), tissue(s) or cell culture(s) may be incubated with a selective CDK9 inhibitor comprised in a composition with appropriate diluents, stabilizers and/or carriers. The molar concentration of the selective CDK9 inhibitor to be used in the contacting/exposing step (comprising, for example, incubating the cell (s), tissue(s) or cell culture(s) with a selective CDK9 inhibitor) are, of course, dependent on the nature of the selective CDK9 inhibitor. Also diluents, stabilizers and/or carriers to be optionally added in the contacting/exposing step will depend on the nature of the selective CDK9 inhibitor. However, a person skilled in the art will know which diluents, stabilizers and/or carriers and the like are to be added and will also be aware of appropriate concentrations of diluents, stabilizers and/or carriers and also of the CDK9 inhibitor(s) to be used in the selection method/method for determining the responsiveness of the present invention. Other methods for exposing cells to the selective CDK9 inhibitor may be the use of cell-matrix or compound arrays, where cells are matrix-bound in array format and can therefore be simultaneously exposed to multiple inhibitors or multiple concentrations of inhibitors or where compounds are matrix-bound in array format an can therefore be simultaneously exposed to multiple types of cells or aliquots of cells.

Also the use of high throughput screening (HTS) is envisaged in context of the present invention, in particular the screening methods of cell(s), tissue(s) and/or cell culture(s) for responsiveness/sensitivity to a selective CDK9 inhibitor. Suitable (HTS) approaches are known in the art and a person skilled in the art is readily in the position to adapt such protocols or known HTS approaches to the performance of the methods of the present invention.

Screening-assays are usually performed in liquid phase, wherein for each cell/tissue/cell culture to be tested at least one reaction batch is made. Typical containers to be used are micro titer plates having for example, 384, 1536, or 3456 wells (i.e. multiples of the "original" 96 reaction vessels). Robotics, data processing and control software, and sensitive detectors, are further commonly used components of a HTS device. Often robot system are used to transport micro titer plates from station to station for addition and mixing of sample(s) and reagent(s), incubating the reagents and final readout (detection). Usually, HTS can be used in the simultaneous preparation, incubation and analysis of many plates.

The assay can be performed in a singly reaction (which is usually preferred), may, however, also comprise washing and/or transfer steps. Detection can be performed taking advantage of radioactivity, luminescence or fluorescence, like fluorescence-resonance-energytransfer (FRET) and fluorescence polarisation (FP) and the like. The biological samples described herein can also be used in such a context. In particular cellular assays and in vivo assays can be employed in HTS. Cellular assays may also comprise cellular extracts, i.e. extracts from cells, tissues and the like. However, preferred herein is the use of cell(s) or tissue(s) as biological sample (in particular a sample obtained from a patient/subject suffering or being prone to suffer from NMC), whereas in vivo assays (wherein suitable animal models are employed, e.g. the herein described mouse models) are particularly useful in the validation/monitoring of the treatment with an CDK9 inhibitor. Depending on the results of a first assay, follow up assays can be performed by re-running the experiment to collect further data on a narrowed set (e.g. samples found "positive" in the first assay), confirming and refining observations.

HITS cannot only be employed in identifying cell(s), tissue(s) and/or animal(s) susceptible/responsive to selective CDK9 inhibitors, or in monitoring the efficacy of a treatment of cancer (in particular NMC) as described herein; HTS is also useful in identifying further CDK9 inhibitors to be used herein. The screening of compound libraries with usually several hundred thousands of substances takes usually between days and weeks. An experimental high throughput screen may be supplemented (or even be replaced) by a virtual screen. For example, if the structure of the target molecule (e.g. CDK9) is known, methods can be employed, which are known under the term "docking". If the structure of several target-binding molecules is known (e.g. the herein described CDK9) methods for Pharmacophor-Modelling can be used aiming at the development new substances which also bind to the target molecule. A suitable readout in animal (in vivo) models is tumor growth (or respectively the complete or partial inhibition of tumor growth and/or its remission).

High-throghput methods for the detection of mutations involve massively parallel sequencing approaches, such as the "picotiter plate pyrosequencing". This approach relies on emulsion PCR- based clonal amplification of a DNA library adapted onto micron-sized beads and subsequent pyrosequencing-by-synthesis (Thomas RK et al. Nature Med 2007) of each clonally amplified template in a picotiter plate, generating over 200,000 unique clonal sequencing reads per experiment. Furthermore, mass spectrometric genotyping approaches (Thomas RK et al.; Nat Gen 2007) and other next generation sequencing methods (Marguerat S et al.; Biochem Soc Trans 2008) may be employed.

The meaning of the terms "cell(s)", "tissue(s)" and "cell culture(s)" is well known in the art and may, for example, be deduced from "The Cell" (Garland Publishing, Inc., third edition). Generally, the term "cell(s) used herein refers to a single cell or a plurality of cells. The term "plurality of cells" means in the context of the present invention a group of cells comprising more than a single cell. Thereby, the cells out of said group of cells may have a similar function. Said cells may be connected cells and/or separate cells. The term "tissue" in the context of the present invention particularly means a group of cells that perform a similar function. The term "cell culture(s)" means in context of the present invention cells as defined herein above which are grown/cultured under controlled conditions. Cell culture(s) comprise in particular cells

(derived/obtained) from multicellular eukaryotes, preferably animals as defined elsewhere herein. It is to be understood that the term "cell culture(s)" as used herein refers also "tissue culture (s)" and/or "organ culture(s)", an "organ" being a group of tissues which perform the some function.

Preferably, the cell(s), tissue(s) or cell culture(s) to be contacted with/exposed to a selective CDK9 inhibitor comprise/are derived from or are (a) tumor cell(s). The tumor cells may, for example, be obtained from a biopsy, in particular a biopsy/biopsies from a patient/subject suffering from NMC or, though less preferred a patient/subject being prone to suffer from NMC. It is preferred herein that said subject is a human. The term "mammalian tumor cell(s)" used herein refers to (a) tumor cell(s) which is derived from or is a tumor cell from a mammal, the term mammal being derived herein below. As described herein above in respect of "cell(s)", "tissue(s)" and "cell culture(s)" the "mammalian tumor cells" may be obtained from a biopsy, in particular a biopsy/biopsies from a patient/subject suffering from NMC or, though less preferred a patient/subject being prone to suffer from NMC. The term "tumor cell" also relates to "cancer cells".

Generally, said tumor cell or cancer cell may be obtained from any biological source/organism, particularly any biological source/organism, suffering from the above-mentioned NMC.

Preferably, the (tumor) cell(s) or (cancer) cell to be contacted is (are) obtained/derived from an animal. More preferably, said (tumor)/(cancer) cell(s) is (are) derived from a mammal. The meaning of the terms "animal" or "mammal" is well known in the art and can, for example, be deduced from Wehner und Gehring (1995; Thieme Verlag). Non-limiting examples for mammals are even-toed ungulates such as sheep, cattle and pig, odd-toed angulates such as horses as well as camivors such as cats and dogs. In the context of this invention, it is particularly envisaged that DNA samples are derived from organisms that are economically, agronomically or scientifically important. Scientifically or experimentally important organisms include, but are not limited to, mice, rats, rabbits, guinea pigs and pigs.

The tumor cell(s) may also be obtained from primates which comprise lemurs, monkeys and apes. The meaning of the terms "primate", "lemur", "monkey" and "ape" is known and may, for example, be deduced by an artisan from Wehner und Gehring (1995, Thieme Verlag). As mentioned above, the tumor or cancer cell(s) is (are) most preferably derived from a human being suffering from the above-mentioned NMCs. In context of this invention particular useful cells, in particular tumor or cancer cells, are, accordingly, human cells. These cells can be obtained from e.g. biopsies or from biological samples but the term "cell" also relates to in vitro cultured cells.

A preferred, however non-limiting cell(s) or cell culture(s) also used in the appended example is cell line 143100 (showing a t15;19 translocation resulting in the formation of a BRD4-NUT-fusion protein). A further cell line to be used in accordance with the present invention is HCC2429 (showing NOTCH3 overexpression in addition to the t15;19 translocation). Further cell lines that can be used include HCC 1143 (NOTCH3 overexpression), PC9 (EGFRmut) or A549 (KRAS mut). These cell lines are well known in the art and may be obtained from ATCC and/ or DSMZ and/or from the U.S. National Cancer Institute (www.lgcpromochem-atcc.com/; www.dsmz.de/; dtp.nci.nih.gov/docs/misc/common_ files/cell_list .html).

In one embodiment, the present invention relates to an in vitro method for the identification of a responder for or a patient sensitive to a selective CDK9 inhibitor, said method comprising the following steps:

evaluating the presence of at least one rearrangement in the NUT gene in a sample obtained from a subject or patient suspected to suffer from or being prone to suffer from NUT midline carcinoma (NMC), whereby the presence of at least one rearrangement in the NUT gene is indicative for a responding patient or is indicative for a sensitivity of said patient to a selective CDK9 inhibitor.

Said sample may, for example, be obtained by (a) biopsy (biopsies). Preferably, said sample is obtained from a patient suspected to suffer from or being prone to suffer from NMC. The NUT midline carcinoma may, in addition to the presence of at least one rearrangement in the NUT gene be characterized by the presence of overexpression in the NOTCH3 gene and/or further rearrangments/mutations/aberrant expression in other genes.

It is preferred herein that said sample is obtained from (a) tumor(s) and, accordingly, is (a) tumor cell(s) or (a) tumor tissue(s) suspected of being a NMC tumour. A person skilled in the art is easily in the position to identify NMC with the herein described rearrangements/mutations/aberrant expression using standard techniques known in the art and methods disclosed herein.

Another embodiment of the present invention relates to the use of an oligo- or polynucleotide capable of detecting (a) mutation(s) of at least one rearrangement in the NUT gene for diagnosing sensitivity to a selective CDK9 inhibitor as defined herein above. Preferably, the oligonucleotide(s) is (are) about 15 to 100 nucleotides in length. A person skilled in the art is, based on his general knowledge and the teaching provided herein, easily in the position to identify and/or prepare (a) an oligo- or polynucleotide capable of detecting at least one rearrangement in the NUT gene and, optionally, overexpression of the NOTCH3 gene and/or further rearrangements/mutations/aerrant expression of other genes. In particular these oligo- or polynucleotides may be used as probe(s) in the detection methods described herein A skilled person will know, for example, computer programs which may be useful for the identification of corresponding probes to be used herein. For example, the NUT nucleic acid sequence (SEQ ID NO: 1) may be used in this context for identifying

specific probes for detecting rearrangements in the NUT gene. Exemplary NUT nucleic acid sequences are available on corresponding databases, such as the NCBI database (www.ncbi.nlm.nih.gov/sites/entrez).
Accordingly, a NUT nucleic acid sequence can be found in this database under the accession number NM_175741.1. As mentioned, an exemplary sequence of a NUT nucleic acid sequence, is also depicted in SEQ ID NOs: 1, respectively.
In a particular embodiment, a method of monitoring the efficacy of a treatment of NUT midline carcinoma (NMC) in a subject or patient suffering from NMC or being prone to suffering from NMC is disclosed herein, comprising the steps of

a) determining in a cell or tissue sample obtained from said subject or patient the presence of at least one rearrangment in the NUT gene; and
b) comparing the rearrangment status in the NUT gene as determined in a) with a reference or control rearrangment status in the NUT gene,

wherein the extent of the difference between said rearrangment status determined in a) and said reference rearrangment status is indicative for said efficacy of a treatment of NMC. Preferably, the treatment of NUT midline carcinoma (NMC) comprises treatment with a selective CDK9 inhibitor as defined herein.
The above monitoring method may, optionally, comprise determining the expression level of the NOTCH3 gene in a cell or tissue sample obtained from said subject or patient; and comparing the expression level of the NOTCH3 gene with a reference or control expression level of the NOTCH3 gene, wherein the extent of the difference between said rearrangment status determined in the first step (e.g. step (a)) and said reference rearrangment status of the second step (e.g. step (b)) is indicative for said efficacy of a treatment of NMC.
The term "expression level" refers to expression on a protein level (e.g. to be determined by Western Blots and the like) or transcriptional level (e.g. spliced, unspliced or partially spliced mRNA, which may be determined by Northern Blots, Real time PCR and the like). The expression of marker genes referred to in the context of the monitoring methods or methods of predicting the efficacy of a treatment is in particular the NOTCH3 gene.
The method of monitoring the efficacy of a treatment of a cancer may comprise a step of determining in a cell or tissue sample obtained from a subject/patient suffering from NMC (e.g. a biopsy) the presence of at one rearrangment in the NUT gene. For example, a rearrangment in the NUT gene (and, optionally, aberrant or overexpression of the NOTCH3 gene) may be present in a sample before start of the treatment of NMC. During or after treatment of NMC, the tumor cells having the rearrangment in the NUT gene (and, optionally, overexpression of the NOTCH3 gene) are erased or otherwise depleted. Thus, the absence of a detectable rearrangement in the NUT gene (and, optionally, absence of overexpression of the NOTCH3 gene) in a sample (cell samples/biopsy samples and the like) obtained from a subject/ patient during or after treatment of a cancer is indicative of the efficacy of the treatment.
The present invention also relates to a method of predicting the efficacy of a treatment of NUT midline carcinoma (NMC) for a subject/patient suffering from said disorder or being prone to suffering from said disorder comprising the steps of

a) determining in a cell or tissue sample obtained from said subject/patient the rearrangment status in the NUT gene; and
b) comparing the rearrangement status in the NUT gene as determined in a) with a reference or control rearrangement status in the NUT gene determined in a cell or tissue sample obtained from a control subject/patient (responder and/or non-response), wherein the extent of the difference between said rearrangement status determined in a) and said reference or control rearrangment status is indicative for the predicted efficacy of a treatment of NMC. Preferably, the treatment of NUT midline carcinoma (NMC) referred to in the above method of predicting the efficacy comprises treatment with a selective CDK9 inhibitor as defined herein.

It has been demonstrated in context of this invention that rearrangments in the NUT gene as disclosed herein act as markers/predictors for susceptibility to a selective CDK9 inhibitor. In particular a responder for or a patient sensitive to a CDK9 inhibitor may be identified in accordance with the present method. Accordingly, the present invention provides the possibility to monitor or predict the efficacy of the treatment of NMC by measuring the expression level of the gene product resulting from a rearrangment in the NUT gene.
Instead of or in addition to the the expression level the activity of the markers/predictors provided herein may be determined. For example, the acitivity of BRD-NUT fusion proteins to induce global histone hypoacetylation and/or transcriptional repression may be determined; such activities and methods for determining same are known in the art; see Schwartz (2011), loc. cit. It has also been shown that Notch3 activates transcription of target genes like myc, CyclinD, raper, hid, HESER, GATA3, Pax2, Lip-1, ErbB2, EGFR and/or Notch; see Bray and Bernard (2010) in: Current Topics in Developmental biology, Volume 92, 253-275. The activity of Notch3 as reflected in the induction or promotion of the above target genes can easily be measured by determining the expression level (e.g. protein or mRNA) of these target genes.

Based on these findings, the present invention provides the particular advantage that the measurement of the expression level and/or activity of at least one of the marker genes allows the rapid determination how efficacious treatment of NMC is (or whether treatment of NMC is efficacious at all, as the case may be). In other words, the efficacy of a treatment of NMC can be monitored or predicted early. Hence, also a potential resistance to the treatment can be recognized early by using the means and method of this invention.

In a particular embodiment, the present invention relates to corresponding means, methods and uses which are based on the early recognition of changes in the rearrangement status in the NUT gene as reflected, for example, in the expression level of NUT fusion genes and/or activity of NUT fusion proteins.

The possibility of recognizing changes in the rearrangement status in the NUT gene early, provides several advantages, like a higher lifespan/likelihood of survival of the subject/patient (for example due to the notice of possible treatment failures and a corresponding change of the treatment regimen) and the possibility of a more efficient therapy (for example due to the possibility to avoid/recognize treatment failures early and, hence, to correspondingly change the treatment regimen early in therapy, i.e. to timely switch to a more suited inhibitor, to discontinue an expensive, ineffective treatment early after diagnosis and to opt for alternative therapy).

In context of the above embodiments of this invention, "early" particularly means prior to (the onset of) a (complete or partial) response. For example, "early" monitoring or predicting the efficacy of a therapy/treatment of NMC may be at least 1, at least 2, at least 3, at least 4, at least 5, at least 6, at least 7, at least 10, or at least 14 days prior to (the onset of) a (partial) response to said therapy/treatment and/or at least 1, at least 2, at least 3, at least 4, at least 5, at least 6, at least 7, at least 10, at least 12, at least 15, or at least 18 month prior to a complete response said therapy/treatment, wherein the longer periods are preferred.

Alternatively, "early" monitoring or predicting the efficacy of a therapy/treatment of said cancer may also be at most 1, at most 2, at most 3, at most 4, at most 5, at most 6, at most 7, at most 10, or at most 14 days after (onset of) the therapy/treatment of said cancer, wherein the shorter periods are preferred. Most preferably, it is envisaged to already monitor the efficacy of a therapy/treatment of said cancer at the day the therapy/treatment was initiated, i.e. once rearrangement status in the NUT gene changes upon said therapy/treatment.

In the following, a non-limiting example of a scheme for (early) monitoring or predicting the efficacy of a therapy/treatment of NMC in accordance with this invention is provided:

- When monitoring the therapy/treatment of NMC with a selective CDK9 inhibitor, the rearrangement status may be determined daily during the first week after initiation of the therapy/treatment, weekly during the first month of the therapy/treatment and, afterwards, monthly.
- The reference activity/expression level may be taken at the day the therapy/treatment is initiated, from the subject/patient to be treated and/or from a corresponding control subject/patient (responder/non-responder); see below.
- If a rise in marker levels is observed in two consecutive samples, or if decrease in marker level is not fast enough (for example not as fast as that of responder or not sufficiently faster than that of a non-responder), change of treatment regimen may be considered.

It is of note that this example is in no way limiting. The skilled person is readily in the position to adapt this scheme to the particular requirements relevant for each individual case, based on the teaching provided herein and on his common general knowledge.

As mentioned, the present invention is particularly useful for monitoring or predicting the efficacy of a therapy/treatment of NMC. Corresponding means, uses and methods are provided herein. In general, monitoring or predicting the efficacy of a certain kind of therapy/treatment is regularly applied in clinical routine, since it allows for preventing the disorder and/or increasing the efficiency of a therapy/treatment and hence, leads to savings in cost and time and to a higher lifespan/likelihood of survival/'Genesung' of the affected patient.

Hence, the skilled person is aware of the meaning of monitoring the efficacy of a certain kind of therapy/treatment. In context of this invention, the meaning of the term "monitoring" encompasses the meaning of terms like "tracking", "discovering" etc.. In particular, the term "monitoring the efficacy of a therapy/treatment of NMC" as used herein refers to monitoring whether a subject/patient suffering from said disorder (or being prone to suffering from said cancer) responds at all to a therapy/treatment of said disorder and/or how the course of said respond is (e.g. how fast/slow the respond is and/or to what extent the respond is).

In context of this invention, the term "predicting the efficacy of a therapy/treatment of NMC" is used in basically the same sense like determining whether, and/or to what extent, a subject/patient exhibits susceptibility to such therapy/treatment, i.e. whether said subject/patient will or would respond at all to a therapy/treatment of said disorder and/or how the course of said respond will or would be (e.g. how fast/slow the respond is and/or to what extent the respond is). In particular, a subject/patient exhibits susceptibility to NMC when its rearrangement status in the NUT gene is aberrant. In one embodiment, the "predicting the efficacy of a therapy/treatment of NMC" in accordance with this invention may be performed after initiation of the therapy/treatment, i.e. during the already ongoing therapy/treatment. In particular,

said "predicting" may be performed during the herein described monitoring the efficacy of a therapy/treatment of said cancer, preferably early after the beginning of said monitoring. Thereby, the predicting may be based on results from said monitoring obtained at a certain point in time of the ongoing therapy/treatment. Preferably, said point in time is an early point in time, like, for example that point in time, when a first result from said monitoring has been obtained. In cases where the "predicting the efficacy of a therapy/treatment of the cancer defined herein" is performed during an already ongoing therapy/treatment, it refers to the following/subsequent efficacy of said therapy/treatment.

In another embodiment, the "predicting the efficacy of a therapy/treatment of the cancer defined herein" in accordance with this invention may be performed (immediately) after diagnosis but, however, prior to initiation of the therapy/treatment. In such cases, "predicting the efficacy of a therapy/treatment of said cancer" refers to the efficacy of a therapy/treatment which has not yet been initiated (or has been initiated substantially at the same point in time when the "predicting" was performed.

In context of this embodiment of the invention, one non-limiting example of a healthy control subject/patient is one having (a) wild-type NUT gene(s). In other words a healthy control subject/patient does not have a rearrangement in the NUT gene.

In accordance with the above, the reference rearrangement status in the NUT gene with respect to the means, methods and uses of monitoring or predicting the efficacy of a treatment of NMC is that determined in (a sample of) the corresponding healthy control subject/patient, i.e. is the "normal" reference rearrangement status in the NUT gene, whereby the "normal" status implies that no rearrangement in the NUT gene has occurred or is present in the NUT gene.

It is to be understood that an aberrant rearrangement status in the NUT gene means that the rearrangement status in the NUT gene as described herein is different from the above described reference rearrangement status in the NUT gene. The reference rearrangement status in the NUT gene is in this context, accordingly, the "normal" rearrangement status in the NUT gene. In particular with respect to the herein disclosed means, methods and uses of monitoring/ predicting the efficacy of a treatment of NMC, the control subject/patient is, in one embodiment, envisaged to be a subject/patient suffering from NMC or being prone to suffering from said cancer, i.e. a subject/patient having, for example, an aberrant rearrangement status in the NUT gene and, hence, not a "normal" rearrangement status in the NUT gene as described in accordance with this invention.

Thereby, it is clear that "different" rearrangement status in the NUT gene means that a rearrangement is present/ detectable in a sample obtained from the subject/patient and is not present/not detectable in a sample obtained from a control subject/patient. The presence or absence of such rearrangements in the NUT gene can easily be determined by herein provided methods such as FISH, CISH and the like. In case the rearrangement status in the NUT gene is determined on basis of the expression level of formed NUT fusion gene products, the presence of a rearrangement in the NUT gene goes along with an expression of such fusion gene products that can an easily be determined by methods described herein, like RT-PCR, Northern Blot and the like.

In this context, a "different" rearrangement status in the NUT gene, in particular the presence of a rearrangement in the NUT gene in a subject/patient, as reflected, for example, in a "higher" expression level of NUT fusion gene products in a subject/patient means higher expression levels than the normal (range of) expression of NUT fusion gene products. For example, "higher expression levels" means at least 1.5 fold, at least 2 fold, at least 2.5 fold, at least 3 fold, at least 4 fold, at least 5 fold, at least 7 fold, at least 10 fold, at least 15 fold, at least 25 fold, at least 50 fold, at least 100 fold, at least 200 fold higher expression levels, wherein the higher values are preferred. Whether and to which extent the rearrangement status as, for example, reflected in the expression level changes can easily be deduced by the skilled person based on the teaching provided herein and the common general knowledge.

It is particularly preferred in this context that the control subject/patient is subjected to the same treatment of the cancer described and defined herein as the subject/patient itself and/or that it is known whether the control subject/ patient is a responder or non-responder to this treatment. Whether a subject/patient is a "responder" or "non-responder" with respect to a certain kind of cancer treatment/therapy can be evaluated by the skilled person on the basis of his common general knowledge and/or the teaching provided herein. Accordingly, the patient responds to cancer treatment/ therapy, if the rearrangment in the NUT gene is reduced upon said treatment/therapy. Preferably, the rearrangment in the NUT gene as, for example, reflected in the expression level of NUT fusion gene products is reduced to control rearrangement status (e.g. control expression level, for example determined in a sample obtained from a person not suffering from said cancer). In other words, a reduction in expression level is indicative for a successful treatment/therapy. A skilled person is readily in the position to determine whether a patient responds to NMC treatment/therapy by evaluation of the presence of rearrangements in the NUT gene as reflected in the expression level of NUT fusion gene products.

In contrast, a patient who does not respond to treatment/therapy does not show a reduced rearrangments in the NUT gene as reflected in the expression level, upon said treatment/therapy. This is in contrast to "responders/responding patients", showing such a reduced expression level.

In particular, a "responder" may be a subject/patient whose (aberrant) rearrangments in the NUT gene as reflected in the expression level change towards their "normal" (protein or mRNA expression) level(s) (in a sufficient manner) upon the cancer treatment/therapy. In one specific embodiment, a "responder" may be a subject/patient not suffering from

one of the herein defined resistances. In particular, a "non-responder" may be a subject/patient whose rearrangments in the NUT gene as reflected in the expression level do not change towards their "normal" (expression) level(s) (in a sufficient manner) upon the cancer treatment/therapy. In one specific embodiment, a "non-responder" may be a subject/patient suffering from one of the herein defined resistances.
In context of this embodiment of the invention, one non-limiting example of a (diseased) control subject/patient (responder and/or non-responder) suffering from NMC or being prone to suffering from a susceptibility thereto is one having a rearrangments in the NUT gene which may be reflected in the formation and, optionally, expression of a NUT fusion gene.

The skilled person is aware of how a typical/desired response to a known therapy/treatment of a NMC should proceed or is intended to proceed. Moreover, the skilled person can consider how a typical/desired response to a (unknown) therapy/treatment of NMC should proceed or is intended to proceed. Based on this knowledge, the means, methods and uses of this invention referring to the efficacy of a therapy/treatment of such a cancer can, for example, also be carried out without employing (a sample of) a particular control subject/patient, i.e. without comparing the "rearrangement status in the NUT gene" (e.g. reflected in the expression level of a NUT fusion gene)" with a "reference rearrangement status in the NUT gene" as defined herein determined in (a sample from) a control subject/patient. Simply by comparing the course of the determined "rearrangement status in the NUT gene" during the therapy/treatment of NMC with the above-mentioned know "typical/desired response", the skilled person is able to consider the efficacy of the therapy/treatment monitored/predicted. If the response of a subject/patient is as fast (or even faster) than the "typical/desired response", the subject/patient is a "responder". If the response of a subject/patient is slower than the "typical/desired response", the subject/patient is a "non-responder" (when no substantial response can be seen) or "weak-responder".

In general, a (desired) efficacy of a treatment of NMC described herein or susceptibility thereto is indicated/predicted, when the aberrant "rearrangement status in the NUT gene" (e.g. reflected in the expression level of a NUT fusion gene)" is shifted back towards the "normal level" of a (healthy) control subject/patient due to/in consequence of said treatment of the cancer or susceptibility thereto.
In context of this invention, the efficacy of a treatment of the cancer defined herein is high, when the subject/patient (to be) treated responds as fast (or even faster) and as complete as a "responder", i.e. exhibits a "typical/desired response". This means that said subject/patient reaches the "normal" "rearrangement status in the NUT gene" (e.g. reflected in the expression level of a NUT fusion gene)" of a healthy subject/patient as fast as a "responder", i.e. in the same manner as in a "typical/desired response".
In context of this invention, the efficacy of a treatment of the cancer defined herein is moderate/low, when the subject/patient (to be) treated responds not as fast and/or not as complete as a "responder", i.e. does not exhibit a "typical/desired response". This means that said subject/patient does not reach the "normal" level of the "rearrangement status in the NUT gene" (e.g. reflected in the expression level of a NUT fusion gene. Accordingly, a moderate/low efficacy means also that the "rearrangement status in the NUT gene" (e.g. reflected in the expression level of a NUT fusion gene)" of a healthy subject/patient is not reached as complete and/or as fast as a "responder", i.e. not in the same manner as in a "typical/desired response".
In context of this invention, there is no efficacy of a treatment of the cancer at all, when the subject/patient (to be) treated does not respond at all.
Particularly, when the efficacy of a treatment of the cancer as monitored/predicted in context of this invention is moderate/low or, if there is no such efficacy, a change of the (planned) therapy/treatment might be considered.

In an alternative embodiment, of the herein disclosed means, methods and uses of monitoring/predicting, the reference "rearrangement status in the NUT gene" (e.g. reflected in the expression level of a NUT fusion gene)" of a control subject/patient can be replaced by a reference "rearrangement status in the NUT gene" (e.g. reflected in the expression level of a NUT fusion gene)" from the subject/patient to be treated itself obtained prior to (or at the beginning of) the treatment/therapy. In this specific case, the "control subject/patient" would be the subject/patient to be treated itself. The efficacy of the cancer treatment would then be assessed on the basis of how the "rearrangement status in the NUT gene" (e.g. reflected in the expression level of a NUT fusion gene)" changes during treatment/therapy compared with said particular reference "rearrangement status in the NUT gene" (e.g. reflected in the expression level of a NUT fusion gene)". The more significant and/or faster said change is the more efficacious is the treatment/therapy.

The explanations and definitions given herein above in context of the monitoring or predicting methods, and, in particular the definitions of a "control subject/patient" which is different to the "subject/patient" to be treated also apply in this context where the "subject/patient" and the "control subject/patient" are actually the same.
For example, if the control subject/patient is a "responder" (e.g. a "positive control"), a minimal or low difference of the "rearrangement status in the NUT gene" (e.g. reflected in the expression level of a NUT fusion gene)" between the "reference" and the sample/patient to be assessed or monitored is indicative for "high efficacy". The same may be evaluated on the basis of a "typical/desired response". Also here a low difference (at a certain point in time) to the "control" indicates a high efficacy. Similarly, in case that the "control subject/patient" is a non-responder (e.g. "negative control") or a patient sample obtained before treatment, a higher difference in reference "rearrangement

status in the NUT gene" (e.g. reflected in the expression level of a NUT fusion gene)" obtained prior to/at the beginning of a therapy/treatment of NMC may also be indicative for a high efficacy. In this context, a high and thereby "positive" difference (at a certain point in time) between a "non-responder sample" or an "own sample before treatment or at the beginning of the treatment" of the given patient and the sample assessed during or after treatment indicates a high efficacy.

As can be deduced from the above, the reference "rearrangement status in the NUT gene" (e.g. reflected in the expression level of a NUT fusion gene)" as referred to herein may be taken at the day of diagnosis, once the therapy/treatment is initiated, in between and/or during therapy/treatment, either from the subject/patient to be treated itself or from a corresponding control subj ect/patient (healthy/responder/non-responder).

If the reference "rearrangement status in the NUT gene" (e.g. reflected in the expression level of a NUT fusion gene)" is obtained from a control subject/patient different from the subject/patient to be treated, it is preferred that the reference "rearrangement status in the NUT gene" (e.g. reflected in the expression level of a NUT fusion gene)" is determined at the same point in time during therapy/treatment. In particular, if the reference "rearrangement status in the NUT gene" (e.g. reflected in the expression level of a NUT fusion gene)" is obtained from the subject/patient to be treated itself, the reference "rearrangement status in the NUT gene" (e.g. reflected in the expression level of a NUT fusion gene)" should be determined at a different point in time during therapy/treatment to allow comparison, for example, at the beginning of (or prior to) the therapy/treatment.

In general, "rearrangement status in the NUT gene" (e.g. reflected in the expression level of a NUT fusion gene) described herein can be determined once or, preferably, several times. For example, "rearrangement status in the NUT gene" (e.g. reflected in the expression level of a NUT fusion gene) can be determined on a daily, weekly, monthly or yearly basis during therapy/treatment. Commonly, the requirements of corresponding studies would be met, if the frequency of determining "rearrangement status in the NUT gene" (e.g. reflected in the expression level of a NUT fusion gene) decreases during process of therapy/treatment. Non-limiting examples of schemes of determining "rearrangement status in the NUT gene" (e.g. reflected in the expression level of a NUT fusion gene)" in accordance with this invention are provided herein.

It is of note that the skilled person is readily in the position to elect (a) suitable control patient(s)/subject(s) and the point (s) in time when the (reference) "rearrangement status in the NUT gene" (e.g. reflected in the expression level of a NUT fusion gene) are determined for each individual setup of the means, methods and uses provided.

In one embodiment, the present invention relates to the use of a (transgenic) cell or a (transgenic) non-human animal having at least one rearrangement in the NUT gene as defined herein for screening and/or validation of a medicament or drug for the treatment of NMC.

The term "cell" as used in this context may also comprise a plurality of cells as well as cells comprised in a tissue. A cell to be used may, for example, be a primary tumor cell. The tumor cell or cell to be used in the screening or validation method may be obtained from samples from a (transgenic) non-human animal (suspected of) suffering from NMC. The tumor cell or cell may also be obtained from patient samples (e.g. biopsies), in particular a biopsy/biopsies from a patient/ subject (suspected of) suffering from NMC. Accordingly, the tumor cell or cell may be a human tumor cell or cell. Again, such a cell to be used in the present screening or validation methods may be comprised in a tissue or tissue sample, like in a sample biopsy.

The used non-human animal or cell may be transgenic or non transgenic. "Transgenic" in this context particularly means that the at least one "rearrangement in the NUT gene" as defined herein may have been introduced by biotechnolgogical means and methods. For example, if a CDK9- inhibitor is to be screened and/or validated, it is preferred that the "rearrangement status in the NUT gene" is reflected in the expression of a NUT fusion gene. The herein described "rearrangement in the NUT gene" (in particular rearrangements in the human NUT gene" may also be used in this context. For example, a "rearranged human NUT gene" (i.e. a rearrangement as found and isolated from the human body, e.g. via biopsy from an NMC tumor) may be employed in the generation of a transgenic animal or cell. In this case, the transgenic animal or cell comprises one or more "rearranged human NUT gene(s)" (e.g. a human NUT/BRD4 and/or NUT/BRD3 fusion gene as described herein).

"Transgenic" in this context may also mean that NOTCH3 is (over) expressed, and/or that the NOTCH3-activity in the transgenic non-human animal or a transgenic cell is enhanced. A preferred (transgenic) non-human animal or (transgenic) cell in context of the invention suffers from NMC for the treatment of which the medicament is to be screened and/or validated. For example, if a medicament for NMC is to be screened and/or validated, the (transgenic) non-human animal or (transgenic) cell is particularly intended to suffer from NMC, i.e. to have, for example, at least one rearrangement in the NUT gene, and, optionally overexpression of the NOTCH3 gene.

The term "transgenic non-human animal" or "transgenic cell" as used herein refers to a non-human animal or cell, not being a human, that comprises genetic material different from the genetic material of a corresponding wild-type animal/ cell. "Genetic material" in this context may be any kind of a nucleic acid molecule, or analogues thereof, for example a nucleic acid molecule, or analogues thereof as defined herein. "Different" in this context means additional or fewer genetic material with respect to the genome of the wild-type animal/cell and/or rearranged genetic material, i.e. genetic material present at a different locus of the genome with respect to the genome of the wild-type animal/cell. An overview

of examples of different expression systems to be used for generating transgenic cell/animal is, for instance, contained in Methods in Enzymology 153 (1987), 385-516, in Bitter et al. (Methods in Enzymology 153 (1987), 516-544) and in (Sawers et al.Applied Microbiology and Biotechnology 46 (1996), 1-9), Billman-Jacobe (Current Opinion in Biotechnology 7 (1996), 500-4), Hockney (Trends in Biotechnology 12 (1994), 456-463), Griffiths et al., (Methods in Molecular Biology 75 (1997), 427-440).

In a preferred embodiment, the (transgenic) non-human animal or (transgenic) cell is or is derived from a mammal. Non-limiting examples of the (transgenic) non-human animal or derived (transgenic) cell are selected from the group consisting of a mouse, a rat, a rabbit, a guinea pig and a Drosophila.

Preferably, the (transgenic) cell in accordance with this invention may be an animal cell, for example, a non-human animal cell. However, also human cells are envisaged to be employed as cells in context of the present invention. In a non limiting example, such cell may be an embryonic stem cell (ES cell), particularly a non-human animal ES, like, for example, a mouse or rat ES cell. The (transgenic) cell as described herein, particularly the ES cell, may also be used for generating the (transgenic) non-human animal as described herein. The ES cell technology for generating transgenic animals is well known in the art and for example is described in Pirity (Methods Cell Biol, 1998, 57:279).

Generally, the (transgenic) cell may be a prokaryotic or eukaryotic cell. For example, the (transgenic) cell may be a bacterial, yeast, fungus, plant or animal cell. In general, the transformation or genetically engineering of a cell with a nucleic acid construct or vector can be carried out by standard methods, as for instance described in Sambrook and Russell (2001), Molecular Cloning: A Laboratory Manual, CSH Press, Cold Spring Harbor, NY, USA; Methods in Yeast Genetics, A Laboratory Course Manual, Cold Spring Harbor Laboratory Press, 1990.

The (transgenic) non-human animal or (transgenic) cell as described or defined in context of this invention is particularly useful in methods for screening and/or validation of a medicament for the treatment of cancers as defined and described herein.

These screening methods may, in particular, performed in vivo using, for example, (transgenic) animals as described herein (e.g. rats, mice and the like) and/or animals comprising (a) cell(s), (a) tissue(s) or (a) cell culture(s) characterized the presence of at least one "rearrangement in the NUT gene" (e.g. reflected in the expression level of a NUT fusion gene)". Said (a) cell(s), (a) tissue(s) or (a) cell culture(s) may, for example, be obtained/derived from (a) NMC tumor cell (s)/tumor(s). In particular, said (a) cell(s), (a) tissue(s) or (a) cell culture(s) may be obtained from a subject/patient suffering from NMC. These in vivo screening methods may in particular comprise measuring and determining differences in tumor volume, for example, in the (transgenic) animals described herein above.

Accordingly, the present invention also relates to such a method for screening and/or validation of a medicament (preferably a selective CDK9 inhbitor) for the treatment of NMC. Said method comprising the steps of

a) administering to a (transgenic) non-human animal or (transgenic) cell as defined herein said medicament or drug to be screened/validated;
b) determining in (a sample from) said animal or cell the "rearrangement status in the NUT gene" (e.g. reflected in the expression level of a NUT fusion gene)" in accordance with this invention;
c) comparing the "rearrangement status in the NUT gene" (e.g. reflected in the expression level of a NUT fusion gene)" determined in b) with a reference "rearrangement status in the NUT gene" (e.g. reflected in the expression level of a NUT fusion gene)" determined in (a sample from) a control (transgenic) non-human animal or (transgenic) cell as defined herein to which said medicament to be screened has not been administered; and
d) selecting said medicament when said "rearrangement status in the NUT gene" (e.g. reflected in the expression level of a NUT fusion gene)" determined in b) differs from said reference "rearrangement status in the NUT gene" (e.g. reflected in the expression level of a NUT fusion gene)" determined in c).

The corresponding definitions and descriptions provided above, for example with respect to "therapy/treatment", "efficacy", "NMC" or "susceptibility"/"responsiveness" thereto, "(control) subject/patient", "(transgenic) non-human animal" or "(transgenic) cell", "rearrangement status in the NUT gene "expression level", "reference expression level" etc., apply here, mutatis mutandis. Particularly the relevant definitions and descriptions provided above with respect to "control subject/patient" also apply to the "control (transgenic) non-human animal" or "(transgenic) cell", mutatis mutandis.

In context of this invention, "screening and/or validation of medicaments" means, on the one hand, whether a given set of compounds comprises one or more compound(s) that can function as (a) medicament(s), and/or, on the other hand, whether (a) given compound(s) can function as (a) medicament(s). It is particularly intended that the medicaments to be screened and/or validated in context of this invention are medicaments for the treatment, prevention and/or amelioration of a NMC.

The skilled person is readily in the position to put this embodiment of the present invention into practice. For example, by doing so, the compound(s)/medicament(s) to be screened and/or validated may be administered to the non-human (transgenic) animal or cell described herein, and, afterwards (for example after a certain period of time sufficient to allow a compound to effect on a cancer as described herein), it is analyzed whether the cancer, or a symptom thereof, of said

animal/cell is ameliorated.

The present invention also relates to a kit for carrying out the methods or uses of this invention. In a preferred embodiment, said kit useful for carrying out the methods and uses described herein comprises oligonucleotides or polynucleotides capable of determining the presence of at least one "rearrangement in the NUT gene". For example, said kit may comprise (a) compound(s) required for specifically determining the "rearrangement in the NUT gene" (e.g. reflected in the expression level of a NUT fusion gene). Moreover, the present invention also relates to the use of (a) compound(s) required for specifically determining the a"rearrangement status in the NUT gene" (e.g. reflected in the expression level of a NUT fusion gene) as defined herein for the preparation of a kit for carrying out the methods or uses of this invention. On the basis of the teaching of this invention, the skilled person knows which compound (s) is (are) required for specifically determining the "rearrangement status in the NUT gene". For example, such compound (s) may be (a) "binding molecule(s)", like, for example, (a) "binding molecule(s)" as defined herein-above. Particularly, such compound(s) may be (a) (nucleotide) probe(s), (a) primer(s) (pair(s)), (an) antibody(ies) and/or (an) aptamer(s) specific for at least one marker gene as described herein or for a product thereof. In a preferred embodiment, the kit (to be prepared in context) of this invention is a diagnostic kit.

In a particularly preferred embodiment of the present invention, the kit (to be prepared in context) of this invention or the methods and uses of the invention may further comprise or be provided with (an) instruction manual(s). For example, said instruction manual(s) may guide the skilled person (how) to determine the (reference) "rearrangement status in the NUT gene" or (reference) expression level of a NUT fusion gene", i.e. (how) to diagnose NMC or a susceptibility thereto, (how) to monitor the efficacy of a treatment of said cancer or a susceptibility thereto or (how) to predict the efficacy of a treatment of said cancer or a susceptibility thereto in accordance with the present invention. Particularly, said instruction manual(s) may comprise guidance to use or apply the herein provided methods or uses.

The kit (to be prepared in context) of this invention may further comprise substances/chemicals and/or equipment suitable/ required for carrying out the methods and uses of this invention. For example, such substances/chemicals and/or equipment are solvents, diluents and/or buffers for stabilizing and/or storing (a) compound(s) required for specifically determining "rearrangement status in the NUT gene" (e.g. reflected in the expression level of a NUT fusion gene)".

The following relates to pharmaceutical compositions and drug combinations to be used in accordance with the present invention. As mentioned above, (a) selective CDK9 inhibitor(s) as defined herein may be used for treating, ameliorating and/or preventing NUT midline carcinoma (NMC). Accordingly, also the use of (a) selective CDK9 inhibitor (s) for the preparation of a pharmaceutical composition for the treatment, amelioration and/or prevention of NUT midline carcinoma (NMC) is envisaged in context herein.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a subject and includes: (a) preventing a disease related to an insufficient immune response from occurring in a subject which may be predisposed to the disease; (b) inhibiting the disease, i.e. arresting its development; or (c) relieving the disease, i.e. causing regression of the disease.

A "patient" or "subject" for the purposes of the present invention includes both humans and other animals, particularly mammals, and other organisms. Thus, the methods are applicable to both human therapy and veterinary applications. In the preferred embodiment the patient is a mammal, and in the most preferred embodiment the patient is human.

In accordance with the above, herein described are drug combinations and pharmaceutical compositions comprising at least one selective CDK9 inhibitors, such as (a) compound(s) of general formula (I) as active ingredient together with at least one pharmaceutically acceptable carrier, excipient and/or diluent and optionally together with one or more other anti-tumor agents As used herein the term "drug combination" refers to a combination of at least to pharmaceutically active agents or therapeutic agents with or without further ingredients, carrier, diluents and/or solvents. As used herein the term "pharmaceutical composition" refers to a galenic formulation of at least one pharmaceutically active agent together with at least one further ingredient, carrier, diluent and/or solvent.

Selective CDK9 inhibitors, such as compounds of formula (I) may be administered as the sole pharmaceutical agent or in combination with one or more additional therapeutic agents, wherein the drug combination causes no unacceptable adverse effects. This combination therapy includes administration of a single pharmaceutical dosage formulation, which contains a selective CDK9 inhibitorand one or more additional therapeutic agents in form of a single pharmaceutical composition, as well as administration of a selective CDK9 inhibitorand each additional therapeutic agent in its own separate pharmaceutical dosage formulation, i.e. in its own separate pharmaceutical composition. For example, a selective CDK9 inhibitor and a therapeutic agent may be administered to the patient together in a single oral dosage composition such as a tablet or capsule, or each agent may be administered in separate pharmaceutical compositions.

Where separate pharmaceutical compositions are used, a selective CDK9 inhibitor and one or more additional therapeutic agents may be administered at essentially the same time (e.g., concurrently) or at separately staggered times (e.g., sequentially).

In particular, the selective CDK9 inhibitos to be used in accordance withrthe present invention may be used in fixed or separate pharmaceutical compositions with other anti-tumor agents such as alkylating agents, anti-metabolites, plant-derived anti-tumor agents, hormonal therapy agents, topoisomerase inhibitors, camptothecin derivatives, kinase inhibitors, targeted drugs, antibodies, interferons and/or biological response modifiers, anti-angiogenic compounds, and other anti-tumor drugs. In this regard, the following is a non-limiting list of examples of secondary agents that may be used in combination with the selective CDK9 inhibitors:

- Alkylating agents include, but are not limited to, nitrogen mustard N-oxide, cyclophosphamide, ifosfamide, thiotepa, ranimustine, nimustine, temozolomide, altretamine, apaziquone, brostallicin, bendamustine, carmustine, estramustine, fotemustine, glufosfamide, mafosfamide, and mitolactol; platinum-coordinated alkylating compounds include, but are not limited to, cisplatin, carboplatin, eptaplatin, lobaplatin, nedaplatin, oxaliplatin, and satraplatin;
- Anti-metabolites include, but are not limited to, methotrexate, 6-mercaptopurine riboside, mercaptopurine, 5-fluorouracil alone or in combination with leucovorin, tegafur, doxifluridine, carmofur, cytarabine, cytarabine ocfosfate, enocitabine, gemcitabine, fludarabin, 5-azacitidine, capecitabine, cladribine, clofarabine, decitabine, eflornithine, ethynylcytidine, cytosine arabinoside, hydroxyurea, melphalan, nelarabine, nolatrexed, ocfosfite, disodium premetrexed, pentostatin, pelitrexol, raltitrexed, triapine, trimetrexate, vidarabine, vincristine, and vinorelbine;
- Hormonal therapy agents include, but are not limited to, exemestane, Lupron, anastrozole, doxercalciferol, fadrozole, formestane, 11-beta hydroxysteroid dehydrogenase 1 inhibitors, 17-alpha hydroxylase/17,20 lyase inhibitors such as abiraterone acetate, 5-alpha reductase inhibitors such as finasteride and epristeride, anti-estrogens such as tamoxifen citrate and fulvestrant Trelstar, toremifene, raloxifene, lasofoxifene, letrozole, anti-androgens such as bicalutamide, flutamide, mifepristone, nilutamide, Casodex, and anti-progesterones and combinations thereof;
- Plant-derived anti-tumor substances include, e.g., those selected from mitotic inhibitors, for example epothilones such as sagopilone, ixabepilone and epothilone B, vinblastine, vinflunine, docetaxel, and paclitaxel;
- Cytotoxic topoisomerase inhibiting agents include, but are not limited to, aclarubicin, doxorubicin, amonafide, belotecan, camptothecin, 10-hydroxycamptothecin, 9-aminocamptothecin, diflomotecan, irinotecan, topotecan, edotecarin, epimbicin, etoposide, exatecan, gimatecan, lurtotecan, mitoxantrone, pirambicin, pixantrone, rubitecan, sobuzoxane, tafluposide, and combinations thereof;
- Immunologicals include interferons such as interferon alpha, interferon alpha-2a, interferon alpha-2b, interferon beta, interferon gamma-1a and interferon gamma-n1, and other immune enhancing agents such as L19-IL2 and other IL2 derivatives, filgrastim, lentinan, sizofilan, TheraCys, ubenimex, aldesleukin, alemtuzumab, BAM-002, dacarbazine, daclizumab, denileukin, gemtuzumab, ozogamicin, ibritumomab, imiquimod, lenograstim, lentinan, melanoma vaccine (Corixa), molgramostim, sargramostim, tasonermin, tecleukin, thymalasin, tositumomab, Vimlizin, epratuzumab, mitumomab, oregovomab, pemtumomab, and Provenge; Merial melanoma vaccine;
- Biological response modifiers are agents that modify defense mechanisms of living organisms or biological responses such as survival, growth or differentiation of tissue cells to direct them to have anti-tumor activity; such agents include, *e.g.,* krestin, lentinan, sizofiran, picibanil, ProMune, and ubenimex;
- Anti-angiogenic compounds include, but are not limited to, acitretin, aflibercept, angiostatin, aplidine, asentar, axitinib, recentin, bevacizumab, brivanib alaninat, cilengtide, combretastatin, DAST, endostatin, fenretinide, halofuginone, pazopanib, ranibizumab, rebimastat, removab, revlimid, sorafenib, vatalanib, squalamine, sunitinib, telatinib, thalidomide, ukrain, and vitaxin;
- Antibodies include, but are not limited to, trastuzumab, cetuximab, bevacizumab, rituximab, ticilimumab, ipilimumab, lumiliximab, catumaxomab, atacicept, oregovomab, and alemtuzumab;
- VEGF inhibitors such as, *e.g.,* sorafenib, DAST, bevacizumab, sunitinib, recentin, axitinib, aflibercept, telatinib, brivanib alaninate, vatalanib, pazopanib, and ranibizumab; Palladia
- EGFR (HER1) inhibitors such as, *e.g.,* cetuximab, panitumumab, vectibix, gefitinib, erlotinib, and Zactima;
- HER2 inhibitors such as, *e.g.,* lapatinib, tratuzumab, and pertuzumab;
- mTOR inhibitors such as, *e.g.,* temsirolimus, sirolimus/Rapamycin, and everolimus;
- c-Met inhibitors;
- PI3K and AKT inhibitors;
- CDK inhibitors;
- Spindle assembly checkpoints inhibitors and targeted anti-mitotic agents such as PLK inhibitors, Aurora inhibitors (*e.g.* Hesperadin), checkpoint kinase inhibitors, and KSP inhibitors;
- HDAC inhibitors such as, *e.g.,* panobinostat, vorinostat, MS275, belinostat, and LBH589;
- HSP90 and HSP70 inhibitors;
- Proteasome inhibitors such as bortezomib and carfilzomib;
- Serine/threonine kinase inhibitors including MEK inhibitors (such as e.g. RDEA 119) and Raf inhibitors such as sorafenib;
- Farnesyl transferase inhibitors such as, *e.g.,* tipifarnib;

- Tyrosine kinase inhibitors including, *e.g.,* dasatinib, nilotibib, DAST, bosutinib, sorafenib, bevacizumab, sunitinib, AZD2171, axitinib, aflibercept, telatinib, imatinib mesylate, brivanib alaninate, pazopanib, ranibizumab, vatalanib, cetuximab, panitumumab, vectibix, gefitinib, erlotinib, lapatinib, tratuzumab, pertuzumab, and c-Kit inhibitors; Palladia, masitinib
- Vitamin D receptor agonists;
- Bcl-2 protein inhibitors such as obatoclax, oblimersen sodium, and gossypol;
- Cluster of differentiation 20 receptor antagonists such as, *e.g.,* rituximab;
- Ribonucleotide reductase inhibitors such as, *e.g.,* gemcitabine;
- Tumor necrosis apoptosis inducing ligand receptor 1 agonists such as, e.*g.,* mapatumumab;
- 5-Hydroxytryptamine receptor antagonists such as, *e.g.,* rEV598, xaliprode, palonosetron hydrochloride, granisetron, Zindol, and AB-1001;
- Integrin inhibitors including alpha5-betal integrin inhibitors such as, *e.g.,* E7820, JSM 6425, volociximab, and endostatin;
- Androgen receptor antagonists including, *e.g.,* nandrolone decanoate, fluoxymesterone, Android, Prost-aid, andromustine, bicalutamide, flutamide, apo-cyproterone, apo-flutamide, chlormadinone acetate, Androcur, Tabi, cyproterone acetate, and nilutamide;
- Aromatase inhibitors such as, *e.g.,* anastrozole, letrozole, testolactone, exemestane, amino-glutethimide, and formestane;
- Matrix metalloproteinase inhibitors;
- Other anti-cancer agents including, *e.g.,* alitretinoin, ampligen, atrasentan bexarotene, bortezomib, bosentan, calcitriol, exisulind, fotemustine, ibandronic acid, miltefosine, mitoxantrone, 1-asparaginase, procarbazine, dacarbazine, hydroxycarbamide, pegaspargase, pentostatin, tazaroten, velcade, gallium nitrate, canfosfamide, darinaparsin, and tretinoin.

The selective CDK9 inhibitors may also be employed in cancer treatment in conjunction with radiation therapy and/or surgical intervention.

Furthermore, the selective CDK9 inhibitorsmay be utilized, as such or in compositions, in research and diagnostic, or as analytical reference standards, and the like, which are well known in the art.

Thus, also envisaged herein is the use of drug combinations comprising at least one selective CDK9 inhibitor such as compounds according to general formula (I) and/or pharmaceutically acceptable salts thereof together with at least one anti-retroviral drug, especially at least one of the drugs mentioned above.

The pharmaceutical compositions to be used in accordance with the present invention comprise at least one selective CDK9 inhibitor as an active ingredient together with at least one pharmaceutically acceptable (i.e. non-toxic) carrier, excipient and/or diluent. The pharmaceutical compositions can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the the pharmaceutical preparations may be used for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one selective CDK9 inhibitor and/or a pharmaceutical acceptable salt thereof as active ingredient.

The pharmaceutical compositions to be used in accordance with the present invention containing at least one selective CDK9 inhibitor and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the selective CDK9 inhibitors (such as 2,4,6-disubstituted pyrimdine derivative according to the general formula (I) or analogues compound thereof) or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrates include starch, methylcellulose, guar gum, and the like. Sweetening and flavoring agents as well as preservatives may also be included,

where appropriate. The disintegrants, diluents, lubricants, binders etc. are discussed in more detail below.

Moreover, the pharmaceutical compositions may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The selective CDK9 inhbitors according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow

characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %. Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

Also disclosed herein is a method for treating, preventing or ameliorating NUT midline carcinoma (NMC) comprising the administration of a selective CDK9 inhibitor as defined herein to a subject in need of such a treatment, prevention or amelioration. Preferably, the subject is a human.

The present invention is further described by reference to the following non-limiting figures and examples.

The Figures show:

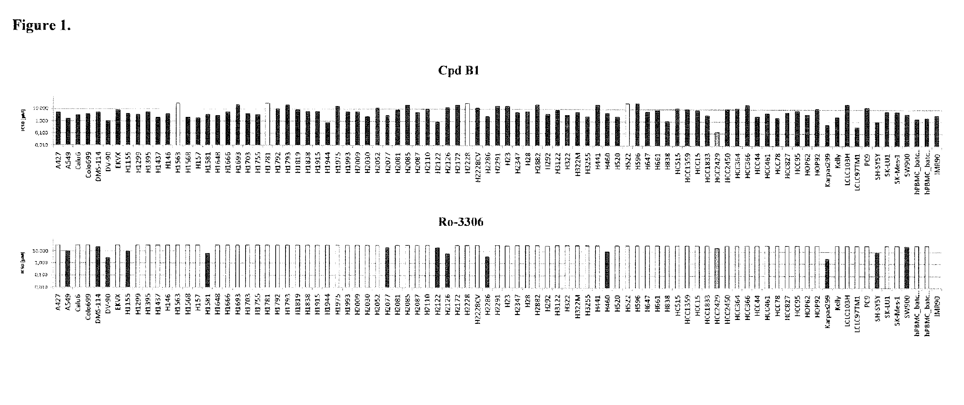

**Figure 1. Proliferation inhibition profile.**

Figure 1 shows a proliferation inhibition profile of a potent specific inhibitor (Cpd B1) and a selective CDK1 inhibitor (Ro-3306).

Proliferation assays were performed as described below under materials and methods. Three compounds (Cpd B1 and Ro-3306) were applied at concentrations between 30 and 0,0137 μM. After 72h incubation with compounds ATP content/proliferation was determined employing CTG (Promega). Relative proliferation values (compared to vehicle control) were used to calculate $IC_{50}$ values (Excel fit; algorithm #205). $IC_{50}$s of the respective compound (Y-axis; logarithmic scale) on proliferation of various cell lines (X-axis) are depicted in black bars.White bars indicate that an $IC_{50}$ could not be determined due to too low activity and therefore was higher than the higest applied concentration in the assays (30 μM). $IC_{50}$s of compounds on CDK9 inhibitor sensitive cell line HCC2429 are presented in grey bars. The $IC_{50}$s of Cpd B1 was determined at 0,151 μM. The specific CDK1 inhibitor Ro-3306 does not affect said cell line potently ($IC_{50}$ initially higher 10μM).

**Figure 2. CDK9 inhibition.**

Figure 2 shows CDK9 inhibition by and selectivity of described compounds. The figure summarizes $IC_{50}$ values of 12 compounds on CDK1/CyclinB1, CDK2/CyclinA, CDK4/CyclinD1, CDK6/CyclinD3, CDK7/CyclinH/Mat1 and CDK9/CyclinT1 activity (methods are described below).

Compounds were either already described (SNS-032: Piperidine-4-carboxylic acid [5-(5-tert-butyl-oxazol-2-ylmethylsulfanyl)-thiazol-2-yl]-amide; Misra RN et al. J Med Chem. 2004, 47(7):1719-28; flavopiridol: 2-(2-Chloro-phenyl)-5,7-dihydroxy-8-(3-hydroxy-1-methyl-piperidin-4-yl)-chromen-4-one, Lloyd R Kelland. Expert Opinion on Investigational Drugs. 2000, 9(12):2903-2911; AX35427: N-(5-((6-(3-aminophenyl)pyrimidin-4-yl)amino)-2-methylphenyl) propane-1-sulfonamide, 848637-29-6P in WO 2005026129; R-547: [4-amino-2-(1-methanesulfonylpiperidin-4-ylamino)pyrimidin-5-yl]-(2,3-difluoro-6-methoxyphenyl)methanone, DePinto W et al., Mol Cancer Ther 2006, 5: 2644-2658;

3-[[6-(2-methoxyphenyl)-4-pyrimidinyl]amino]-Benzenemethanesulfonamide compound 1073485-20-7P in WO 2008132138.

AX38679: 3-((6-(2-methoxyphenyl)pyrimidin-4-yl)amino)benzenesulfonamide, 848637-62-7P in WO 2005026129; PHA767491: 1,5,6,7-tetrahydro-2-(4-pyridinyl)-4H-pyrrolo[3,2-c]pyridin-4-one, Montagnoli, A. Nat Chem Biol 2008, 4(6) 357-365; BS181: N5-(6-aminohexyl)-3-(1-methylethyl)-N7-(phenylmethyl), Ali S et al. Cancer Res. 2009, 69 (15):6208-15) or mentioned above (Cpd 24, Cpd C1, Cpd B1 and Cpd B2).

**Figure 3. CDK9 inhibitors 1073485-20-7P and Cpd B1.**

Figure 3 shows general kinase selectivity of two typical selective CDK9 inhibitors 1073485-20-7P and Cpd B1. Employing enzymatic in vitro assays on the activity of 23 different kinases incubated with 10 μM Cpd B1 or DMSO as control resulted in the depicted residual activities (compared to the vehicle control). As a result it is demonstrated that Cpd B1 inhibits only CDK9 with high potency and is a selective CDK9 kinase inhibitor in accordance with the present invention.

**Figure 4.**

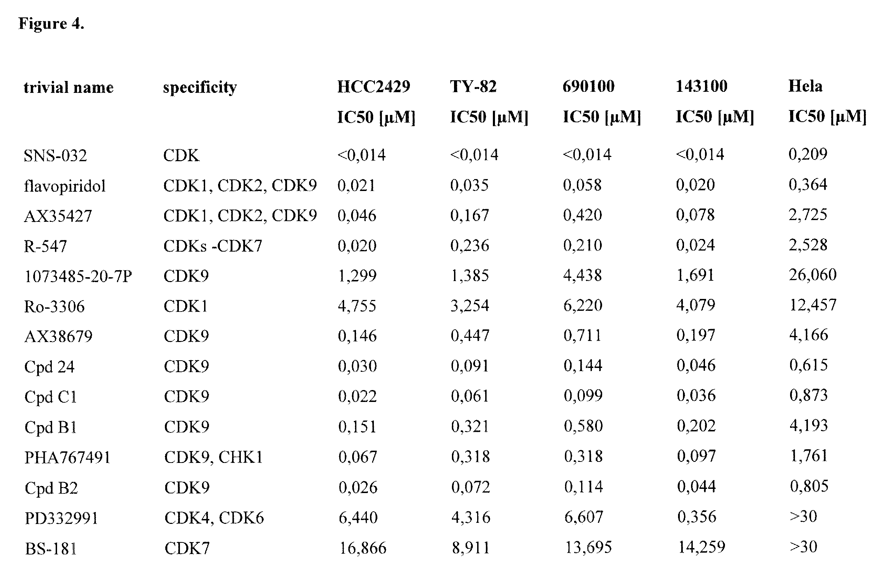

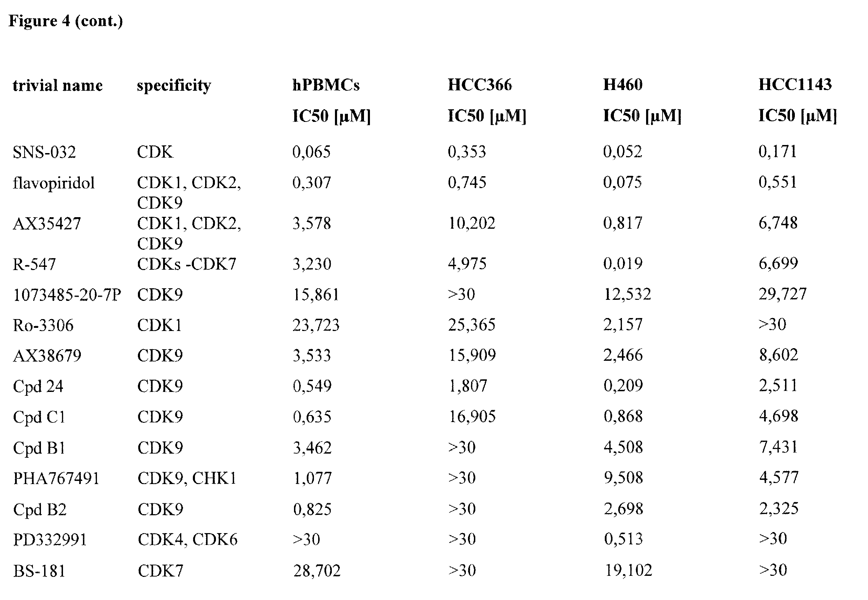

Figure 4 displays proliferation assay results of selected CDK9 inhibitors as well as other CDK standard inhibitors on various Brd4-Nut mutated as well as wild type cell lines. The proliferation results are presented as $IC_{50}$ in μM. First, the anti-proliferative effect of CDK9 inhibitors is much more pronounced on BrdNut mutated cell lines (=sensitive cell lines). Second, within the table it is clearly visible, that the anti-proliferative effect of the compounds is directly correlated to the Even with relatively weak CDK9 Inhibitors like 1073485-20-7P proliferation inhibition is much more potent in mutated, sensitive cell lines (HCC2429, TY-82, 690100, 143100) than in normal wilde type cell lines (Hela, HCC366, H460 HCC1143 and hPBMCs).

**Figure 5.**

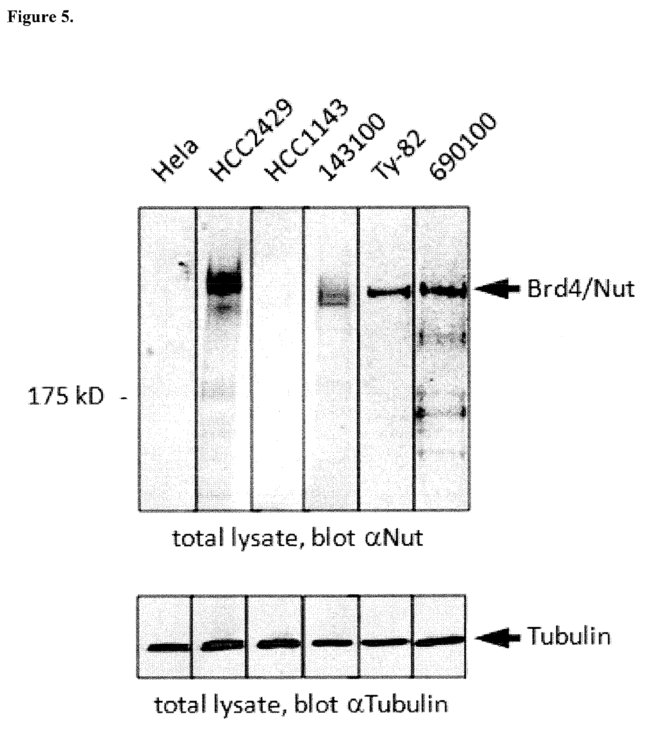

Figure 5 shows the expression of BrdNut fusion proteins in various cell lines (Hela, HCC2429, Ty-82, 143100, 69100 and HCC1143). Cell lysates were analyzed as described in materials and methods. Fusion proteins were detected as high molecular weight bands employing an antibody directed against Nut proteins. As a loading control same lysates were analyzed for their tubulin content.

The Examples illustrate the invention.

**Example 1:**

**Material and Methods**

***Material***

NSCLC cells (A427, A549, Calu6, Colo699, DMS-114, DV-90, EKVX, H1155, H1299, H1395, H1437, H146, H1563, H1568, H157, H1581, H1648, H1666, H1693, H1703, H1755, H1781, H1792, H1793, H1819, H1838, H1915, H1944, H1975, H1993, H2009, H2030, H2052, H2077, H2081, H2085, H2087, H2110, H2122, H2126, H2172, H2228, H2228CV, H2286, H2291, H23, H2347, H28, H2882, H292, H3122, H322, H322M, H3255, H441, H460, H520, H522, H596, H647, H661, H838, HC515, HCC1359, HCC15, HCC1143, HCC1833, HCC2429, HCC2450, HCC364, HCC366, HCC44, HCC461, HCC78, HCC827, HCC95, HOP62, HOP92, Karpas299, Kelly, LCLC103H, LCLC97TM1, PC9, SH-SY5Y, SK-LU1, SK-Mes-1, SW900, 690100 and 143100) are described in PCT patent application WO 2010/020618 and WO 2010/020619. TY-82 cells were purchased from Health Science Research Resource Bank (Osaka, Japan). Peripheral blood mononuclear cells (hPBMCs) were provided by the Blutspendedienst Hagen (DRK West, Hagen). All other cell lines (e.g. Hela cells) have been purchased from LGC Standards (ATCC, Wesel) or the DSMZ (Deutsche Sammlung von Mikroorganismen und Zellkulturen, Braunschweig).

***Cell Viability Assays***

Cell lines were maintained in RPMI 1640 cell culture medium + glutamine (PAN Biotech GmbH, Aidenbach, Germany; order no. P04-22100; P04-05500) supplemented with 10% fetal calf serum "Gold" (PAA Laboratories GmbH, Pasching, Austria; order no. A15-151) and grown in a humidified atmosphere at 37 °C, 5 % CO2. As a first step, an optimal cell density for each cell line was determined to guarantee linearity. For viability assays with compounds, cells were then seeded at a density of 200 to 1000 per well in 25 μl in 384-well plates (Greiner Bio-One, Frickenhausen, Germany; order no. 781080). After overnight incubation at 37 °C/5 % CO2, 25nl or 75nl compounds were added to each sample well by using BIOMEK $FX^P$ Laboratory Automation Workstation (Beckman Coulter, USA). Wells with cells and 0.1 % or 0,3 % DMSO in culture medium were used as positive controls, wells with cells and 10 μM staurosporine (Selleck Chemicals, Huston, USA) in culture medium were used as negative controls. Upon incubation with compounds for 72 h at 37 °C/5 % CO2 25 μl Cell Titer Glo reagent (Promega, Madison, USA, order no. G7573; 1:2 diluted with cell culture medium) was added to each well to determine cell viability. 384well-plates were placed for 2 min on an orbital microplate shakes and incubated for further 10 min at room temperature resulting in a stabilization of light signal. Luminescence was measured by Envision Plate Reader (Perkin Elmer, USA). IC50 values were calculate with the software Excel Fit (IDBS, Guildford, UK) from 3-fold dilution series comprising 8 concentrations in duplicates.

***Immunoblot analysis***

Cellular proteins were solubilized in CLB-A buffer (Zeptosens, Switzerland). After addition of 3xLämmli buffer (30 % v/v glycerol, 6 % SDS, 150 mM Tris/HCl [pH 6,8], 0,3 % w/v bromophenol blue, 300 mM DTT) proteins were denatured by incubation at 95 °C for 5 min. Thereon, proteins were separated on SDS-FAGE and transferred to PVDF membranes (Immobilon®, Milipore, Schwalbach). After blocking, bound proteins were incubated with antibodies against □-tubulin (T59840R, Biozol; clone B-5-1-2, Sigma-Aldrich) or Nut (C52B1, Cell Signaling, Frankfurt a. M.) diluted in blocking buffer (Li-Cor biosciences, Bad Homburg, Germany).

***Measurement of half maximal inhibitory concentration to CDKs***

This protocol describes how the Lance Ultra KinaSelect Assay was performed to determine half maximal inhibitory concentration ($IC_{50}$) of compounds of general formula (I) and CDK/Cyclin complexes. The principle behind this enzymatic assay is based upon the phosphorylation of the Ulight-Peptide Substrat. It is detected by using a specific EU-labeled anti-phospho peptide antibody. The binding of the Eu labeled anti-phospho peptide antibody to the phosphorylated U*Light* labeled peptide gives rise to a FRET-signal. Binding of an inhibitor to the kinase prevents phosphorylation of the U*light*-MBP Substrat, resulting in a loss of FRET.

**Table 3** Reagents, stock concentrations and final assay concentrations

| Kinase | Supplier | Kinase-conc. [nM] | ATP-conc.[μm] | Substrat | Supplier | Substrat-conc. [nM] | Antibody | Supplier | Antibody-conc. [nM] |
|---|---|---|---|---|---|---|---|---|---|
| **CDK1/CyclinB1 (91 kDa)** | Carna | 2 | 20 | Ulight MBP | Perkin Elmer | 50 | Eu-anti-P-MBP | Perkin Elmer | 0,25 |
| **CDK2/CyclinA [135 kDa)** | Proqinase | 5 | 3 | Ulight MBP | Perkin Elmer | 50 | Eu-anti-P-MBP | Perkin Elmer | 0,25 |
| **CDK4/CyclinD1 (123 kDa)** | Invitrogen | 10 | 90 | Ulight MBP | Perkin Elmer | 50 | Eu-anti-P-MBP | Perkin Elmer | 0,25 |
| **CDK6/cyclinD3 (123 kDa)** | Carna | 5 | 55 | Ulight MBP | Perkin Elmer | 50 | Eu-anti-P-MBP | Perkin Elmer | 0,025 |
| **CDK7/CyclinH/ MAT1 (126 kDa)** | Invitrogen | 10 | 25 | Ulight MBP | Perkin Elmer | 50 | Eu-anti-P-MBP | Perkin Elmer | 0,25 |
| **CDK9/cyclinT1 (132 kDa)** | Invitrogen | 10 | 25 | Ulight MBP | Perkin Elmer | 50 | Eu-anti-P-MBP | Perkin Elmer | 0,25 |
| **CDK9/CyclinK (92 kDa)** | Invitrogen | 10 | 125 | Ulight MBP | Perkin Elmer | 50 | Eu-anti-P-MBP | Perkin Elmer | 0,25 |

The selective CDK9 inhibitors described herein above (see also Table 1 and Table 2) were diluted from a 10 mM DMSO stock solution 1:10 in a total volume of 15 µl DMSO. This compound predilution was then serial diluted 1:3 over 8 steps in DMSO and briefly spun down. Each compound solution was now diluted 1:20 in Enzymatic Buffer (HEPES: 50 mM, pH: 7.5; $MgCl_2$: 10 mM; EGTA: 1 mM; DTT: 2mM; Tween-20: 0.01%), mixed thoroughly and spun down. For every sample, 2 µl of the diluted compound were mixed with 6 µl CDK/Cyclin/Substrat solution and 2 µl ATP solution in a well of a small volume 384 well plate (Corning Incorporated, Corning, NY, USA; order no. 3673). The CDK/Cyclin was diluted to the appropriate concentration (see Table 3 and the ATP concentration was adjusted to its $IC_{50}$ concentration for the CDK/Cyclin, which was 3 µM for CDK2/ Cyclic A, 20 µM for CDK1/ Cyclin B1, 25 µM CDK7/Cyclin H and CDK9/Cyclin T1, 55 µM CDK6/Cyclin D3, 90 µM CDK4/Cyclin D1 and 125 µM for CDK9/Cyclin K. For negative controls, in each well 2 µl of DMSO solution (1% final DMSO assay concentration) was mixed with 6 µl substrat solution (50 nM Ulight MBP final assay concentration) and 2 µl ATP solution (appropriate final concentration see Table 3. For positive controls, in each well 2 µl of DMSO solution (1% final DMSO assay concentration) was mixed with 6 µl CDK/Cyclin/ Substrat (appropriate final concentration see Table 3 and 2 pl Tracer ATP solution (appropriate final concentration see Table 3. Positive and negative controls were calculated from at least 8 different sample wells. The 384 well plates were mixed in a Teleshaker plate mixer (Beckman Coulter, Brea, CA, USA) at 2000 rpm for 40 sec, and incubated for 1 h at room temperature. Before reading, 10 µl the detection buffer (Lance Detection Buffer 1X ; EDTA: 20nM; Eu-Anti-P-MBP: see Table 3 was added. The FRET signal was measured at 340 nm excitation, 665 nm and 615 nm emission (for the kinase tracer and LanthaScreen Eu-AB, respectively) with an Envision spectrophotometer (Perkin Elmer, Waltham, MA, USA) with 50 µs delay and 300 µs integration time. $IC_{50}$ values were determined from the sigmoidal dose response curves with the software Quattro Workflow (Quattro GmbH, Munich, Germany).

***Other Kinase assays:***

A radiometric protein kinase assay (33PanQinase® Activity Assay) was used for measuring the kinase activity of the 333 protein kinases. All kinase assays were performed in 96-well FlashPlatesTM from Perkin Elmer (Boston, MA, USA) in a 50 µl reaction volume. The reaction cocktail was pipetted in 4 steps in the following order:

- 10 µl of non-radioactive ATP solution (in H2O)
- 25 µl of assay buffer/ [γ-33P]-ATP mixture
- 5 µl of test sample in 10% DMSO
- 10 µl of enzyme/substrate mixture

The assay for all enzymes contained 70 mM HEPES-NaOH, pH 7.5, 3 mM MgC12, 3 mM MnC12, 3 µM Na-orthovanadate, 1.2 mM DTT, ATP/[γ-33P]-ATP (variable amounts, corresponding to the apparent ATP-Km of the respective kinase, see Table 4 below / approx. 8 x 1005 cpm per well), protein kinase (variable amounts; see Table 4), and substrate (variable amounts; see Table 4). All protein kinases provided by ProQinase were expressed in Sf9 insect cells or in E.coli as recombinant GST-fusion proteins or His-tagged proteins. All kinases were produced from human cDNAs. Kinases were purified by affinity chromatography using either GSH-agarose or Ni-NTA-agarose. The purity of the protein kinases was examined by SDS-PAGE/Coomassie staining. The identity of the protein kinases was checked by mass spectroscopy. The concentrations of enzymes and substrates used for the assays are shown in Table 4 below.
The reaction cocktails were incubated at 30° C for 60 minutes. The reaction was stopped with 50 µl of 2 % (v/v) H3PO4, plates were aspirated and washed two times with 200 µl 0.9 % (w/v) NaCl. All assays were performed with a Beckman-Coulter Biomek 2000/SL robotic system. Incorporation of 33Pi (counting of "cpm") was determined with a microplate scintillation counter (Microbeta, Wallac).

Table 4: Conditions of additional kinase assays

| **Kinase** | | | **Kinase Conc.** | | **ATP Conc.** | **Substrate** | | |
|---|---|---|---|---|---|---|---|---|
| **Name** | **PQ Lot** | **External Vendor Lot** | **ng/50µl** | **nM*** | **µM** | **Name** | **Lot** | **µg/50µl** |
| ABL1wt | 5 | | 25 | 6,6 | 0,3 | Poly(Ala,Glu,Lys, Tyr)6:2:5:1 | SIG_53H5516 | 0,125 |
| AKT1 | 7 | | 25 | 6,4 | 3 | GSK3(14-27) | 11 | 2 |
| Aurora-A | 4 | | 50 | 13,1 | 10 | tetra(LRRWSLG) | 6 | 0,5 |
| CDK9/CycT | 4 | | 15 | 1,9 | 1 | RBER-CHKtide | 25 | 1 |

(continued)

| Kinase | | | Kinase Conc. | | ATP Conc. | Substrate | | |
|---|---|---|---|---|---|---|---|---|
| Name | PQ Lot | External Vendor Lot | ng/50μl | nM* | μM | Name | Lot | μg/50μl |
| CK2-alpha1 | 3 | | 20 | 5,6 | 0,1 | Casein | SIG_83K7430 | 1 |
| EGF-R wt | 17 | | 20 | 4,5 | 0,3 | Poly(Glu,Tyr)4:1 | SIG_20K5903 | 0,125 |
| EPHB4 | 7 | | 10 | 2,6 | 1 | Poly(Glu,Tyr)4:1 | SIG_20K5903 | 0,125 |
| FGF-R1 | 1 | INV_31517 | 3 | 1,2 | 1 | Poly(Glu,Tyr)4:1 | SIG_20K5903 | 0,125 |
| FLT3 wt | 11 | | 25 | 6,1 | 3 | Poly(Ala,Glu,Lys, Tyr)6:2:5:1 | SIG_53H5516 | 0,125 |
| GSK3-beta | 3 | | 50 | 13,1 | 0,3 | RBER-CHKtide | 24 | 1 |
| IGF1-R | 12 | | 10 | 2,6 | 1 | Poly(Glu,Tyr)4:1 | SIG_20K5903 | 0,125 |
| IKK-beta | 8 | | 50 | 8,3 | 0,3 | RBER-CHKtide | 24 | 2 |
| JNK1 | 5 | | 5 | 2,3 | 0,3 | ATF2 | 11 | 2 |
| LCK | 3 | | 20 | 4,3 | 0,3 | Poly(Glu,Tyr)4:1 | SIG_20K5903 | 0,125 |
| MAPKAPK3 | 1 | | 10 | 4,6 | 0,1 | tetra(LRRWSLG) | 6 | 0,25 |
| MEK1 | 2 | 50 | 23 | 3 | | ERK2 K54R (kinase-dead) | 6 | 2 |
| MET wt | 12 | | 20 | 5,1 | 0,3 | Poly(Ala,Glu,Lys, Tyr)6:2:5:1 | SIG_53H5516 | 0,125 |
| PDGFR-beta | 13 | | 50 | 11,4 | 0,3 | Poly(Ala,Glu,L: ys,Tyr)6:2:5:1 | SIG_53H5516 | 0,125 |
| PLK1 | 13 | | 15 | 3,0 | 1 | RBER-CHKtide | 25 | 2 |
| p38-alpha | 5 | | 10 | 4,8 | 1 | ATF2 | 10 | 2 |
| ROCK2 | 2 | | 5 | 1,1 | 0,3 | S6-Peptide | 6 | 1 |
| TGFB-R1 | 3 | | 5 | 1,6 | 1 | GSK3(14-27) | 9 | 1 |
| VEGF-R2 | 15 | | 25 | 5,7 | 1 | Poly(Glu,Tyr)4:1 | SIG_20K5903 | 0,125 |

**Results**

Within a screening approach to profile more than 1600 kinase inhibitors and other compounds on proliferation of 86 cell lines we identified HCC2429 cells to be sensitive for CDK9 inhibitors (specific as well as unspecific). This initial finding was verified by dose response experiments (see figure 1). In these experiments selective CDK9 inhibitors (Cpd B1) potently affected proliferation of HCC2429 cells whereas a specific CDK1 inhibitor (Ro-3306) did not show comparable effects (figure 1). HCC2429 cells overexpress Notch3 and are known to contain a t(15, 19) translocation. The later one results in the expression of a Brd4/Nut fusion protein. We confirmed the selectivity of a set of additional compounds by in vitro kinase assays (figure 2). To this end we performed assays for CDKs as well as for other kinases (ABL1 wt, AKT1, Aurora-A, CDK1/CycA, CDK1/CycE, CDK2/CycA, CDK3/CycE, CDK4/CycD1, CDK6/CycD1, CDK7/CycH/MAT1, CDK9/CycT, CK2-alpha1, EGF-R wt, EPHB4, FGF-R1 wt, FLT3 wt, GSK3-beta, IGFI-R, IKK-beta, JNK1, LCK, MAPKAPK3, MEK1 wt, MET, PDGFR-beta, PLK1, p38-alpha, ROCK2, TGFB-R1, VEGF-R2). Alltogether these experiments corroborate the enormous selectivity of the pyridine, triazine and pyrimidine compounds.Profiling of selected CDK9 inhibitors on cells bearing a similar t(15, 19) translocation (Ty-82, 690100, 143100 together with HCC2429) or control cells (HCC1143, H3122, PC9, A549, Hela, H460 and hPBMCs) confirmed that this rearrangement sensitizes for CDK9 inhibition (figure 4), Interestingly, overexpression of Notch3 did not result in sensitization as shown by Notch3 overexpressing cell lines HCC1143 and others (H1793, H1819, H441 and H647; data not shown). The expression of the fusion gene was confirmed in said cells by western blot analysis (figure 5).

The present invention refers to the following nucleotide and amino acid sequences:

The sequences provided herein are available in the NCBI database and can be retrieved from www.ncbi.nlm.nih.gov/sites/entrez?db=gene; Theses sequences also relate to annotated and modified sequences. The present invention also provides techniques and methods wherein homologous sequences, and also genetic allelic variants and the like of the concise sequences provided herein are used. Preferably, such "variants" are genetic variants.

SEQ ID No. 1:
Nucleotide sequence encoding homo sapiens nut gene (alias Homo sapiens chromosome 15 open reading frame 55 (C15orf55); accession number NM_175741.1):

```
  1 gagttccgta ttctagttct gtgtgatctg atctttacct tcccttcctt ggatccctgt
 61 gcacctactg gagccaggtt actctgggtc ctggacctga ctgcctcatt ctggaggctt
121 ccagacagcc acagttagtg cccaaacctg agaggatggc ttcagatgga gcatctgcat
181 tgccgggacc ggatatgagc atgaaaccta gtgccgccct gtctccatcc cctgcacttc
```

```
 241 cctttctccc accaacttct gacccaccag accacccacc cagggagcca cctccacagc
 301 ccatcatgcc ttcagtattc tctccagaca accctctgat gctctctgct ttccccagct
 361 cactgttggt gacaggggac gggggccctt gcctcagtgg ggctggggct ggcaaggtca
 421 ttgtcaaagt caagacagaa ggggggtcag ctgagccctc tcaaactcag aactttatcc
 481 ttactcagac tgccctcaat tcgactgccc cgggcactcc ctgtggaggc cttgagggtc
 541 ctgcacctcc atttgtgaca gcatctaatg tgaagaccat tctgccctct aaggctgttg
 601 gtgtcagcca ggagggtcct ccaggccttc cgcctcagcc tccaccacca gttgctcaac
 661 tggtccccat tgtgcccctg gaaaaagctt ggccagggcc acatgggaca accggggaag
 721 gaggtcctgt ggccactcta tccaagcctt ccctaggtga ccgctccaaa atttccaagg
 781 acgtttatga gaacttccgt cagtggcagc gttacaaagc cttggcccgg aggcacctat
 841 cccagagtcc tgacacagaa gctctttcct gttttcttat cccagtgctt cgttccctgg
 901 cccggctgaa gcccactatg accctggagg agggactgcc attggctgtg caggagtggg
 961 agcacaccag caactttgac cggatgatct tttatgagat ggcagaaagg ttcatggagt
1021 ttgaggctga ggagatgcag attcagaaca cacagctgat gaatgggtct cagggcctgt
1081 ctcctgcaac cccttttgaaa cttgatcctc tagggcccct ggcctctgag gtttgccagc
1141 agccagtgta cattccgaag aaggcagcct ccaagacacg ggcccccgc cggcgtcagc
1201 gtaaagccca gagacctcct gctcctgagg cacccaagga gatcccacca gaagctgtga
1261 aggagtatgt tgacatcatg gaatggctgg tggggactca cttggccact ggggagtcag
1321 atggaaaaca agaggaagaa gggcagcagc aggaggagga aggatgtat ccagatccag
1381 gtctcctgag ctacatcaat gagctgtgtt ctcagaaggt ctttgtctcc aaggtggagg
1441 ctgtcattca ccctcaattt ctggcagatc tgctgtcccc agaaaaacag agagatccct
1501 tggccttaat tgaggagcta gagcaagaag aaggactcac tcttgcccag ctggtccaga
1561 agcgactcat ggccttggaa gaggaggaag atgcagaggc gcctccaagt ttcagtggcg
1621 ctcagttgga ctcaagtcct tctggttctg ttgaggatga agatggggat gggcggcttc
1681 ggccctcacc tgggcttcag ggggctgggg gcgccgcttg ccttggaaag gtttcttctt
1741 caggaaaacg ggcaagagaa gtgcatggtg ggcaggagca agccctagat agccccagag
1801 ggatgcacag ggatgggaac actctgccat cccccagcag ctgggacctg cagccagaac
1861 ttgcagctcc acagggaact ccgggaccct tgggtgtgga gaggagaggg tctgggaagg
1921 ttataaacca ggtatctcta catcaggatg gccatctagg aggcgctggg cctcctgggc
1981 actgcctggt ggctgatagg acttcagagg ctctgcccct ttgttggcag ggaggcttcc
2041 agcctgagag cactcccagt ttggatgctg gacttgcaga gctggctcct ctgcaaggac
2101 aagggttaga aaagcaagtc ctgggattgc agaaaggaca acaaacaggg ggtcgtggag
2161 tgcttcctca agggaaggag cctttagcag tgccctggga aggctcttca ggagccatgt
2221 ggggagatga cagaggtacc cccatggctc agagttatga tcagaatcct tcccctagag
2281 cagctgggga gagggacgat gtctgtctca gcccaggagt ttggctgagc agtgagatgg
2341 atgctgtagg cttggagctg cctgtacaaa tagaggaggt catagagagc ttccaagttg
2401 agaagtgtgt aactgagtat caggaaggct gccagggact gggctccagg ggcaacattt
2461 ccctgggtcc tggagaaacc ctagtacctg gggatacgga gagcagtgtg attccctgtg
2521 gaggcacagt tgcggcagct gccctagaaa agagaaacta ttgcagcttg ccaggacctt
2581 tgagggccaa cagcccaccc ttgaggtcca aagaaaatca agaacagagc tgtgaaaccg
2641 tagggcatcc cagtgatctg tgggcagaag gttgcttccc attgctagaa agtggtgatt
2701 ccacactggg gtcttccaaa gaaacccttc cacccacatg ccaaggcaat ctccttatca
2761 tggggactga ggatgcctcc tccttgcctg aagccagtca agaggcaggg agcagaggca
2821 attccttttc tcctctgttg gaaaccatag aacctgtcaa catactagat gttaaagatg
2881 actgtggcct ccaactaagg gtcagcgagg acacctgccc actgaatgtt cattcttatg
2941 acccccaagg agaaggcagg gtggatcctg atctgtccaa gcctaaaaac cttgctcctt
3001 tacaagagag tcaggagtct tacacaactg ggactcccaa agcaacatct tctcaccagg
3061 gccttggaag cactttgcct agaaggggaa ccaggaatgc catagttccg agagaaactt
3121 ctgttagtaa aacacacagg tcagcagaca gggccaaagg aaaggagaaa aagaaaaagg
3181 aagcagagga agaggatgag gaactctcca actttgctta cctcttggcc tctaaactta
3241 gcctctcacc aagggagcat cccctcagtc ctcaccatgc ctcaggaggt cagggcagcc
3301 agagagcatc ccacctgctc cctgctggag caaaaggccc cagcaaactt ccatatcctg
3361 ttgccaagtc tgggaagcga gctctagctg gaggtccagc ccctactgaa aagacacccc
3421 actcaggagc tcaacttggg gtccccaggg agaaacccct agctctggga gtagttcgac
3481 cctcacagcc tcgtaaaagg cggtgtgaca gttttgtcac gggcagaagg aagaaacgac
3541 gtcgtagcca gtagggagca gcgggaccat ctgaccccac ttgccagtcc ctaaaggtgg
3601 gtgccccaga gtagattcca cccctgctgc ccaccaatgg agaatcccaa tgttgaatct
3661 catcccaatg ttgttttgtt gttctgcaaa agtggcaagc atggagagag aggtcagact
3721 ggctaggctg caggggggaat taccctttgga aggagctata tagaaaaaaa atgaataaag
3781 tgttttgttg gaaaa
```

The coding region ranges from nucleotide 156 to nucleotide 3554.

SEQ ID No. 2:
Amino acid sequence of homo sapiens Nut protein:

```
MASDGASALPGPDMSMKPSAALSPSPALPFLPPTSDPPDHPPREPPPQPIMPSVFSPDNPLMLSAFPSSLLVTGDGGPCL
SGAGAGKVIVKVKTEGGSAEPSQTQNFILTQTALNSTAPGTPCGGLEGPAPPFVTASNVKTILPSKAVGVSQEGPPGLPP
QPPPPVAQLVPIVPLEKAWPGPHGTTGEGGPVATLSKPSLGDRSKISKDVYENFRQWQRYKALARRHLSQSPDTEALSCF
LIPVLRSLARLKPTMTLEEGLPLAVQEWEHTSNFDRMIFYEMAERFMEFEAEEMQIQNTQLMNGSQGLSPATPLKLDPLG
PLASEVCQQPVYIPKKAASKTRAPRRRQRKAQRPPAPEAPKEIPPEAVKEYVDIMEWLVGTHLATGESDGKQEEEGQQQE
EEGMYPDPGLLSYINELCSQKVFVSKVEAVIHPQFLADLLSPEKQRDPLALIEELEQEEGLTLAQLVQKRLMALEEEEDA
EAPPSFSGAQLDSSPSGSVEDEDGDGRLRPSPGLQGAGGAACLGKVSSSGKRAREVHGGQEQALDSPRGMHRDGNTLPSP
SSWDLQPELAAPQGTPGPLGVERRGSGKVINQVSLHQDGHLGGAGPPGHCLVADRTSEALPLCWQGGFQPESTPSLDAGL
AELAPLQGQGLEKQVLGLQKGQQTGGRGVLPQGKEPLAVPWEGSSGAMWGDDRGTPMAQSYDQNPSPRAAGERDDVCLSP
GVWLSSEMDAVGLELPVQIEEVIESFQVEKCVTEYQEGCQGLGSRGNISLGPGETLVPGDTESSVIPCGGTVAAAALEKR
NYCSLPGPLRANSPPLRSKENQEQSCETVGHPSDLWAEGCFPLLESGDSTLGSSKETLPPTCQGNLLIMGTEDASSLPEA
SQEAGSRGNSFSPLLETIEPVNILDVKDDCGLQLRVSEDTCPLNVHSYDPQGEGRVDPDLSKPKNLAPLQESQESYTTGT
PKATSSHQGLGSTLPRRGTRNAIVPRETSVSKTHRSADRAKGKEKKKKEAEEEDEELSNFAYLLASKLSLSPREHPLSPH
HASGGQGSQRASHLLPAGAKGPSKLPYPVAKSGKRALAGGPAPTEKTPHSGAQLGVPREKPLALGVVRPSQPRKRRCDSF
VTGRRKKRRRSQ
```

SEQ ID No. 3:
Nucleotide sequence encoding homo sapiens brd4 (accession number NM_058243.2;):

```
   1 attctttgga atactactgc tagaagtctg acttaagacc cagcttatgg gccacatggc
  61 acccagctgc ttctgcagag aaggcaggcc actgatgggt acagcaaagt gtggtgctgc
 121 tggccaagcc aaagacccgt gtaggatgac tgggcctctg ccccttgtgg gtgttgccac
 181 tgtgcttgag tgcctggtga agaatgtgat gggatcacta gcatgtctgc ggagagcggc
 241 cctgggacga gattgagaaa tctgccagta atgggggatg gactagaaac ttcccaaatg
 301 tctacaacac aggcccaggc ccaaccccag ccagccaacg cagccagcac caaccccccg
 361 cccccagaga cctccaaccc taacaagccc aagaggcaga ccaaccaact gcaatacctg
 421 ctcagagtgg tgctcaagac actatggaaa caccagtttg catggccttt ccagcagcct
 481 gtggatgccg tcaagctgaa cctccctgat tactataaga tcattaaaac gcctatggat
 541 atgggaacaa taaagaagcg cttggaaaac aactattact ggaatgctca ggaatgtatc
 601 caggacttca acactatgtt tacaaattgt tacatctaca acaagcctgg agatgacata
 661 gtcttaatgg cagaagctct ggaaaagctc ttcttgcaaa aaataaatga gctacccaca
 721 gaagaaaccg agatcatgat agtccaggca aaaggaagag gacgtgggag gaaagaaaca
 781 gggacagcaa aacctggcgt ttccacggta ccaaacacaa ctcaagcatc gactcctccg
 841 cagacccaga cccctcagcc gaatcctcct cctgtgcagg ccacgcctca ccccttccct
 901 gccgtcaccc cggacctcat cgtccagacc cctgtcatga cagtggtgcc tccccagcca
 961 ctgcagacgc cccgccagt gcccccccag ccacaacccc cacccgctcc agctccccag
1021 cccgtacaga gccacccacc catcatcgcg gccaccccac agcctgtgaa gacaaagaag
1081 ggagtgaaga ggaaagcaga caccaccacc cccaccacca ttgaccccat tcacgagcca
1141 ccctcgctgc ccccggagcc caagaccacc aagctgggcc agcggcggga gagcagccgg
1201 cctgtgaaac ctccaaagaa ggacgtgccc gactctcagc agcacccagc accagagaag
1261 agcagcaagg tctcggagca gctcaagtgc tgcagcggca tcctcaagga gatgtttgcc
1321 aagaagcacg ccgcctacgc ctggcccttc tacaagcctg tggacgtgga ggcactgggc
1381 ctacacgact actgtgacat catcaagcac cccatggaca tgagcacaat caagtctaaa
1441 ctggaggccc gtgagtaccg tgatgctcag gagtttggtg ctgacgtccg attgatgttc
1501 tccaactgct ataagtacaa ccctcctgac catgaggtgg tggccatggc ccgcaagctc
1561 caggatgtgt tcgaaatgcg ctttgccaag atgccggacg agcctgagga gccagtggtg
```

```
1621 gccgtgtcct ccccggcagt gccccctccc accaaggttg tggccccgcc ctcatccagc
1681 gacagcagca gcgatagctc ctcggacagt gacagttcga ctgatgactc tgaggaggag
1741 cgagcccagc ggctggctga gctccaggag cagctcaaag ccgtgcacga gcagcttgca
1801 gccctctctc agccccagca gaacaaacca aagaaaaagg agaaagacaa gaaggaaaag
1861 aaaaaagaaa agcacaaaag gaaagaggaa gtggaagaga ataaaaaaag caaagccaag
1921 gaacctcctc ctaaaaagac gaagaaaaat aatagcagca acagcaatgt gagcaagaag
1981 gagccagcgc ccatgaagag caagccccct cccacgtatg agtcggagga agaggacaag
2041 tgcaagccta tgtcctatga ggagaagcgg cagctcagct tggacatcaa caagctcccc
2101 ggcgagaagc tgggccgcgt ggtgcacatc atccagtcac gggagccctc cctgaagaat
2161 tccaaccccg acgagattga aatcgacttt gagaccctga agccgtccac actgcgtgag
2221 ctggagcgct atgtcacctc ctgtttgcgg aagaaaagga aacctcaagc tgagaaagtt
2281 gatgtgattg ccggctcctc caagatgaag ggcttctcgt cctcagagtc ggagagctcc
2341 agtgagtcca gctcctctga cagcgaagac tccgaaacag agatggctcc gaagtcaaaa
2401 aagaagggc accccgggag ggagcagaag aagcaccatc atcaccacca tcagcagatg
2461 cagcaggccc cggctcctgt gccccagcag ccgccccccgc ctccccagca gcccccaccg
2521 cctccacctc cgcagcagca acagcagccg ccaccccgc ctcccccacc ctccatgccg
2581 cagcaggcag ccccggcgat gaagtcctcg cccccaccct tcattgccac ccaggtgccc
2641 gtcctggagc cccagctccc aggcagcgtc tttgacccca tcggccactt cacccagccc
2701 atcctgcacc tgccgcagcc tgagctgccc cctcacctgc cccagccgcc tgagcacagc
2761 actccacccc atctcaacca gcacgcagtg gtctctcctc cagctttgca caacgcacta
2821 ccccagcagc catcacggcc cagcaaccga gccgctgccc tgcctcccaa gcccgcccgg
2881 cccccagccg tgtcaccagc cttgacccaa acacccctgc tcccacagcc ccccatggcc
2941 caaccccccc aagtgctgct ggaggatgaa gagccacctg ccccacccct cacctccatg
3001 cagatgcagc tgtacctgca gcagctgcag aaggtgcagc cccctacgcc gctactccct
3061 tccgtgaagg tgcagtccca gcccccaccc cccctgccgc ccccacccca cccctctgtg
3121 cagcagcagc tgcagcagca gccgccacca cccccaccac cccagcccca gcctccaccc
3181 cagcagcagc atcagccccc tccacggccc gtgcacttgc agcccatgca gttttccacc
3241 cacatccaac agcccccgcc accccagggc cagcagcccc cccatccgcc cccaggccag
3301 cagccacccc cgccgcagcc tgccaagcct cagcaagtca tccagcacca ccattcaccc
3361 cggcaccaca agtcggaccc ctactcaacc ggtcacctcc gcgaagcccc ctccccgctt
3421 atgatacatt ccccccagat gtcacagttc cagagcctga cccaccagtc tccaccccag
3481 caaaacgtcc agcctaagaa acaggagctg cgtgctgcct ccgtggtcca gccccagccc
3541 ctcgtggtgg tgaaggagga gaagatccac tcacccatca tccgcagcga gcccttcagc
3601 ccctcgctgc ggccggagcc ccccaagcac ccggagagca tcaaggcccc cgtccacctg
3661 ccccagcggc cggaaatgaa gcctgtggat gtcgggaggc ctgtgatccg gcccccagag
3721 cagaacgcac cgccaccagg ggcccctgac aaggacaaac agaaacagga gccgaagact
3781 ccagttgcgc ccaaaaagga cctgaaaatc aagaacatgg gctcctgggc cagcctagtg
3841 cagaagcatc cgaccacccc ctcctccaca gccaagtcat ccagcgacag cttcgagcag
3901 ttccgccgcg ccgctcggga gaaagaggag cgtgagaagg ccctgaaggc tcaggccgag
3961 cacgctgaga aggagaagga gcggctgcgg caggagcgca tgaggagccg agaggacgag
4021 gatgcgctgg agcaggcccg gcgggcccat gaggaggcac gtcggcgcca ggagcagcag
4081 cagcagcagc gccaggagca acagcagcag cagcaacagc aagcagctgc ggtggctgcc
4141 gccgccaccc cacaggccca gagctcccag ccccagtcca tgctggacca gcagagggag
4201 ttggcccgga agcgggagca ggagcgaaga cgccgggaag ccatggcagc taccattgac
4261 atgaatttcc agagtgatct attgtcaata tttgaagaaa atcttttctg agcgcaccta
4321 ggtggcttct gactttgatt ttctggcaaa acattgactt tccatagtgt taggggcggt
4381 ggtggaggtg ggatcagcgg ccaggggatg cctcagggcc tggccctcct gcatgctatg
4441 cccggggcag gcctgacggg cagctgagga ttgcagagcc tgtctgcctt acggccagtc
4501 ggacagacgt cccgccaccc accacccctc acaggacgtc cgctcagcac acgccttgtt
4561 acgagcaagt gccggctgga cccaagccct gcatccccac atgcggggca gaggcccttc
4621 tctccgccaa atgtctacac agtatacaca ggacatcgtt gctgccgccg tgactggttt
4681 tctgtcccca agaacgtgac gttcgtgatg tcctgcccgc cgggagtctt tccccacacc
4741 ccagccatcg ccgcccgctc ccaggaggcc agggcaggcc tgcgtgggct ggaggcgggc
4801 gaggccggcc cacccccctcg ctggcactga ctttgccttg aacagacccc ccgaccctcc
4861 cccacaagcc tttaattgag agccgctctc tgtaagtgtt tgcttgtgca aaagggaata
4921 gtgccgtgga ggtgtgtgtg tccatggcat ccggagcgag gcgactgtcc tgcgtgggta
4981 gccctcggcc ggggagtgag gccaccaacc aaagtcagtt ccttcccacc tgtgtttctg
5041 tttcgttttt tttttctttt tttttctata tatatttttt gttgaattct attttatttt
5101 taattctctc ttctcctcca gacacaatgg cactgcttat ctccgaaatg gtgtgatcgt
5161 ctcctcattg agcagcggct gccaccgcgc tgtgggta
```

The coding region ranges from nucleotide 223 to nucleotide 4311.

SEQ II) No. 4:
Amino acid sequence of homo sapiens Brd4 protein:

```
MSAESGPGTRLRNLPVMGDGLETSQMSTTQAQAQPQPANAASTNPPPPETSNPNKPKRQTNQLQYLLRVVLKTLWKHQFA
WPFQQPVDAVKLNLPDYYKIIKTPMDMGTIKKRLENNYYWNAQECIQDFNTMFTNCYIYNKPGDDIVLMAEALEKLFLQK
INELPTEETEIMIVQAKGRGRGRKETGTAKPGVSTVPNTTQASTPPQTQTPQPNPPPVQATPHPFPAVTPDLIVQTPVMT
VVPPQPLQTPPPVPPQPQPPPAPAPQPVQSHPPIIAATPQPVKTKKGVKRKADTTTPTTIDPIHEPPSLPPEPKTTKLGQ
RRESSRPVKPPKKDVPDSQQHPAPEKSSKVSEQLKCCSGILKEMFAKKHAAYAWPFYKPVDVEALGLHDYCDIIKHPMDM
STIKSKLEAREYRDAQEFGADVRLMFSNCYKYNPPDHEVVAMARKLQDVFEMRFAKMPDEPEEPVVAVSSPAVPPPTKVV
APPSSSDSSSDSSSDSDSSTDDSEEERAQRLAELQEQLKAVHEQLAALSQPQQNKPKKKEKDKKEKKKEKHKRKEEVEEN
KKSKAKEPPPKKTKKNNSSNSNVSKKEPAPMKSKPPPTYESEEEDKCKPMSYEEKRQLSLDINKLPGEKLGRVVHIIQSR
EPSLKNSNPDEIEIDFETLKPSTLRELERYVTSCLRKKRKPQAEKVDVIAGSSKMKGFSSSESESSSESSSSDSEDSETE
MAPKSKKKGHPGREQKKHHHHHHQQMQQAPAPVPQQPPPPPQQPPPPPPPQQQQQPPPPPPPSMPQQAAPAMKSSPPPF
IATQVPVLEPQLPGSVFDPIGHFTQPILHLPQPELPPHLPQPPEHSTPPHLNQHAVVSPPALHNALPQQPSRPSNRAAAL
PPKPARPPAVSPALTQTPLLPQPPMAQPPQVLLEDEEPPAPPLTSMQMQLYLQQLQKVQPPTPLLPSVKVQSQPPPPLPP
PPHPSVQQQLQQQPPPPPPPQPQPPPQQQHQPPPRPVHLQPMQFSTHIQQPPPPQGQQPPHPPPGQQPPPPQPAKPQQVI
QHHHSPRHHKSDPYSTGHLREAPSPLMIHSPQMSQFQSLTHQSPPQQNVQPKKQELRAASVVQPQPLVVVKEEKIHSPII
RSEPFSPSLRPEPPKHPESIKAPVHLPQRPEMKPVDVGRPVIRPPEQNAPPPGAPDKDKQKQEPKTPVAPKKDLKIKNMG
SWASLVQKHPTTPSSTAKSSSDSFEQFRRAAREKEEREKALKAQAEHAEKEKERLRQERMRSREDEDALEQARRAHEEAR
RRQEQQQQQRQEQQQQQQQAAAVAAAATPQAQSSQPQSMLDQQRELARKREQERRRREAMAATIDMNFQSDLLSIFEEN
LF
```

SEQ ID No. 5:
Nucleotide sequence encoding homo sapiens brd3 (accession number NM_007371.3).

```
   1 tgccggggcc ggcgagccaa agaggagccg gccgcgcggg ccgggagggg acggccgccg
  61 gagccgcgag gccaactgtc gcctggttgg gcccggaaat gggacgtcgc gctttctcag
 121 ggagcgtaga agcagccagg gcctctccaa gccgctgctg tgacagaaag tgagtgagct
 181 gccggaggat gtccaccgcc acgacagtcg cccccgcggg gatcccggcg accccgggcc
 241 ctgtgaaccc accccccccg gaggtctcca accccagcaa gcccggccgc aagaccaacc
 301 agctgcagta catgcagaat gtggtggtga agacgctctg gaaacaccag ttcgcctggc
 361 ccttctacca gcccgtggac gcaatcaaat tgaacctgcc ggattatcat aaaataatta
 421 aaaacccaat ggatatgggg actattaaga agagactaga aaataattat tattggagtg
 481 caagcgaatg tatgcaggac ttcaacacca tgtttacaaa ttgttacatt tataacaagc
 541 ccacagatga catagtgcta atggcccaag ctttagagaa aatttttcta caaaaagtgg
 601 cccagatgcc ccaagaggaa gttgaattat taccccctgc tccaaagggc aaaggtcgga
 661 agccggctgc gggagcccag agcgcaggta cacagcaagt ggcggccgtg tcctctgtct
 721 ccccagcgac cccctttcag agcgtgcccc ccaccgtctc ccagacgccc gtcatcgctg
 781 ccacccctgt accaaccatc actgcaaacg tcacgtcggt cccagtcccc ccagctgccg
 841 ccccacctcc tcctgccaca cccatcgtcc ccgtggtccc tcctacgccg cctgtcgtca
 901 agaaaaaggg cgtgaagcgg aaagcagaca caaccactcc cacgacgtcg gccatcactg
 961 ccagccggag tgagtcgccc ccgccgttgt cagaccccaa gcaggccaaa gtggtggccc
1021 ggcgggagag tggtggccgc cccatcaagc ctcccaagaa ggacctggag gacggcgagg
1081 tgccccagca cgcaggcaag aagggcaagc tgtcggagca cctacgctac tgcgacagca
1141 tcctcaggga gatgctatcc aagaagcacg cggcctacgc ctggcccttc tacaagccag
1201 tggatgccga ggccctggag ctgcacgact accacgacat catcaagcac ccgatggacc
1261 tcagcaccgt gaaaaggaag atggatggcc gagagtaccc agacgcacag ggctttgctg
1321 ctgatgtccg gctgatgttc tcgaattgct acaaatacaa tcccccagac cacgaggttg
1381 tggccatggc ccggaagctc caggacgtgt ttgagatgag gtttgccaag atgccagatg
1441 agcccgtgga ggcaccggcg ctgcctgccc ccgcggcccc catggtgagc aagggcgctg
1501 agagcagccg tagcagtgag gagagctctt cggactcagg cagctcggac tcggaggagg
1561 agcgggccac caggctggcg gagctgcagg agcagctgaa ggccgtgcac gagcagctgg
```

```
1621 ccgccctgtc tcaggcccca gtaaacaaac caaagaagaa gaaggagaag aaggagaagg
1681 agaagaagaa gaaggacaag gagaaggaga aggagaagca caaagtgaag gccgaggaag
1741 agaagaaggc caaggtggct ccgcctgcca agcaggctca gcagaagaag gctcctgcca
1801 agaaggccaa cagcacgacc acggccggca gacagctgaa gaaaggcggc aagcaggcat
1861 ctgcctccta cgactcagag gaagaggagg agggcctgcc catgagctac gatgaaaagc
1921 gccagcttag cctggacatc aaccggctgc ccggggagaa gctgggccgg gtagtgcaca
1981 tcatccaatc tcgggagccc tcgctcaggg actccaaccc cgacgagata gaaattgact
2041 ttgagactct gaaacccacc actttgcggg aactggagag atatgtcaag tcttgtttac
2101 agaaaaagca aaggaaaccg ttctcagcaa gcgggaagaa acaggcagcc aagtcgaaag
2161 aggagctagc tcaggaaaag aagaaggagc tggaaaagcg tctgcaggat gtcagcgggc
2221 agctgagcag cagcaagaag cccgccccgga aagagaagcc cggctcagca ccctcagggg
2281 gcccgtccag gctcagcagc agcagctcct ccgagtctgg gagcagcagc tccagcgggt
2341 ccagctctga cagcagtgac tcagaatgaa ctggcttcgg acagaacagg acagatggat
2401 gtcgcacacg ccgagactct gccgtacccc tctgtggttc atattactac ttctgttcca
2461 tggtgtgcag gtctgcttct taattcagtg ttatgatatc ttccagtttt tgctttcata
2521 ggtcagagat ctatcttgtg tgtgcgttag acttgatgag aaggtgtgaa ctctgcagaa
2581 agtctcttct tcatcactga attcagtcac ttggagatga caacttcaaa tgctaacccg
2641 atgaccccag aaaaccgtgt gagattcgta ccgaagaacc ttgtggaatc cctttgctta
2701 ggcccaacct ggtcgatagc tcgagaaaga atttttttcca aggaaatgtc tcggatatgg
2761 gtactgtatt tgaaagctgt tagctttgtc aacacgcatt gtccttgtca tttgggcccc
2821 gagctctgac cctcgtgtct gacgcggcca cctctttctg gaggggctga ggacagatgt
2881 gcctgcttgt ggagaccagg ctgggcctaa gcgaagggtc atcgcagccc cagcccggag
2941 cgtggagccc ttggggggtg gtcgggtggg atgtgcgttc tccgctcgtg gtgatgtcag
3001 gagctcctcg gagggaacag agcggctgtg tatgcagcct gcaggtttcc atacactgaa
3061 gcttttacct caactttaaa aaaaaaaaaa gaagaaaaga tttaaaaaaa aaaaaaaagg
3121 agactttttt tgtaaggttc ggcaattttc tacggaagtc cagcccgctg tgagtccccg
3181 cctggctagc gctgcccctg ctgctgctgc cgcgccaccc ttgggaacca cttcccgagc
3241 ttatgtgtat ataaatagtt atttattaac atcattggtc atttttaaaa aaaagaaaat
3301 aagaaaaaac cgcagaagaa atgcattcac acagtcgcag agatgcaggc cttgccagtg
3361 gtgtgccggg cgcgggtcct gtctggcggc ggcctgtcgt ctccaggtgc taactcctgc
3421 caccgcgcgg tgctcaccca cgtctgttcg cgcgctcgcc cctgggtttg ttgggttttt
3481 tggttttttt ctttgtggtt tttttttttt tttttttttg tatgaaactt ggaggcttac
3541 aggtatagac agctttcagc tacagcacat tctaattttt tattttgttt agttcttttg
3601 tattcacttc tggtctcttt aagactgttt taaaagaaat caatttaggg aaccccagtt
3661 atataatata aactttgtaa tctgagagaa aaaatgtata gtaaatctaa gtcttgattt
3721 ttaactttct attgtaaaaa ataataatat acagagttta atagaaggtg atgttttggt
3781 tttgttttcc cagaggctgc catatggtct ttgagtacgg ggatgtccca aactggccca
3841 ccaatgagca tggcggctcc ggccaggaat gccagagtta gcctcccagg cttgcgggtg
3901 gacatgcctg ctccctgcca gcctccagtg gcctggccag gccctcccga gcctgtctgc
3961 cctccccagg ggtggaggag tctctgggcc ccaggaggat tccctcccgg agactcgcac
4021 ggtgctccct gctcacgcgt tgtcacagtt agtccggaaa tgactgaaac caggcattct
4081 cccggacctc agcgtggggg agcctccagg cagacgctgg gtatggagct gtggtgtggt
4141 ctgtcctgta tggtggccag tgctttctgc cagcatttct ggatggatat agggactatc
4201 attagtatcc taatacacgg tgattttaaa acaaccataa aattgattca gagtccactg
4261 acccttacag atgtaggtat acccttactg gagagggaac tctgatgagg agatgctggt
4321 aaattatcat tttttaaatt gctggtgagt ctgacacttg gtgagttttc agccagtttg
4381 ttaaactttt aattaagttt tgtttataat aaaaatataa atggatttga aagtttccat
4441 tttttaaagt taccctcgtt ttcaaaggta ttttctaaac agatctttaa tggactattt
4501 aaaccgaatt taaggaattc acacacgaca gttgacaggt cttcacgcag gctggttggt
4561 aacgtgctgc cagcacaggg ctgggtgata cgtacaccct aagccggggg tgcctggggc
4621 tggggggcgc tccttgcaat gcccctccag ccacagggca gtgaggtgct gcctgtgtga
4681 gccgtcgggg gagcggccgg ctgtgggggc agcgcagcag gagcatcgtg ggcctttcc
4741 ttctcggctg gttctctgtg acggtggcgt cggctcgcct ctgctccttt catctagaaa
4801 gaagccactg accctgacag cccacggcgg gtacactgag cagctgcatt ggtgctgtca
4861 ctttttttaag gctttctgtc cagacttcaa cactggtttc ttttcagagt ttcgaaggat
4921 taatgacttc ctcagcgccc ttgctggcgg gctgagggtg acagtcacgt ccgtttcttc
4981 tgtattagaa ggctgcggtg attcaattag attgtcccac tgctgagacc tgtagggcag
5041 cttctaacat gcttttttca aggggagagg agtagtgaca agtcgtgtgt cggaattgga
5101 tttgagaaca ctctgaatga cccctggagg ccgagggggc aggcttcggg cgtgaactga
5161 actccagacc cctctttgtg ttgggcagtg tcatcttgct tacaaactgt aagacacatt
```

5221 tttttgtgtg tttgtttttg ttgttgttct tttgcagcac tcacgcctct gacagtcttt
5281 tgggaaagag taacacccac atacagaatt tgtcacatcc agagtagcac tgttccttaa
5341 tactggcata atgcttccag gaagtttttc ttttttatat ttaaaatgtt acttttctgt
5401 atgatgtgca tgcaagttta ccgtaacttt tcttaaactt tttagtgccg tttctagtat
5461 attcctgtaa atgtcagtta ctgaaaatga gtccaatgta agtagtttag cttgtttatt
5521 gcaatgctgg cctcaacaca acagaataaa aatggtagaa agtactcttt gatgtttctg
5581 gtaatcatgg acccttctcc tggggcattt gttttgtttt cataataaaa agcaaaaaaa
5641 aaaaaaaaaa aaaaaaaaaa aaaaaaaaaa aaa

The coding region ranges from nucleotide 189 to nucleotide 2369.

SEQ ID No. 6:
Amino acid sequence of homo sapiens Brd3:

MSTATTVAPAGIPATPGPVNPPPPEVSNPSKPGRKTNQLQYMQNVVVKTLWKHQFAWPFYQPVDAIKLNLPDYHKIIKNP
MDMGTIKKRLENNYYWSASECMQDFNTMFTNCYIYNKPTDDIVLMAQALEKIFLQKVAQMPQEEVELLPPAPKGKGRKPA
AGAQSAGTQQVAAVSSVSPATPFQSVPPTVSQTPVIAATPVPTITANVTSVPVPPAAAPPPPATPIVPVVPPTPPVVKKK
GVKRKADTTTPTTSAITASRSESPPPLSDPKQAKVVARRESGGRPIKPPKKDLEDGEVPQHAGKKGKLSEHLRYCDSILR
EMLSKKHAAYAWPFYKPVDAEALELHDYHDIIKHPMDLSTVKRKMDGREYPDAQGFAADVRLMFSNCYKYNPPDHEVVAM
ARKLQDVFEMRFAKMPDEPVEAPALPAPAAPMVSKGAESSRSSEESSSDSGSSDSEEERATRLAELQEQLKAVHEQLAAL
SQAPVNKPKKKKEKKEKEKKKKDKEKEKEKHKVKAEEEKKAKVAPPAKQAQQKKAPAKKANSTTTAGRQLKKGGKQASAS
YDSEEEEEGLPMSYDEKRQLSLDINRLPGEKLGRVVHIIQSREPSLRDSNPDEIEIDFETLKPTTLRELERYVKSCLQKK
QRKPFSASGKKQAAKSKEELAQEKKKELEKRLQDVSGQLSSSKKPARKEKPGSAPSGGPSRLSSSSSSESGSSSSSGSSS
DSSDSE

SEQ ID No. 7:
Nucleotide sequence encoding homo sapiens cdk9 (accession number NM_001261.3, alias TAK, C-2k, CTK1, CDC2L4, PITALRE):

1 aagtggccgtggaggcggaagtggcgcggccgcggaggggcctggagtgcggcggcggcg
61 ggacccggagcaggagcggcggcagcagcgactgggggcggcggcggcgcgttggaggcg
121 gccatggcaaagcagtacgactcggtggagtgcccttttttgtgatgaagtttccaaatac
181 gagaagctcgccaagatcggccaaggcaccttcggggaggtgttcaaggccaggcaccgc
241 aagaccggccagaaggtggctctgaagaaggtgctgatggaaaacgagaaggaggggttc
301 cccattacagccttgcgggagatcaagatccttcagcttctaaaacacgagaatgtggtc
361 aacttgattgagatttgtcgaaccaaagcttcccccctataaccgctgcaagggtagtata
421 tacctggtgttcgacttctgcgagcatgaccttgctgggctgttgagcaatgttttggtc
481 aagttcacgctgtctgagatcaagagggtgatgcagatgctgcttaacggcctctactac
541 atccacagaaacaagatcctgcatagggacatgaaggctgctaatgtgcttatcactcgt
601 gatggggtcctgaagctggcagactttgggctggcccgggccttcagcctggccaagaac
661 agccagcccaaccgctacaccaaccgtgtggtgacactctggtaccggcccccggagctg
721 ttgctcggggagcgggactacggccccccattgacctgtggggtgctgggtgcatcatg
781 gcagagatgtggacccgcagccccatcatgcagggcaacacggagcagcaccaactcgcc
841 ctcatcagtcagctctgcggctccatcacccctgaggtgtggccaaacgtggacaactat
901 gagctgtacgaaaagctggagctggtcaagggccagaagcggaaggtgaaggacaggctg
961 aaggcctatgtgcgtgacccatacgcactggacctcatcgacaagctgctggtgctggac
1021 cctgcccagcgcatcgacagcgatgacgccctcaaccacgacttcttctggtccgacccc
1081 atgccctccgacctcaagggcatgctctccacccacctgacgtccatgttcgagtacttg
1141 gcaccaccgcgccggaagggcagccagatcacccagcagtccaccaaccagagtcgcaat
1201 cccgccaccaccaaccagacggagtttgagcgcgtcttctgagggccggcgcttgccact
1261 agggctcttgtgtttttttttcttctgctatgtgacttgcatcgtggagacagggcatttg
1321 agtttatatctctcatgcatattttatttaatccccaccctgggctctgggagcagcccg
1381 ctgagtggactggagtggagcattggctgagagaccaggagggcactggagctgtcttgt
1441 ccttgctggttttctggatggttcccagagggtttccatggggtaggaggatgggctcgc
1501 ccaccagtgactttttctaagagctcccggcgtggtggaagaggggacaggtccctcacc

```
1561 cacccacaatcctattctcgggctgagaaccctgcgtggggacagggctcgcctcaggaa
1621 tgggctgtttttggcctaaccctcagaaacactggggctggcacaaactcttggtttctt
1681 caacaggagaattttactgtgtttcttttggttccattgtttggagacattcctgggcac
1741 agtttggtccgttagaattaaaagttgaatttttttttttttaaatttttttttttcct
1801 ccaggacttgtgtgttttgttctgcgcacacaccgccaactgttcccccacagtcagcag
1861 caggttgggcctgaccattgggacttgattgtcaagtcactggaggtcttgacttttta
1921 tctcagttcatgttctcttccataattggaaaggacctttgtctgtttttcctcttgggt
1981 gccttccagaacgcatctcatgtccctggtgagggaattggtgagggcctgctgtgagct
2041 gctgtggctgcgatggtcacccagctgggcaaatcactggagtgacaatttgacctgtca
2101 cctgagaaggatggtccctcagactgctgggtagagggcctggggcaggctggagagaga
2161 aagtgggcagagggtgaagggatcacaggggtcttggaaggtggcagtagtttggacggg
2221 ggtggggagtatgtgggagaaaaaaacagactgaaggtagaatccttgggaacctttgag
2281 gagcggcacattctggcaggcacgttttctgtgagcctgaagttaggaagagacattctg
2341 gaggtcaatttcctacatcctcttacaggcggagaccttgaagtggggccaggaaggaag
2401 gttggcaaaacctttgaccagaactgtccttcatttacagaaactgacccagaccacaac
2461 acaaaaggccag
```

The coding region ranges from nucleotide 124 to nucleotide 1242.

SEQ ID No. 8:
Amino acid sequence of homo sapiens CDK9:

```
MAKQYDSVECPFCDEVSKYEKLAKIGQGTFGEVFKARHRKTGQKVALKKVLMENEKEGFPITALREIKILQLLKHENVVN
LIEICRTKASPYNRCKGSIYLVFDFCEHDLAGLLSNVLVKFTLSEIKRVMQMLLNGLYYIHRNKILHRDMKAANVLITRD
GVLKLADFGLARAFSLAKNSQPNRYTNRVVTLWYRPPELLLGERDYGPPIDLWGAGCIMAEMWTRSPIMQGNTEQHQLAL
ISQLCGSITPEVWPNVDNYELYEKLELVKGQKRKVKDRLKAYVRDPYALDLIDKLLVLDPAQRIDSDDALNHDFFWSDPM
PSDLKGMLSTHLTSMFEYLAPPRRKGSQITQQSTNQSRNPATTNQTEFERVF
```

SEQ ID No. 9:
Nucleotide sequence encoding homo sapiens cyclinT1 (accession number NM_001240.2):

```
   1 gggaagatac acggttttta agaaagaact tctaacccca ctccaccgcc caacggtcac
  61 gtgacgcgcc tgcgtcctcg cctcaaggtt ttttctggtc gcatattggc tgttaactcg
 121 gctacggggt gtagcgaggt gcattccgga agtaggtcct ttgacttttg cttcctctac
 181 ggaggcaagt aacaacccgg cggtcgacgc ttagcggaag ttcgtcaagt cgcagttgcc
 241 cccgcacagc ggatgtgggt cgcctcttag gtgacgctgg gaagtgcctg caaccttcgc
 301 cgctgccttc tggttgaagc actatggagg gagagaggaa gaacaacaac aaacggtggt
 361 atttcactcg agaacagctg gaaaatagcc catcccgtcg ttttggcgtg gacccagata
 421 aagaactttc ttatcgccag caggcggcca atctgcttca ggacatgggg cagcgtctta
 481 acgtctcaca attgactatc aacactgcta tagtatacat gcatcgattc tacatgattc
 541 agtccttcac acagttccct ggaaattctg tggctccagc agccttgttt ctagcagcta
 601 aagtggagga gcagcccaaa aaattggaac atgtcatcaa ggtagcacat acttgtctcc
 661 atcctcagga atcccttcct gatactagaa gtgaggctta tttgcaacaa gttcaagatc
 721 tggtcatttt agaaagcata attttgcaga ctttaggctt tgaactaaca attgatcacc
 781 cacatactca tgtagtaaag tgcactcaac ttgttcgagc aagcaaggac ttagcacaga
 841 cttcttactt catggcaacc aacagcctgc atttgaccac atttagcctg cagtacacac
 901 ctcctgtggt ggcctgtgtc tgcattcacc tggcttgcaa gtggtccaat tgggagatcc
 961 cagtctcaac tgacgggaag cactggtggg agtatgttga cgccactgtg accttggaac
1021 ttttagatga actgacacat gagtttctac agattttgga gaaaactccc aacaggctca
1081 aacgcatttg gaattggagg gcatgcgagg ctgccaagaa aacaaaagca gatgaccgag
1141 gaacagatga aaagacttca gagcagacaa tcctcaatat gatttcccag agctcttcag
1201 acacaaccat tgcaggttta atgagcatgt caacttctac cacaagtgca gtgccttccc
1261 tgccagtctc cgaagagtca tccagcaact taaccagtgt ggagatgttg ccgggcaagc
1321 gttggctgtc ctcccaacct tctttcaaac tagaacctac tcaggtcat cggactagtg
1381 agaatttagc acttacagga gttgatcatt ccttaccaca ggatggttca aatgcattta
```

```
1441 tttcccagaa gcagaatagt aagagtgtgc catcagctaa agtgtcactg aaagaatacc
1501 gcgcgaagca tgcagaagaa ttggctgccc agaagaggca actggagaac atggaagcca
1561 atgtgaagtc acaatatgca tatgctgccc agaatctcct ttctcatcat gatagccatt
1621 cttcagtcat tctaaaaatg cccatagagg gttcagaaaa ccccgagcgg ccttttctgg
1681 aaaaggctga caaaacagct ctcaaaatga gaatcccagt ggcaggtgga gataaagctg
1741 cgtcttcaaa accagaggag ataaaaatgc gcataaaagt ccatgctgca gctgataagc
1801 acaattctgt agaggacagt gttacaaaga gccgagagca caaagaaaag cacaagactc
1861 acccatctaa tcatcatcat catcataatc accactcaca caagcactct cattcccaac
1921 ttccagttgg tactgggaac aaacgtcctg gtgatccaaa acatagtagc cagacaagca
1981 acttagcaca taaaacctat agcttgtcta gttctttttc ctcttccagt tctactcgta
2041 aaaggggacc ctctgaagag actggagggg ctgtgtttga tcatccagcc aagattgcca
2101 agagtactaa atcctcttcc ctaaatttct ccttcccttc acttcctaca atgggtcaga
2161 tgcctgggca tagctcagac acaagtggcc tttccttttc acagcccagc tgtaaaactc
2221 gtgtccctca ttcgaaactg gataaagggc ccactggggc caatggtcac aacacgaccc
2281 agacaataga ctatcaagac actgtgaata tgcttcactc cctgctcagt gcccagggtg
2341 ttcagcccac tcagcccact gcatttgaat ttgttcgtcc ttatagtgac tatctgaatc
2401 ctcggtctgg tggaatctcc tcgagatctg gcaatacaga caaaccccgg ccaccacctc
2461 tgccatcaga acctcctcca ccacttccac cccttcctaa gtaaaaaaag aaaaagaaga
2521 ggagaaaaaa acttctttaa aaaaacacat aatttttctt tttttttt
```

The coding region ranges from nucleotide 324 to nucleotide 2504.

SEQ ID No. 10:
Amino acid sequence of homo sapiens CyclinT1:

```
MEGERKNNNKRWYFTREQLENSPSRRFGVDPDKELSYRQQAANLLQDMGQRLNVSQLTINTAIVYMHRFYMIQSFTQFPG
NSVAPAALFLAAKVEEQPKKLEHVIKVAHTCLHPQESLPDTRSEAYLQQVQDLVILESIILQTLGFELTIDHPHTHVVKC
TQLVRASKDLAQTSYFMATNSLHLTTFSLQYTPPVVACVCIHLACKWSNWEIPVSTDGKHWWEYVDATVTLELLDELTHE
FLQILEKTPNRLKRIWNWRACEAAKKTKADDRGTDEKTSEQTILNMISQSSSDTTIAGLMSMSTSTTSAVPSLPVSEESS
SNLTSVEMLPGKRWLSSQPSFKLEPTQGHRTSENLALTGVDHSLPQDGSNAFISQKQNSKSVPSAKVSLKEYRAKHAEEL
AAQKRQLENMEANVKSQYAYAAQNLLSHHDSHSSVILKMPIEGSENPERPFLEKADKTALKMRIPVAGGDKAASSKPEEI
KMRIKVHAAADKHNSVEDSVTKSREHKEKHKTHPSNHHHHHNHHSHKHSHSQLPVGTGNKRPGDPKHSSQTSNLAHKTYS
LSSSFSSSSSTRKRGPSEETGGAVFDHPAKIAKSTKSSSLNFSFPSLPTMGQMPGHSSDTSGLSFSQPSCKTRVPHSKLD
KGPTGANGHNTTQTIDYQDTVNMLHSLLSAQGVQPTQPTAFEFVRPYSDYLNPRSGGISSRSGNTDKPRPPPLPSEPPPP
LPPLPK
```

SEQ ID No. 11:
Nucleotide sequence encoding homo sapiens Notch3.

```
  1 gcggcgcgga ggctggcccg ggacgcgccc ggagcccagg gaaggaggga ggaggggagg
 61 gtcgcggccg gccgccatgg ggccgggggc ccgtggccgc cgccgccgcc gtcgccccgat
121 gtcgccgcca ccgccaccgc cacccgtgcg ggcgctgccc ctgctgctgc tgctagcggg
181 gccgggggct gcagcccccc cttgcctgga cggaagcccg tgtgcaaatg gaggtcgttg
241 cacccagctg ccctcccggg aggctgcctg cctgtgcccg cctggctggg tgggtgagcg
301 gtgtcagctg gaggacccct gtcactcagg cccctgtgct ggccgtggtg tctgccagag
361 ttcagtggtg gctggcaccg cccgattctc atgccggtgc cccccgtggct tccgaggccc
421 tgactgctcc ctgccagatc cctgcctcag cagcccttgt gcccacggtg cccgctgctc
481 agtggggccc gatggacgct tcctctgctc ctgcccacct ggctaccagg gccgcagctg
541 ccgaagcgac gtggatgagt gccgggtggg tgagccctgc cgccatggtg gcacctgcct
601 caacacacct ggctccttcc gctgccagtg tccagctggc tacacagggc cactatgtga
661 gaaccccgcg gtgccctgtg caccctcacc atgccgtaac gggggcacct gcaggcagag
721 tggcgacctc acttacgact gtgcctgtct tcctgggttt gagggtcaga attgtgaagt
781 gaacgtggac gactgtccag gacaccgatg tctcaatggg gggacatgcg tggatggcgt
841 caacacctat aactgccagt gccctcctga gtggacaggc cagttctgca cggaggacgt
901 ggatgagtgt cagctgcagc ccaacgcctg ccacaatggg ggtacctgct tcaacacgct
```

961 gggtggccac agctgcgtgt gtgtcaatgg ctggacaggc gagagctgca gtcagaatat
1021 cgatgactgt gccacagccg tgtgcttcca tggggccacc tgccatgacc gcgtggcttc
1081 tttctactgt gcctgcccca tgggcaagac tggcctcctg tgtcacctgg atgacgcctg
1141 tgtcagcaac ccctgccacg aggatgctat ctgtgacaca aatccggtga acggccgggc
1201 catttgcacc tgtcctcccg gcttcacggg tggggcatgt gaccaggatg tggacgagtg
1261 ctctatcggc gccaacccct gcgagcactt gggcaggtgc gtgaacacgc agggctcctt
1321 cctgtgccag tgcggtcgtg gctacactgg acctcgctgt gagaccgatg tcaacgagtg
1381 tctgtcgggg ccctgccgaa accaggccac gtgcctcgac cgcataggcc agttcacctg
1441 tatctgtatg gcaggcttca caggaaccta ttgcgaggtg gacattgacg agtgtcagag
1501 tagcccctgt gtcaacggtg gggtctgcaa ggaccgagtc aatggcttca gctgcacctg
1561 cccctcgggc ttcagcggct ccacgtgtca gctggacgtg gacgaatgcg ccagcacgcc
1621 ctgcaggaat ggcgccaaat gcgtggacca gcccgatggc tacgagtgcc gctgtgccga
1681 gggctttgag ggcacgctgt gtgatcgcaa cgtggacgac tgctcccctg acccatgcca
1741 ccatggtcgc tgcgtggatg gcatcgccag cttctcatgt gcctgtgctc ctggctacac
1801 gggcacacgc tgcgagagcc aggtggacga atgccgcagc cagccctgcc gccatggcgg
1861 caaatgccta gacctggtgg acaagtacct ctgccgctgc ccttctggga ccacaggtgt
1921 gaactgcgaa gtgaacattg acgactgtgc cagcaacccc tgcacctttg gagtctgccg
1981 tgatggcatc aaccgctacg actgtgtctg ccaacctggc ttcacagggc cccttttgtaa
2041 cgtggagatc aatgagtgtg cttccagccc atgcggcgag ggaggttcct gtgtggatgg
2101 ggaaaatggc ttccgctgcc tctgcccgcc tggctccttg cccccactct gcctcccccc
2161 gagccatccc tgtgcccatg agccctgcag tcacggcatc tgctatgatg cacctggcgg
2221 gttccgctgt gtgtgtgagc ctggctggag tggcccccgc tgcagccaga gcctggcccg
2281 agacgcctgt gagtcccagc cgtgcagggc cggtgggaca tgcagcagcg atggaatggg
2341 tttccactgc acctgcccgc ctggtgtcca gggacgtcag tgtgaactcc tctcccccctg
2401 caccccgaac ccctgtgagc atggggggccg ctgcgagtct gcccctggcc agctgcctgt
2461 ctgctcctgc ccccagggct ggcaaggccc acgatgccag caggatgtgg acgagtgtgc
2521 tggccccgca ccctgtggcc ctcatggtat ctgcaccaac ctggcaggga gtttcagctg
2581 cacctgccat ggagggtaca ctggcccttc ctgcgatcag gacatcaatg actgtgaccc
2641 caacccatgc ctgaacggtg gctcgtgcca agacggcgtg ggctcctttt cctgctcctg
2701 cctccctggt ttcgccggcc cacgatgcgc ccgcgatgtg gatgagtgcc tgagcaaccc
2761 ctgcggcccg ggcacctgta ccgaccacgt ggcctccttc acctgcacct gcccgccagg
2821 ctacggaggc ttccactgcg aacaggacct gcccgactgc agccccagct cctgcttcaa
2881 tggcgggacc tgtgtggacg gcgtgaactc gttcagctgc ctgtgccgtc ccggctacac
2941 aggagcccac tgccaacatg aggcagaccc ctgcctctcg cggccctgcc tacacggggg
3001 cgtctgcagc gccgcccacc ctggcttccg ctgcacctgc ctcgagagct tcacgggccc
3061 gcagtgccag acgctggtgg attggtgcag ccgccagcct tgtcaaaacg gggtcgctg
3121 cgtccagact ggggcctatt gcctttgtcc ccctggatgg agcggacgcc tctgtgacat
3181 ccgaagcttg ccctgcaggg aggccgcagc ccagatcggg gtgcggctgg agcagctgtg
3241 tcaggcgggt gggcagtgtg tggatgaaga cagctcccac tactgcgtgt gcccagaggg
3301 ccgtactggt agccactgtg agcaggaggt ggacccctgc ttggcccagc cctgccagca
3361 tgggggggacc tgccgtggct atatgggggg ctacatgtgt gagtgtcttc ctggctacaa
3421 tggtgataac tgtgaggacg acgtggacga gtgtgcctcc cagccctgcc agcacggggg
3481 ttcatgcatt gacctcgtgg cccgctatct ctgctcctgt cccccaggaa cgctgggggt
3541 gctctgcgag attaatgagg atgactgcgg cccaggccca ccgctggact caggcccccg
3601 gtgcctacac aatggcacct gcgtggacct ggtgggtggt ttccgctgca cctgtccccc
3661 aggatacact ggtttgcgct gcgaggcaga catcaatgag tgtcgctcag gtgcctgcca
3721 cgcggcacac acccgggact gcctgcagga cccaggcgga ggtttccgtt gcctttgtca
3781 tgctggcttc tcaggtcctc gctgtcagac tgtcctgtct ccctgcgagt cccagccatg
3841 ccagcatgga ggccagtgcc gtcctagccc gggtcctggg ggtgggctga ccttcacctg
3901 tcactgtgcc cagccgttct ggggtccgcg ttgcgagcgg gtggcgcgct cctgccggga
3961 gctgcagtgc ccggtgggcg tcccatgcca gcagacgccc cgcgggccgc gctgcgcctg
4021 ccccccaggg ttgtcgggac cctcctgccg cagcttcccg gggtcgccgc cggggggccag
4081 caacgccagc tgcgcggccg ccccctgtct ccacgggggc tcctgccgcc ccgcgccgct
4141 cgcgcccttc ttccgctgcg cttgcgcgca gggctggacc gggccgcgct gcgaggcgcc
4201 cgccgcggca cccgaggtct cggaggagcc gcggtgcccg cgcgccgcct gccaggccaa
4261 gcgcggggac cagcgctgcg accgcgagtg caacagccca ggctgcggct gggacggcgg
4321 cgactgctcg ctgagcgtgg gcgacccctg gcggcaatgc gaggcgctgc agtgctggcg
4381 cctcttcaac aacagccgct gcgaccccgc ctgcagctcg cccgcctgcc tctacgacaa
4441 cttcgactgc cacgccggtg gccgcgagcg cacttgcaac ccggtgtacg agaagtactg
4501 cgccgaccac tttgccgacg gccgctgcga ccagggctgc aacacggagg agtgcggctg

```
4561 ggatgggctg gattgtgcca gcgaggtgcc ggccctgctg gcccgcggcg tgctggtgct
4621 cacagtgctg ctgccgccag aggagctact gcgttccagc gccgactttc tgcagcggct
4681 cagcgccatc ctgcgcacct cgctgcgctt ccgcctggac gcgcacggcc aggccatggt
4741 cttcccttac caccggccta gtcctggctc cgaaccccgg gcccgtcggg agctggcccc
4801 cgaggtgatc ggctcggtag taatgctgga gattgacaac cggctctgcc tgcagtcgcc
4861 tgagaatgat cactgcttcc ccgatgccca gagcgccgct gactacctgg gagcgttgtc
4921 agcggtggag cgcctggact tcccgtaccc actgcgggac gtgcgggggg agccgctgga
4981 gcctccagaa cccagcgtcc cgctgctgcc actgctagtg gcgggcgctg tcttgctgct
5041 ggtcattctc gtcctgggtg tcatggtggc ccggcgcaag cgcgagcaca gcaccctctg
5101 gttccctgag ggcttctcac tgcacaagga cgtggcctct ggtcacaagg gccggcggga
5161 acccgtgggc caggacgcgc tgggcatgaa gaacatggcc aagggtgaga gcctgatggg
5221 ggaggtggcc acagactgga tggacacaga gtgcccagag gccaagcggc taaaggtaga
5281 ggagccaggc atgggggctg aggaggctgt ggattgccgt cagtggactc aacaccatct
5341 ggttgctgct gacatccgcg tggcaccagc catggcactg acaccaccac agggcgacgc
5401 agatgctgat ggcatggatg tcaatgtgcg tggcccagat ggcttcaccc cgctaatgct
5461 ggcttccttc tgtggggggg ctctggagcc aatgccaact gaagaggatg aggcagatga
5521 cacatcagct agcatcatct ccgacctgat ctgccagggg gctcagcttg ggcacggac
5581 tgaccgtact ggcgagactg ctttgcacct ggctgcccgt tatgcccgtg ctgatgcagc
5641 caagcggctg ctggatgctg gggcagacac caatgcccag gaccactcag gccgcactcc
5701 cctgcacaca gctgtcacag ccgatgccca gggtgtcttc cagattctca tccgaaaccg
5761 ctctacagac ttggatgccc gcatggcaga tggctcaacg gcactgatcc tggcggcccg
5821 cctggcagta gagggcatgg tggaagagct catcgccagc catgctgatg tcaatgctgt
5881 ggatgagctt gggaaatcag ccttacactg ggctgcggct gtgaacaacg tggaagccac
5941 tttggccctg ctcaaaaatg gagccaataa ggacatgcag gatagcaagg aggagacccc
6001 cctattcctg gccgcccgcg agggcagcta tgaggctgcc aagctgctgt tggaccactt
6061 tgccaaccgt gagatcaccg accacctgga caggctgccg cgggacgtag cccaggagag
6121 actgcaccag gacatcgtgc gcttgctgga tcaacccagt gggccccgca gcccccccgg
6181 tccccacggc ctggggcctc tgctctgtcc tccaggggcc ttcctccctg gcctcaaagc
6241 ggcacagtcg gggtccaaga agagcaggag gcccccccggg aaggcggggc tggggccgca
6301 ggggccccgg gggcggggca agaagctgac gctggcctgc ccgggccccc tggctgacag
6361 ctcggtcacg ctgtcgcccg tggactcgct ggactccccg cggcctttcg gtgggccccc
6421 tgcttcccct ggtggcttcc cccttgaggg gccctatgca gctgccactg ccactgcagt
6481 gtctctggca cagcttggtg gcccaggccg ggcgggtcta gggcgccagc cccctggagg
6541 atgtgtactc agcctgggcc tgctgaaccc tgtggctgtg cccctcgatt gggcccggct
6601 gcccccacct gcccctccag gcccctcgtt cctgctgcca ctggcgccgg gaccccagct
6661 gctcaaccca gggacccccg tctccccgca ggagcggccc ccgccttacc tggcagtccc
6721 aggacatggc gaggagtacc cggcggctgg ggcacacagc agcccccccaa aggcccgctt
6781 cctgcgggtt cccagtgagc acccttacct gaccccatcc cccgaatccc ctgagcactg
6841 ggccagcccc tcacctccct ccctctcaga ctggtccgaa tccacgccta gcccagccac
6901 tgccactggg gccatggcca ccaccactgg ggcactgcct gcccagccac ttcccttgtc
6961 tgttcccagc tcccttgctc aggcccagac ccagctgggg ccccagccgg aagttacccc
7021 caagaggcaa gtgttggcct gagacgctcg tcagttctta gatcttgggg gcctaaagag
7081 acccccgtcc tgcctccttt ctttctctgt ctcttccttc cttttagtct ttttcatcct
7141 cttctctttc caccaaccct cctgcatcct tgccttgcag cgtgaccgag ataggtcatc
7201 agcccagggc ttcagtcttc ctttatttat aatgggtggg ggctaccacc caccctctca
7261 gtcttgtgaa gagtctggga cctccttctt ccccacttct ctcttccctc attcctttct
7321 ctctccttct ggcctctcat ttccttacac tctgacatga atgaattatt attattttta
7381 tttttctttt tttttttaca ttttgtatag aaacaaattc atttaaacaa acttattatt
7441 attatttttt acaaaatata tatatggaga tgctccctcc ccctgtgaac cccccagtgc
7501 ccccgtgggg ctgagtctgt gggcccattc ggccaagctg gattctgtgt acctagtaca
7561 caggcatgac tgggatcccg tgtaccgagt acacgaccca ggtatgtacc aagtaggcac
7621 ccttgggcgc acccactggg gccaggggtc gggggagtgt tgggagcctc ctccccaccc
7681 cacctccctc acttcactgc attccagatg ggacatgttc catagccttg ctggggaagg
7741 gcccactgcc aactccctct gccccagccc caccccttggc catctccctt tgggaactag
7801 ggggctgctg gtgggaaatg ggagccaggg cagatgtatg cattcctttg tgtccctgta
7861 aatgtgggac tacaagaaga ggagctgcct gagtggtact ttctcttcct ggtaatcctc
7921 tggcccagcc tcatggcaga atagaggtat ttttaggcta tttttgtaat atggcttctg
7981 gtcaaaatcc ctgtgtagct gaattcccaa gccctgcatt gtacagcccc ccactcccct
8041 caccacctaa taaaggaata gttaacactc aaaaaaaaaa aaaaaaaaa
```

The coding region ranges from nucleotide 77 to nucleotide 7042.

SEQ ID No. 12:
Amino acid sequence of homo sapiens Notch3:

```
MGPGARGRRRRRRPMSPPPPPPPVRALPLLLLLAGPGAAAPPCLDGSPCANGGRCTQLPSREAACLCPPGWVGERCQLED
PCHSGPCAGRGVCQSSVVAGTARFSCRCPRGFRGPDCSLPDPCLSSPCAHGARCSVGPDGRFLCSCPPGYQGRSCRSDVD
ECRVGEPCRHGGTCLNTPGSFRCQCPAGYTGPLCENPAVPCAPSPCRNGGTCRQSGDLTYDCACLPGFEGQNCEVNVDDC
PGHRCLNGGTCVDGVNTYNCQCPPEWTGQFCTEDVDECQLQPNACHNGGTCFNTLGGHSCVCVNGWTGESCSQNIDDCAT
AVCFHGATCHDRVASFYCACPMGKTGLLCHLDDACVSNPCHEDAICDTNPVNGRAICTCPPGFTGGACDQDVDECSIGAN
PCEHLGRCVNTQGSFLCQCGRGYTGPRCETDVNECLSGPCRNQATCLDRIGQFTCICMAGFTGTYCEVDIDECQSSPCVN
GGVCKDRVNGFSCTCPSGFSGSTCQLDVDECASTPCRNGAKCVDQPDGYECRCAEGFEGTLCDRNVDDCSPDPCHHGRCV
DGIASFSCACAPGYTGTRCESQVDECRSQPCRHGGKCLDLVDKYLCRCPSGTTGVNCEVNIDDCASNPCTFGVCRDGINR
YDCVCQPGFTGPLCNVEINECASSPCGEGGSCVDGENGFRCLCPPGSLPPLCLPPSHPCAHEPCSHGICYDAPGGFRCVC
EPGWSGPRCSQSLARDACESQPCRAGGTCSSDGMGFHCTCPPGVQGRQCELLSPCTPNPCEHGGRCESAPGQLPVCSCPQ
GWQGPRCQQDVDECAGPAPCGPHGICTNLAGSFSCTCHGGYTGPSCDQDINDCDPNPCLNGGSCQDGVGSFSCSCLPGFA
GPRCARDVDECLSNPCGPGTCTDHVASFTCTCPPGYGGFHCEQDLPDCSPSSCFNGGTCVDGVNSFSCLCRPGYTGAHCQ
HEADPCLSRPCLHGGVCSAAHPGFRCTCLESFTGPQCQTLVDWCSRQPCQNGGRCVQTGAYCLCPPGWSGRLCDIRSLPC
REAAAQIGVRLEQLCQAGGQCVDEDSSHYCVCPEGRTGSHCEQEVDPCLAQPCQHGGTCRGYMGGYMCECLPGYNGDNCE
DDVDECASQPCQHGGSCIDLVARYLCSCPPGTLGVLCEINEDDCGPGPPLDSGPRCLHNGTCVDLVGGFRCTCPPGYTGL
RCEADINECRSGACHAAHTRDCLQDPGGGFRCLCHAGFSGPRCQTVLSPCESQPCQHGGQCRPSPGPGGGLTFTCHCAQP
FWGPRCERVARSCRELQCPVGVPCQQTPRGPRCACPPGLSGPSCRSFPGSPPGASNASCAAAPCLHGGSCRPAPLAPFFR
CACAQGWTGPRCEAPAAAPEVSEEPRCPRAACQAKRGDQRCDRECNSPGCGWDGGDCSLSVGDPWRQCEALQCWRLFNNS
RCDPACSSPACLYDNFDCHAGGRERTCNPVYEKYCADHFADGRCDQGCNTEECGWDGLDCASEVPALLARGVLVLTVLLP
PEELLRSSADFLQRLSAILRTSLRFRLDAHGQAMVFPYHRPSPGSEPRARRELAPEVIGSVVMLEIDNRLCLQSPENDHC
FPDAQSAADYLGALSAVERLDFPYPLRDVRGEPLEPPEPSVPLLPLLVAGAVLLLVILVLGVMVARRKREHSTLWFPEGF
SLHKDVASGHKGRREPVGQDALGMKNMAKGESLMGEVATDWMDTECPEAKRLKVEEPGMGAEEAVDCRQWTQHHLVAADI
RVAPAMALTPPQGDADADGMDVNVRGPDGFTPLMLASFCGGALEPMPTEEDEADDTSASIISDLICQGAQLGARTDRTGE
TALHLAARYARADAAKRLLDAGADTNAQDHSGRTPLHTAVTADAQGVFQILIRNRSTDLDARMADGSTALILAARLAVEG
MVEELIASHADVNAVDELGKSALHWAAAVNNVEATLALLKNGANKDMQDSKEETPLFLAAREGSYEAAKLLLDHFANREI
TDHLDRLPRDVAQERLHQDIVRLLDQPSGPRSPPGPHGLGPLLCPPGAFLPGLKAAQSGSKKSRRPPGKAGLGPQGPRGR
GKKLTLACPGPLADSSVTLSPVDSLDSPRPFGGPPASPGGFPLEGPYAAATATAVSLAQLGGPGRAGLGRQPPGGCVLSL
GLLNPVAVPLDWARLPPPAPPGPSFLLPLAPGPQLLNPGTPVSPQERPPPYLAVPGHGEEYPAAGAHSSPPKARFLRVPS
EHPYLTPSPESPEHWASPSPPSLSDWSESTPSPATATGAMATTTGALPAQPLPLSVPSSLAQAQTQLGPQPEVTPKRQVL
A
```

All references cited herein are fully incorporated by reference. Having now fully described the invention, it will be understood by a person skilled in the art that the invention may be practiced within a wide and equivalent range of conditions, parameters and the like, without affecting the spirit or scope of the invention or any embodiment thereof.

## Claims

1. A method of selecting (a) cell(s), (a) tissue(s) or (a) cell culture(s) with susceptibility to a selective CDK9 inhibitor, comprising the steps:

    (a) determining the presence of a rearrangement in the NUT gene in said cell, tissue or cell culture;
    (b) selecting (a) cell(s), tissue(s) or cell cultures(s) with at least one rearrangement in the NUT gene;
    (c) contacting said cell(s), tissue(s) or cell culture(s) with a selective CDK9 inhibitor; and
    (d) evaluating viability of said cell(s), tissue(s) or cell culture(s) contacted with said selective CDK9 inhibitor, wherein a decreased viability is indicative for susceptibility to said selective CDK9 inhibitor.

2. A method for determining the responsiveness of a mammalian tumor cell or cancer cell to a selective CDK9 inhibitor, said method comprising determining the presence of at least one rearrangement in the NUT gene in said tumor or cancer cell, wherein said rearrangement in the NUT gene is indicative of whether the cell is likely to respond or is responsive to said selective CDK9 inhibitor.

3. In vitro method for the identification of a responder for or a patient sensitive to a selective CDK9 inhibitor, said method comprising evaluating the presence of at least one rearrangement in the NUT gene in a sample obtained

from a subject/patient suspected to suffer from or being prone to suffer from NUT midline carcinoma (NMC), whereby the presence of at least one rearrangement in the NUT gene is indicative for a responding patient or is indicative for a sensitivity of said patient to a selective CDK9 inhibitor.

**4.** The method of claim 3, wherein said selective CDK9 inhibitor is a compound having the general formula (I)

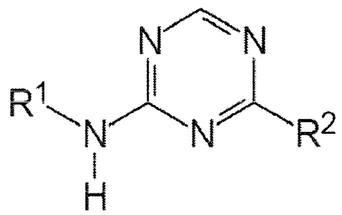

Formula (I)

wherein

**$R^1$** is

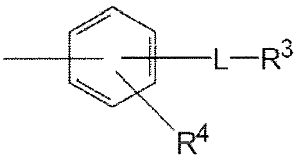 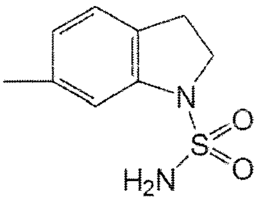 or 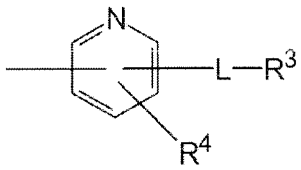

**L** is a bond or $-CR^5R^6-$, $-CR^5R^6-CR^7R^8-$, $-CR^5R^6-CR^7-CR^8R^{10}-$, $-CR^5R^6-CR^7R^8-CR^9R^{10}-CR^{11}R^{12}-$;
**$R^5$- $R^{12}$** represent independently of each other -H, $-CH_3$. $-C_2H_5$: $-C_3H_7$, -F, -Cl, -Br, -I;
**$R^3$** is selected from -H, $-NO_2$, $-NH_2$, -CN, -F, -Cl, -Br, -I, $-CH_3$, $-C_2H_5$, -Ph, $-C_3H_7$, $-CH(CH_3)_2$, $-C_4H_9$, $-CH_2-CH(CH_3)_2$, $-CH(CH_3)-C_2H_5$, $-C(CH_3)_3$, $-O-CH_3$, $-O-C_2H_5$, $-O-C_3H_7$, $-O-CH(CH_3)_2$, $-O-C_4H_9$, $-O-CH_2-CH(CH_3)_2$, $-O-CH(CH_3)-C_2H_5$, $-O-C(CH_3)_3$, $-CR^{13}R^{14}R^{21}$, $-CR^{13}R^{14}-CR^{15}R^{16}R^{21}$, $-O-CR^{13}R^{14}R^{21}$ $-CR^{13}R^{14}-CR^{15}R^{16}-CR^{17}R^{18}R^{21}$, $-CR^{13}R^{14}-CR^{15}R^{16}-CR^{17}R^{18}-CR^{19}R^{20}R^{21}$ $-O-CR^{13}R^{14}-CR^{15}R^{16}R^{21}$, $-O-CR^{13}R^{14}-CR^{15}R^{16}-CR^{17}R^{18}R^{21}$, $-SO_2R^{22}$, $-CONR^{23}R^4$, $-NR^{25}COR^{22}$, $-O-CR^{13}R^{14}-CR^{15}R^{16}-CR^{17}R^{18}-CR^{19}R^{20}R^{21}$, $-NR^{25}SO_2NR^{23}R^{24}$, $NR^{25}SO_2R^{22}$, $-NR^{25}CONR^{23}R^{24}$, $-SO_2NR^{23}R^{24}$, $-SO(NR^{26})R^{27}$, -NH-CO-NH-Ph;
**$R^{13}$-$R^{21}$, $R^{29}$-$R^{32}$** and **$R^{33}$ - $R^{48}$** represent independently of each other -H, -F, -Cl, -Br, -I;
**$R^{26}$** is -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$,- $C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-C_2H_4-C(CH_3)_3$, $-CH(CH_3)-C(CH_3)_3$, $-CR^{13}R^{14}R^{21}$, $-COR^{28}$, $-CR^{13}R^{14}-CR^{15}R^{16}R^{21}$, $-CR^{13}R^{14}-CR^{15}R^{16}-CR^{17}R^{18}-CR^{19}R^{20}-CR^{29}R^{30}R^{21}$, $-CR^{13}R^{14}-CR^{15}R^{16}-CR^{17}R^{18}R^{21}$, $-CR^{13}R^{14}-CR^{15}R^{16}-CR^{17}R^{18}-CR^{19}R^{20}R^{21}$, $-CR^{13}R^{14}-CR^{15}R^{16}-CR^{17}R^{18}-CR^{19}R^{20}-CR^{29}R^{30}-CR^{31}R^{32}R^{21}$, $-COOR^{28}$,

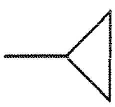 , 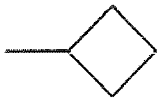 , 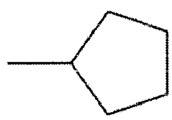 , 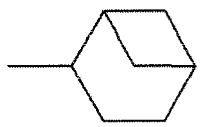 ,

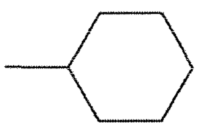 , 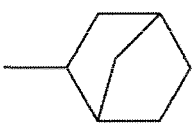 , 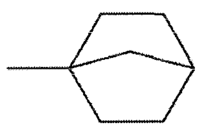 , 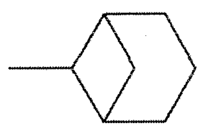 ,

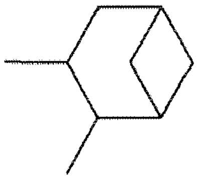 , 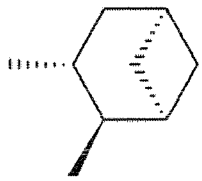 , 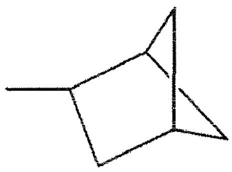 , 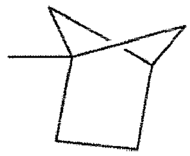 ,

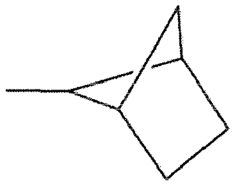 , 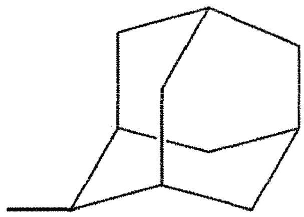 , 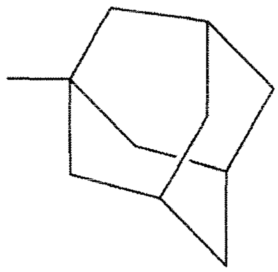 ,

these $C_3$-$C_6$-cycloalkyl groups may further be substituted by one, two, three, four, five or more substituents selected from the group consisting of $R^{33}$ - $R^{48}$;

**$R^{22}$, $R^{27}$,** and **$R^{28}$** are independently selected from $-CR^{49}R^{50}R^{51}$, $-CR^{49}R^{50}-CR^{52}R^{53}R^{51}$, $-CR^{49}R^{50}-CR^{52}R^{53}-CR^{54}R^{55}-CR^{56}R^{57}-CR^{58}R^{59}R^{51}$, $-CR^{49}R^{50}-CR^{52}R^{53}-CR^{54}R^{55}R^{51}$, $-CR^{49}R^{50}-CR^{52}R^{53}-CR^{54}R^{55}-CR^{56}R^{57}R^{51}$, $-CR^{49}R^{50}-CR^{52}R^{53}-CR^{54}R^{55}-CR^{56}R^{57}-CR^{58}R^{59}-CR^{60}R^{61}R^{51}$, $-CH_2Ph$, $-CH_2Ph$ the phenyl group of which may further be substituted by one, two, three, four or five substituents selected from the group consisting of $R^5$ - $R^{12}$;

$C_3$-$C_6$-cycloalkyl groups listed for $R^{26}$, which may further be substituted by one, two, three, four, five or more substituents selected from the group consisting of $R^{33}$ - $R^{48}$;

**$R^{49}$ - $R^{61}$** represent independently of each other -H, $-CH_3$, $-C_2H_5$, $-C_3H_7$, $-C_4H_9$, -F, -Cl, -Br, -I, -OH, $-NO_2$, $-NH_2$;

**$R^{23}$** and **$R^{24}$** are independently selected from -H, $-CR^{49}R^{50}R^{51}$, $-CR^{49}R^{50}-CR^{52}R^{53}R^{51}$, $-CR^{49}R^{50}-CR^{52}R^{53}-CR^{54}R^{55}-CR^{56}R^{57}-CR^{58}R^{59}R^{51}$, $-CR^{49}R^{50}-CR^{52}R^{53}-CR^{54}R^{55}R^{51}$, $-CR^{49}R^{50}-CR^{52}R^{53}-CR^{54}R^{55}-CR^{56}R^{57}R^{51}$, $-CR^{49}R^{50}-CR^{52}R^{53}-CR^{54}R^{55}-CR^{56}R^{57}-CR^{58}R^{59}-CR^{60}R^{61}R^{51}$, $-CR^{49}R^{50}-CR^{52}R^{53}-O-R^{51}$, $-CR^{49}R^{50}-CR^{52}R^{53}-CR^{54}R^{55}-O-R^{51'}$, $-CR^{49}R^{50}-CR^{52}R^{53}-NR^{51'}R^{51''}$, $-CR^{49}R^{50}-CR^{52}R^{53}-CR^{54}R^{55}-NR^{51'}R^{51''}$, $-CR^{49}R^{50}-CR^{52}R^{53}-CR^{54}R^{55}-CR^{56}R^{57}-NR^{51'}R^{51''}$, $-CR^{49}R^{50}-CR^{52}R^{53}-CR^{54}R^{55}-CR^{56}R^{57}$ $-CR^{58}R^{59}-NR^{51'}R^{51''}$, phenyl, substituted phenyl, benzyl, substituted benzyl, or both residues **$R^{23}$** and **$R^{24}$** together form with the nitrogen atom to which they are attached a azetidine, pyrrolidine, piperidine, piperazine, azepane, or morpholine ring;

**$R^{51'}$** and **$R^{51''}$** represent independently of each other -H, $-CH_3$, $-C_2H_5$, $-C_3H_7$, $-C_4H_9$, $-CH_2Ph$, $-COOC(CH_3)_3$, $-COOCH_3$, $-COOCH_2CH_3$, $-COOCH_2CH_2CH_3$, $-COOCH(CH_3)_2$, $-COOCH_2Ph$, $-COCH_3$;

and **$R^{25}$** is -H, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$;

**$R^4$** is selected from -H, $-NO_2$, $NH_2$, -CN, -F, -Cl, -Br, -I, $-CR^{62}R^{63}R^{64}$,

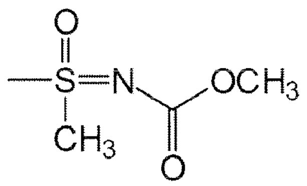 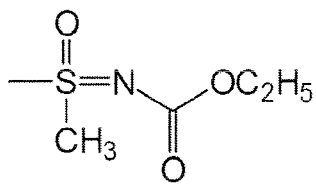 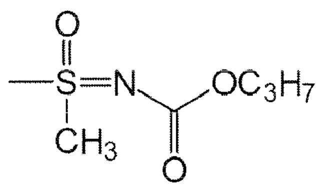

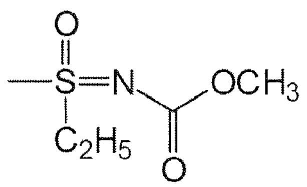 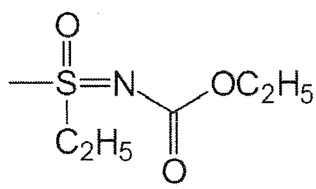 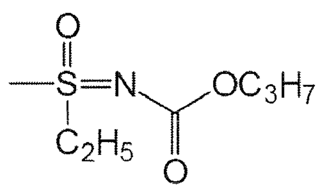

$-CONH_2$, $-SO_2CH_3$, $-SO_2C_2H_5$, $-SO_2C_3H_7$, $-NH-SO_2-CH_3$, $-NH-SO_2-C_2H_5$, $-NH-SO_2-C_3H_7$, $-NHCO-CH_3$, $-NHCO-C_2H_5$, $-NHCO-C_3H_7$, $-SO_2NR^{23}R^{24}$, $-CH_2-SO_2NR^{23}R^{24}$, $-C_2H_4-SO_2NR^{23}R^{24}$, $-C_3H_6-SO_2NR^{23}R^{24}$, $-SO_2-NH_2$, $-CH_2-SO_2NH_2$, $-C_2H_4-SO_2NH_2$, $-C_3H_6-SO_2NH_2$, $-O-CR^{62}R^{63}-CR^{65}R^{66}R^{64}$, $-O-CR^{62}R^{63}-CR^{65}R^{66}-CR^{67}R^{68}R^{64}$, $-CR^{62}R^{63}-CR^{65}R^{66}-CR^{67}R^{68}-CR^{69}R^{70}R^{64}$,- $O-CR^{62}R^{63}-CR^{65}R^{66}-CR^{67}R^{68}-CR^{69}R^{70}R^{64}$, $-CR^{62}R^{63}-CR^{65}R^{66}-CR^{67}R^{68}R^{64}$ , $-O-CR^{62}R^{63}-CR^{65}R^{66}-CR^{67}R^{68}-CR^{69}R^{70}-CR^{71}R^{72}R^{64}$, $-CR^{62}R^{63}-CR^{65}R^{66}R^{64}$, $-O-CR^{62}R^{63}-CR^{65}R^{66}-CR^{67}R^{68}-CR^{69}R^{70}-CR^{71}R^{72}-CR^{73}R^{74}R^{64}$, $-O-CR^{62}R^{63}R^{64}$, $-CR^{62}R^{63}-CR^{65}R^{66}-CR^{67}R^{68}-CR^{69}R^{70}-CR^{71},R^{72}R^{64}$, $-CR^{62}R^{63}-CR^{65}R^{66}-CR^{67}R^{68}-CR^{69}R^{70}-CR^{71}R^{72}-CR^{73}R^{74}R^{64}$, $-OCH_2Ph$,

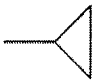 , 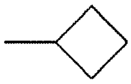 , 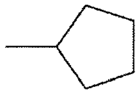 , 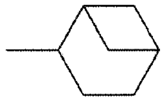 ,

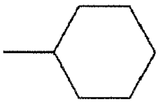 , 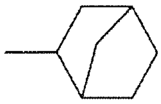 , 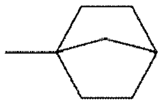 , 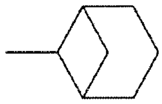 ,

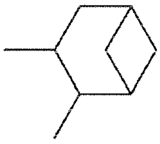 , 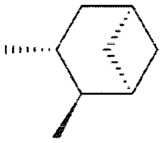 , 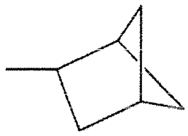 , 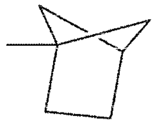 ,

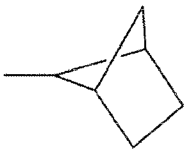 , 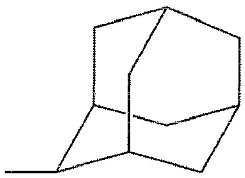 , 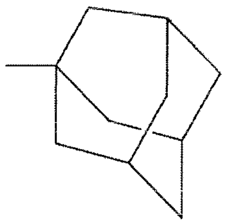 ,

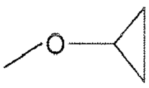 , 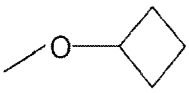 , 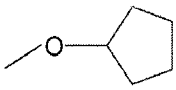 , 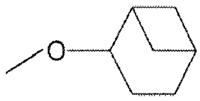 ,

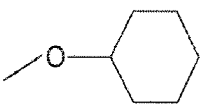 , 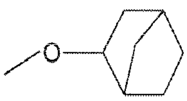 , 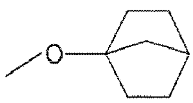 , 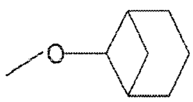 ,

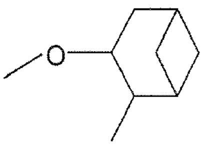 , 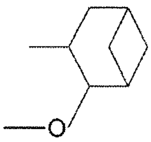 , 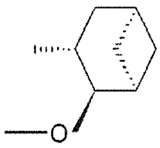 , 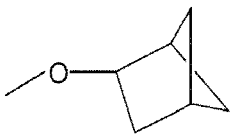 ,

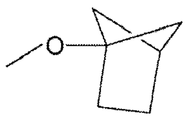 , 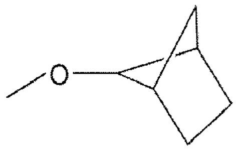 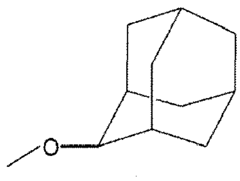 , 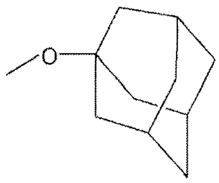 ,

these $C_3$-$C_6$-cycloalkoxy groups and $C_3$-$C_6$-cycloalkyl groups may further be substituted by one, two, three, four, five or more substituents selected from the group consisting of $R^{33}$ - $R^{48}$;

**$R^{62}$ - $R^{74}$** represent independently of each other -H, -cyclo-$C_3H_5$, -cyclo-$C_4H_7$, -cyclo-$C_5H_9$, -$CR^{75}R^{76}R^{77}$, -$CR^{75}R^{76}$-$CR^{78}R^{79}R^{77}$, -$CR^{75}R^{76}$-$CR^{78}R^{79}$-$CR^{80}R^{81}R^{77}$, -$CR^{75}R^{76}$-$CR^{78}R^{79}$-$CR^{80}R^{79}$-$CR^{82}R^{81}R^{77}$, -F, -Cl, -Br, -I, -Ph;

**$R^{75}$ - $R^{82}$** represent independently of each other -H, -F -Cl, -Br, -I, -$NH_2$;

**$R^4$** together with $R^{22}$, $R^{23}$ $R^{24}$ or $R^{25}$ may form a group -$CH_2CH_2$- or -$CH_2CH_2CH_2$- if $R^4$ is attached ortho to -L-$R^3$;

**$R^2$** is

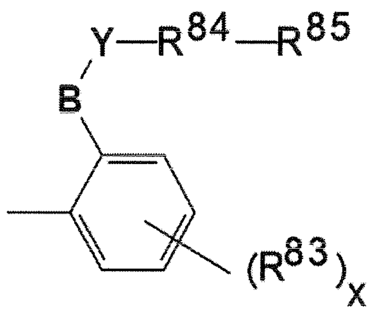 or 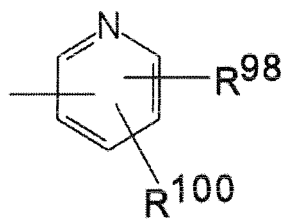 ;

**$R^{83}$** is selected from -H, -OH, $NO_2$, -CN, -F, -Cl, -Br, -I, , -$CF_3$, -$NR^{23'}R^{24'}$, -$CR^{62}R^{63}R^{64}$, -$CR^{62}R^{63}$-$NR^{23'}R^{24'}$, -$CR^{62}R^{63}$-$CR^{65}R^{66}R^{64}$, -$CR^{62}R^{63}$-$CR^{65}R^{66}$-$NR^{23'}R^{24'}$, -$CR^{62}R^{63}$-$CR^{65}R^{66}$-$CR^{67}R^{68}R^{64}$, -$CR^{62}R^{63}$-$CR^{65}R^{66}$-$CR^{67}R^{68}$-$NR^{23'}R^{24'}$, -O-$CR^{62}R^{63}R^{64}$, -O-$CR^{62}R^{63}$-$CR^{65}R^{66}R^{64}$ , -O-$CR^{62}R^{63}$-$CR^{65}R^{66}$-$CR^{67}R^{68}R^{64}$, -CHO, -$CH_2OH$, -$CR^{23'}O$, -$CH_2OR^{23'}$;

**$R^{23'}$** and **$R^{24'}$** represent independently of each other -H, -$CH_3$, -$C_2H_5$, -$C_3H_7$, -$CH(CH_3)_2$, -$C_4H_9$, -$CH_2$-$CH(CH_3)_2$, -$CH(CH_3)$-$C_2H_5$, -$C(CH_3)_3$; -(cyclo-$C_3H_5$);

x is a value between 0 and 3;

**B** is a bond, -$CR^{86}R^{87}$-, -$CR^{86}R^{87}$-$CR^{88}R^{89}$-, -$CR^{86}R^{87}$-$CR^{88}R^{89}$-$CR^{90}R^{91}$-, -$CR^{86}R^{87}$-$CR^{88}R^{89}$-$CR^{90}R^{91}$-$CR^{92}R^{93}$-, -$CR^{86}R^{87}$-$CR^{88}R^{89}$-$CR^{90}R^{91}$-$CR^{92}R^{93}$-$CR^{94}R^{95}$-, -$CR^{86}R^{87}$-$CR^{88}R^{89}$-$CR^{90}R^{91}$-$CR^{92}R^{93}$-$CR^{94}R^{95}$-$CR^{96}R^{97}$-;

**$R^{86}$** - **$R^{97}$** represent independently of each other -H, -$CH_3$, -$C_2H_5$, -$C_3H_7$, -$C_4H_9$, -F, -Cl, -Br, -I;

**Y** is a bond, -O-, -S-, -SO-, -$SO_2$-, -$SO_2NH$-, -$NHSO_2$-, -CO-, -COO-, -OOC-, -CONH-, -NHCO-, -NH-, -$N(CH_3)$-, -NH-CO-NH-, -O-CO-NH-, -NH-CO-O-;

**$R^{84}$** is selected from a bond, -$CR^{86}R^{87}$-, -$CR^{86}R^{87}$-$CR^{88}R^{89}$-$CR^{90}R^{91}$-, -$CR^{86}R^{87}$-$CR^{88}R^{89}$-$CR^{90}R^{91}$-$CR^{92}R^{93}$-, -$CR^{86}R^{87}$-$CR^{88}R^{89}$-$CR^{90}R^{91}$-$CR^{92}R^{93}$-$CR^{94}R^{95}$-, -$CR^{86}R^{87}$-$CR^{88}R^{89}$-, -$CR^{86}R^{87}$-$CR^{88}R^{89}$-$CR^{90}R^{91}$-$CR^{92}$-$R^{93}$-$CR^{94}R^{95}$-$CR^{96}R^{97}$-;

**$R^{85}$** is selected from

(i) -H, -OH, -$OCH_3$, -$OC_2H_5$, -$OC_3H_7$, -O-cyclo-$C_3H_5$, -$OCH(CH_3)_2$, -$OC(CH_3)_3$, -$OC_4H_9$, -Ph, -OPh, -$OCH_2$-Ph, -$OCPh_3$, -SH, -$SCH_3$, -$SC_2H_5$, -$SC_3H_7$, -S-cyclo-$C_3H_5$, -$SCH(CH_3)_2$, -$SC(CH_3)_3$, -$SC_4H_9$, -$NO_2$, -F, -Cl, -Br, -I, -$P(O)(OH)_2$, -$P(O)(OCH_3)_2$, -$P(O)(OC_2H_5)_2$, -$P(O)(OCH(CH_3)_2)_2$, -$Si(CH_3)_2(C(CH_3)_3)$, -$Si(C_2H_5)_3$, -$Si(CH_3)_3$, -CN, -CHO, -$COCH_3$, -$COC_2H_5$, -$COC_3H_7$, -CO-cyclo-$C_3H_5$, -$COCH(CH_3)_2$, -$COC(CH_3)_3$, -$COC_4H_9$, -COOH, -$COOCH_3$, -$COOC_2H_5$, -$COOC_3H_7$, -$COOC_4H_9$, -COO-cyclo-$C_3H_5$, -$COOCH(CH_3)_2$, -$COOC(CH_3)_3$, -OOC-$CH_3$, -OOC-$C_2H_5$, -OOC-$C_3H_7$, -OOC-$C_4H_9$, -OOC-cyclo-$C_3H_5$, -OOC-$CH(CH_3)_2$, -OOC-$C(CH_3)_3$, -$CONR^{23'}R^{24'}$, -$NHCOCH_3$, -$NHCOC_2H_5$, -$NHCOC_3H_7$, NHCO-cyclo-$C_3H_5$, -NH-CO-$CH(CH_3)_2$, -$NHCOC_4H_9$, -NHCO-$C(CH_3)_3$, -NHCO-$OCH_3$, -NHCO-$OC_2H_5$, -NHCO-$OC_3H_7$, -NHCO-O-cyclo-$C_3H_5$, -NHCO-$OC_4H_9$, NHCO-$OCH(CH_3)_2$, -NHCO-$OC(CH_3)_3$, -NHCO-$OCH_2Ph$, -$NR^{23}R^{24}$, -$CF_3$, -$SOCH_3$, -$SOC_2H_5$, -$SOC_3H_7$, -SO-cyclo-$C_3H_5$, -$SOCH(CH_3)_2$, -$SOC(CH_3)_3$, -$SO_2CH_3$, -$SO_2C_2H_5$, -$SO_2C_3H_7$, -$SO_2$-cyclo-$C_3H_5$, -$SO_2CH(CH_3)_2$, -$SO_2C_4H_9$, -$SO_2C(CH_3)_3$, -$SO_3H$, -$SO_2NR^{23'}R^{24'}$, -$OCF_3$,

$-OC_2F_5$, $-O-COOCH_3$, $-O-COOC_2H_5$, $-O-COOC_3H_7$, $-O-COO$-cyclo-$C_3H_5$, $-O-COOC_4H_9$, $-O-COOCH(CH_3)_2$, $-O-COOCH_2Ph$, $-O-COOC(CH_3)_3$, $NH-CO-NH_2$, $-NH-CO-NHCH_3$, $-NH-CO-NHC_2H_5$, $-NH-CO-NHC_3H_7$, $-NH-CO-NHC_4H_9$, $-NH-CO-NH$-cyclo-$C_3H_5$, $-OCH_2$-cyclo-$C_3H_5$, $-NH-CO-NH[CH(CH_3)_2]$, $-NH-CO-NH[C(CH_3)_3]$, $-NH-CO-N(CH_3)_2$, $-NH-CO-N(C_2H_5)_2$, $-NH-CO-N(C_3H_7)_2$, $-NH-CO-N(C_4H_9)_2$, $-NH-CO-N$(cyclo-$C_3H_5)_2$, $-NH-CO-N[CH(CH_3)_2]_2$, $-NH-CO-N[C(CH_3)_3]_2$, $-NH-C(=NH)-NH_2$, $-NH-C(=NH)-NHCH_3$, $-NH-C(=NH)-NHC_2H_5$, $-NH-C(=NH)-NHC_3H_7$, $-NH-C(=NH)-NHC_4H_9$, $-NH-C(=NH)-NH$-cyclo-$C_3H_5$, $-NH-C(=NH)-NH[CH(CH_3)_2]$, $-NH-C(=NH)-NH[C(CH_3)_3]$, $NH-C(=NH)-N(CH_3)_2$, $-NH-C(=NH)-N(C_2H_5)_2$, $-NH-C(=NH)-N(C_3H_7)_2$, $-NH-C(=NH)-N$(cyclo-$C_3H_5)_2$, $-NH-C(=NH)-N(C_4H_9)_2$, $-NH-C(=NH)-N[CH(CH_3)_2]_2$, $-NH-C(=NH)-N[C(CH_3)_3]_2$, $-O-CO-NH_2$, $-O-CO-NHCH_3$, $-O-CO-NHC_2H_5$, $-O-CO-NHC_3H_7$, $-O-CO-NHC_4H_9$, $-O-CO-NH$-cyclo-$C_3H_5$, $-O-CO-NH[CH(CH_3)_2]$, $-O-CO-NH[C(CH_3)_3]$, $-O-CO-N(CH_3)_2$, $-O-CO-N(C_2H_5)_2$, $-O-CO-N(C_3H_7)_2$, $-O-CO-N(C_4H_9)_2$, $-O-CO-N$(cyclo-$C_3H_5)_2$, $-O-CO-N[CH(CH_3)_2]_2$, $-O-CO-N[C(CH_3)_3]_2$,

(ii) an aromatic or heteroaromatic mono- or bicyclic ring selected from 2-thienyl, 3-thienyl, 2-furanyl, 3-furanyl, 2-oxazolyl, 3-oxazolyl, 4-oxazolyl, 2-thiazolyl, 3-thiazolyl, 4-thiazolyl, 1-pyrazolyl, 3-pyrazolyl, 4-pyrazolyl, 5-pyrazolyl, 1-imidazolyl, 2-imidazolyl, 4-imidazolyl, 5-imidazolyl, phenyl, 1-naphthyl, 2-naphthyl, 2-pyridyl, 3-pyridyl, 4-pyridyl, 2-pyrimidinyl, 4-pyrimidinyl, 5-pyrimidinyl, 2-pyrazinyl, 3-pyridazinyl, 4-pyridazinyl, 1,3,5-triazin-2-yl,

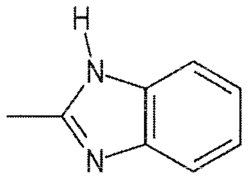 , 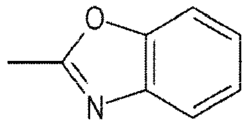 , 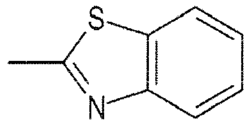 ,

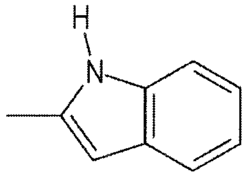 , 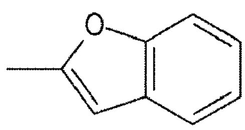 , 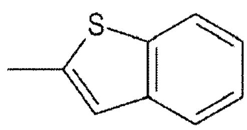 ,

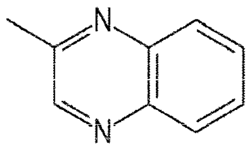 , 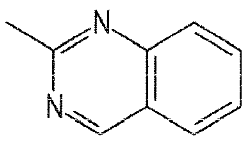 , 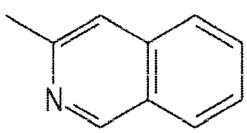 ,

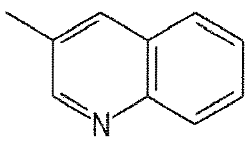 , 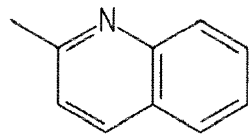 , 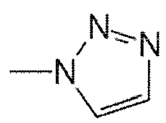 ,

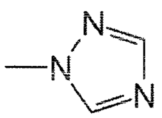 , 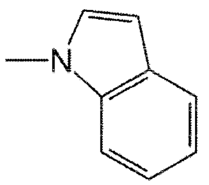 , 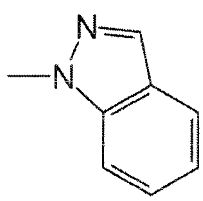 , 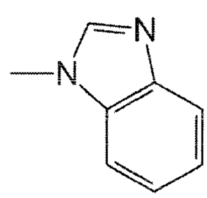 ,

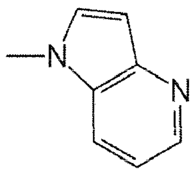, 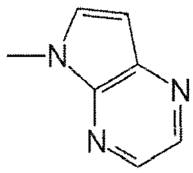, 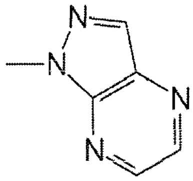, 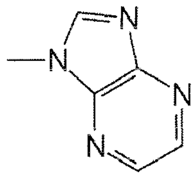,

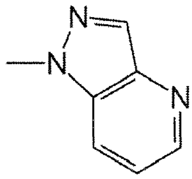, 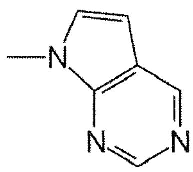, 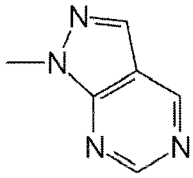, 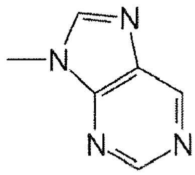,

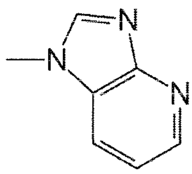, 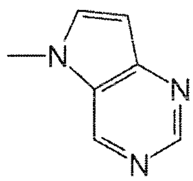, 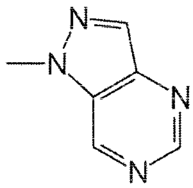, 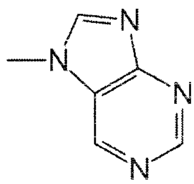,

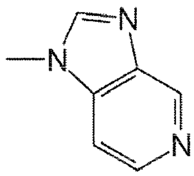 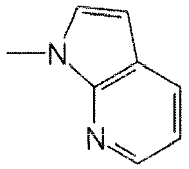, 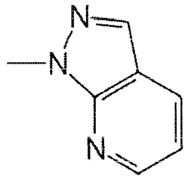, 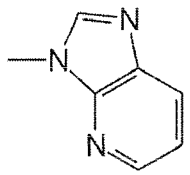,

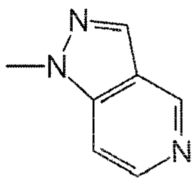, 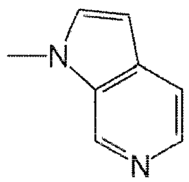, 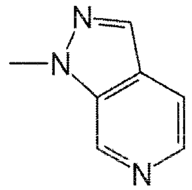, 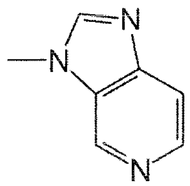,

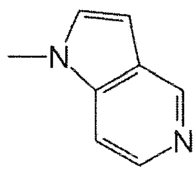, 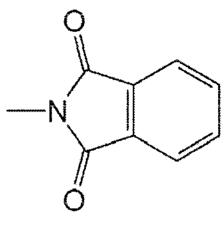.

which optionally may be substituted by one or two substituents selected from -F, -Cl, - Br, -I, $-OCH_3$, $-CH_3$, $-NO_2$, -CN, $-CF_3$;

(iii) a saturated ring selected from cyclopentyl, azetidin-1-yl,

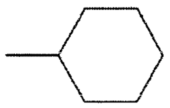,
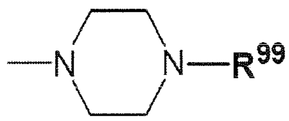,
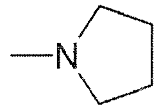,

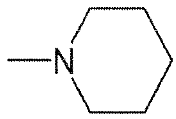,
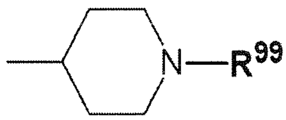,
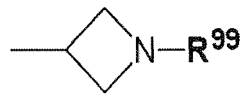,

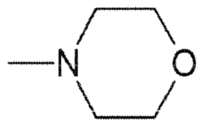;
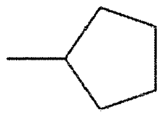,
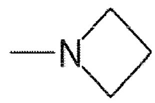;

**$R^{99}$** represents -H, $-CH_3$, $-CH_2Ph$, $-COOC(CH_3)_3$, $-COOCH_3$, $-COOCH_2CH_3$, $-COOCH_2CH_2CH_3$, $-COOCH(CH_3)_2$, $-COOCH_2Ph$, $-COCH_3$;
the group -B-Y-$R^{84}$-$R^{85}$ together with one substituent $R^{83}$ may form a group $-OCH_2O-$, if $R^{83}$ is attached in position ortho to -B-Y-$R^{84}$-$R^{85}$;
with the proviso that $R^{83}$ is not -H if the group -B-Y-$R^{84}$-$R^{85}$ is hydrogen.
**$R^{98}$** is selected from $-NO_2$, -CN, -F, -Cl, -Br, -I, $-NH_2$, -OH, $-CR^{62}R^{63}R^{64}$, $-CR^{62}R^{63}$-$CR^{65}R^{66}R^{64}$, $-CR^{62}R^{63}$-$CR^{65}R^{66}$-$CR^{67}R^{68}R^{64}$, $-CR^{62}R^{63}$-$CR^{65}R^{66}$-$CR^{67}R^{68}$-$CR^{69}R^{70}R^{64}$, -O-$CR^{62}R^{63}R^{64}$, -O-$CR^{62}R^{63}$-$CR^{65}R^{66}R^{64}$, -O-$CR^{62}R^{63}$-$CR^{65}R^{66}$-$CR^{65}R^{68}R^{64}$, -O-$CR^{62}R^{63}$-$CR^{65}R^{66}$-$CR^{67}R^{68}$-$CR^{69}R^{70}R^{64}$, -O-$CR^{62}R^{63}$-$CR^{65}R^{66}$-$CR^{67}R^{68}$-$CR^{69}R^{70}$-$CR^{71}R^{72}R^{64}$, -O-$CR^{62}R^{63}$-$CR^{65}R^{66}$-$CR^{67}R^{68}$-$CR^{69}R^{70}$-$CR^{71}R^{72}$-$CR^{73}R^{74}R^{64}$, $-CR^{62}R^{63}$-O-$R^{65}R^{66}R^{64}$, $-CR^{62}R^{63}$-O-$CR^{65}R^{66}$-$CR^{67}R^{68}R^{64}$, $-CR^{62}R^{63}$-O-$CR^{65}R^{66}$-$CR^{67}R^{68}$-$CR^{69}R^{70}R^{64}$, $-CR^{62}R^{63}$-O-$CR^{65}R^{66}$-$CR^{67}R^{68}$-$CR^{69}R^{70}$-$CR^{71}R^{72}R^{64}$, $-CR^{62}R^{63}$-O-$CR^{65}R^{66}$-$CR^{67}R^{68}$-$CR^{69}R^{70}$-$CR^{71}R^{72}$-$CR^{73}R^{74}R^{64}$ , $-CR^{62}R^{63}$-$CR^{65}R^{66}$-$CR^{67}R^{68}$-$CR^{69}R^{70}$-$CR^{71}R^{72}R^{64}$, $-CR^{62}R^{63}$-$CR^{65}R^{66}$-$CR^{67}R^{68}$-$CR^{69}R^{70}$-$CR^{71}R^{72}$-$CR^{73}R^{74}R^{64}$, $-OCH_2Ph$, $-OCH_2$-$CH_2$-Ph, $-CH_2$-O-$CH_2$-Ph;
with the proviso that $R^{98}$ is attached to a position ortho to the bond between the pyridine and the triazine ring if $R^{98}$ is not an amino group in para position to the bond between the pyridine and the triazine ring;
**$R^{100}$** is selected from -H, $-NO_2$, -CN, -F, -Cl, -Br, -I, $-NH_2$, -OH, $-CF_3$, $-CH_3$, $-CH_2CH_3$, $-CH_2CH_2CH_3$, $-CH(CH_3)_2$, $-OCH_3$, $-OCH_2CH_3$, $-OCH_2CH_2CH_3$, $-OCH(CH_3)_2$, $-OCF_3$, $-OCH_2Ph$;
and with the proviso that if $R^1$ is a phenyl moiety and $R^2$ is also a phenyl moiety a chloro substituent is only allowed on the $R^1$ phenyl moiety or on the $R^2$ phenyl moiety but not on both simultaneously;
and with the proviso that the compound 4-[4-(2-benzoylaminophenyl)-[1,3,5]triazin-2-ylamino]benzamide is excluded;
and enantiomers, stereoisomeric forms, mixtures of enantiomers, diastereomers, mixtures of diastereomers, prodrugs, hydrates, solvates, acid salt forms, tautomers, and racemates of the above mentioned compounds and pharmaceutically acceptable salts thereof or salts of solvates thereof.

**5.** The method of claim 4, wherein

**$R^1$** represents

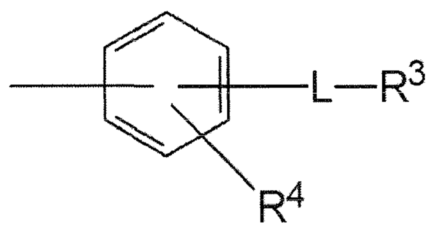

in which
**L** is a bond, $-CH_2-$, $-CH_2CH_2-$, or $-CF_2-$;
**$R^3$** is $-SO_2NH_2$, $-SO_2NH(CH_3)$, $-SO_2N(CH_3)_2$, $-SO_2NH(CH_2CH_2OCH_3)$, $-NHSO_2CH_3$, $-NHSO_2CH_2CH_3$, $-NH-SO_2CH_2CH_2CH_3$, $-NHSO_2CF_3$, $-SO_2CH_3$, $NHSO_2NH_2$, $-SO(NH)CH_3$;
**$R^4$** is -H, $-CH_3$, -F, -Cl, or $-CF_3$;
**$R^2$** represents

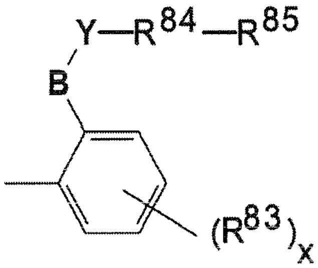

or

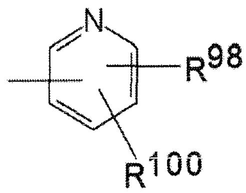

;

in which the group **$-B-Y-R^{84}-R^{85}$** is $-OCH_3$, $-OCH_2CH_3$, $-OCH_2CH_2CH_3$, $-OCH_2CH_2CH_2CH_3$, $-OCH(CH_3)_2$, -OPh, $-OCH_2Ph$, $-OCH_2$(4-pyridyl);
**$R^{83}$** is -H, -F, or -Cl;
x is 0, 1, or 2;
**$R^{98}$** is $-OCH_3$ and **$R^{100}$** is -H,
with the proviso that $R^{98}$ is attached to a position ortho to the bond between the pyridine and the triazine ring.

**6.** The method of claim 4 or 5, wherein

**$R^1$** represents

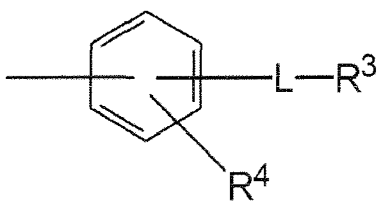

in which
the substituent **$-L-R^3$** is $-SO_2NH_2$, $-CH_2SO_2NH_2$, $-CH_2CH_2SO_2NH_2$, $-CF_2SO_2NH_2$, $-NHSO_2NH_2$, $-CH_2NHSO_2NH_2$, $-SO_2CH_3$, $-SO(NH)CH_3$, $-CH_2SO(NH)CH_3$;
**$R^4$** is -H;
**$R^2$** represents 2-methoxyphenyl, 4-fluoro-2-methoxyphenyl, or 6-fluoro-2-methoxyphenyl.

**7.** The method of claim 4, wherein

$R^1$ is

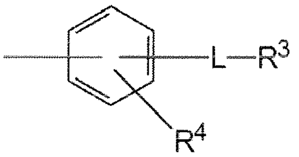

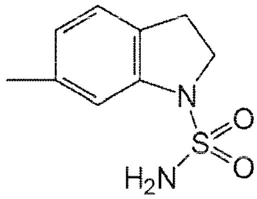

or

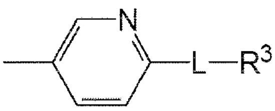

**L** is a bond, $-CH_2-$, or $-CH_2CH_2-$;
**$R^3$** is -H, $-SO_2NR^{23}R^{24}$, $-CONR^{23}R^{24}$, $-NO_2$, $-NH_2$, $-NHSO_2R^{22}$, $NHCOR^{22}$, $-SO_2R^{22}$, -NH-CO-NH-Ph, or -Ph,
**$R^4$** is -H, $-CH_2-SO_2NR^{23}R^{24}$, $-SO_2NR^{23}R^{24}$, $-CONH_2$, $-C_2H_4-SO_2NR^{23}R^{24}$, $-NH-SO_2-CH_3$, $-NH-SO_2-C_2H_5$, $-NH-SO_2-C_3H_7$, $N-H-CO-CH_3$, $-NHCO-C_2H_5$, $-NO_2$, $-NH_2$, $-SO_2CH_3$, or

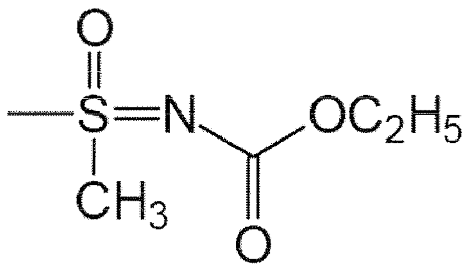

$R^{23}$ and $R^{24}$ are independently selected from -H, $-CH_3$, $-C_2H_5$, $-C_3H_7$, -(cyclo-$C_3H_5$), - $CH_2$-$CH_2$-$CH_2$-$CH_2$-$NH_2$, or -$CH_2$-$CH_2$-$CH_2$-$CH_2$-NH-COOC$(CH_3)_3$,
**$R^2$** represents

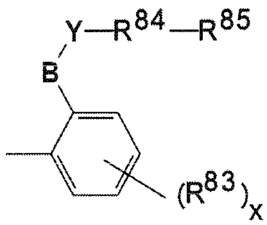 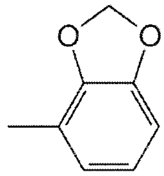 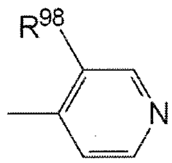 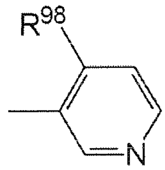

or

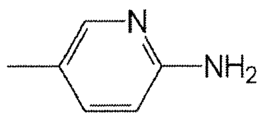

**B** is a bond or $-CH_2-$;
**Y** is a bond, -O-, or -NH-;
**$R^{83}$** is selected from -H, -CN, -F, -Cl, -O-$CR^{62}R^{63}R^{64}$, $-CF_3$, $-CH_2OR^{23'}$, $-CR^{23'}O$, $-CR^{62}R^{63}$-$NR^{23'}R^{24'}$, $-CR^{62}R^{63}R^{64}$;
**$R^{23'}$** and **$R^{24'}$** represent independently of each other -H, $-CH_3$, -(cyclo-$C_3H_5$);
**$R^{62}$** - **$R^{64}$** represent independently of each other -H, $-CH_3$, -Ph, -F, -cyclo-$C_3H_5$;
**$R^{84}$** is selected from a bond, $-CH_2-$, or -$CH_2$-$CH_2$-$CH_2$-$CH_2$-;
**$R^{85}$** is selected from -H, $-CF_3$, $-OCH_3$, $-OCH(CH_3)_2$, -CN, $-NHCOCH_3$, $-OCH_2$-cyclo-$C_3H_5$, $-NR^{23}R^{24}$, -Ph, -OPh, -NHCO-OC$(CH_3)_3$,

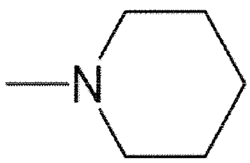 , 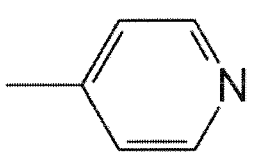 , 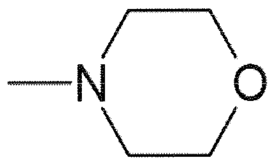 ;

**$R^{98}$** represents $-OCH_3$;
and salts, solvates or salts of solvates of the afore-mentioned compounds and especially the hydrochloride salt or the trifluoroacetate salt of these compounds.

**8.** The method of claim 4, wherein

**$R^1$** represents

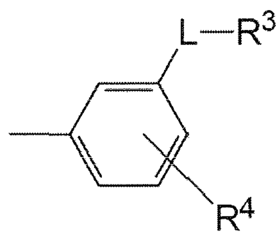

in which
the substituent -L-$R^3$ is -$SO_2NH_2$ or -$CH_2SO_2NH_2$,
$R^4$ is -H;
$R^2$ represents 2-methoxyphenyl, 4-fluoro-2-methoxyphenyl or 2-benzyloxyphenyl,
or their salts, solvates or salts of solvates and especially the hydrochloride salt or the trifluoroacetate salt.

**9.** The method of claim 4, wherein the compound is selected from the group consisting of

3-[(4-(2-Methoxyphenyl)-1,3,5-triazin-2-yl)amino]benzenemethanesulfonamide,
3-[(4-(4-Fluoro-2-methoxyphenyl)-1,3,5-triazin-2-yl)amino]benzenemethane-sulfonamide,
3-[(4-(5-Fluoro-2-methoxyphenyl)-1,3,5-triazin-2-yl)amino]benzenemethane-sulfonamide,
3-[(4-(6-Fluoro-2-methoxyphenyl)-1,3,5-triazin-2-yl)amino]benzenemethane-sulfonamide,
3-[(4-(3,5-Difluoro-2-methoxyphenyl)-1,3,5-triazin-2-yl)amino]benzenemethanesulfonamide,
3-[(4-(4-Chloro-2-methoxyphenyl)-1,3,5-triazin-2-yl)amino]benzenemethane-sulfonamide,
3-[(4-(5-Chloro-2-methoxyphenyl)-1,3,5-triazin-2-yl)amino]benzenemethane-sulfonamide,
3-[(4-(2-Methoxy-4-trifluorometyl-phenyl)-1,3,5-triazin-2-yl)amino]benzenemethanesulfonamide,
3-[(4-(2-Methoxy-5-trifluoromethyl-phenyl)-1,3,5-triazin-2-yl)amino]benzenemethanesulfonamide,
3-[(4-(5-Hydroxymethyl-2-methoxyphenyl)-1,3,5-triazin-2-yl)amino]benzenemethanesulfonamide,
3-[(4-(5-Formyl-2-methoxyphenyl)-1,3,5-triazin-2-yl)amino]benzenemethane-sulfonamide,
3-[(4-(2-Ethoxyphenyl)-1,3,5-triazin-2-yl)amino]benzenemethanesulfonamide,
3-[(4-(2-Benzyloxyphenyl)-1,3,5-triazin-2-yl)amino] benzenemethanesulfonamide,
1-(3-{ [4-(2-phenoxyphenyl)-1,3,5-triazin-2-yl]amino }phenyl)methanesulfonamide,
3-[(4-(1,3-Benzodioxol-4-yl)-1,3,5-triazin-2-yl)amino]benzenemethanesulfonamide,
3-[(4-(2-((4-Pyridinyl)methoxy)phenyl)-1,3,5-triazin-2-yl)amino]benzenemethanesulfonamide,
3-[(4-(2-(4-(tert-Butoxycarbonylamino)butoxy)phenyl)-1,3,5-triazin-2-yl)amino]-benzenemethanesulfonamide,
3-[(4-(4-Methoxypyridin-3-yl)-1,3,5-triazin-2-yl)amino]benzenemethane-sulfonamide,
3-[(4-(3-Methoxypyridin-4-yl)-1,3,5-triazin-2-yl)amino]benzenemethane-sulfonamide,
3-[(4-(2-((Morpholin-4-yl)methyl)phenyl)-1,3,5-triazin-2-yl)amino]benzenemethanesulfonamide,
3-[(4-(2-((Piperidin-1-yl)methyl)phenyl)-1,3,5-triazin-2-yl)amino]benzenemethanesulfonamide,
3-[(4-(2-(Cyclopropylamino-methyl)phenyl)-1,3,5-triazin-2-yl)amino]benzenemethanesulfonamide,
3-[(4-(6-Aminopyridin-3-yl)-1,3,5-triazin-2-yl)amino]benzenemethane-sulfonamide,
3-[(4-(2-(Methoxymethyl)phenyl)-1,3,5-triazin-2-yl)amino]benzene-methanesulfonamide,
3-[(4-(2-Methoxyphenyl)-1,3,5-triazin-2-yl)amino]benzenesulfonamide,
2-[3-((4-(2-Methoxyphenyl)-1,3,5-triazin-2-yl)amino)phenyl]ethanesulfonamide,
2-[3-((4-(4-Fluoro-2-methoxyphenyl)-1,3,5-triazin-2-yl)amino)phenyl]ethane-sulfonamide,
3-[(4-(2-Methoxyphenyl)-1,3,5-triazin-2-yl)amino]benzamide,
6-[(4-(2-Methoxyphenyl)-1,3,5-triazin-2-yl)amino]-2,3-dihydro-1H-indole-1-sulfonamide, rac-S-[3-((4-(2-Methoxyphenyl)-1,3,5-triazin-2-yl)amino)phenyl]-N-ethoxy-carbonyl-S-methyl-sulfoximide,
4-(2-Methoxyphenyl)-N-(3-nitrophenyl)-1,3,5-triazine-2-amine,
3-[(4-(2-(4-Aminobutoxy)phenyl)-1,3,5-triazin-2-yl)amino]benzenemethane-sulfonamide,
N-(3-Aminophenyl)-4-(2-methoxyphenyl)-1,3,5-triazine-2-amine,
N-[3-((4-(2-Methoxyphenyl)-1,3,5-triazin-2-yl)amino)phenyl]-methanesulfonamide,
N-[3-((4-(2-Methoxyphenyl)-1,3,5-triazin-2-yl)amino)phenyl]-propanesulfonamide,
N-[3-((4-(2-Methoxyphenyl)-1,3,5-triazin-2-yl)amino)phenyl]acetamide,
N-[3-((4-(2-Methoxyphenyl)-1,3,5-triazin-2-yl)amino)phenyl]-N'-phenyl-urea,
3-[(4-(2-Methoxy-5-(methylamino-methyl)phenyl)-1,3,5-triazin-2-yl)amino]-benzenemethanesulfonamide,
4-(2-Methoxyphenyl)-N-phenyl-1,3,5-triazine-2-amine,
tert-Butyl-[4-((3-((4-(4-Fluoro-2-methoxyphenyl)-1,3,5-triazin-2-yl)amino)phenyl)-methylsulfonamido) butyl]carbamate,
N-(4-Aminobutyl)-1-[3-((4-(4-fluoro-2-methoxyphenyl)-1,3,5-triazin-2-yl)amino)-phenyl]methanesulfonamide,
4-(2-Methoxyphenyl)-N-(3-(methylsulfonyl)phenyl)-1,3,5-triazin-2-amine,
4-[(4-(2-Methoxyphenyl)-1,3,5-triazin-2-yl)amino]benzenemethane-sulfonamide,
1-[3-({4-[4-fluoro-2-(trifluoromethyl)phenyl]-1,3,5-triazin-2-yl}amino)phenyl]-methanesulfonamide,
1-[3-({4-[4-fluoro-2-(propan-2-yloxy)phenyl]-1,3,5-triazin-2-yl}amino)phenyl]-methanesulfonamide,
1-(3-{[4-(2-cyano-4-fluorophenyl)-1,3,5-triazin-2-yl]amino}phenyl)methane-sulfonamide,
N-[5-fluoro-2-(4-{[3-(sulfamoylmethyl)phenyl]amino}-1,3,5-triazin-2-yl)phenyl]-acetamide,
1-[3-({4-[2-(cyclopropylmethoxy)-4-fluorophenyl]-1,3,5-triazin-2-yl}amino)phenyl]-methanesulfonamide,
1-(3-{[4-(3,4-difluoro-2-methoxyphenyl)-1,3,5-triazin-2-yl]amino}phenyl)methane-sulfonamide,
1-(3-{[4-(4,5-difluoro-2-methoxyphenyl)-1,3,5-triazin-2-yl]amino}phenyl)methane-sulfonamide,
4-(4-fluoro-2-methoxyphenyl)-N-[6-(methylsulfonyl)pyridin-3-yl]-1,3,5-triazin-2-amine,
3-[(4-(2-Methoxyphenyl)-1,3,5-triazin-2-yl)amino]benzenemethanesulfonamide trifluoroacetic acid salt,
1-(3-{[4-(4-fluoro-2-methoxyphenyl)-1,3,5-triazin-2-yl]amino}phenyl)methane-sulfonamide hydrochloride,
3-[(4-(4-Fluoro-2-methoxyphenyl)-1,3,5-triazin-2-yl)amino]benzene-methanesulfonamide trifluoroacetic acid salt,
3-[(4-(2-Benzyloxyphenyl)-1,3,5-triazin-2-yl)amino] benzenemethanesulfon-amide trifluoroacetic acid salt,
3-[(4-(2-Methoxyphenyl)-1,3,5-triazin-2-yl)amino]benzenesulfonamide trifluoroacetic acid salt.

**10.** The method of any of claims 1 to 3, wherein said inhibitor is selected from the group consisting of

3-[(4-(2-Methoxyphenyl)-1,3,5-triazin-2-yl)amino]benzenemethanesulfonamide (Cpd B1);
3-[(4-(4-Fluoro-2-methoxyphenyl)-1,3,5-triazin-2-yl)amino] benzenemethanesulfonamide (Cpd B2);
3-[(4-(5-Fluoro-2-methoxyphenyl)-1,3,5-triazin-2-yl)amino] benzenemethanesulfonamide (Cpd B3);
3-[(4-(6-Fluoro-2-methoxyphenyl)-1,3,5-triazin-2-yl)amino] benzenemethanesulfonamide (Cpd B4);
3-[(4-(4-Chloro-2-methoxyphenyl)-1,3,5-triazin-2-yl)amino] benzenemethanesulfonamide (Cpd B6);
3-[(4-(5-Chloro-2-methoxyphenyl)-1,3,5-triazin-2-yl)amino] benzenemethanesulfonamide (Cpd B7);
3-[(4-(2-Ethoxyphenyl)-1,3,5-triazin-2-yl)amino]benzenemethanesulfonamide (Cpd B12);
3-[(4-(2-Benzyloxyphenyl)-1,3,5-triazin-2-yl)amino] benzenemethanesulfonamide (Cpd B13);
1-(3-{[4-(2-phenoxyphenyl)-1,3,5-triazin-2-yl]amino}phenyl)methanesulfonamide (Cpd B14);
3-[(4-(2-((4-Pyridinyl)methoxy)phenyl)-1,3,5-triazin-2-yl)amino]benzenemethanesulfonamide (Cpd B 16);
3-[(4-(2-(4-(tert-Butoxycarbonylamino)butoxy)phenyl)-1,3,5-triazin-2-yl)amino]benzenemethanesulfonamide (Cpd B 17);
3-[(4-(3-Methoxypyridin-4-yl)-1,3,5-triazin-2-yl)amino]benzenemethanesulfonamide (Cpd B18);
3-[(4-(6-Aminopyridin-3-yl)-1,3,5-triazin-2-yl)amino]benzenemethanesulfonamide (Cpd B23);
3-[(4-(2-Methoxyphenyl)-1,3,5-triazin-2-yl)amino]benzenesulfonamide (Cpd B24);
2-[3-((4-(2-Methoxyphenyl)-1,3,5-triazin-2-yl)amino)phenyl]ethanesulfonamide (Cpd C1);
N-[3-((4-(2-Methoxyphenyl)-1,3,5-triazin-2-yl)amino)phenyl]-methanesulfonamide (Cpd D1);
N-[3-((4-(2-Methoxyphenyl)-1,3,5-triazin-2-yl)amino)phenyl]-propanesulfonamide (Cpd L1);
tert-Butyl [4-((3-((4-(4-Fluoro-2-methoxyphenyl)-1,3,5-triazin-2-yl)amino)phenyl)methylsulfonamido) butyl]carbamate (Cpd Q1);
N-(4-Aminobutyl)-1-[3-((4-(4-fluoro-2-methoxyphenyl)-1,3,5-triazin-2-yl)amino)phenyl]methanesulfonamide (Cpd R1);
4-(2-Methoxyphenyl)-N-(3-(methylsulfonyl)phenyl)-1,3,5-triazin-2-amine (Cpd S1);
1-[3-({4-[4-Fluoro-2-(propan-2-yloxy)phenyl]-1,3,5-triazin-2-yl}amino)phenyl]methanesulfonamide (Cpd U2);
1-[3-({4-[2-(Cyclopropylmethoxy)-4-fluorophenyl]-1,3,5-triazin-2-yl}amino)phenyl]methanesulfonamide (Cpd U5);
1-(3-{[4-(4,5-Difluoro-2-methoxyphenyl)-1,3,5-triazin-2-yl]amino}phenyl)methanesulfonamide (Cpd U7);
3-[(4-(2-Methoxyphenyl)pyridin-2-yl)amino]benzenesulfonamide (Cpd 24);
4-(2-Methoxyphenyl)-N-(3-(methylsulfonyl)phenyl)pyridin-2-amine (Cpd 25);
[3-((4-(4-Fluoro-2-methoxyphenyl)pyridin-2-yl)amino)phenyl]methanesulfonamide (Cpd 26);
[3-((4-(2-Methoxyphenyl)pyridin-2-yl)amino)phenyl]methanesulfonamide (Cpd 27);
1-[3-((4-(4-Fluoro-2-methoxyphenyl)pyridm-2-yl)amino)phenyl]-N,N-dimethylmethanesulfonamide (Cpd 28);
2-[3-((4-(2-Methoxyphenyl)pyridin-2-yl)amino)phenyl]ethanesulfonamide (Cpd 29);
N-[3-((4-(4-Fluoro-2-methoxyphenyl)pyridin-2-yl)amino)phenyl]methanesulfonamide (Cpd 30);
N-[3-((4-(4-Fluoro-2-methoxyphenyl)pyridin-2-yl)amino)phenyl]acetamide (Cpd 31);
1-[3-((4-(4-Fluoro-2-methoxyphenyl)pyridin-2-yl)amino)phenyl]-N-propylmethanesulfonamide (Cpd 32);
(R)-Methyl 1-[4-((3-(Sulfamoylmethyl)phenyl)amino)-1,3,5-triazin-2-yl]piperidine-2-carboxylate (Cpd 33);

(R)-3-[(4-(2-(Methoxymethyl)pyrrolidin-1-yl)-1,3,5-triazin-2-yl)amino]benzenemethanesulfonamide (Cpd 34);
(R)-Methyl 1-[4-((3-(Sulfamoylmethyl)phenyl)amino)-1,3,5-triazin-2-yl]pyrrolidine-2-carboxylate)Cpd 35);
rac-3-[(4-(2-Phenylpyrrolidin-1-yl)-1,3,5-triazin-2-yl)amino]benzenemethanesulfonamide (Cpd 36);
(R)-3-[(4-(2-Phenylpyrrolidin-1-yl)-1,3,5-triazin-2-yl)amino]benzenemethanesulfonamide (Cpd 37);
3-[(4-(7,8-Dihydro-1,6-naphthyridin-6(5H)-yl)-1,3,5-triazin-2-yl)amino]benzenemethanesulfonamide (Cpd 38);
3-[(4-(3,4-Dihydroquinolin-I(2H)-yl)-1,3,5-triazin-2-yl)amino]benzenemethanesulfonamide (Cpd 39);
3-[(4-(6,7-Dihydro-3H-imidazo[4,5-c]pyridin-5(4H)-yl)-1,3,5-triazin-2-yl)amino]benzenemethanesulfonamide (Cpd 40);
3-[(4-(1H-Pyrrolo[3,4-c]pyridin-2(3H)-yl)-1,3,5-triazin-2-yl)amino]benzenemethanesulfonamide (Cpd 41);
3-[(4-(Pyrrolo[3,4-c]pyrazol-5(1H,4H,6H)-yl)-1,3,5-triazin-2-yl)amino]benzenemethanesulfonamide (Cpd 42);
3-[(4-(Indolin-1-yl)-1,3,5-triazin-2-yl)amino]benzenemethanesulfonamide (Cpd 43); and
(S)-3-[(4-(2-Methylpyrrolidin-1-yl)-1,3,5-triazin-2-yl)amino]benzenemethanesulfonamide (Cpd 44).

**11.** The method of any one of claims 1 to 3, wherein said inhibitor is selected from the group consisting of

Piperidine-4-carboxylic acid [5-(5-tert-butyl-oxazol-2-ylmethylsulfanyl)-thiazol-2-yl]-amide (SNS-032);
2-(2-Chloro-phenyl)-5,7-dihydroxy-8-(3-hydroxy-1-methyl-piperidin-4-yl)-chromen-4-one, (flavopiridol);
N-(5-((6-(3-aminophenyl)pyrimidin-4-yl)amino)-2-methylphenyl)propane-1-sulfonamide (AX35427);
[4-amino-2-(1-methanesulfonylpiperidin-4-ylamino)pyrimidin-5-yl]-(2,3-difluoro-6-methoxyphenyl)methanone (R-547);
3-[[6-(2-methoxyphenyl)-4-pyrimidinyl]amino]-Benzenemethanesulfonamide compound (1073485-20-7P);
3-((6-(2-methoxyphenyl)pyrimidin-4-yl)amino)benzenesulfonamide (AX38679);
1,5,6,7-tetrahydro-2-(4-pyridinyl)-4H-pyrrolo[3,2-c]pyridin-4-one, (PHA767491);
5,6-dichloro-1-b-ribofuranosyl-benzimidazole (DRB);
6-Benzylamino-2[(R)-(1'-ethyl-2'-hydroxyethylamino)]-9-isopropylpurine (Roscovitine);
4-[[4-Amino-5-(2,6-difluorobenzoyl)thiazol-2-yl]amino]-N-((R)-2-dimethylamino-1-methylethyl)benzamide (AG-012986);
4H-1-Benzopyran-4-one, 2-(2-chlorophenyl)-5,7-dihydroxy-8-[(2R,3S)-2-(hydroxymethyl)-1-methyl-3-pyrrolidinyl]-, hydrochloride (1:1) (P276-00);
4-[3-Chloro-5-(4-methylpiperazi-n-1-yl)benzoylamino]-1H-pyrazole-3-carboxylic acid cyclohexylamide (ZK 304709);
4-(2,6-Dichlorobenzoylamino)-1H-pyrazole-3-carboxylic acid N-(piperidin-4-yl)amide (AT7519);
N-[2-(dimethylamino)ethyl]-2-fluoro-4-[[5-fluoro-4-[2-methyl-1-(1-methylethyl)-1H-imidazol-5-yl]-2-pyrimidinyl] amino] (Compound 7d);
[4-[[5-fluoro-4-[2-methyl-1-(1-methylethyl)-1H-imidazol-5-yl]-2-pyrimidinyl] amino]phenyl][(3S)-3-(methylamino)-1-pyrrolidinyl](AZD5597);
N-[1,4-dihydro-3-[4-[[4-(2-methoxyethyl)-1-piperazinyl]methyl]phenyl]-4-oxoindeno[1,2-c]pyrazol-5-yl]-N'-4-morpholinyl-, hydrochloride (1:2) (RGB-286638); and
4-(2,6-dichlorobenzamido)-N-(1-(methylsulfonyl)piperidin-4-yl)-1H-pyrazole-3-carboxamide (LCQ195/AT9311).

**12.** The method of any one of claims 1 to 11, wherein rearrangement in the NUT gene is a t15;19 translocation as reflected in formation of a Brd4/NUT fusion gene.

**13.** The method of any one of claims 1 to 11, wherein said rearrangement in the NUT gene is a formation of a NUT variant fusion gene.

**14.** The method of claim 13, wherein said NUT variant fusion gene is a Brd3/NUT fusion gene.

**15.** The method of any one of claims 12 to 14, wherein said rearrangement in the NUT gene is reflected in expression of the formed NUT fusion gene and whereby the expression level of the formed NUT fusion gene is detected.

**16.** The method of claim 15, wherein the expression level is detected by an immunohistochemical method, real-time PCR, and/or Northern Blot.

**17.** The method of any one of claims 1 to 14, wherein said rearrangement in the NUT gene is detected by an in situ hybridization method.

**18.** The method of claim 17, wherein the in situ hybridization method is selected from the group consisting of fluorescent in situ hybridization (FISH), chromogenic in situ hybridization (CISH) and silver in situ hybridization (SISH).

**19.** Use of an oligo- or polynucleotide capable of detecting at least one rearrangement in the NUT gene for diagnosing sensitivity to a selective CDK9 inhibitor as defined in any one of claims 1 to 11.

**20.** The use of claim 19 wherein said oligonucleotide is about 1 to 100 nucleotides in length.

**21.** A method of monitoring the efficacy of a treatment of NUT midline carcinoma (NMC) in a subject/patient suffering from said disorder or being prone to suffering from said disorder comprising the steps:

a) determining in a cell or tissue sample obtained from said subject/patient the presence of at least one rearrangement in the NUT gene; and
b) comparing the rearrangement status in the NUT gene as determined in a) with a reference or control rearrangement status in the NUT gene,

wherein the extent of the difference between said rearrangement status determined in a) and said reference rearrangement status is indicative for said efficacy of a treatment of NUT midline carcinoma (NMC).

**22.** The method of claim 21, wherein the treatment of NUT midline carcinoma (NMC) comprises treatment with a selective CDK9 inhibitor as defined in any one of claims 1 to 11.

**23.** A method of predicting the efficacy of a treatment of NUT midline carcinoma (NMC) for a subject/patient suffering from said disorder or being prone to suffering from said disorder comprising the steps of

a) determining in a cell or tissue sample obtained from said subject/patient the rearrangement status in the NUT gene; and
b) comparing the rearrangement status in the NUT gene as determined in a) with a reference or control rearrangement status in the NUT gene determined in a cell or tissue sample obtained from a control subject/patient (responder and/or non-responder),

wherein the extent of the difference between said rearrangement status determined in a) and said reference rearrangement status is indicative for the predicted efficacy of a treatment of NUT midline carcinoma (NMC).

**24.** The method of claim 23, wherein the treatment of NUT midline carcinoma (NMC) comprises treatment with a selective CDK9 inhibitor as defined in any one of claims 1 to 11.

Figure 1.

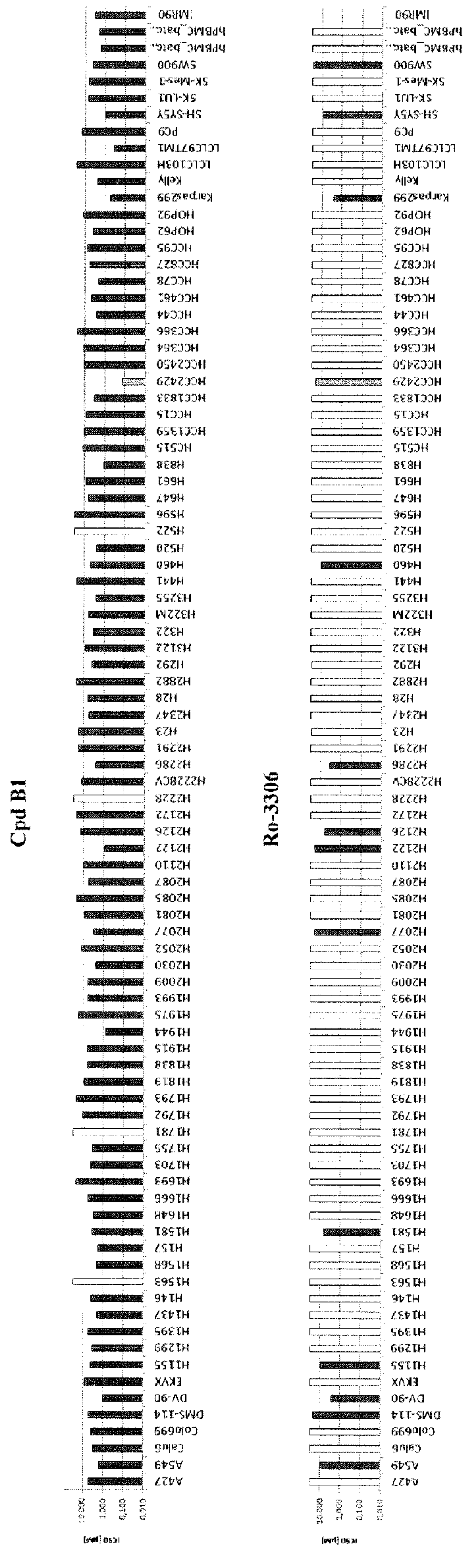

**Figure 2.**

| trivial name | specificity | CDK1 LANCE Assay IC50 [µM] | CDK2 LANCE Assay IC50 [µM] | CDK4 LANCE Assay IC50 [µM] | CDK6 LANCE Assay IC50 [µM] | CDK7 LANCE Assay IC50 [µM] | CDK9 LANCE Assay IC50 [µM] |
|---|---|---|---|---|---|---|---|
| SNS-032 | CDK | 0,047 | 0,007 | 0,067 | 1,357 | 0,058 | 0,005 |
| flavopiridol | CDK1, CDK2, CDK9 | 0,037 | 0,053 | 0,211 | 0,793 | 0,246 | 0,016 |
| AX35427 | CDK1, CDK2, CDK9 | 0,455 | 0,291 | >1 | >1 | >1 | 0,007 |
| R-547 | CDKs -CDK7 | 0,005 | 0,005 | 0,005 | 0,011 | 0,149 | 0,010 |
| 1073485-20-7P | CDK9 | 5,505 | 2,780 | 5,929 | >10 | >10 | 0,111 |
| Ro-3306 | CDK1 | | | | | | |
| AX38679 | CDK9 | 3,744 | 0,545 | 6,953 | >10 | >10 | 0,030 |
| Cpd 24 | CDK9 | 2,469 | 0,831 | >10 | >10 | >10 | 0,015 |
| Cpd C1 | CDK9 | 1,349 | 0,781 | 1,505 | >10 | >10 | 0,021 |
| Cpd B1 | CDK9 | 12,050 | 3,083 | >10 | >10 | >10 | 0,096 |
| PHA767491 | CDK9, CHK1 | 0,853 | 0,280 | >10 | >10 | >10 | 0,031 |
| Cpd B2 | CDK9 | 0,899 | 0,568 | 3,205 | >10 | >10 | 0,011 |
| PD332991 | CDK4, CDK6 | | | | | | |
| BS-181 | CDK7 | 21,060 | 3,178 | >10 | >10 | 0,057 | 1,944 |

**Figure 3.**

| Compound | 1073485-20-7P | Cpd B1 |
|---|---|---|
| ABL1 wt | 92 | 61 |
| AKT1 | 114 | 104 |
| Aurora-A | 89 | 52 |
| CDK9/CycT | 3 | 1 |
| CK2-alpha1 | 97 | 97 |
| EGF-R wt | 87 | 47 |
| EPHB4 | 101 | 76 |
| FGF-R1 wt | 101 | 74 |
| FLT3 wt | 59 | 63 |
| GSK3-beta | 77 | 64 |
| IGF1-R | 105 | 97 |
| IKK-beta | 104 | 95 |
| JNK1 | 107 | 107 |
| LCK | 97 | 80 |
| MAPKAPK3 | 93 | 86 |
| MEK1 wt | 106 | 89 |
| MET | 103 | 96 |
| PDGFR-beta | 85 | 52 |
| PLK1 | 109 | 109 |
| p38-alpha | 111 | 99 |
| ROCK2 | 105 | 109 |
| TGFB-R1 | 104 | 92 |
| VEGF-R2 | 105 | 63 |

**Figure 4.**

| **trivial name** | **specificity** | **HCC2429 IC50 [µM]** | **TY-82 IC50 [µM]** | **690100 IC50 [µM]** | **143100 IC50 [µM]** | **Hela IC50 [µM]** |
|---|---|---|---|---|---|---|
| SNS-032 | CDK | <0,014 | <0,014 | <0,014 | <0,014 | 0,209 |
| flavopiridol | CDK1, CDK2, CDK9 | 0,021 | 0,035 | 0,058 | 0,020 | 0,364 |
| AX35427 | CDK1, CDK2, CDK9 | 0,046 | 0,167 | 0,420 | 0,078 | 2,725 |
| R-547 | CDKs -CDK7 | 0,020 | 0,236 | 0,210 | 0,024 | 2,528 |
| 1073485-20-7P | CDK9 | 1,299 | 1,385 | 4,438 | 1,691 | 26,060 |
| Ro-3306 | CDK1 | 4,755 | 3,254 | 6,220 | 4,079 | 12,457 |
| AX38679 | CDK9 | 0,146 | 0,447 | 0,711 | 0,197 | 4,166 |
| Cpd 24 | CDK9 | 0,030 | 0,091 | 0,144 | 0,046 | 0,615 |
| Cpd C1 | CDK9 | 0,022 | 0,061 | 0,099 | 0,036 | 0,873 |
| Cpd B1 | CDK9 | 0,151 | 0,321 | 0,580 | 0,202 | 4,193 |
| PHA767491 | CDK9, CHK1 | 0,067 | 0,318 | 0,318 | 0,097 | 1,761 |
| Cpd B2 | CDK9 | 0,026 | 0,072 | 0,114 | 0,044 | 0,805 |
| PD332991 | CDK4, CDK6 | 6,440 | 4,316 | 6,607 | 0,356 | >30 |
| BS-181 | CDK7 | 16,866 | 8,911 | 13,695 | 14,259 | >30 |

**Figure 4 (cont.)**

| trivial name | specificity | hPBMCs IC50 [µM] | HCC366 IC50 [µM] | H460 IC50 [µM] | HCC1143 IC50 [µM] |
|---|---|---|---|---|---|
| SNS-032 | CDK | 0,065 | 0,353 | 0,052 | 0,171 |
| flavopiridol | CDK1, CDK2, CDK9 | 0,307 | 0,745 | 0,075 | 0,551 |
| AX35427 | CDK1, CDK2, CDK9 | 3,578 | 10,202 | 0,817 | 6,748 |
| R-547 | CDKs -CDK7 | 3,230 | 4,975 | 0,019 | 6,699 |
| 1073485-20-7P | CDK9 | 15,861 | >30 | 12,532 | 29,727 |
| Ro-3306 | CDK1 | 23,723 | 25,365 | 2,157 | >30 |
| AX38679 | CDK9 | 3,533 | 15,909 | 2,466 | 8,602 |
| Cpd 24 | CDK9 | 0,549 | 1,807 | 0,209 | 2,511 |
| Cpd C1 | CDK9 | 0,635 | 16,905 | 0,868 | 4,698 |
| Cpd B1 | CDK9 | 3,462 | >30 | 4,508 | 7,431 |
| PHA767491 | CDK9, CHK1 | 1,077 | >30 | 9,508 | 4,577 |
| Cpd B2 | CDK9 | 0,825 | >30 | 2,698 | 2,325 |
| PD332991 | CDK4, CDK6 | >30 | >30 | 0,513 | >30 |
| BS-181 | CDK7 | 28,702 | >30 | 19,102 | >30 |

Figure 5.

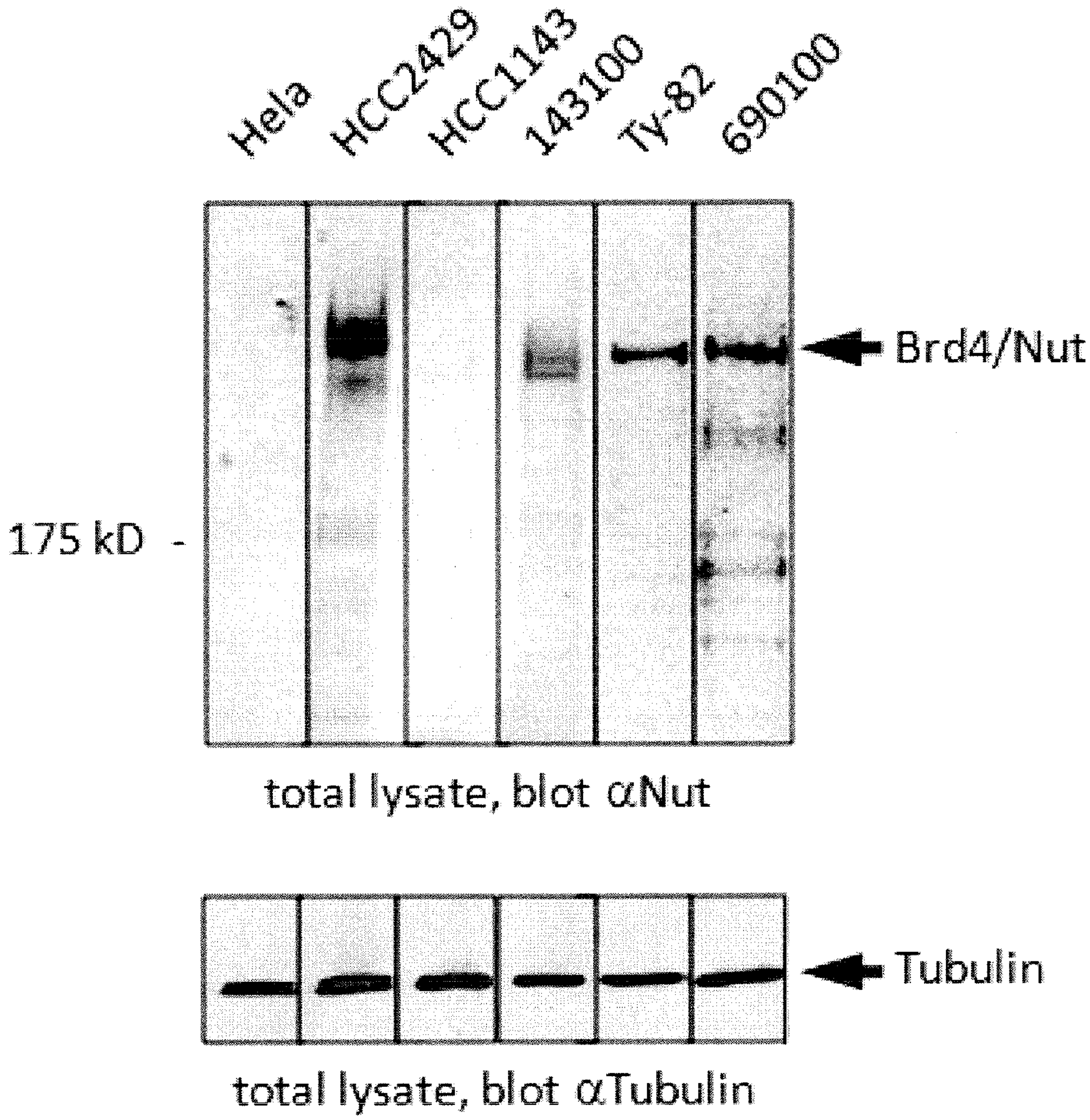
Hela
HCC2429
HCC1143
143100
Ty-82
690100
Brd4/Nut
175 kD -
total lysate, blot αNut
Tubulin
total lysate, blot αTubulin

Europäisches Patentamt
European Patent Office
Office européen des brevets

# EUROPEAN SEARCH REPORT

Application Number
EP 11 17 8334

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 2010/011700 A2 (BRIGHAM & WOMENS HOSPITAL [US]; DANA FARBER CANCER INST INC [US]; FREN) 28 January 2010 (2010-01-28)<br>* page 4, line 9 - page 5, line 11 * | 21,23<br><br><br>1-20,22, 24 | INV.<br>C12Q1/68 |
| A | ENGLESON JENS ET AL: "Midline carcinoma with t(15;19) and BRD4-NUT fusion oncogene in a 30-year-old female with response to docetaxel and radiotherapy",<br>BMC CANCER, BIOMED CENTRAL, LONDON, GB,<br>vol. 6, no. 1, 16 March 2006 (2006-03-16), page 69, XP021016272,<br>ISSN: 1471-2407, DOI: 10.1186/1471-2407-6-69<br>* abstract * | 1-24 | |
| A | FREDRIK MERTENS ET AL: "Successful treatment of a child with t(15;19)-positive tumor",<br>PEDIATRIC BLOOD & CANCER,<br>vol. 49, no. 7,<br>1 December 2007 (2007-12-01), pages 1015-1017, XP55021163,<br>ISSN: 1545-5009, DOI: 10.1002/pbc.20755<br>* page 1015, column 2 * | 1-24 | TECHNICAL FIELDS SEARCHED (IPC)<br>C12Q |
| A,P | MULLER SUSANNE ET AL: "Bromodomains as therapeutic targets",<br>EXPERT REVIEWS IN MOLECULAR MEDICINE,<br>vol. 13, 13 September 2011 (2011-09-13), pages 1-21 URL, XP002670944,<br>* pages 7-10; page 13, last paragraph * | 1-24 | |

-/--

The present search report has been drawn up for all claims

| Place of search | Date of completion of the search | Examiner |
|---|---|---|
| Munich | 8 March 2012 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

2

Europäisches Patentamt
European Patent Office
Office européen des brevets

# EUROPEAN SEARCH REPORT

Application Number
EP 11 17 8334

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | J. YAN ET AL: "Perturbation of BRD4 Protein Function by BRD4-NUT Protein Abrogates Cellular Differentiation in NUT Midline Carcinoma", JOURNAL OF BIOLOGICAL CHEMISTRY, vol. 286, no. 31, 5 August 2011 (2011-08-05), pages 27663-27675, XP55021164, ISSN: 0021-9258, DOI: 10.1074/jbc.M111.246975 * page 27669; figure 4; page 27674, last sentance * ----- | 1-24 | |
| Y | AARON J DONNER ET AL: "CDK8 is a positive regulator of transcriptional elongation within the serum response network", NATURE STRUCTURAL & MOLECULAR BIOLOGY, vol. 17, no. 2, 1 February 2010 (2010-02-01), pages 194-201, XP55021166, ISSN: 1545-9993, DOI: 10.1038/nsmb.1752 * page 198 column 1 bottom * ----- | 1-24 | TECHNICAL FIELDS SEARCHED (IPC) |
| A | VLADIMÍR KRYŠTOF ET AL: "Pharmacological targeting of CDK9 in cardiac hypertrophy", MEDICINAL RESEARCH REVIEWS, 1 January 2009 (2009-01-01), pages N/A-N/A, XP55021167, ISSN: 0198-6325, DOI: 10.1002/med.20172 * page 651 * ----- -/-- | 1-24 | |

The present search report has been drawn up for all claims

| Place of search | Date of completion of the search | Examiner |
|---|---|---|
| Munich | 8 March 2012 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 11 17 8334

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TONG WEI-GANG ET AL: "Phase I and pharmacologic study of SNS-032, a potent and selective Cdk2, 7, and 9 inhibitor, in patients with advanced chronic lymphocytic leukemia and multiple myeloma.", JOURNAL OF CLINICAL ONCOLOGY : OFFICIAL JOURNAL OF THE AMERICAN SOCIETY OF CLINICAL ONCOLOGY 20 JUN 2010 LNKD- PUBMED:20479412, vol. 28, no. 18, 20 June 2010 (2010-06-20), pages 3015-3022, XP002670945, ISSN: 1527-7755<br>* abstract *<br>----- | 1-24 | |
| T | WO 2011/116951 A1 (LEAD DISCOVERY CT GMBH [DE]; BAYER PHARMA AG [DE]; EICKHOFF JAN [DE];) 29 September 2011 (2011-09-29)<br>* claims *<br>----- | 1-24 | |
| Y | WO 2008/079933 A2 (NOVARTIS AG [CH]; BECKWITH ROHAN ERIC JOHN [US]; CURTIS DANIEL TIM [US] 3 July 2008 (2008-07-03)<br>* claims *<br>----- | 1-24 | TECHNICAL FIELDS SEARCHED (IPC) |

The present search report has been drawn up for all claims

2

| Place of search | Date of completion of the search | Examiner |
|---|---|---|
| Munich | 8 March 2012 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 17 8334

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2012

| Patent document cited in search report | Publication date | Patent family member(s) | Publication date |
|---|---|---|---|
| WO 2010011700 A2 | 28-01-2010 | US 2011213012 A1 | 01-09-2011 |
| | | WO 2010011700 A2 | 28-01-2010 |
| WO 2011116951 A1 | 29-09-2011 | UY 33286 A | 31-10-2011 |
| | | WO 2011116951 A1 | 29-09-2011 |
| WO 2008079933 A2 | 03-07-2008 | AU 2007336933 A1 | 03-07-2008 |
| | | CA 2672518 A1 | 03-07-2008 |
| | | CN 101568529 A | 28-10-2009 |
| | | EA 200900799 A1 | 30-12-2009 |
| | | EP 2094682 A2 | 02-09-2009 |
| | | JP 2010514689 A | 06-05-2010 |
| | | KR 20090091306 A | 27-08-2009 |
| | | US 2010048597 A1 | 25-02-2010 |
| | | WO 2008079933 A2 | 03-07-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010011700 A
- EP 2011001445 W
- EP 10075131 A
- EP 11075037 A
- EP 11075038 A
- EP 2011001445 PCT
- WO 2009047359 A
- WO 2010003133 A
- WO 200879933 A
- WO 2411012661 A
- WO 2005026129 A
- WO 2008132138 A
- WO 9720842 A1
- WO 2010020618 A
- WO 2010020619 A


### Non-patent literature cited in the description


- **FRENCH.** *J Clin Oncology,* 2004, vol. 22 (20), 4135-4139
- **FRENCH.** *J Clin Pathol,* 2010, vol. 63, 492-496
- **KUZUME.** *Int J Cancer,* 1992, vol. 50, 259-264
- **FRENCH.** *Am J Pathol,* 2001, vol. 159 (6), 1987-1992
- **FRENCH.** *Cancer Genetics and Cytogenetics,* 2010, vol. 203, 16-20
- **ZIAI.** *Head and Neck Pathol,* 2010, vol. 4, 163-168
- **FRENCH.** *Cancer Res,* 2003, vol. 63, 304-307
- **FRENCH.** *Oncogene,* 2008, vol. 27, 2237-2242
- **FRENCH.** *J Clin Pathol,* 2010
- **HAACK.** *Am J Surg Pathol,* 2009, vol. 33 (7), 984-991
- **SCHWARTZ.** *Cancer Res,* 2011, vol. 71 (7), 2686-2696
- **ALSARRAJ.** *Cancer Res,* 2011
- **MOSHINSKY DJ ; RUSLIM L ; BLAKE RA ; TANG F.** *J Biomol Screen.,* August 2003, vol. 8 (4), 447-52
- *J. Org. Chem.,* 1995, vol. 60, 8428-8430
- **KRYSTOF.** *Medicinal Research Reviews,* 2009
- **MISRA RN et al.** *J Med Chem.,* 2004, vol. 47 (7), 1719-28
- **LLOYD R KELLAND.** *Expert Opinion on Investigational Drugs,* 2000, vol. 9 (12), 2903-2911
- **DEPINTO W et al.** *Mol Cancer Ther,* 2006, vol. 5, 2644-2658
- **MONTAGNOLI, A.** *Nat Chem Biol,* 2008, vol. 4 (6), 357-365
- **ALI S et al.** *Cancer Res.,* 2009, vol. 69 (15), 6208-15
- **MANCEBO HS ; LEE G ; FLYGARE J ; TOMASSINI J ; LUU P ; ZHU Y ; PENG J ; BLAU C ; HAZUDA D ; PRICE D.** P-TEFb kinase is required for HIV Tat transcriptional activation in vivo and in vitro. *Genes Dev,* 1997, vol. 11, 2633-2644
- **ZHANG C ; LUNDGREN K ; YAN Z ; ARANGO ME ; PRICE S ; HUBER A ; HIGGINS J ; TROCHE G ; SKAPTASON J ; KOUDRIAKOVA T.** Pharmacologic properties of AG-012986, a pan-cyclin-dependent kinase inhibitor with antitumor efficacy. *Mol Cancer Ther,* 2008, vol. 7, 818-828
- **JOSHI, KALPANA S. ; RATHOS, MAGGIE J. ; JOSHI, RAJENDRA D. ; SIVAKUMAR, MEENAKSHI ; MASCARENHAS, MALCOLM ; KAMBLE, SHRIKANT ; LAL, BANSI ; SHARMA, SOMESH.** In vitro antitumor properties of a novel cyclin-dependent kinase inhibitor, P276-00. *Molecular Cancer Therapeutics,* 2007, vol. 6 (3), 918-925
- **SIEMEISTER, G. ; LUECKING, U. ; WAGNER, C. ; DETJEN, K. ; MC COY, C. ; BOSSLET, KLAUS.** Molecular and pharmacodynamic characteristics of the novel multi-target tumor growth inhibitor ZK304709. *Biomedicine & Pharmacotherapy,* 2006, vol. 60 (6), 269-272
- **SQUIRES MS ; FELTELL RE ; LOCK V ; SMITH D ; LEWIS EJ ; HIGGINS J ; YULE M ; THOMPSON NT ; COOKE L ; CROCE DELLA K.** AT7519, a potent CDK inhibitor, is active in leukemia models and primary CLL patient samples. *49th Annual Meeting and Exposition of American Society for Hematology,* 08 December 2007

- **JONES CD ; ANDREWS DM ; BARKER AJ ; BLADES K ; DAUNT P ; EAST S ; GEH C ; GRAHAM MA ; JOHNSON KM ; LODDICK SA.** The discovery of AZD5597, a potent imidazole pyrimidine amide CDK inhibitor suitable for intravenous dosing. *Bioorg Med Chem Lett,* 2008, vol. 18, 6369-6373
- **KRYSTOF V ; CHAMRÁD I ; JORDA R ; KOHOUTEK J.** *Med Res Rev.,* July 2010, vol. 30 (4), 646-66
- **MCMILLIN, DOUGLAS W. ; DELMORE, JAKE ; NEGRI, JOSEPH ; BUON, LEUTZ ; JACOBS, HANNAH M. ; LAUBACH, JACOB ; JAKUBIKOVA, JANA ; OOI, MELISSA ; HAYDEN, PATRICK ; SCHLOSSMAN, ROBERT.** Molecular and cellular effects of multi-targeted cyclin-dependent kinase inhibition in myeloma: biological and clinical implications. *British Journal of Haematology,* 2011, vol. 152 (4), 420-432
- **ELBASHIR.** *Nature,* 2001, vol. 411, 494-498
- **PADDISON.** *Genes Dev.,* 2002, vol. 16, 948-958
- **ELBASHIR.** *Methods,* 2002, vol. 26, 199-213
- **NOVINA.** *Mat. Med.,* 03 June 2002
- **DONZE.** *Nucl. Acids Res.,* 2002, vol. 30, e46
- **PAUL.** *Nat. Biotech,* 2002, vol. 20, 505-508
- **LEE.** *Nat. Biotech.,* 2002, vol. 20, 500-505
- **MIYAGASHI.** *Nat. Biotech.,* 2002, vol. 20, 497-500
- **YU.** *PNAS,* 2002, vol. 99, 6047-6052
- **BRUMMELKAMP.** *Science,* 2002, vol. 296, 550-553
- **SAMBROOK ; RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press Oxford, 1985
- **THOMPSON.** *Nucl. Acids Res.,* 1994, vol. 2, 4673-4680
- *Brutlag Comp. App. Biosci.,* 1990, vol. 6, 237-245
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **HENIKOFF.** *PNAS,* 1989, vol. 89, 10915
- **BRUTLAG.** *Comp. App. Biosci.,* 1990, vol. 6, 237-245
- **FRENCH CA.** *NUT midline carcinoma. French CA. Cancer Genet Cytogenet.,* November 2010, vol. 203 (1), 16-20
- **FRENCH.** *Cancer Genet Cytogenet,* 2010
- *Cancer Research,* 1999, vol. 59, 5169-5175
- *Pathol Int,* 1996, vol. 46, 801-804
- **VOGESER.** *Dtsch Arztebl,* 2007, vol. 104 (31-32), A2194-200
- **THOMAS RK et al.** *Nature Med,* 2007
- **THOMAS RK et al.** *Nat Gen,* 2007
- **MARGUERAT S et al.** *Biochem Soc Trans,* 2008
- The Cell. Garland Publishing, Inc,
- **BRAY ; BERNARD.** *Current Topics in Developmental biology,* 2010, vol. 92, 253-275
- *Methods in Enzymology,* 1987, vol. 153, 385-516
- **BITTER et al.** *Methods in Enzymology,* 1987, vol. 153, 516-544
- **SAWERS et al.** *Applied Microbiology and Biotechnology,* 1996, vol. 46, 1-9
- **BILLMAN-JACOBE.** *Current Opinion in Biotechnology,* 1996, vol. 7, 500-4
- **HOCKNEY.** *Trends in Biotechnology,* 1994, vol. 12, 456-463
- **GRIFFITHS et al.** *Methods in Molecular Biology,* 1997, vol. 75, 427-440
- **PIRITY.** *Methods Cell Biol,* 1998, vol. 57, 279
- **SAMBROOK ; RUSSELL.** Molecular Cloning: A Laboratory Manual. Cold Spring Harbor, 2001
- Methods in Yeast Genetics, A Laboratory Course Manual. Cold Spring Harbor Laboratory Press, 1990